# FASCICULE DE BREVET EUROPEEN

(11) **EP 0 970 684 B1**
(45) Date de publication et mention de la délivrance du brevet: **08.06.2005**
(21) Numéro de dépôt: 99401521.2
(22) Date de dépôt: 18.06.1999
(51) Int. Cl.: A61K 7/13

(54) **Composition de teinture pour fibres kérantiniques avec un colorant direct cationique et un polymère épaississant**
Färbemittel für Keratinfasern, das ein Kationischer Farbstoffe und eine Verdickendes Polymer enthält
Composition for dyeing keratinous fibres containing a cationic direct dye and a thickening polymer

(30) Priorité: 09.07.1998 FR 9808835
(43) Date de publication de la demande: 12.01.2000
(73) Titulaire: L'OREAL, 75008 Paris (FR)
(72) Inventeur: Lang, Gérard, 95390 Saint Prix (FR); Cotteret, Jean, 78480 Verneuil/Seine (FR)
(74) Mandataire: Miszputen, Laurent

(56) Documents cités:
- EP-A- 0 827 738
- EP-A- 0 827 739
- EP-A- 0 850 637
- EP-A- 0 850 638
- WO-A-95/01772
- WO-A-95/15144
- WO-A-99/20234
- WO-A-99/20235
- FR-A- 2 140 205
- FR-A- 2 189 006
- FR-A- 2 282 860
- FR-A- 2 285 851
- FR-A- 2 751 533

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins un polymère épaississant particulier.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la puissance, de l'homogénéité de la couleur répartie le long de la fibre, on dit alors que la coloration est trop sélective, et sur le plan de la tenacité, en terme de résistance aux diverses agressions que peuvent subir les cheveux (lumière, intempéries,shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations plus puissantes et néanmoins peu sélectives et résistant bien aux diverses agressions que peuvent subir les cheveux, en associant au moins un polymère épaississant particulier à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, **(i)**au moins un colorant direct cationique dont la structure répond aux formules (I) à (IV) définies ci-après, caractérisée par le fait qu'elle contient en outre **(ii)**au moins un polymère épaississant particulier.

(i) Le colorant direct cationique utilisable selon la présente invention est un composé choisi parmi ceux de formules (I), (II), (III), (III'), (IV) suivantes:
**a) les composés de formule (I) suivante :** 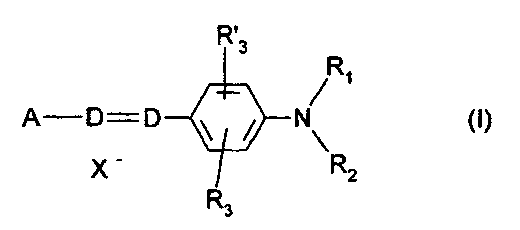 dans laquelle :
   D représente un atome d'azote ou le groupement -CH,
   R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
   X ⁻ représente un anion de préférence choisi parmi le chlorure, le méthyl sulfate et l'acétate,
   A représente un groupement choisi par les structures A1 à A18 suivantes : 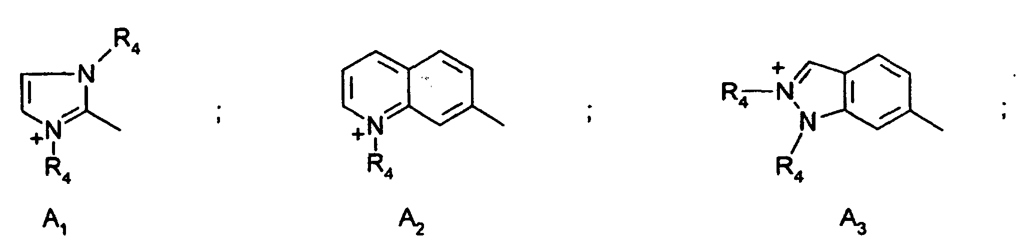 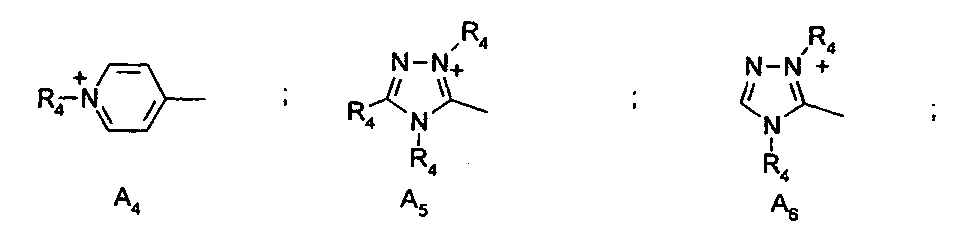 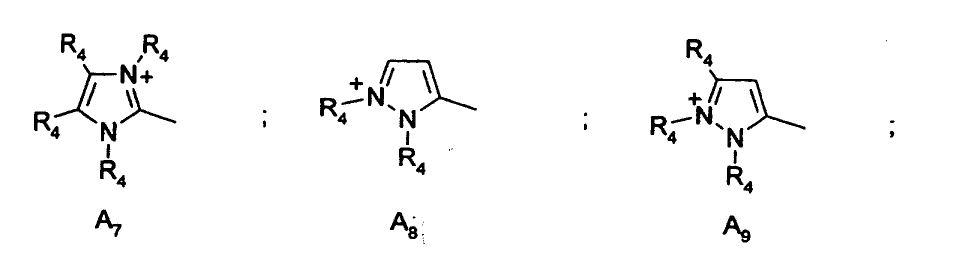 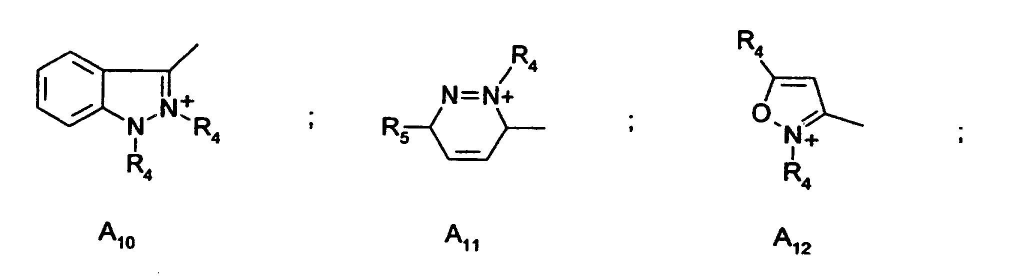 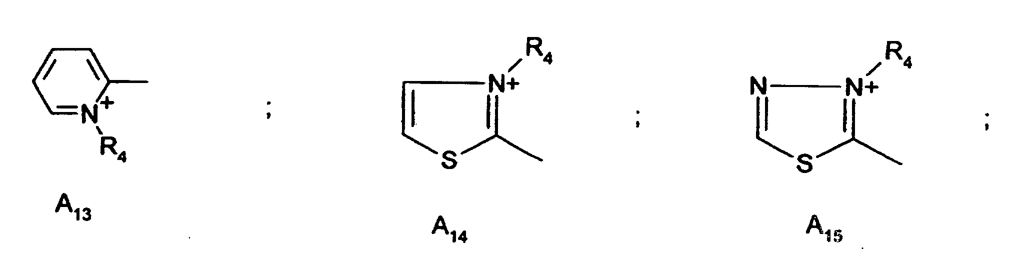 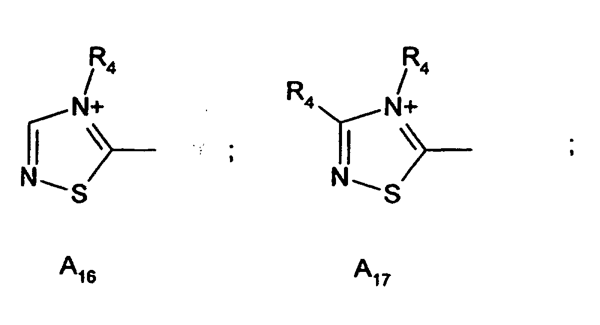 et 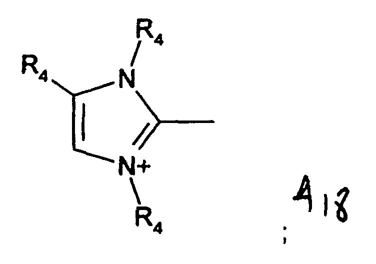 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 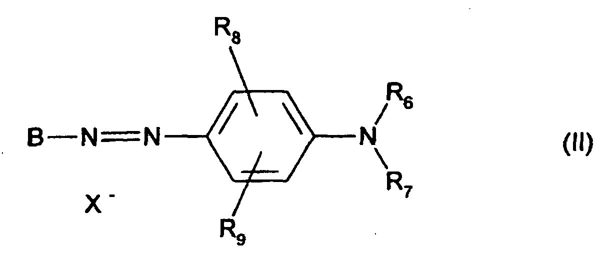 dans laquelle:
   R₆ représente un atome d'hydrogène ou un radical alkyle en C₁-C₄,
   R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
   R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   X⁻ représente un anion de préférence choisi parmi le chlorure, le méthyl sulfate et l'acétate,
   B représente un groupement choisi par les structures B1 à B6 suivantes : 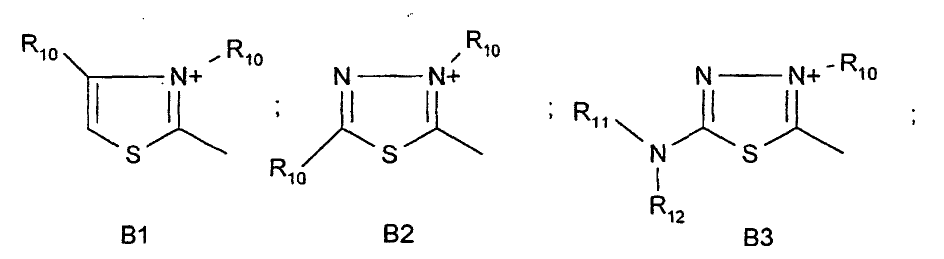 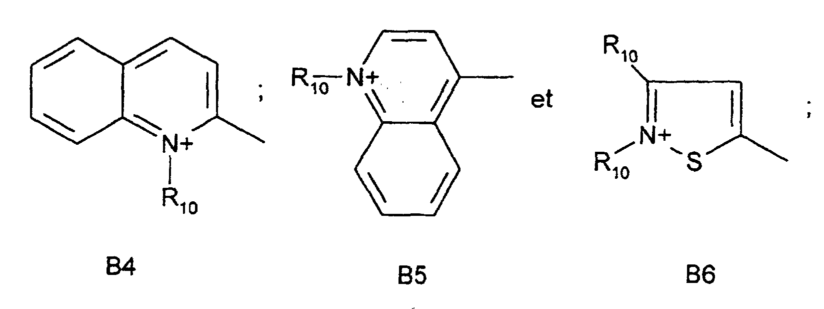 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) les composés de formules (III) et (III') suivantes :** 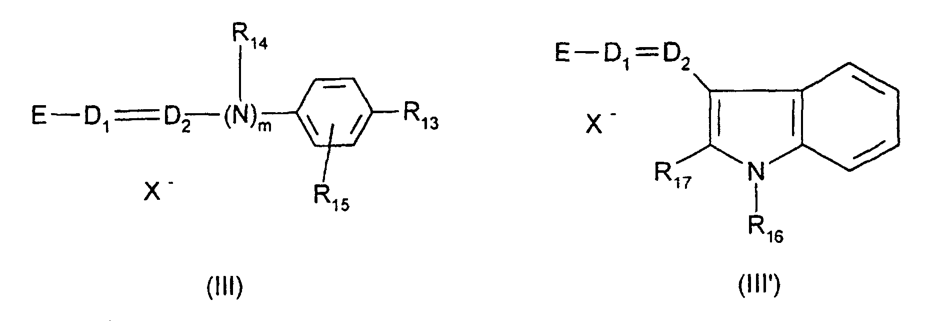 dans lesquelles :
   R₁₃ représente un atome d'hydrogène, un radical alcoxy en C₁-C₄, un atome d'halogène tel que le brome, le chlore, l'iode ou le fluor ou un radical amino,
   R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₁₅ représente un atome d'hydrogène ou d'halogène tel que le brome, le chlore, l'iode ou le fluor,
   R₁₆ et R₁₇, identiques ou différents, représentent un atome d'hydrogène ou un radical alkyle en C₁-C₄,
   D₁ et D₂, identiques ou différents, représentent un atome d'azote ou le groupement -CH,
   m = 0 ou 1,
   étant entendu que lorsque R₁₃ représente un groupement amino non substitué, alors D₁ et D₂ représentent simultanément un groupement -CH et m = 0,
   X⁻ représente un anion de préférence choisi parmi le chlorure, le méthyl sulfate et l'acétate,
   E représente un groupement choisi par les structures E1 à E8 suivantes : 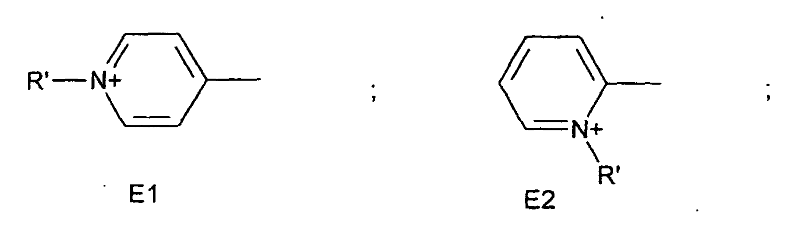 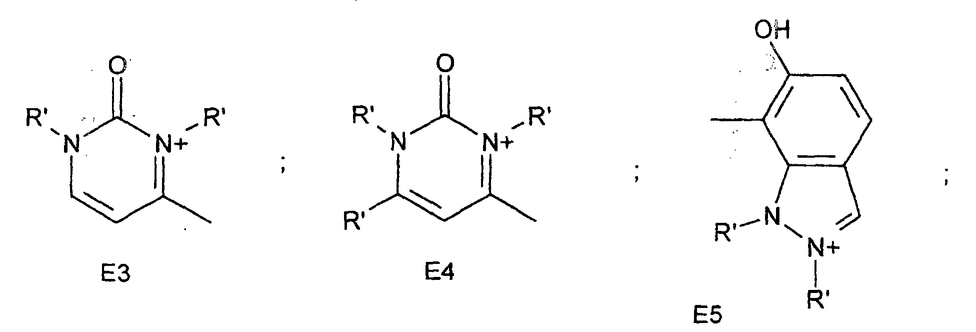 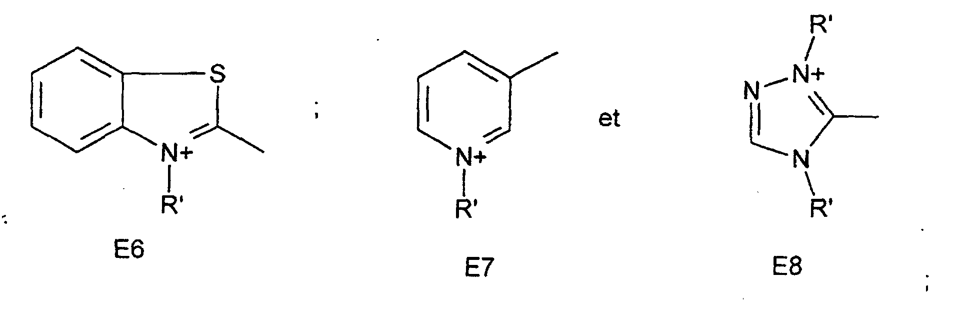 dans lesquelles R' représente un radical alkyle en C₁-C₄ ;
   lorsque m = 0 et que D₁ représente un atome d'azote, alors E peut également désigner un groupement de structure E9 suivante : 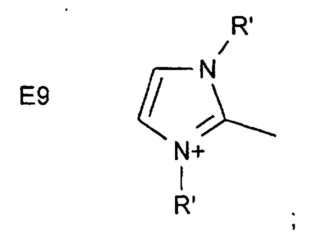 dans laquelle R' représente un radical alkyle en C₁-C₄.
**d) les composés de formule (IV) suivante :**

   G-N = N-J (IV)

   dans laquelle :
   **le symbole G** représente un groupement choisi parmi les structures G₁ à G₃ suivantes : 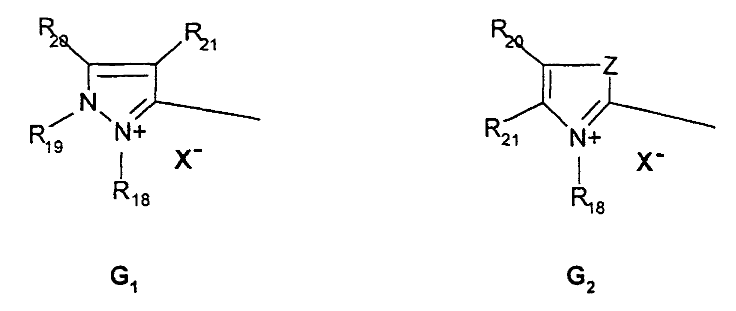 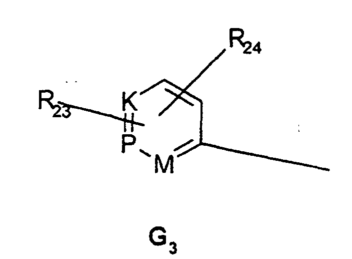 structures G₁ à G₃ dans lesquelles,
      R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor;
      R₁₉ désigne un radical alkyle en C₁-C₄ ou un radical phényle;
      R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄ , alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂;
      R₂₀ peut désigner en outre un atome d'hydrogène;
      Z désigne un atome d'oxygène, de soufre ou un groupement -NR₁₉;
      M représente un groupement -CH, -CR (R désignant alkyle en C₁-C₄), ou -NR₂₂(X⁻)ᵣ;
      K représente un groupement -CH, -CR (R désignant alkyle en C₁-C₄), ou -NR₂₂(X⁻)ᵣ;
      P représente un groupement -CH, -CR (R désignant alkyle en C₁-C₄), ou -NR₂₂(X⁻)ᵣ; r désigne zéro ou 1;
      R₂₂ représente un atome O⁻, un radical alcoxy en C₁-C₄, ou un radical alkyle en C₁-C₄;
      R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
      X⁻ représente un anion de préférence choisi parmi le chlorure, l'iodure, le méthyl sulfate, l'éthyl sulfate, l'acétate et le perchlorate;
      sous réserve que,
      si R₂₂ désigne O⁻, alors r désigne zéro;
      si K ou P ou M désignent -N-alkyle C₁-C₄ X⁻, alors R₂₃ ou R₂₄ est différent d'un atome d'hydrogène;
      si K désigne -NR₂₂(X⁻)ᵣ, alors M= P= -CH, -CR;
      si M désigne -NR₂₂(X⁻)ᵣ, alors K= P= -CH, -CR;
      si P désigne -NR₂₂(X⁻)ᵣ , alors K= M et désignent -CH ou -CR;
      si Z désigne un atome de soufre avec R₂₁ désignant alkyle en C₁-C₄, alors R₂₀ est différent d'un atome d'hydrogène;
      si Z désigne -NR₁₉ avec R₁₉ désignant alkyle en C₁-C₄ , alors au moins l'un des radicaux R₁₈, R₂₀ ou R₂₁ du groupement de structure G₂ est différent d'un radical alkyle en C₁-C₄;
   **le symbole J** représente :
      - **(a)** un groupement de structure J₁ suivante : 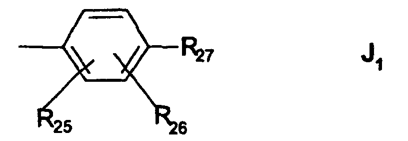 structure J₁ dans laquelle,
         R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
         R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
         R₂₇ représente un atome d'hydrogène, un radical -OH, un radical -NHR₂₈, un radical -NR₂₉R₃₀;
         R₂₈ représente un atome d'hydrogène, un radical alkyle en C₁-C₄, un radical
         monohydroxyalkyle en C₁-C₄, polyhydroxyalkyle en C₂-C₄, un radical phényle; R₂₉ et R₃₀, identiques ou différents, représentent un radical alkyle en C₁-C₄, un radical monohydroxyalkyle en C₁-C₄, polyhydroxyalkyle en C₂-C₄;
      - **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante : 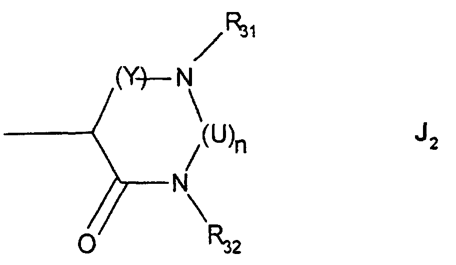 structure J₂ dans laquelle,
         R₃₁ et R₃₂, identiques ou différents, représentent un atome d'hydrogène, un radical alkyle en C₁-C₄, un radical phényle;
         Y désigne le radical -CO- ou le radical 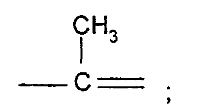 n = 0 ou 1, avec, lorsque n désigne 1, U désigne le radical -CO- .

Dans les structures (I) à (IV) définies ci-dessus le groupement alkyle ou alcoxy en C₁-C₄ désigne de préférence méthyle, éthyle, butyle, méthoxy, éthoxy.

Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954. Ceux de formule (IV) utilisables dans les compositions tinctoriales conformes à l'invention, sont des composés connus et sont décrits par exemple dans les demandes de brevets FR-2189006, FR-2285851, FR-2282860 et FR-2140205 et ses certificats d'addition.

Parmi les colorants directs cationiques de formule (I) utilisables dans les compositions tinctoriales conformes à l'invention, on peut plus particulièrement citer les composés répondant aux structures (I1) à (I54) suivantes :

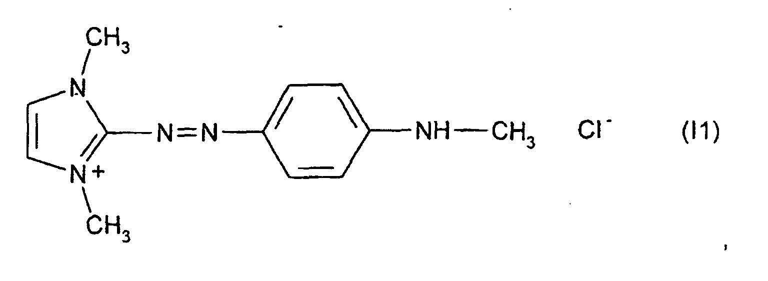

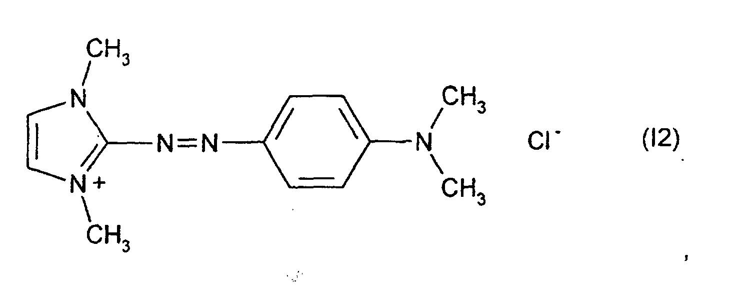

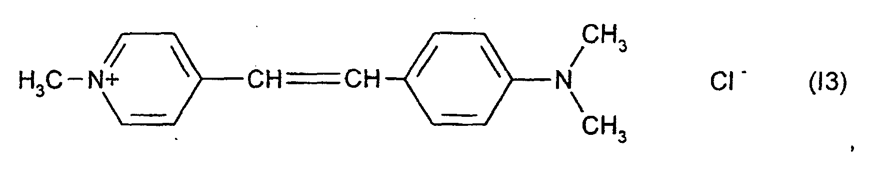

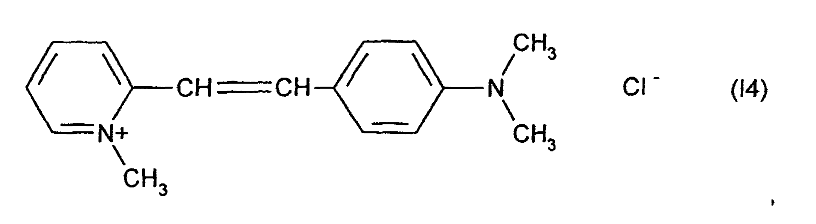

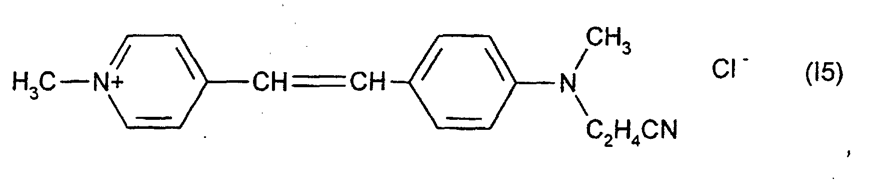

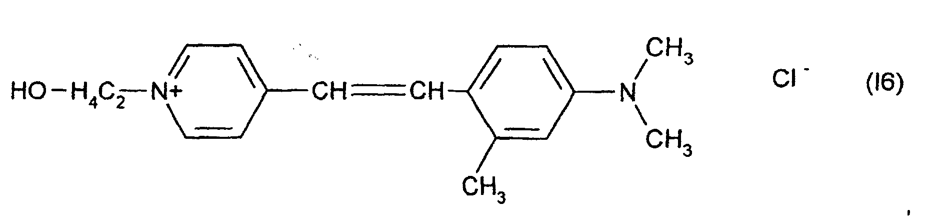

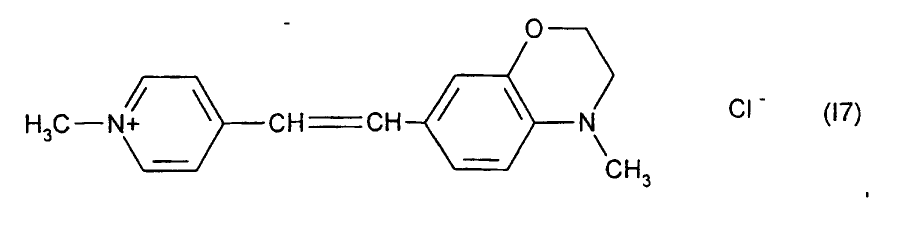

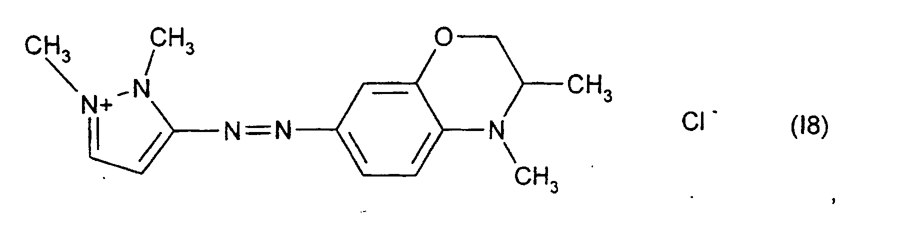

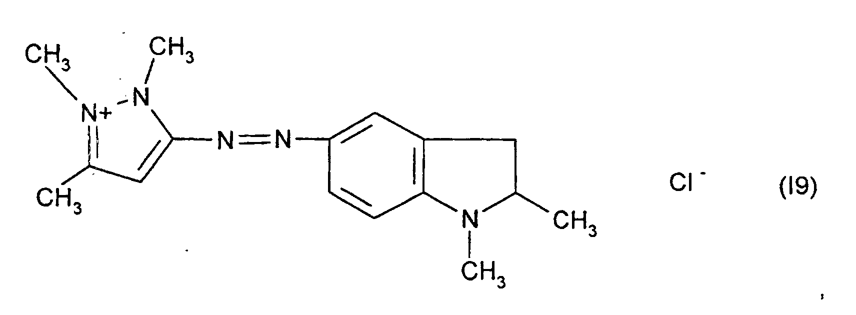

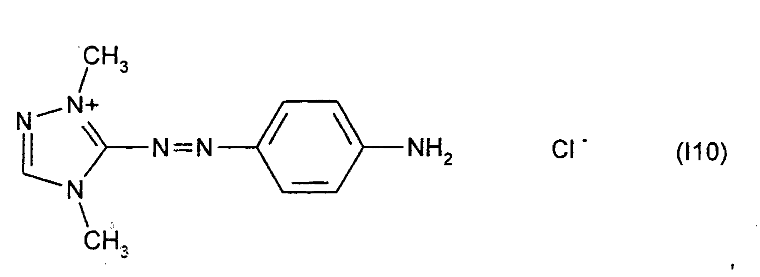

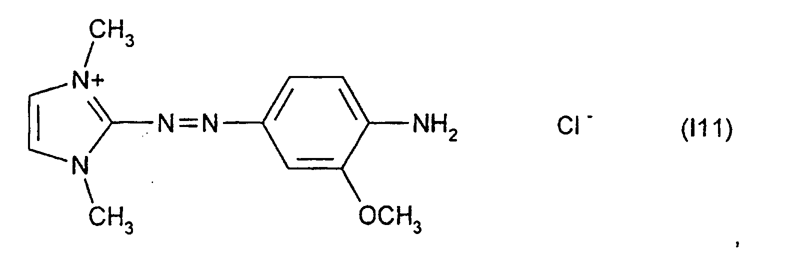

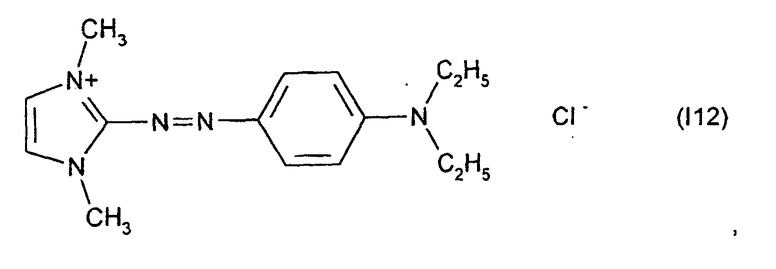

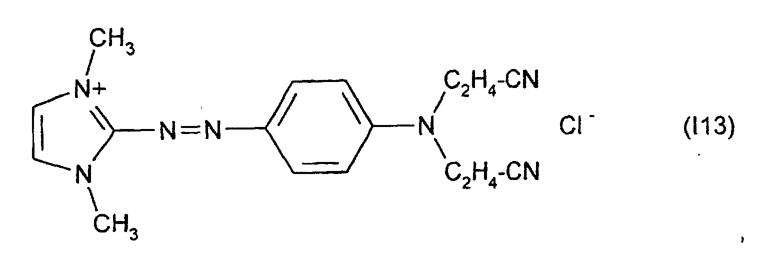

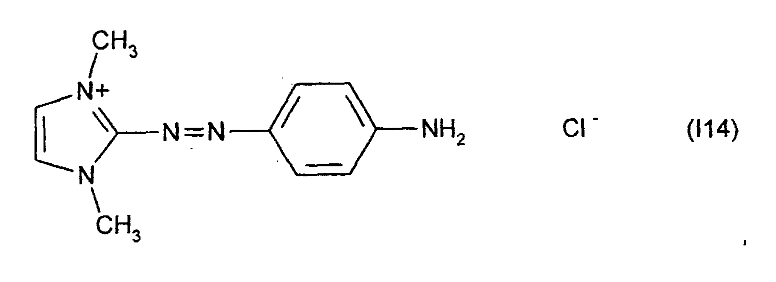

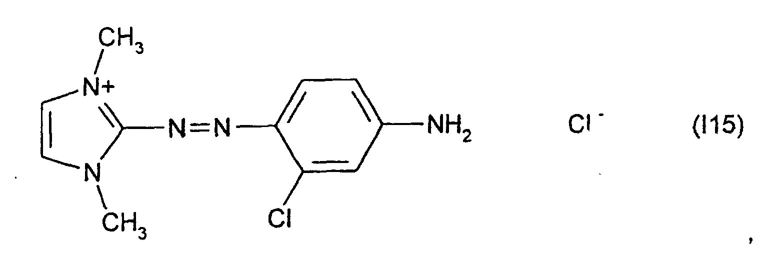

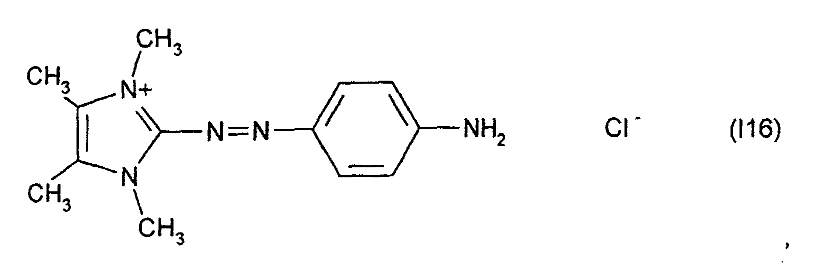

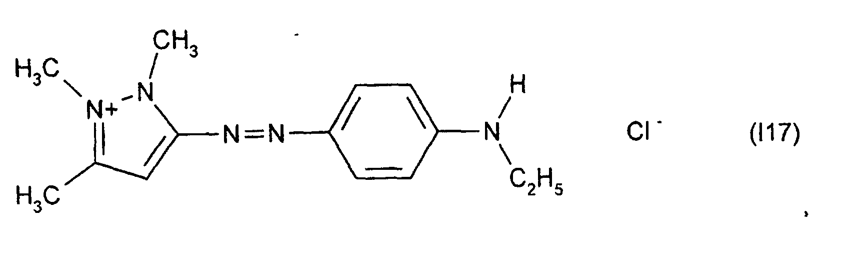

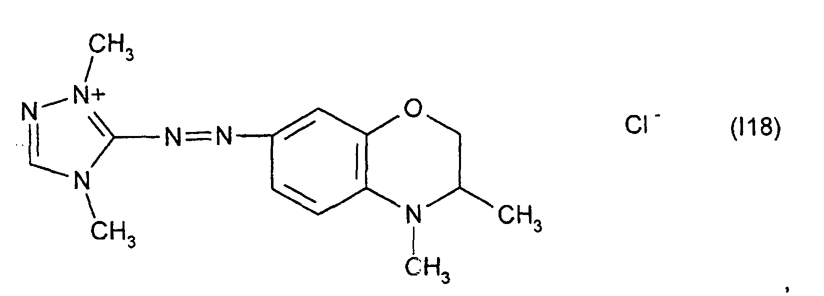

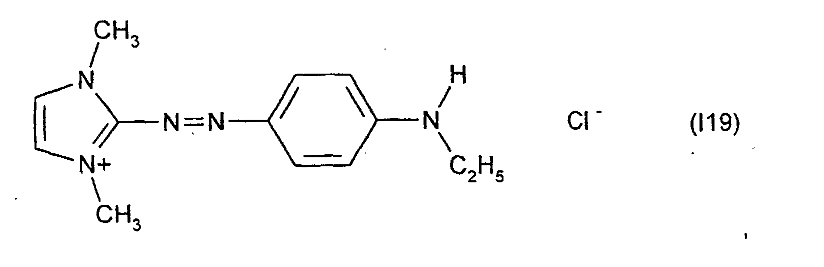

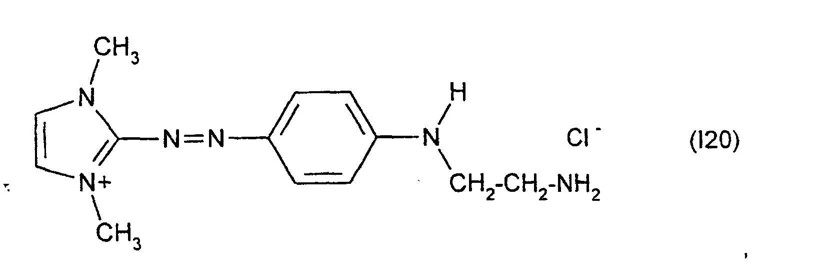

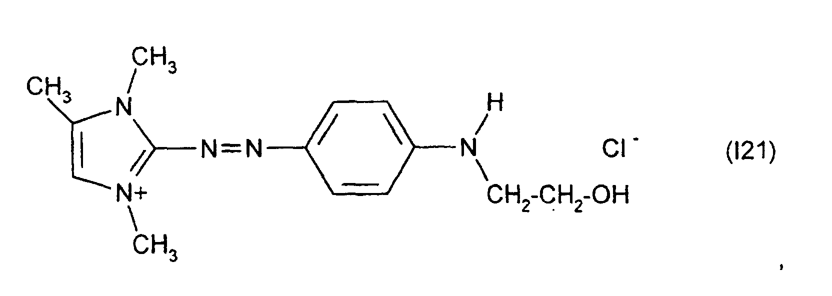

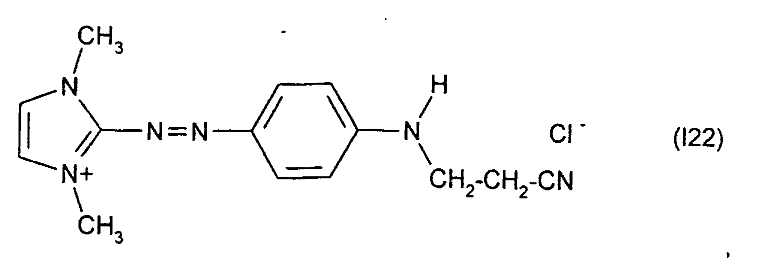

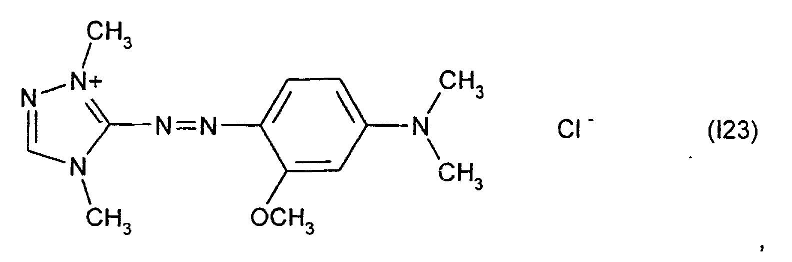

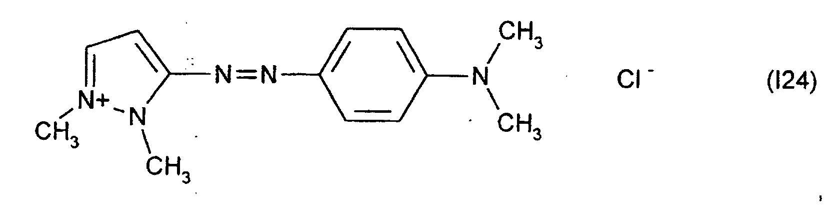

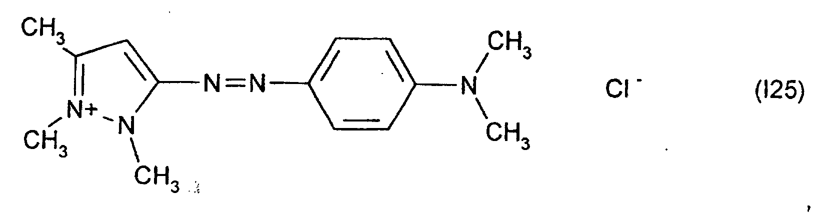

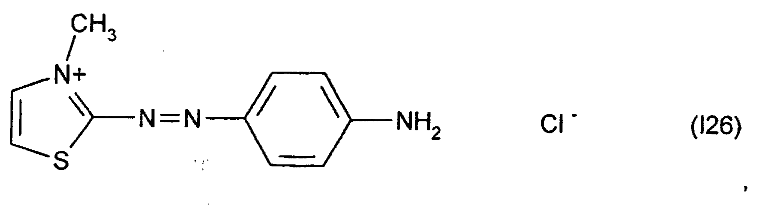

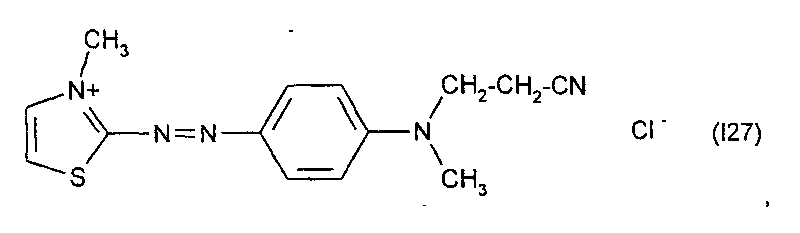

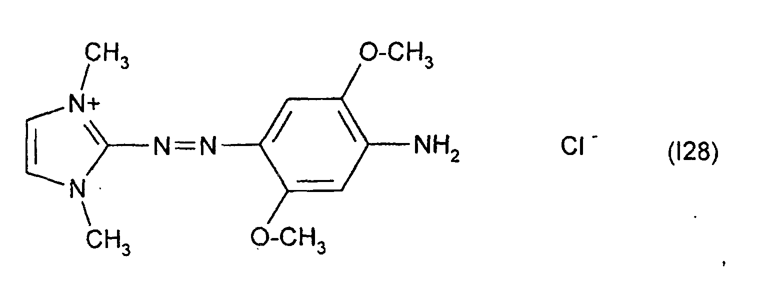

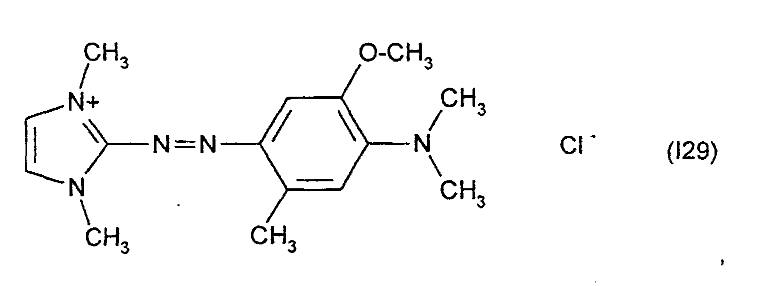

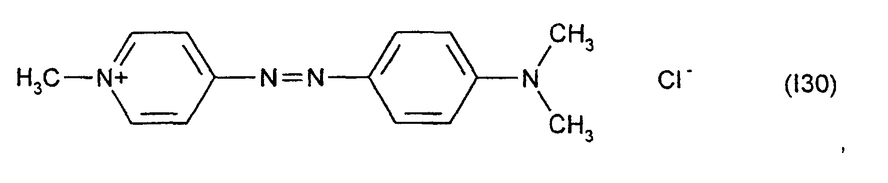

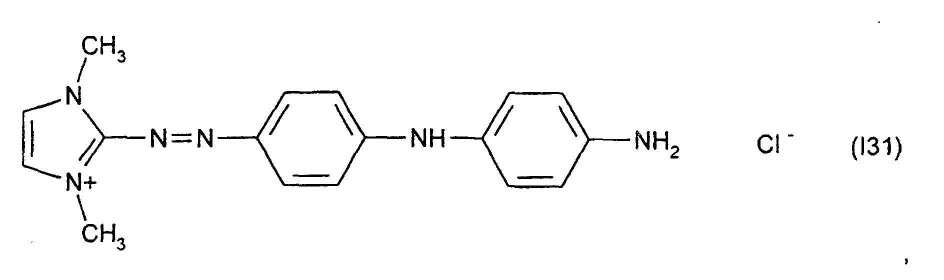

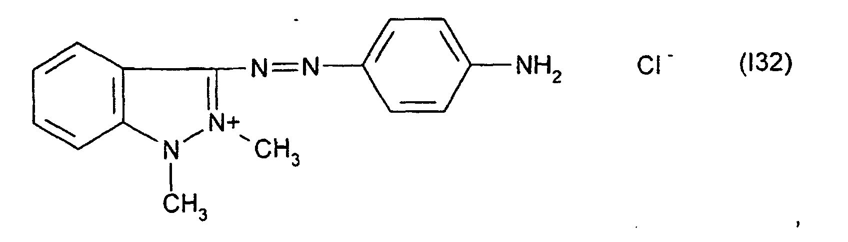

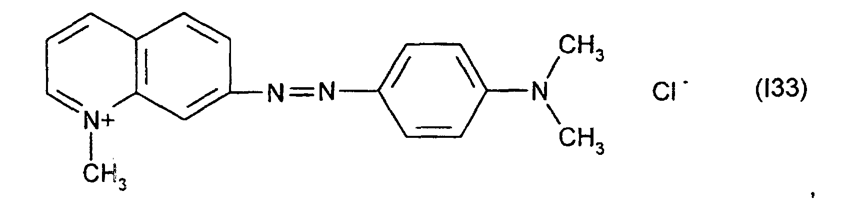

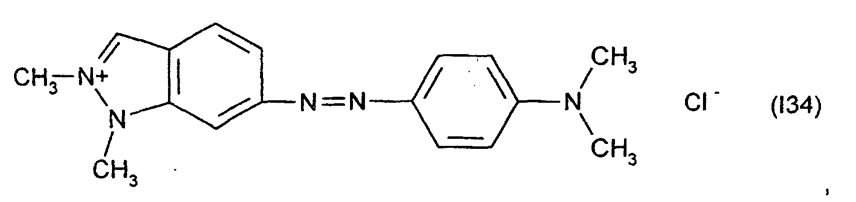

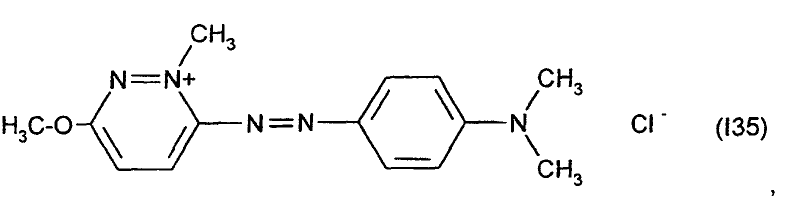

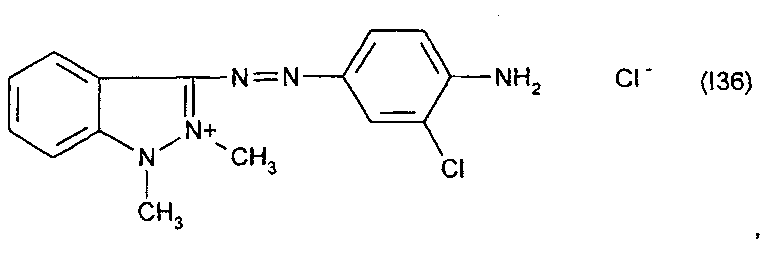

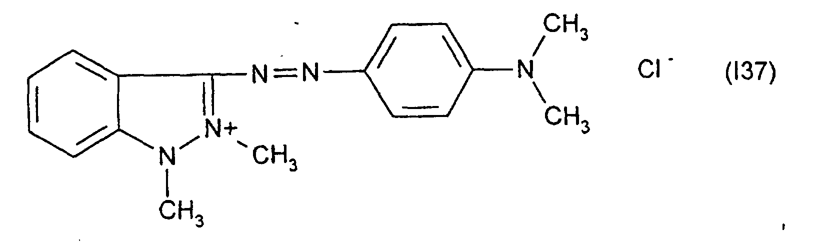

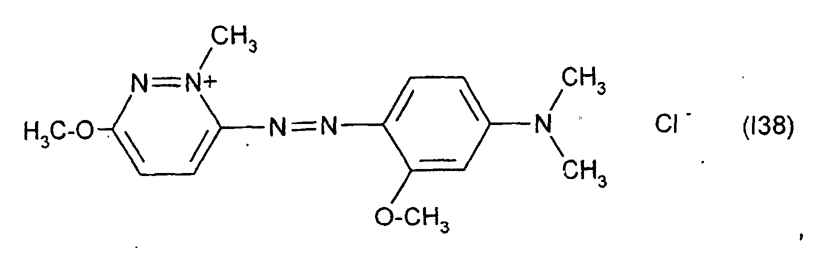

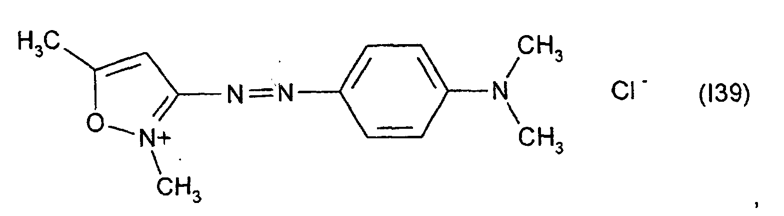

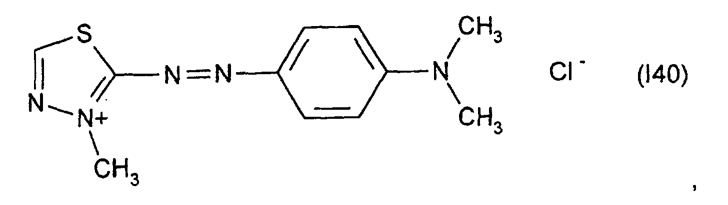

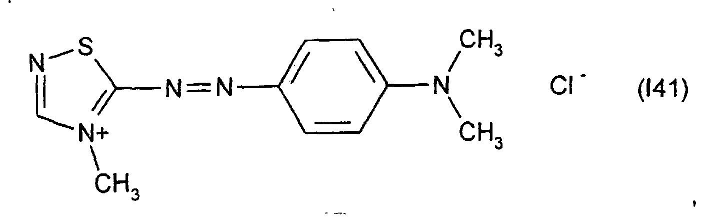

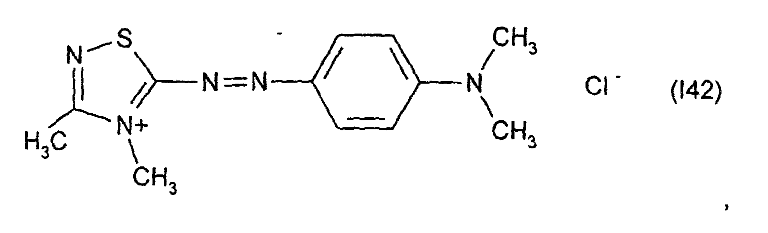

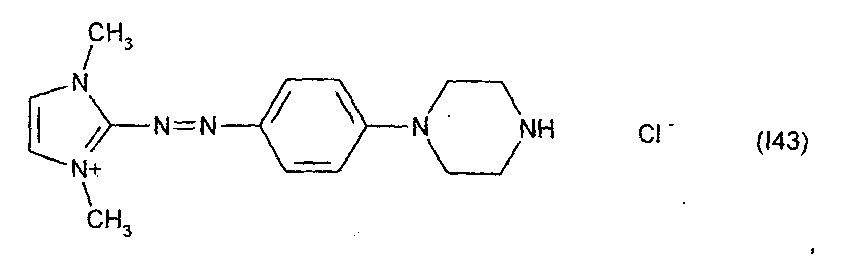

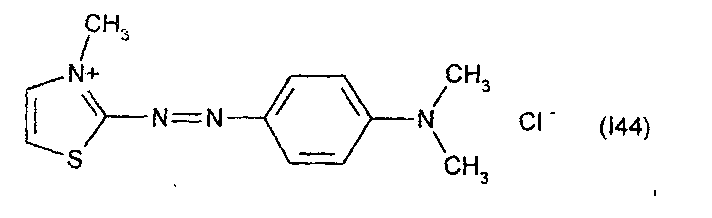

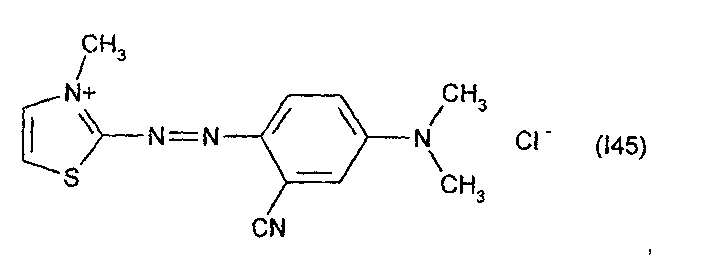

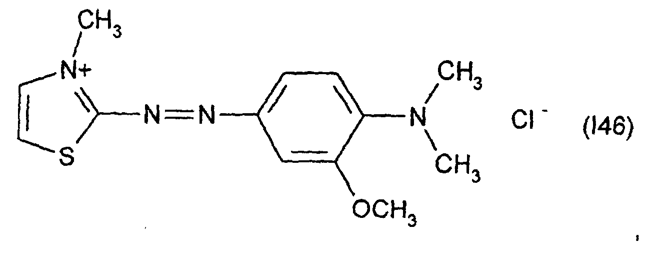

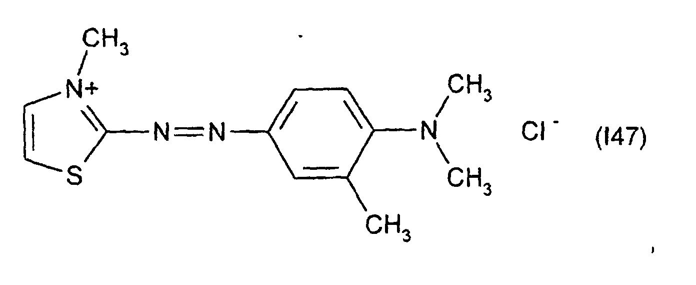

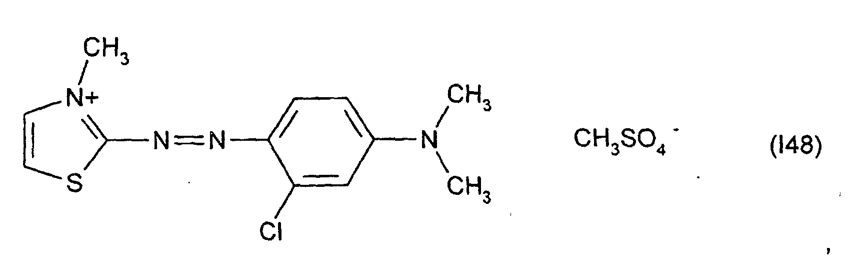

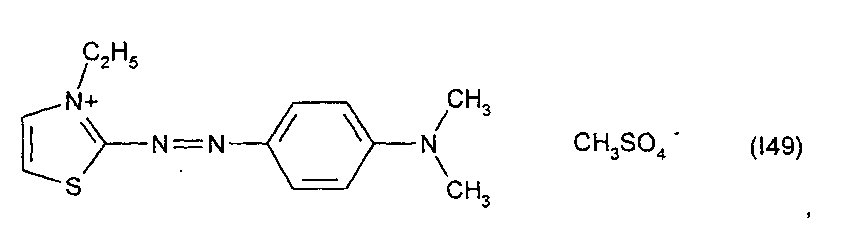

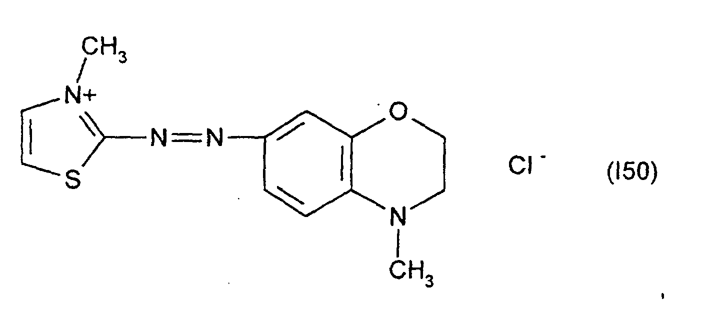

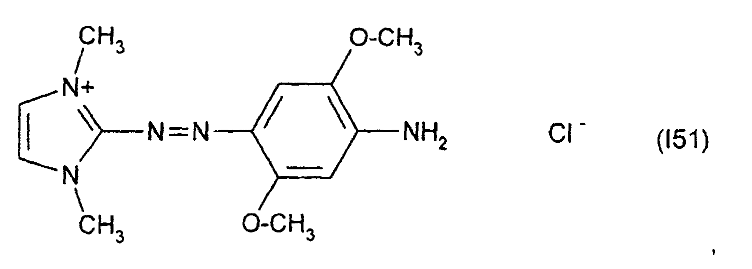

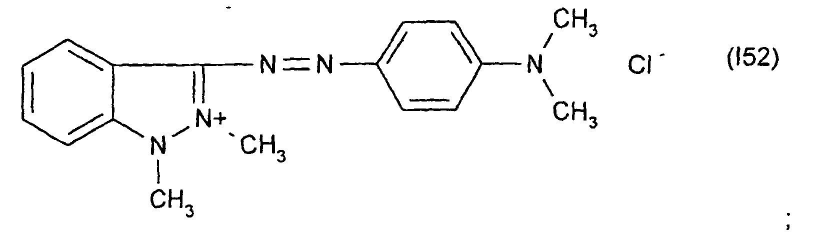

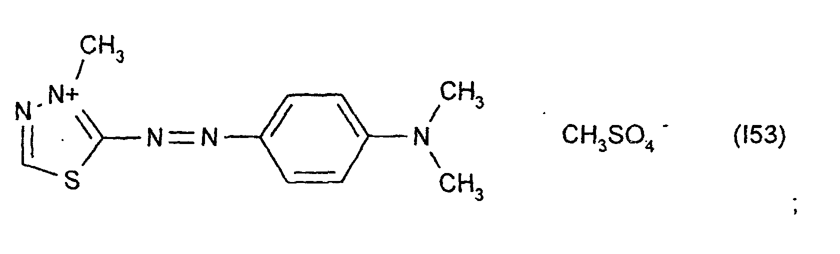

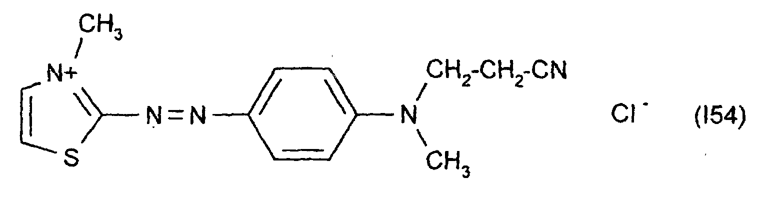

Parmi les composés de structures (I1) à (I54) décrits ci-dessus, on préfère tout particulièrement les composés répondant aux structures (I1), (I2), (I14) et (I31).

Parmi les colorants directs cationiques de formule (II) utilisables dans les compositions tinctoriales conformes à l'invention, on peut plus particulièrement citer les composés répondant aux structures (II1) à (II9) suivantes : 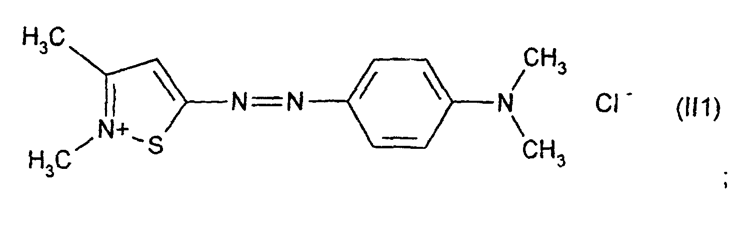 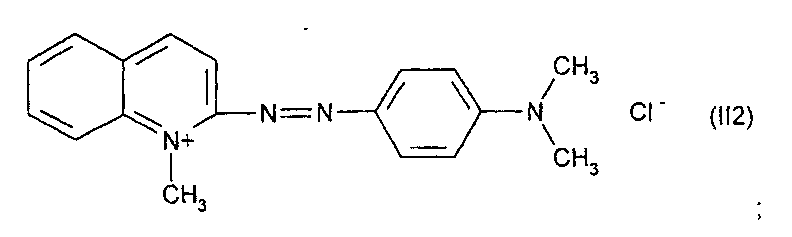 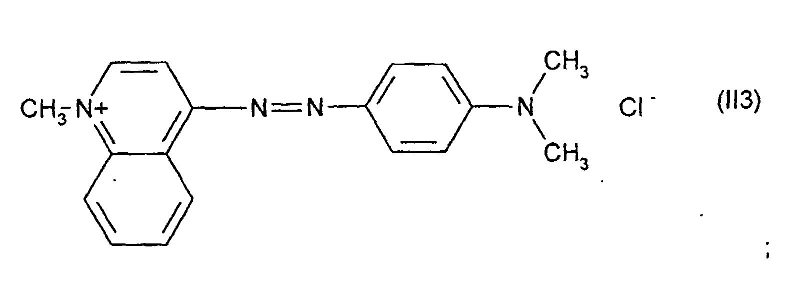 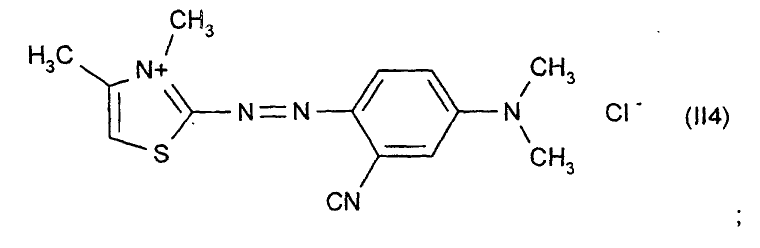 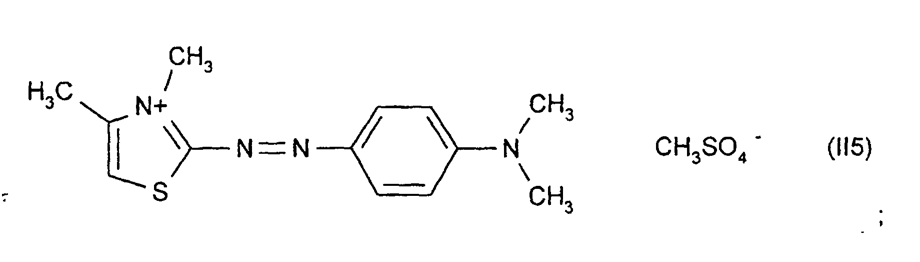 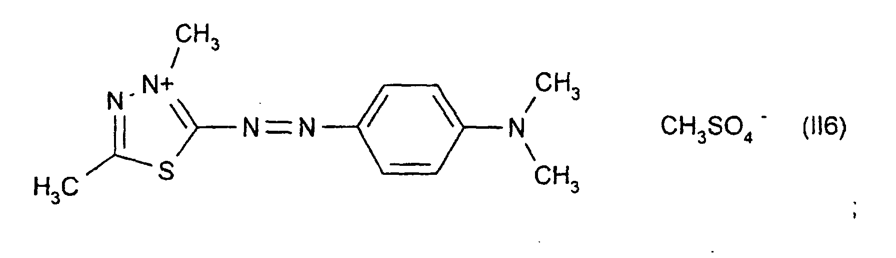 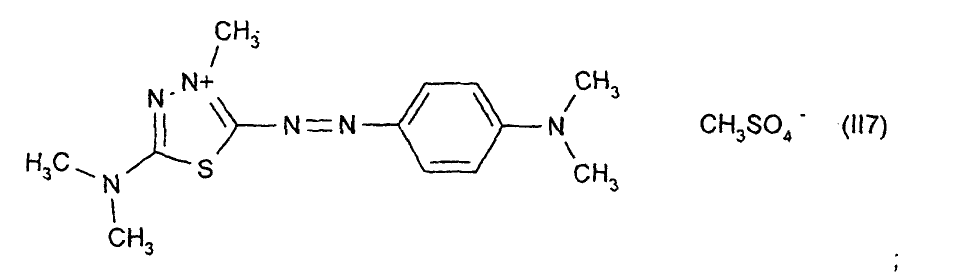 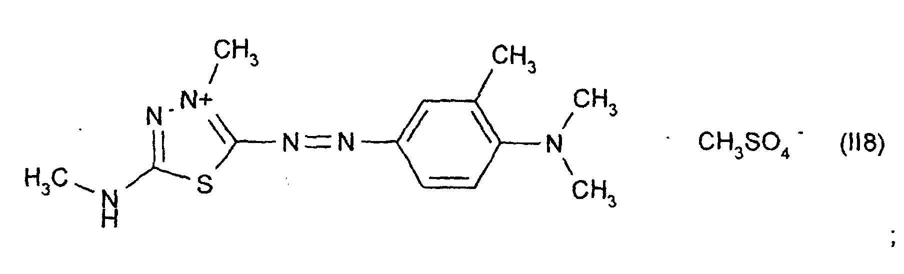 et

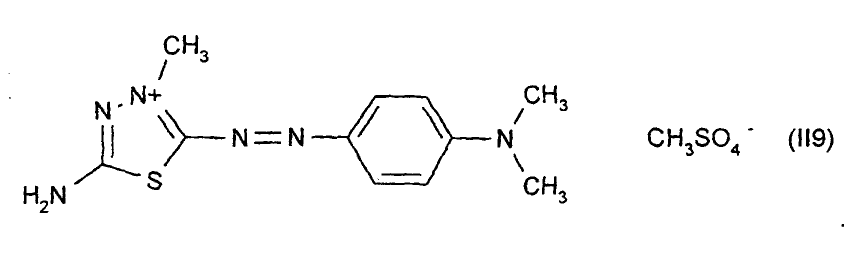

Parmi les colorants directs cationiques de formule (III), utilisables dans les compositions tinctoriales conformes à l'invention, on peut plus particulièrement citer les composés répondant aux structures (III1) à (III18) suivantes : 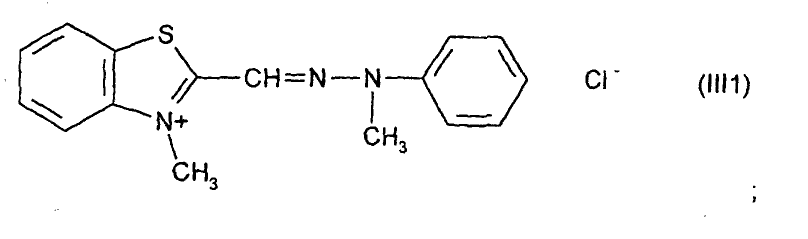 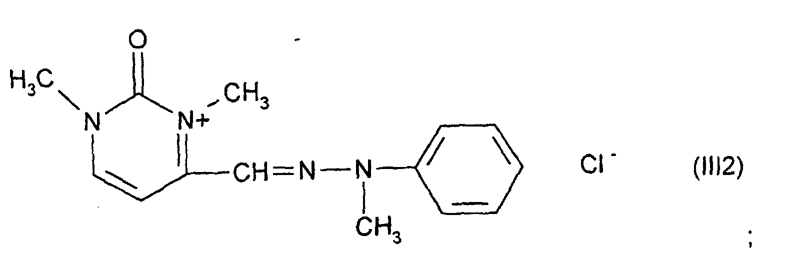 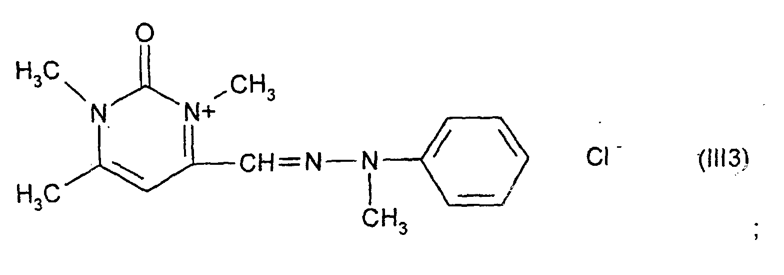 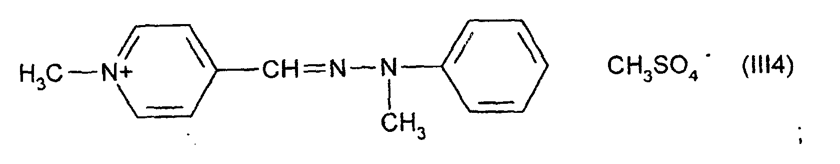 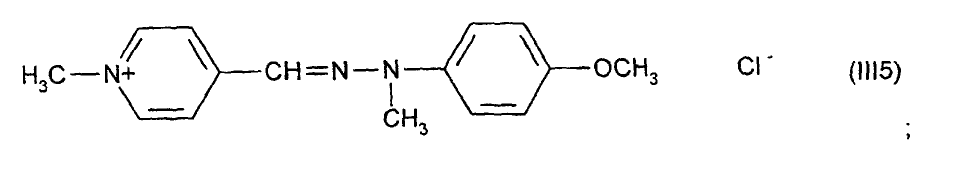 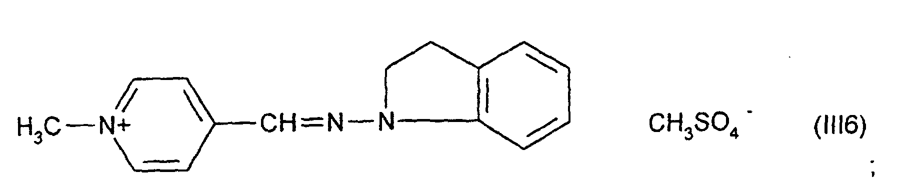 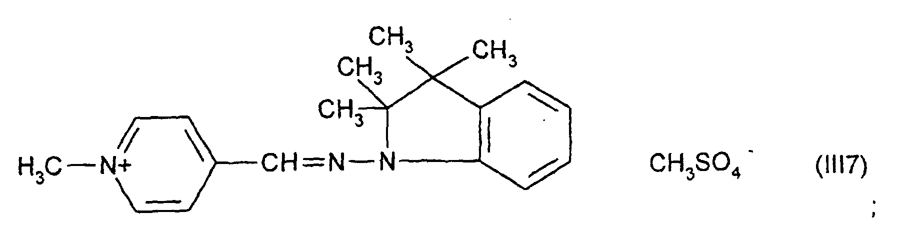 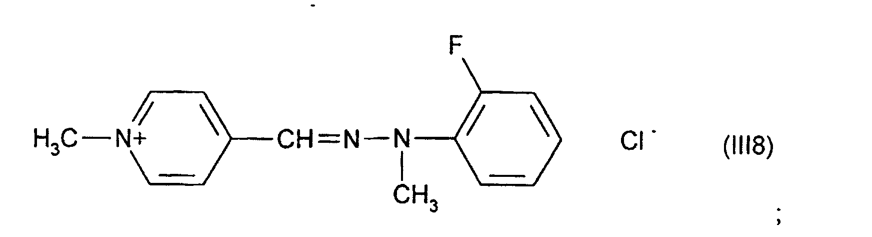 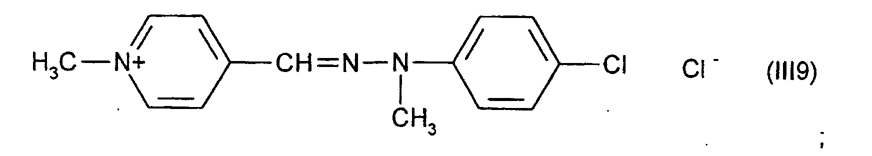 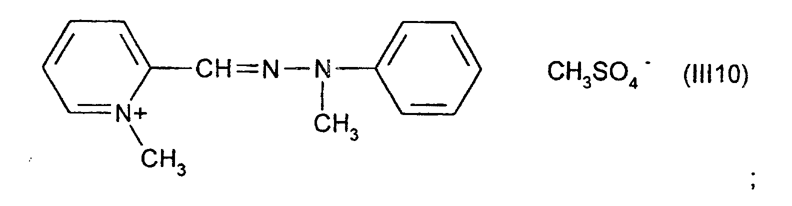 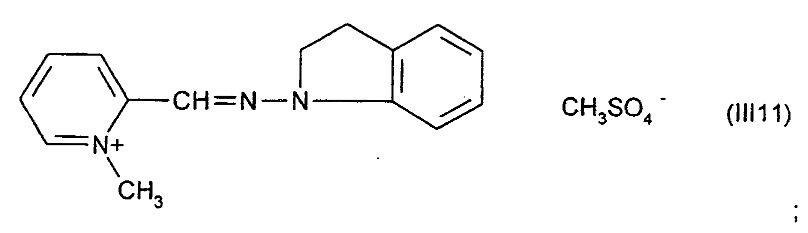 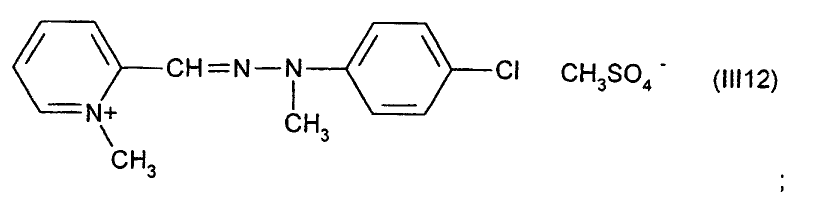 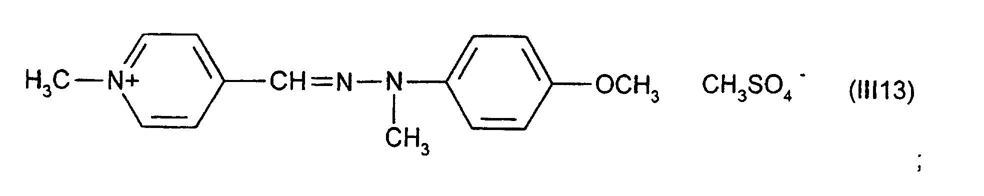 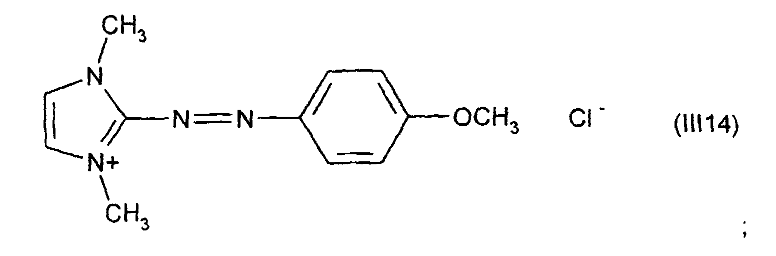 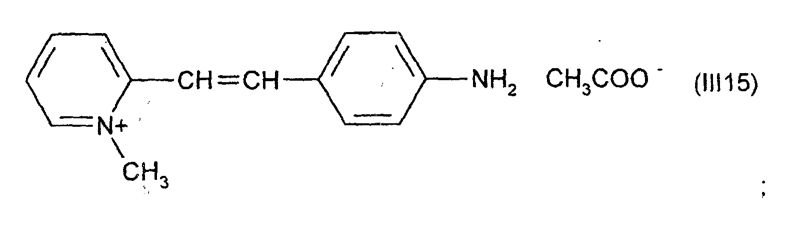 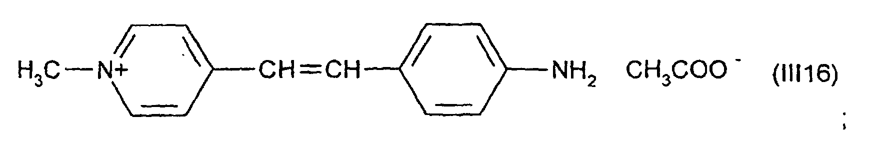 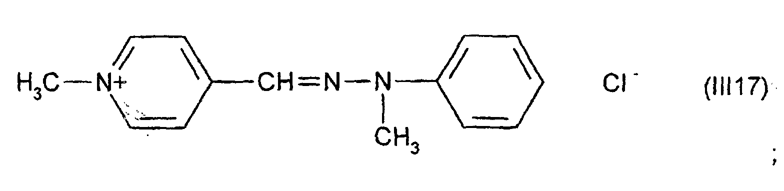 et

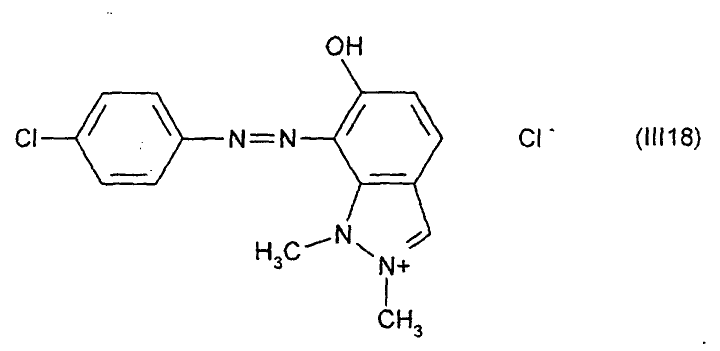

Parmi les composés particuliers de structures (III1) à (III18) décrits ci-dessus, on préfère tout particulièrement les composés répondant aux structures (III4), (III5) et (III13).

Parmi les colorants directs cationiques de formule (III'), utilisables dans les compositions tinctoriales conformes à l'invention, on peut plus particulièrement citer les composés répondant aux structures (III'1 ) à (III'3) suivantes : 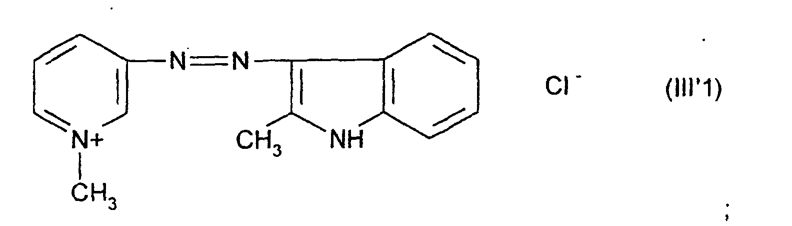 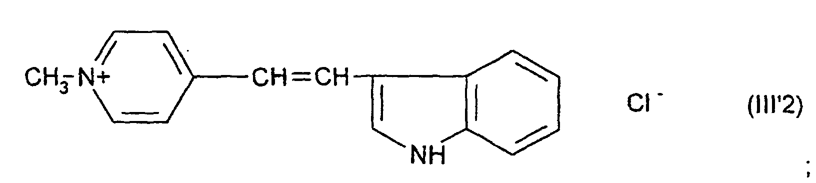 et

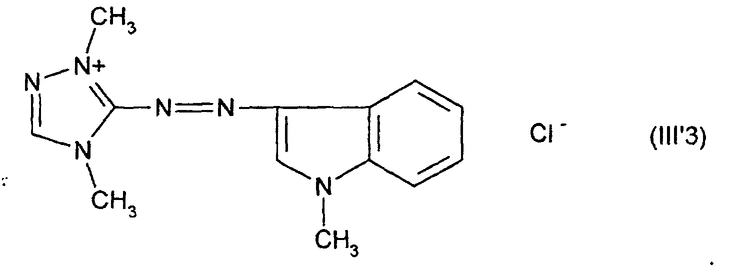

Parmi les colorants directs cationiques de formule (IV) utilisables dans les compositions tinctoriales conformes à l'invention, on peut citer plus particulièrement les composés de structures (IV)₁ à (IV)₇₇ suivantes :

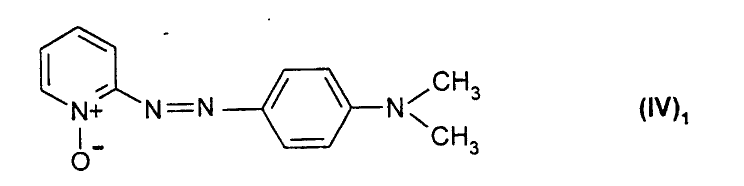

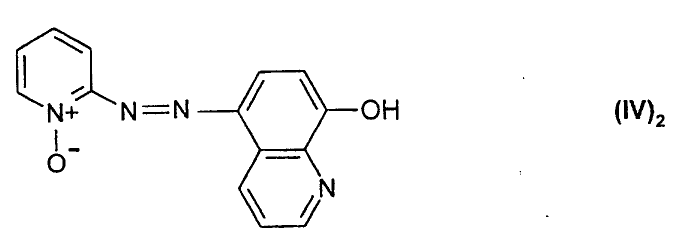

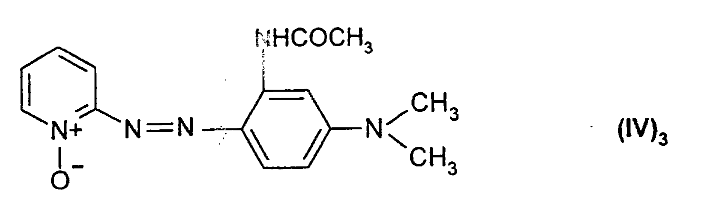

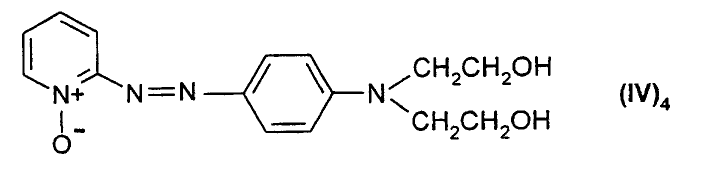

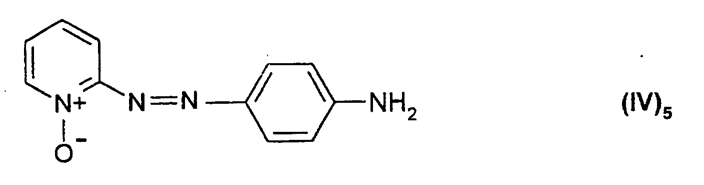

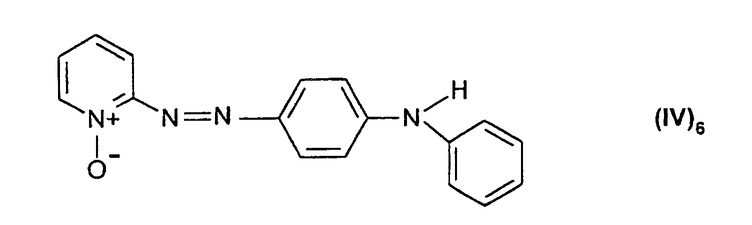

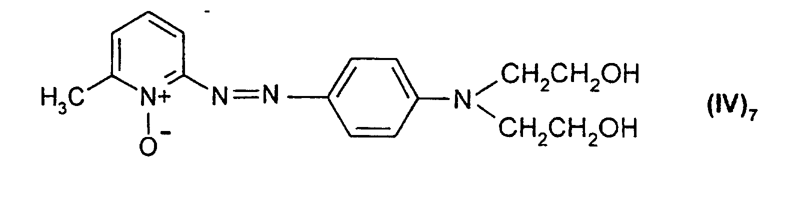

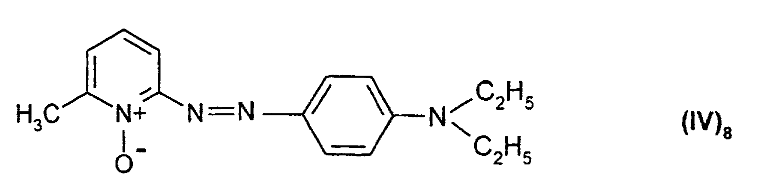

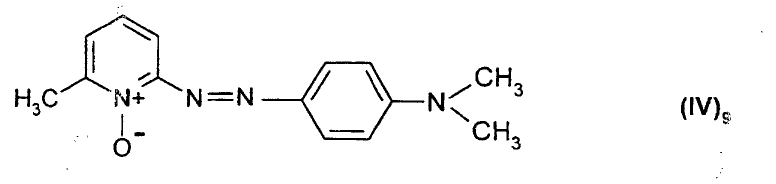

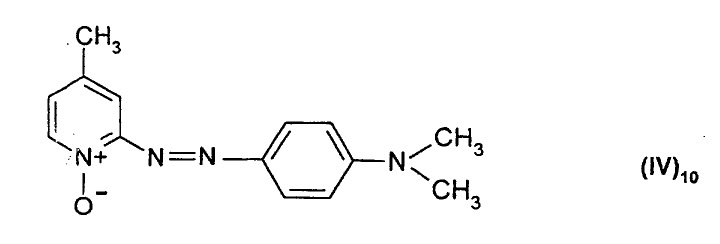

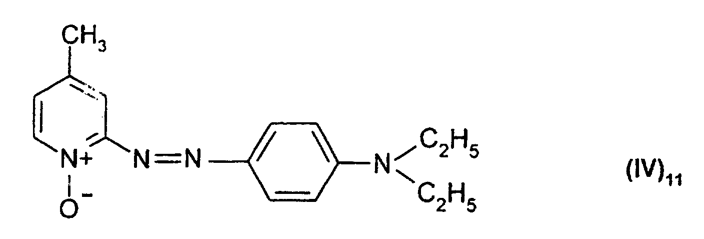

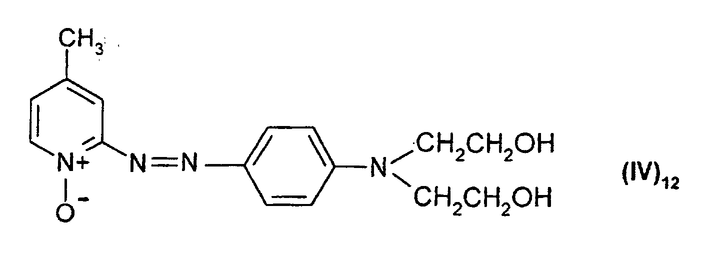

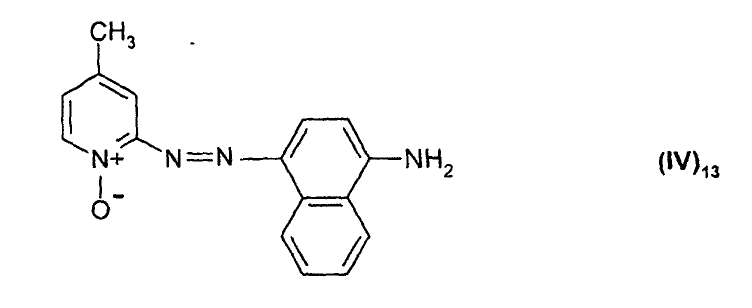

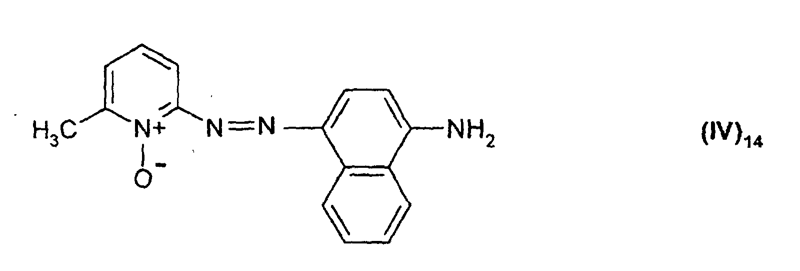

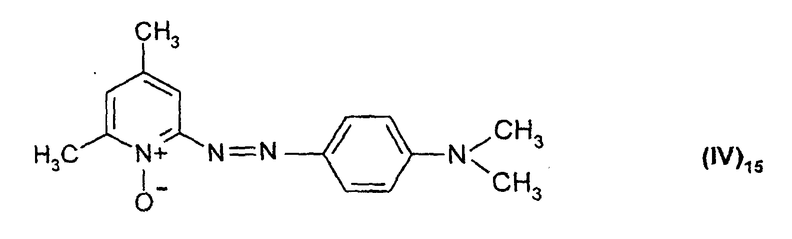

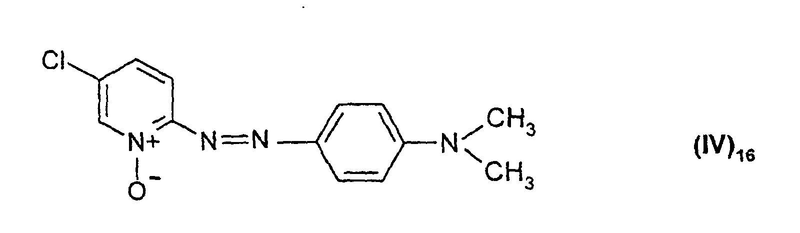

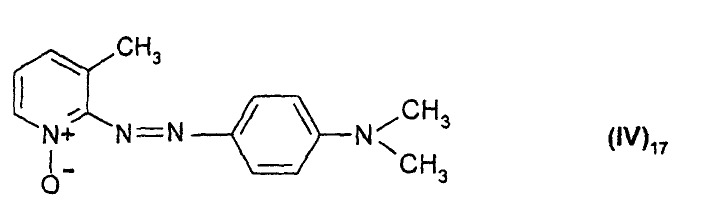

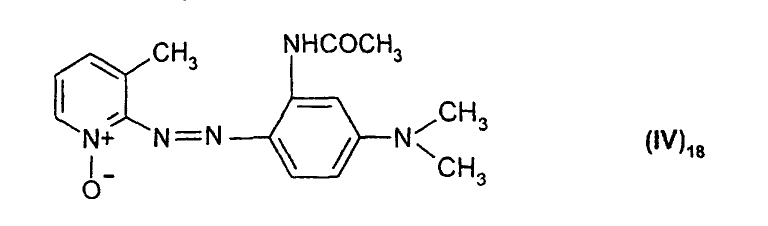

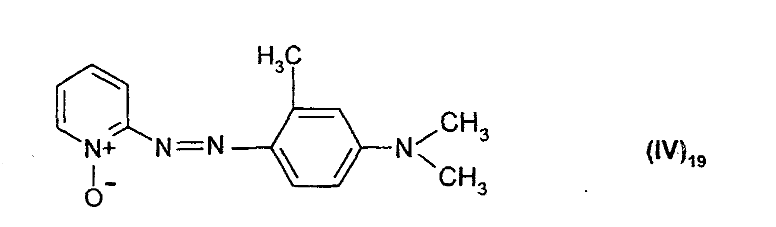

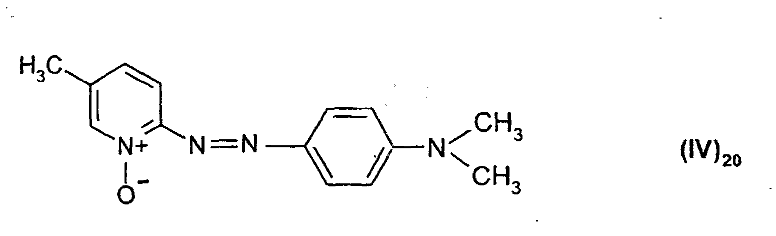

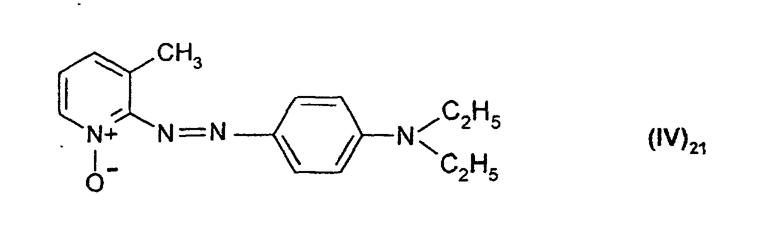

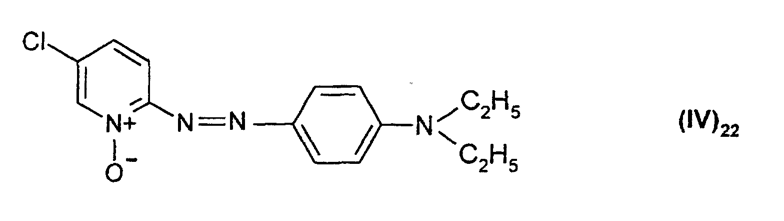

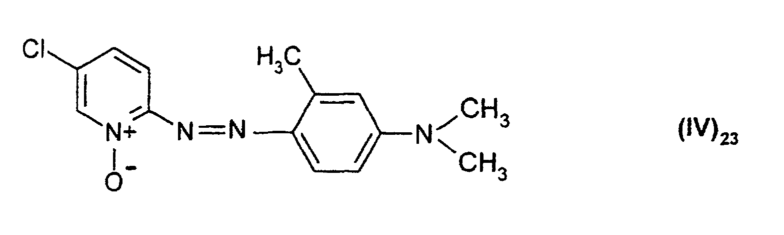

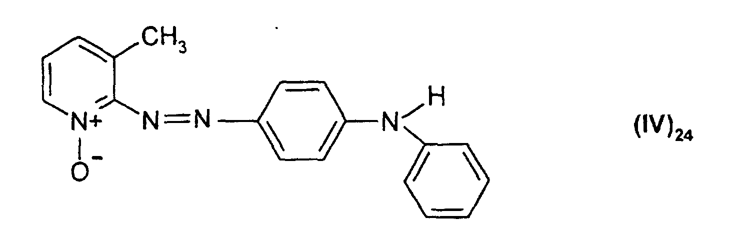

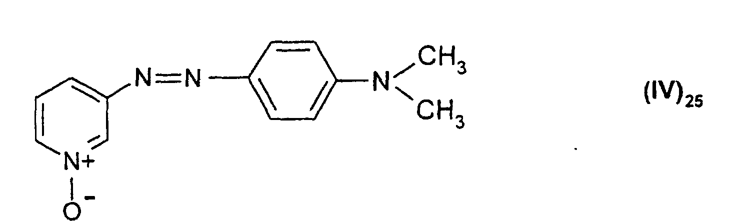

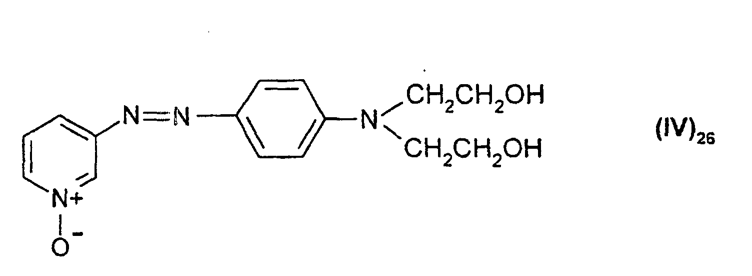

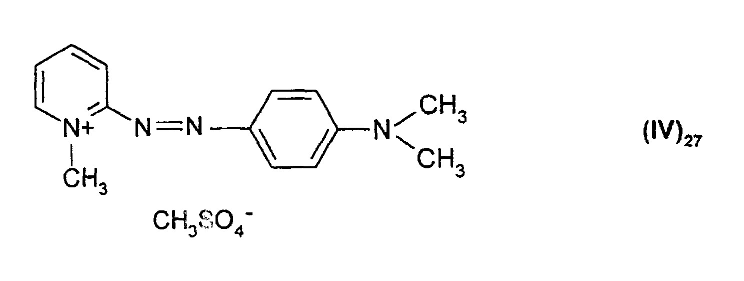

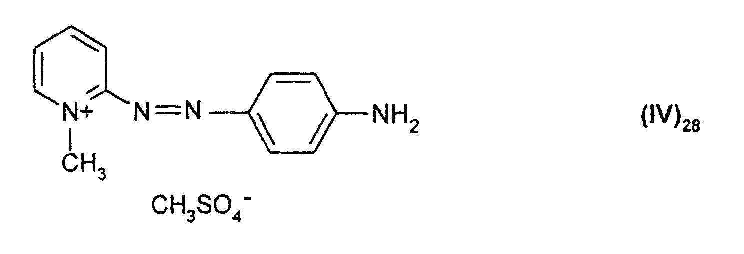

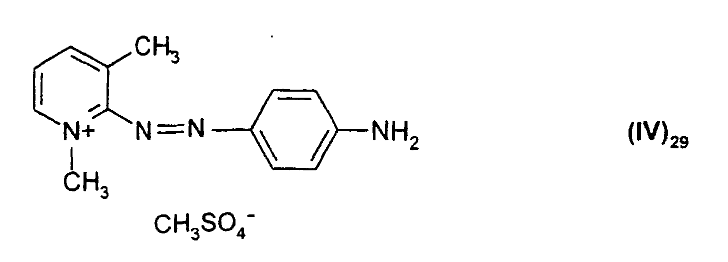

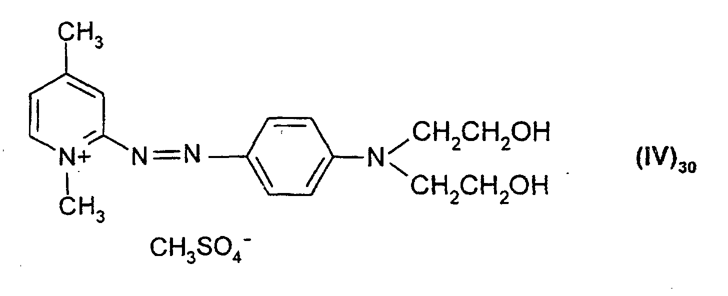

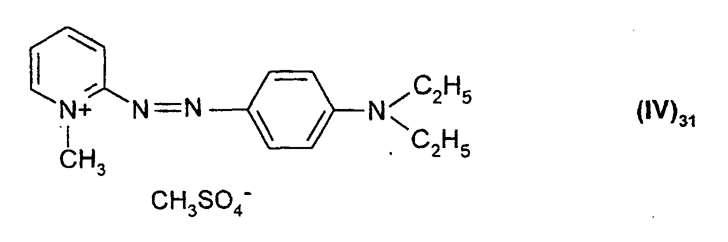

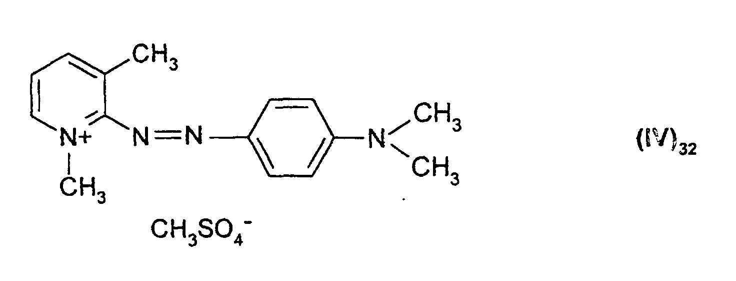

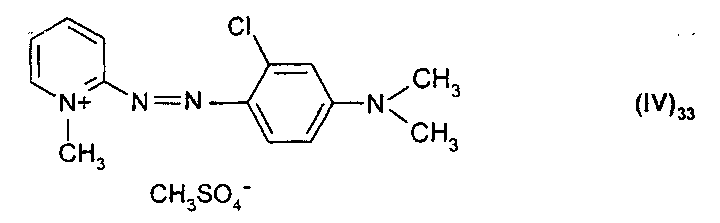

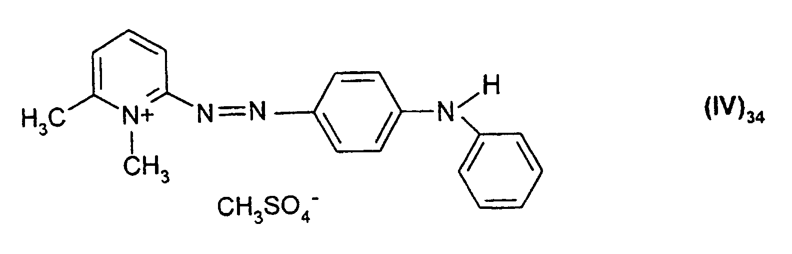

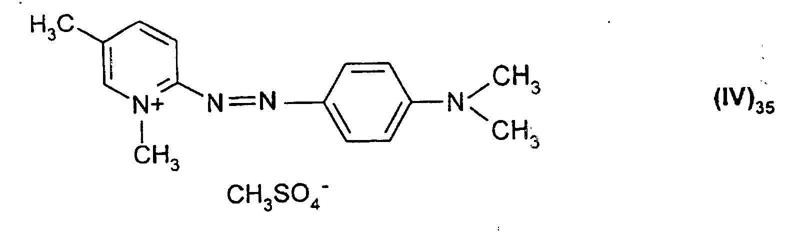

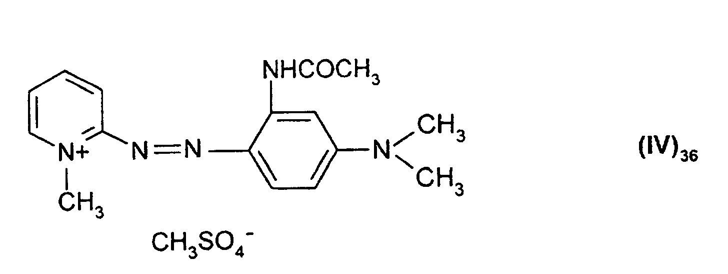

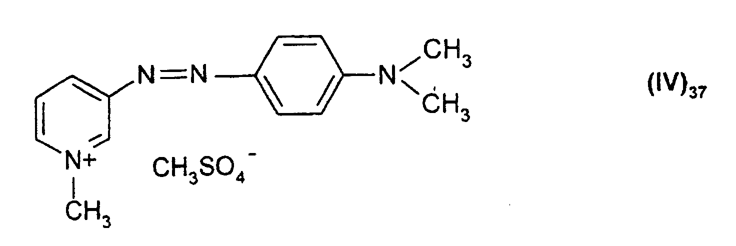

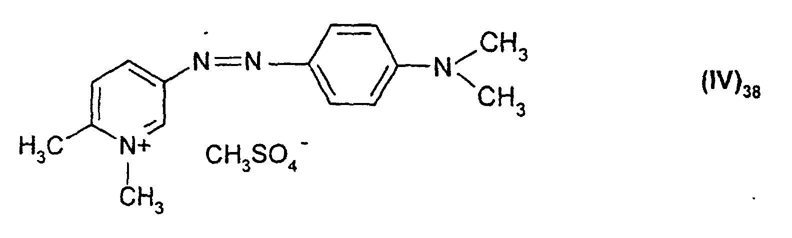

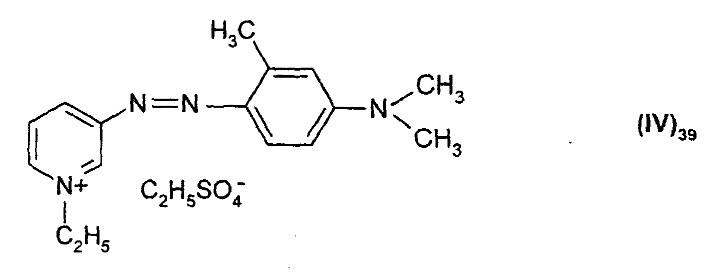

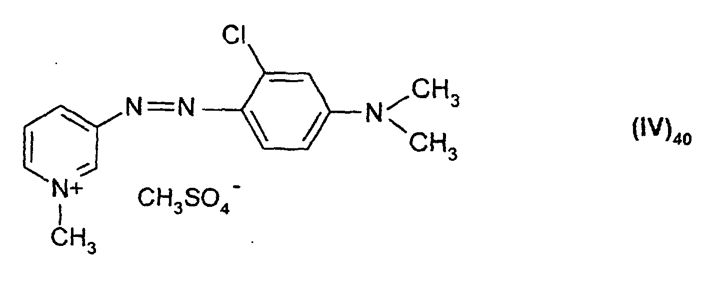

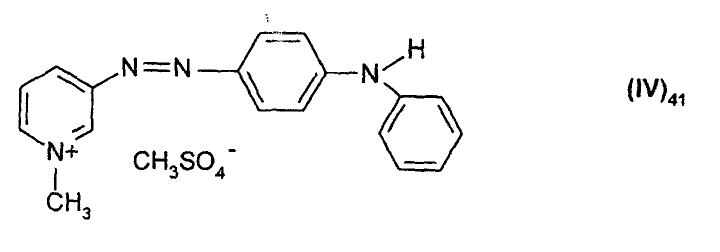

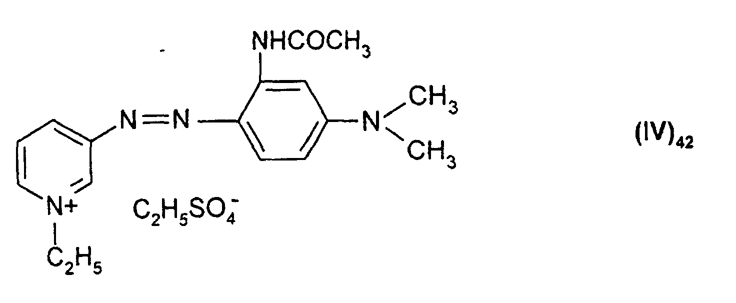

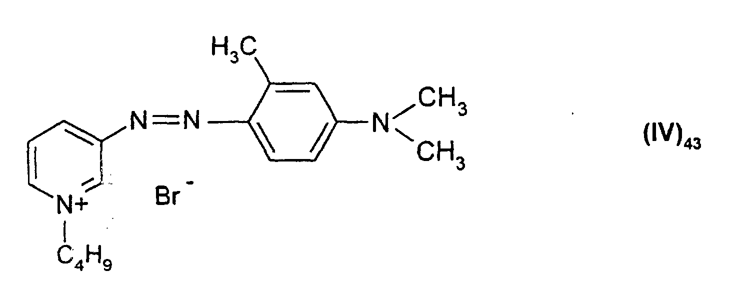

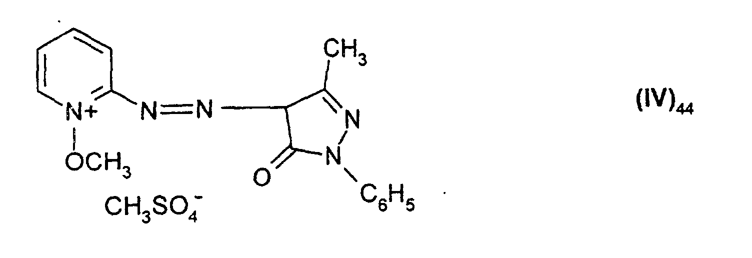

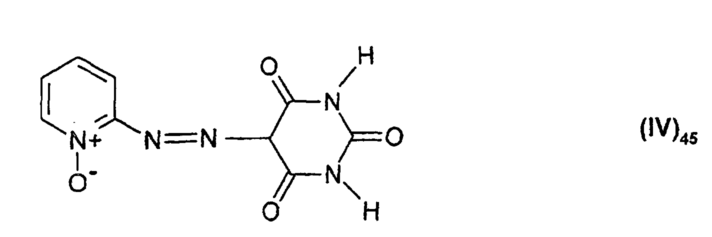

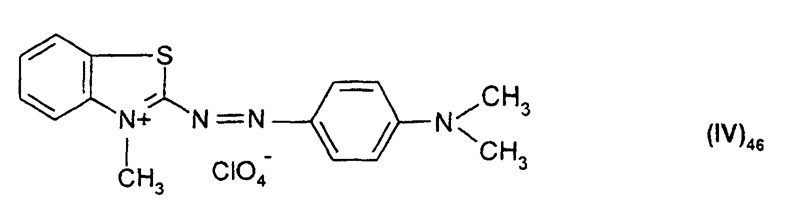

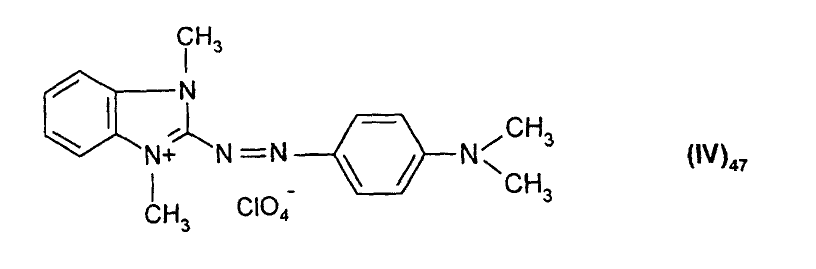

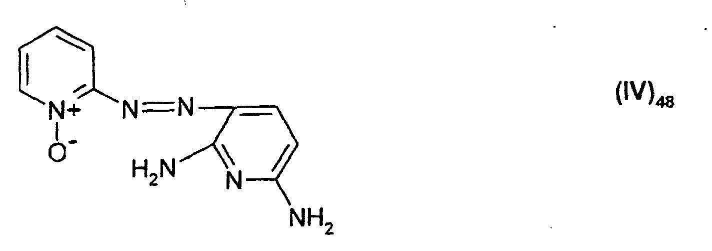

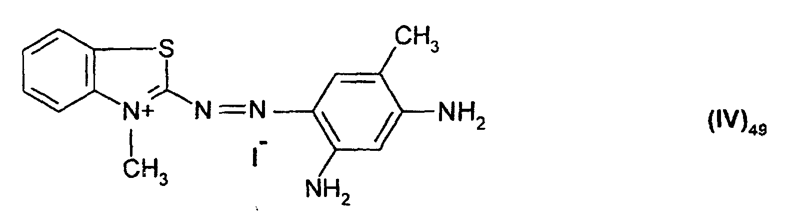

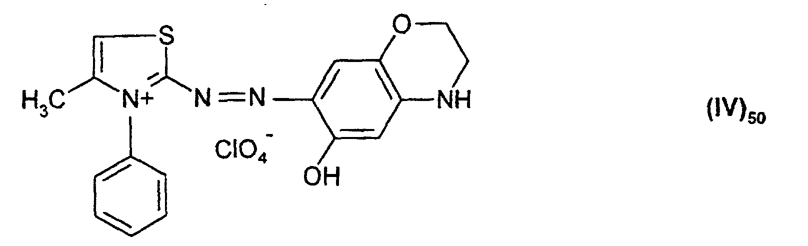

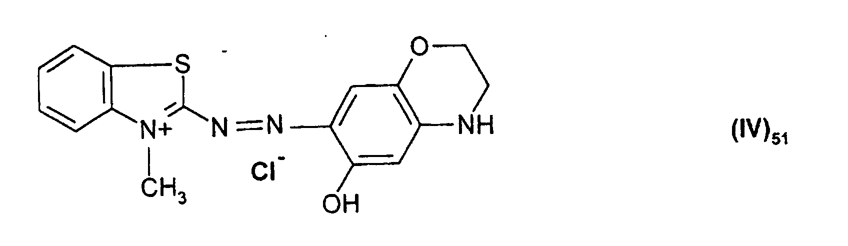

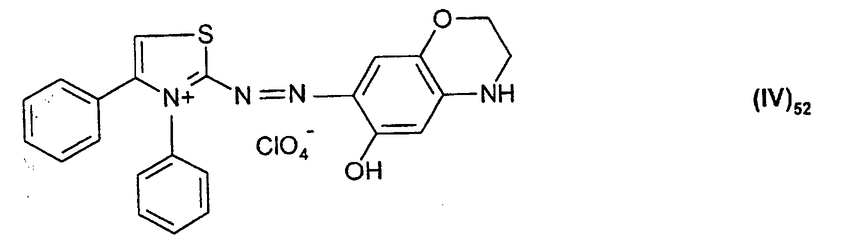

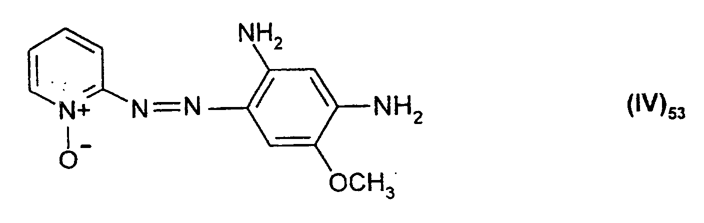

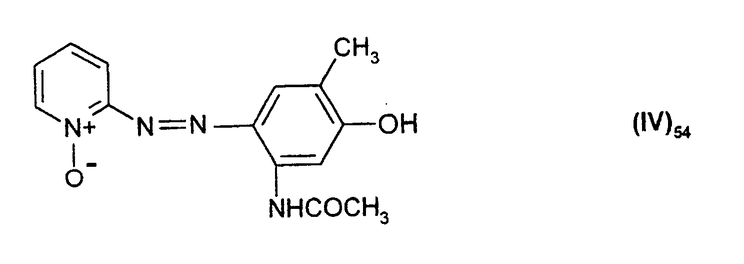

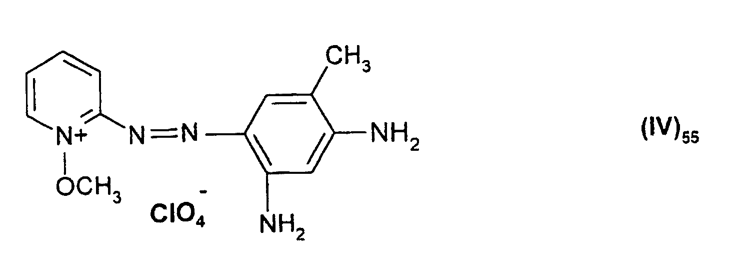

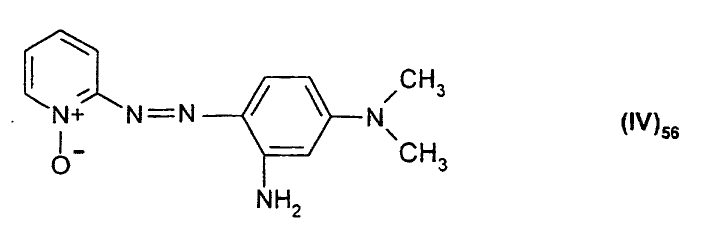

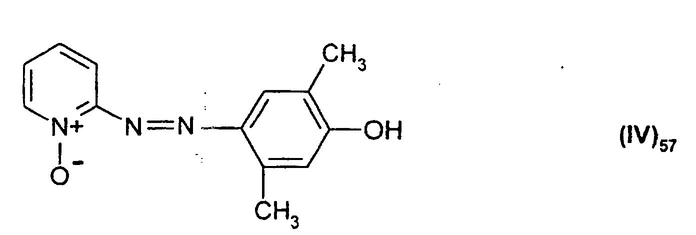

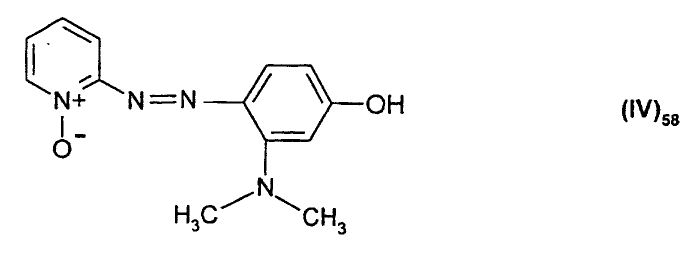

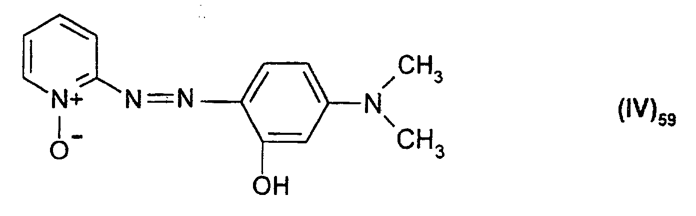

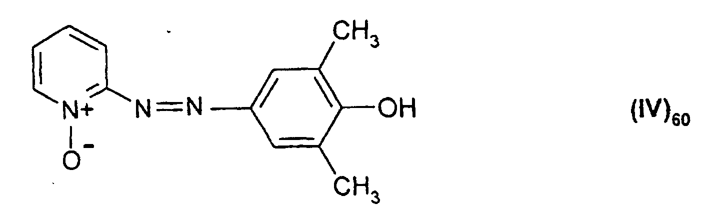

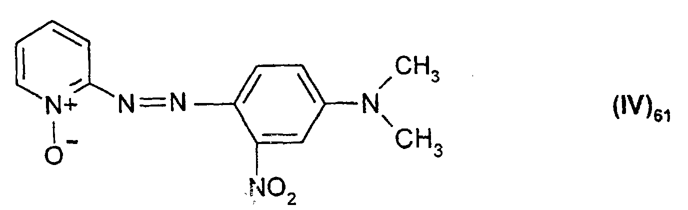

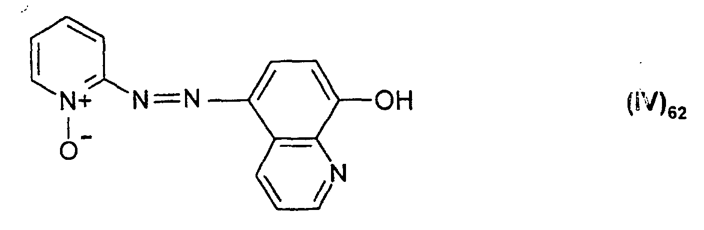

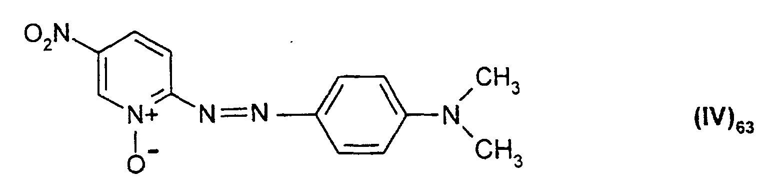

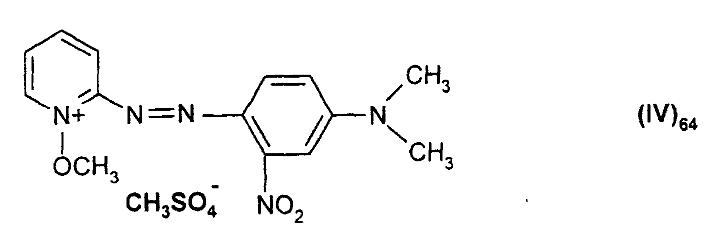

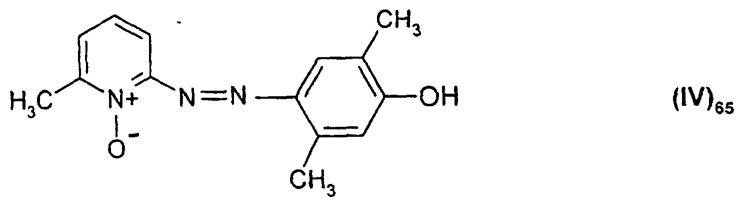

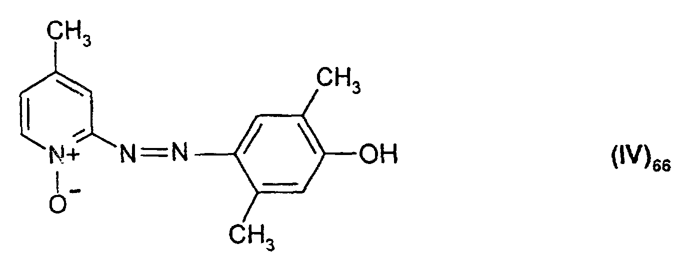

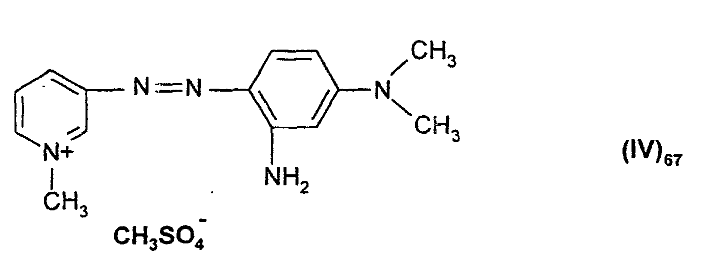

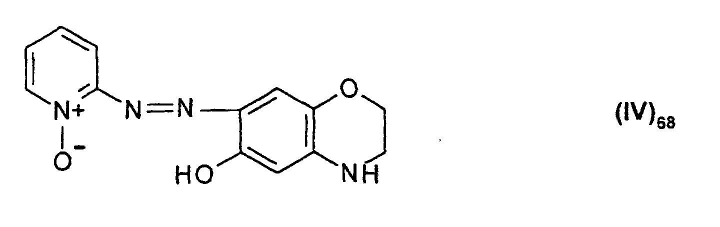

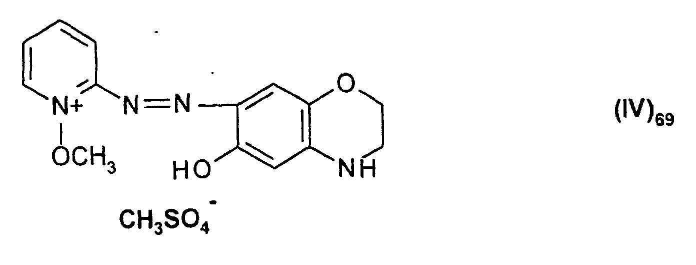

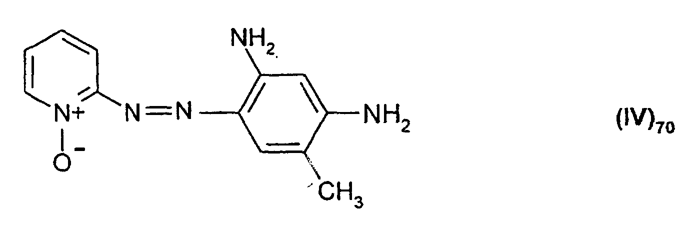

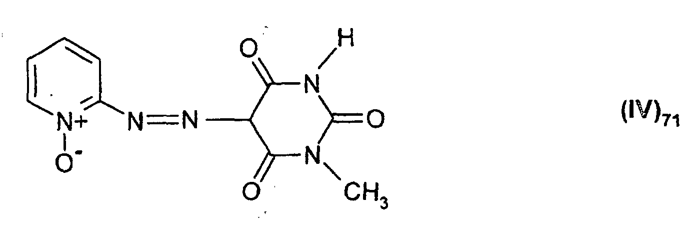

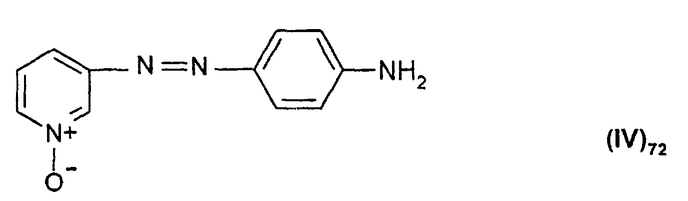

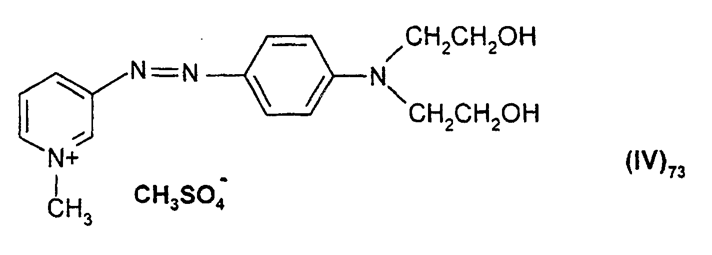

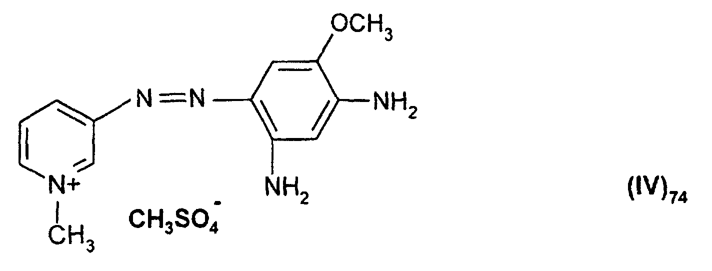

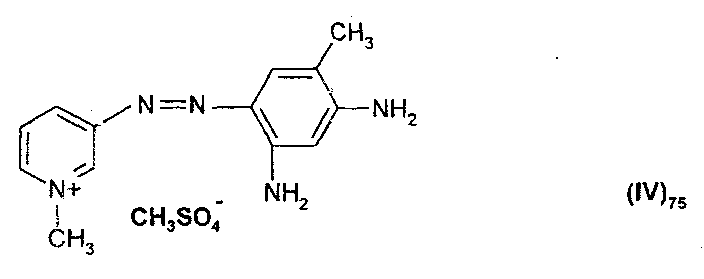

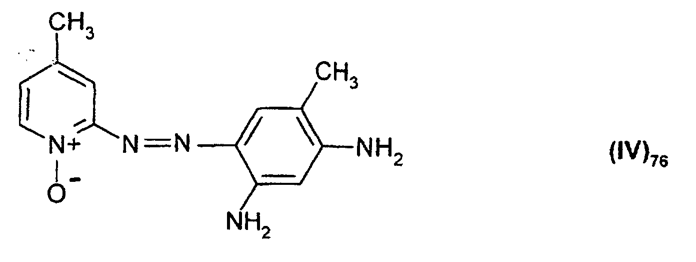

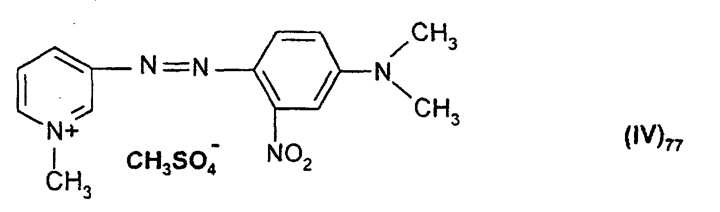

Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.

**(ii)** Le polymère épaississant utilisable selon la présente invention est choisi dans le groupe constitué par:
**(ii)**_{**1**} **-** les polymères amphiphiles non-ioniques comportant au moins un motif hydrophile et au moins un motif à chaîne grasse ;
**(ii)**_{**2**} **-** les polymères amphiphiles anioniques comportant au moins un motif hydrophile et au moins un motif à chaîne grasse ;
**(ii)**_{**3**} **-** les polymères amphiphiles cationiques comportant au moins un motif hydrophile et au moins un motif à chaîne grasse ;

Les polymères amphiphiles non-ioniques comportant au moins un motif hydrophile et au moins un motif à chaîne grasse **(ii)**_{**1**}**,** utilisés selon l'invention, sont choisis de préférence parmi :
**(ii)**_{**1**}**(a)** les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - les hydroxyéthylcelluloses modifiées par des groupements comportant au moins un groupe polyalkylène glycol éther d'alkyl phénol, tel que le produit AMERCELL POLYMER HM-1500 (polyéthylène glycol (15) éther de nonyl phénol) vendu par la société AMERCHOL.
**(ii)**_{**1**}**(b)** les hydroxypropylguars modifiés par des groupements comportant au moins une chaîne grasse tel que le produit ESAFLOR HM 22 (chaîne alkyle en C₂₂) vendu par la société LAMBERTI, les produits MIRACARE XC95-3 (chaîne alkyle en C₁₄) et RE205-1 (chaîne alkyle en C₂₀) vendus par la société RHONE POULENC.
**(ii)**_{**1**}**(c)** les uréthanes polyéthers comportant au moins une chaîne grasse telle que des groupes alkyle ou alcényle en C₈-C₃₀, comme les produits DAPRAL T 210 et DAPRAL T 212 vendus par la société AKZO.
**(ii)**_{**1**}**(d)** les copolymères de vinyl pyrrolidone et de monomères hydrophobes à chaîne grasse ;
   on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
**(ii)**_{**1**}**(e)** les copolymères de méthacrylates ou d'acrylates d'alkyles en C₁-C₆ et de monomères amphiphiles comportant au moins une chaîne grasse tels que par exemple le copolymère méthacrylate de méthyle/acrylate de stéaryle oxyéthyléné vendu par la société GOLDSCHMIDT sous la dénomination ANTIL 208.
**(ii)**_{**1**}**(f)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

Les polymères amphiphiles anioniques **(ii)**_{**2**} peuvent être choisis parmi ceux :
**(ii)**_{**2**}**(a)** comportant au moins un motif hydrophile et au moins un motif éther d'allyl à chaîne grasse et de préférence parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyl à chaîne grasse correspond au monomère de formule (V) suivante :

   CH₂=C R' CH₂ O Bₙ R (V)

   dans laquelle R' désigne H ou CH₃, B désigne le radical éthylèneoxy, n est nul ou désigne un entier allant de 1 à 100, R désigne un radical hydrocarboné choisi parmi les radicaux alkyl ou cycloalkyl, comprenant 8 à 30 atomes de carbone, de préférence 10 à 24, et plus particulièrement encore de 12 à 18 atomes de carbone, et tout particulièrement un radical alkyl en C₁₀-C₂₄.

Un motif de formule (V) plus particulièrement préféré selon la présente invention est un motif dans lequel R' désigne H, n est égal à 10, et R désigne un radical stéaryl (C₁₈).
Des polymères amphiphiles anioniques de ce type sont décrits et préparés, selon un procédé de polymérisation en émulsion, dans le brevet EP-0216479 B2.

Parmi lesdits polymères amphiphiles anioniques cités **(ii)**_{**2**}**(a),** on préfère utiliser particulièrement selon l'invention, les polymères formés à partir de 20 à 60% en poids d'acide acrylique et/ou d'acide méthacrylique, de 5 à 60% en poids de (méth)acrylates d'alkyls inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyl, le (méth)acrylate d'allyl, le divinylbenzène, le diméthacrylate de (poly)éthylèneglycol, et le méthylène-bis-acrylamide.
Et parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

Les polymères amphiphiles anioniques **(ii)**_{**2**} peuvent être également choisis parmi ceux :
**(ii)**_{**2**}**(b)** comportant au moins un motif hydrophile de type acide carboxylique insaturé oléfinique, et au moins un motif à chaîne grasse exclusivement de type ester d'alkyl (C₁₀-C₃₀) d'acide carboxylique insaturé, et de préférence parmi ceux dont le motif hydrophile de type acide carboxylique insaturé oléfinique correspond au monomère de formule (VI) suivante : 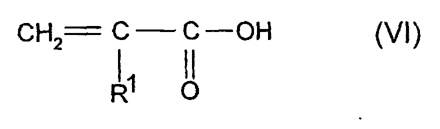 formule dans laquelle, R¹ désigne H ou CH₃ ou C₂H₅, c'est-à-dire des motifs acide acrylique, acide méthacrylique ou acide éthacrylique, et dont le motif à chaîne grasse de type ester d'alkyl (C₁₀-C₃₀) d'acide carboxylique insaturé correspond au monomère de formule (VII) suivante : 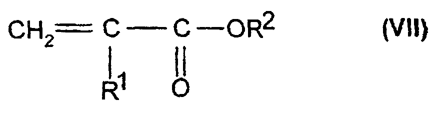 formule dans laquelle, R¹ désigne H ou CH₃ ou C₂H₅ (c'est-à-dire des motifs acrylates, méthacrylates ou éthacrylates) et de préférence H (motifs acrylates) ou CH₃ (motifs méthacrylates), R² désignant un radical alkyle en C₁₀-C₃₀, et de préférence en C₁₂-C₂₂.
Des esters d'alkyls (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.

Des polymères amphiphiles anioniques de ce type **(ii)**_{**2**}**(b)** sont par exemple décrits et préparés, selon les brevets US-3 915 921 et 4 509 949.

Des polymères amphiphiles anioniques **(ii)**_{**2**}**(b)** utilisables dans le cadre de la présente invention peuvent désigner plus particulièrement des polymères formés à partir d'un mélange de monomères comprenant :
(i) essentiellement de l'acide acrylique et un ester de formule (VII) décrite ci-dessus dans laquelle R¹ désigne H ou CH₃, R² désignant un radical alkyle ayant de 12 à 22 atomes de carbone, et un agent réticulant, tels que par exemple ceux constitués de 95 à 60% en poids d'acide acrylique (motif hydrophile), 4 à 40% en poids d'acrylate d'alkyles en C₁₀-C₃₀ (motif à chaîne grasse), et 0 à 6% en poids de monomère polymérisable réticulant, ou 98 à 96% en poids d'acide acrylique (motif hydrophile), 1 à 4% en poids d'acrylate d'alkyles en C₁₀-C₃₀ (motif à chaîne grasse), et 0,1 à 0,6% en poids de monomère polymérisable réticulant,
(ii) essentiellement de l'acide acrylique et du méthacrylate de lauryle tel que celui formé à partir de 66% en poids d'acide acrylique et 34% en poids de méthacrylate de lauryle.

Ledit réticulant est un monomère contenant un groupe CH₂=C〈 avec au moins un autre groupement polymérisable dont les liaisons insaturées sont non conjuguées l'une par rapport à l'autre. On peut notamment citer les polyallyléthers tels que notamment le polyallylsucrose et le polyallylpentaérythritol.

Parmi lesdits polymères cités dans la classe **(ii)**_{**2**}**(b),** on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.C. sous la dénomination COATEX SX.

Les polymères amphiphiles cationiques (ii)₃ utilisés selon l'invention sont choisis de préférence parmi les dérivés de cellulose quaternisée et les polyacrylates à groupements latéraux aminés.

Les dérivés de cellulose quaternisée sont, en particulier,
**(ii)**_{**3**}**(a)** les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
**(ii)**_{**3**}**(b)** les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les polyacrylates à groupements latéraux aminés **(ii)**_{**3**}**(c),** quaternisés ou non, possèdent par exemple des groupements hydrophobes du type stéareth 20 [alcool stéarylique polyoxyéthyléné (20)].

Les radicaux alkyle portés par les cellluloses ou hydroxycelluloses quaternisées ci-dessus comportent de préférence de 8 à 30 atomes de carbone.
Les radicaux aryle désignent de préférence les groupements phényle, benzyle, naphtyle ou anthryle.

On peut indiquer comme exemples d'alkylhydroxyéthylcelluloses quaternisées à chaînes grasses en C₈-C₃₀, les produits QUATRISOFT LM200, QUATRISOFT LM-X529-18-A, QUATRISOFT LM-X529-18-B (alkyle en C₁₂) et QUATRISOFT LM-X529-8 (alkyle en C₁₈) commercialisés par la société AMERCHOL et les produits CRODACEL QM, CRODACEL QL (alkyle en C₁₂), et CRODACEL QS (alkyle en C₁₈) commercialisés par la société CRODA.

Comme exemples de polyacrylates à chaînes latérales aminées, on peut citer les polymères 8781-124B ou 9492-103 de la société NATIONAL STARCH.

On préfère plus particulièrement, selon la présente invention, utiliser les polymères amphiphiles de type non-ionique **(ii)**_{**1**} et de type anionique **(ii)**_{**2**} décrits ci-avant et plus particulièrement encore les polymères amphiphiles de classe **(ii)**_{**1**}**(a)** et **(ii)**_{**1**}**(c)** et de classe **(ii)**_{**2**}**(a)** et **(ii)**_{**2**}**(b).**

Les polymères épaississants amphiphiles de type non-ionique, anionique ou cationique utilisés dans les compositions de la présente invention sont présents de préférence à raison de 0,01 à 10% environ en poids, en particulier à raison de 0,1 à 5% environ en poids par rapport au poids total de la composition de teinture appliquée sur les fibres kératiniques.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VIII) suivante : 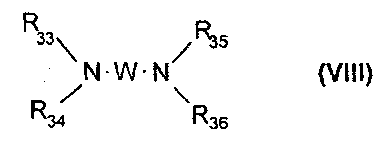 dans laquelle W est un reste propylène éventuellement substitué par un groupement hydroxyle ou un radical alkyle en C₁-C₆ ; R₃₃, R₃₄, R₃₅ et R₃₆, identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques.
Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et du polymère épaississant (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents tensioactifs, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant le polymère épaississant particulier.

Lorsque l'association du colorant direct cationique (i) et du polymère épaississant (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydoréductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère épaississant (ii) tel que défini précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère épaississant tel que défini précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 à 3 :

On a préparé les trois compositions de teinture directe réunies dans le tableau suivant:
(*toutes teneurs exprimées en grammes*)

| | **Exemple 1** | **Exemple 2** | **Exemple 3** |
|---|---|---|---|
| Colorant direct cationique de formule (I1) | 0,2 | | |
| Colorant direct cationique de formule (I14) | | 0,2 | |
| Colorant direct cationique de formule IV(27) | | | 0,1 |
| Diuréthanne (HMD) d'alcools C₁₆ -C₁₈ oxyéthylénés (66OE) et oxypropylénés (14OP), vendu sous la dénomination DAPRAL T212 par la société AKZO | 1,0 MA * | | |
| Terpolymère acide méthacrylique/acrylate d'éthyle/stéareth 10 allyl éther réticulé vendu en émulsion à 30% poids sous la dénomination SALCARE SC90 par la société ALLIED COLLOID | | 1,0 MA * | |
| Copolymère acide acrylique/acrylate d'alkyle C₁₀ -C₃₀ réticulé vendu sous la dénomination PEMULEN TR1 par la société GOODRICH | | | 1,0 MA * |
| Ethanol | 10 | 10 | 10 |
| 2-amino-2-méthyl-1-propanol qs | pH 9 | pH 9 | pH 9 |
| Eau déminéralisée qsp | 100 | 100 | 100 |

| | | | |
|---|---|---|---|
| MA* désigne Matière Active | | | |

Les compositions ci-dessus ont été appliquées chacune pendant 30 minutes sur des mèches de cheveux gris naturels à 90 % de blancs. Les mèches de cheveux ont ensuite été rincées, lavées avec un shampooing standard puis séchées.

Les mèches ont été teintes dans les nuances suivantes :

| **Exemples** | **Nuances obtenues** |
|---|---|
| 1 | Rouge puissant |
| 2 | Orangé puissant |
| 3 | Pourpre puissant |

## Revendications

1. Composition pour la teinture des fibres kératiniques et en particulier des fibres kératiniques humaines telles que les cheveux, renfermant dans un milieu approprié pour la teinture, (i) au moins composé choisi parmi ceux de formules (I), (II), (III), (III'), (IV) suivantes :
**a) les composés de formule (I) suivante :** 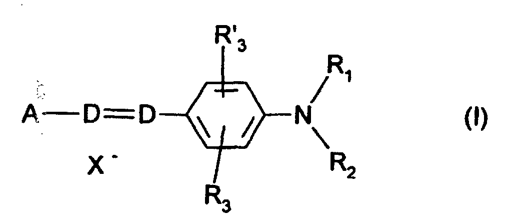 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X ⁻ représente un anion de préférence choisi parmi le chlorure, le méthyl sulfate et l'acétate,
A représente un groupement choisi par les structures A1 à A18 suivantes : 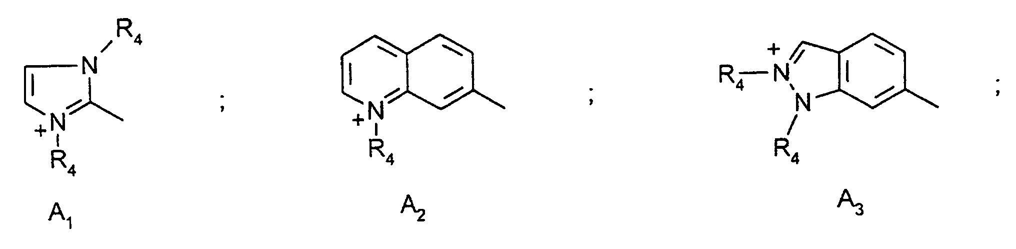 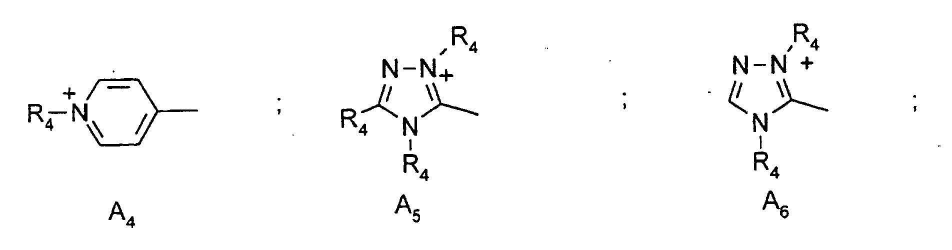 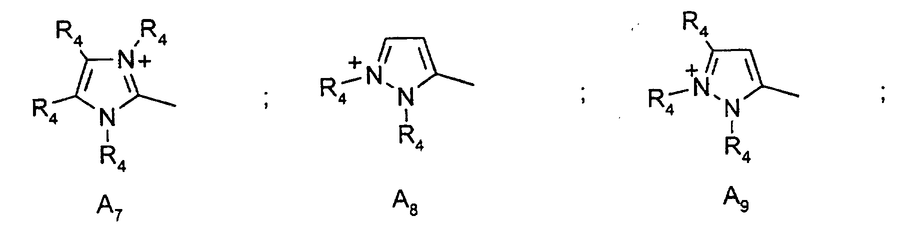 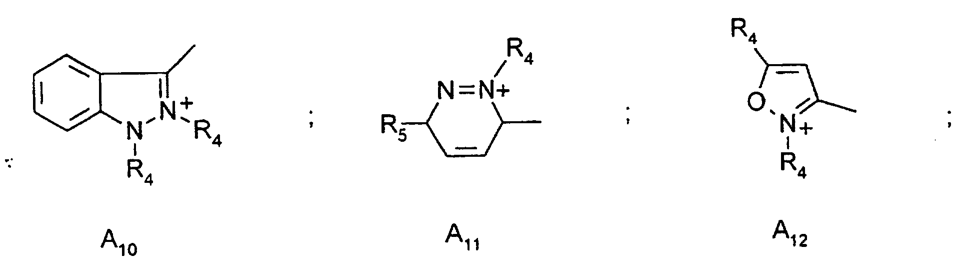 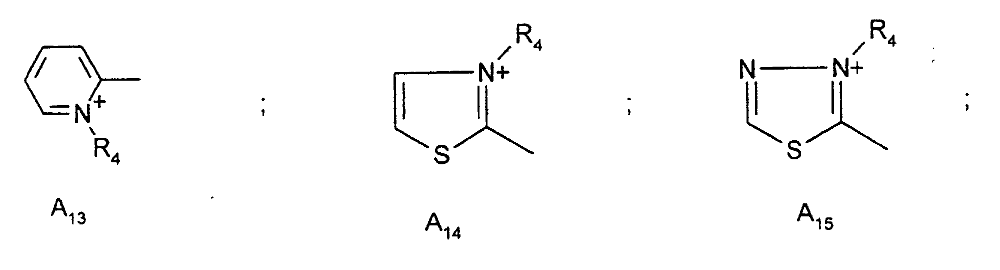 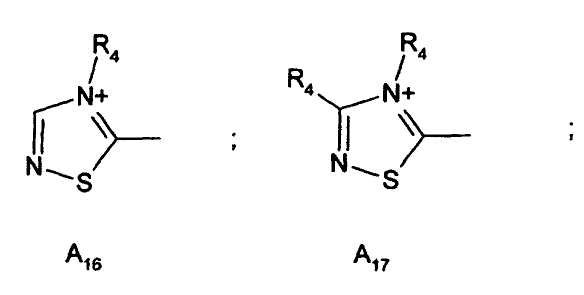 et 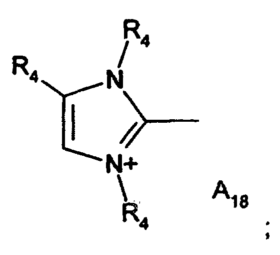 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**b) les composés de formule (II) suivante :** 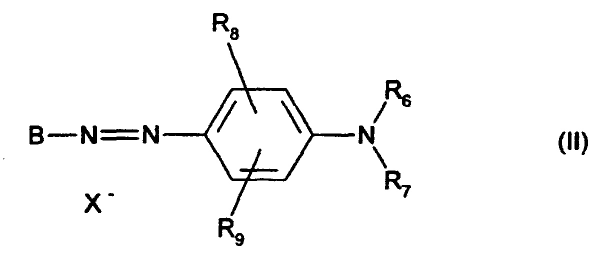 dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B6 suivantes : 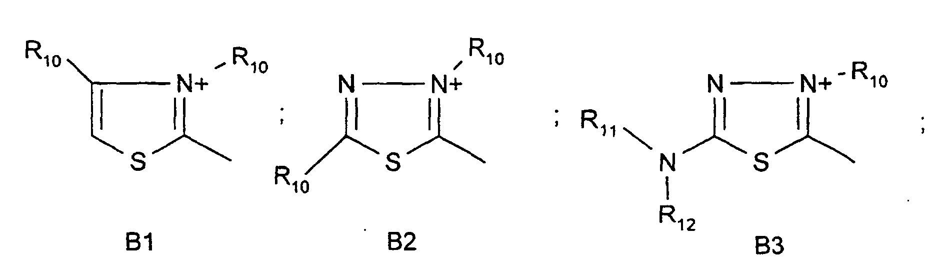 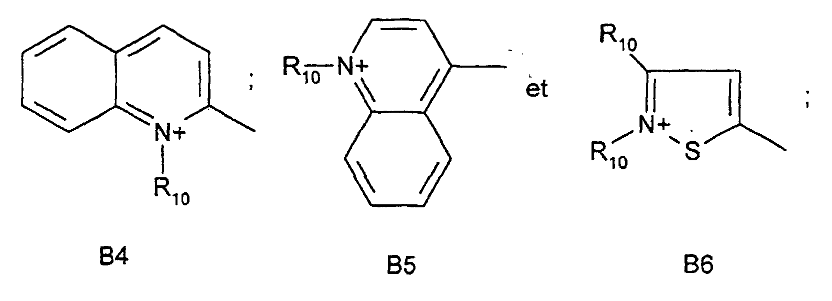 dans lesquelles R₁₀ représente un radical alkyle en C₁-C₄, R₁₁ et R₁₂, identiques ou différents, représentent un atome d'hydrogène ou un radical alkyle en C₁-C₄ ;
**c) les composés de formules (III) et (III') suivantes :** 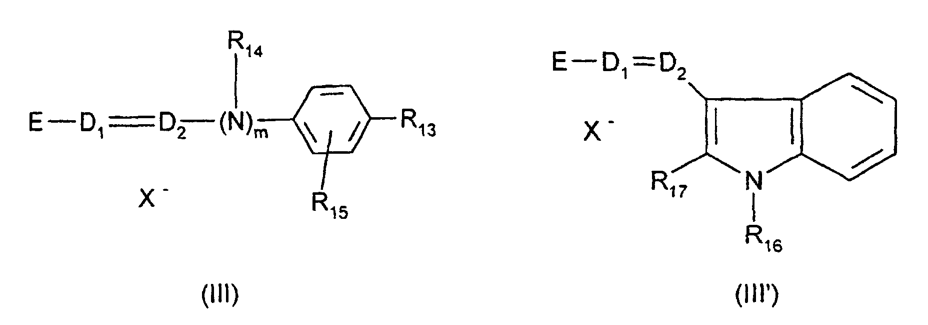 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 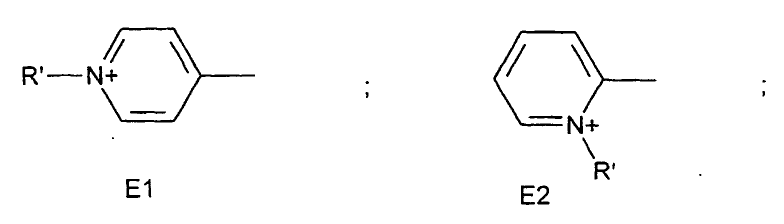 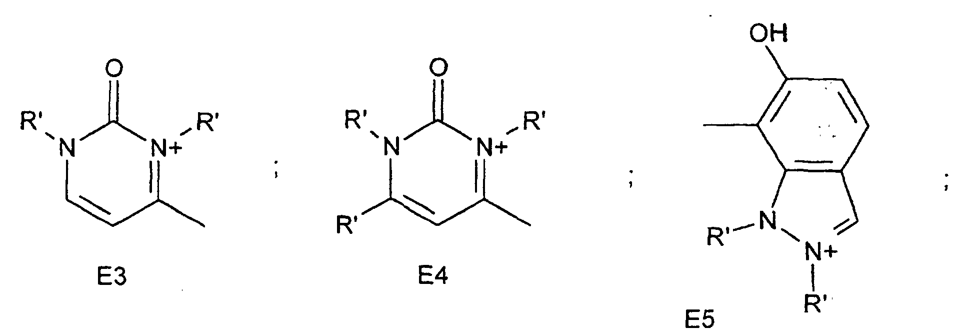 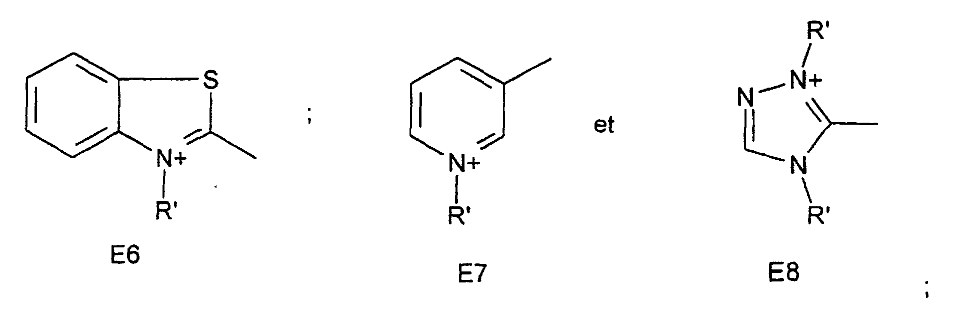 dans lesquelles R' représente un radical alkyle en C₁-C₄ ;
lorsque m = 0 et que D₁ représente un atome d'azote, alors E peut également désigner un groupement de structure E9 suivante : 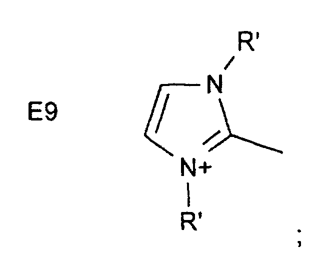 dans laquelle R' représente un radical alkyle en C₁-C₄.
**d) les composés de formule (IV) suivante :**
G-N = N-J (IV)
dans laquelle :
**le symbole G** représente un groupement choisi parmi les structures G₁ à G₃ suivantes : 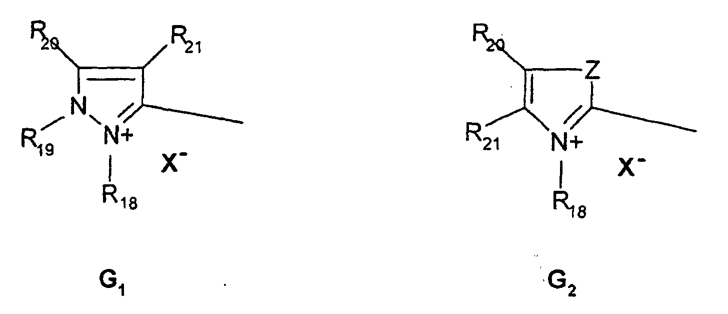 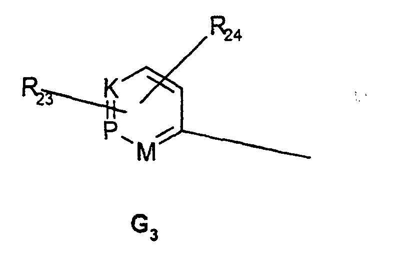 structures G₁ à G₃ dans lesquelles,
R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₁₉ désigne un radical alkyle en C₁-C₄ ou un radical phényle;
R₂₀ et R₂₁, identiques ou différents, représentent un radical alkyle en C₁-C₄ , un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄ , alcoxy en C₁-C₄, ou NO₂;
R₂₀ peut désigner en outre un atome d'hydrogène;
Z désigne un atome d'oxygène, de soufre ou un groupement -NR₁₉;
M représente un groupement -CH, -CR (R désignant alkyle en C₁-C₄), ou -NR₂₂(X⁻)ᵣ;
K représente un groupement -CH, -CR (R désignant alkyle en C₁-C₄), ou -NR₂₂(X⁻)ᵣ;
P représente un groupement -CH, -CR (R désignant alkyle en C₁-C₄), ou -NR₂₂(X⁻)ᵣ; r désigne zéro ou 1;
R₂₂ représente un atome O⁻, un radical alcoxy en C₁-C₄, ou un radical alkyle en C₁-C₄;
R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
X⁻ représente un anion de préférence choisi parmi le chlorure, l'iodure, le méthyl sulfate, l'éthyl sulfate, l'acétate et le perchlorate;
sous réserve que,
si R₂₂ désigne O⁻, alors r désigne zéro;
si K ou P ou M désignent -N-alkyle C₁-C₄ X⁻, alors R₂₃ ou R₂₄ est différent d'un atome d'hydrogène;
si K désigne -NR₂₂(X⁻)ᵣ, alors M= P= -CH, -CR;
si M désigne -NR₂₂(X⁻)ᵣ, alors K= P= -CH, -CR;
si P désigne -NR₂₂(X⁻)ᵣ, alors K= M et désignent -CH ou -CR;
si Z désigne un atome de soufre avec R₂₁ désignant alkyle en C₁-C₄ , alors R₂₀ est différent d'un atome d'hydrogène;
si Z désigne -NR₁₉ avec R₁₉ désignant alkyle en C₁-C₄ , alors au moins l'un des radicaux R₁₈, R₂₀ ou R₂₁ de G₂ est différent d'un radical alkyle en C₁-C₄;
**le symbole J** représente :
- **(a)** un groupement de structure J₁ suivante : 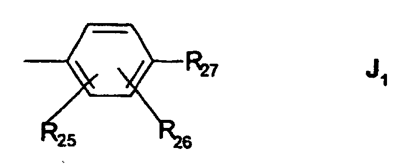 structure J₁ dans laquelle,
R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre;
R₂₇ représente un atome d'hydrogène, un radical -OH, un radical -NHR₂₈, un radical -NR₂₉R₃₀;
R₂₈ représente un atome d'hydrogène, un radical alkyle en C₁-C₄, un radical monohydroxyalkyle en C₁-C₄, polyhydroxyalkyle en C₂-C₄, un radical phényle;
R₂₉ et R₃₀, identiques ou différents, représentent un radical alkyle en C₁-C₄ , un radical monohydroxyalkyle en C₁-C₄, polyhydroxyalkyle en C₂-C₄;
- **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
et notamment un groupement de structure J₂ suivante : 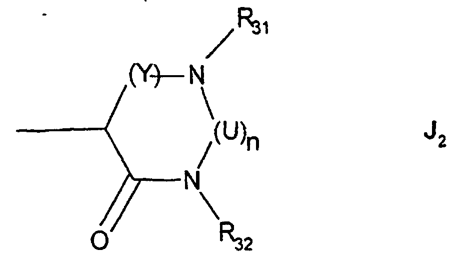 structure J₂ dans laquelle,
R₃₁ et R₃₂, identiques ou différents, représentent un atome d'hydrogène, un radical alkyle en C₁-C₄ , un radical phényle;
Y désigne le radical -CO- ou le radical 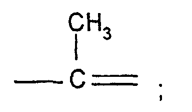 n = 0 ou 1, avec, lorsque n désigne 1, U désigne le radical -CO- .
ladite composition étant **caractérisée par le fait qu'**elle contient en outre **(ii)**au moins un polymère épaississant choisi dans le groupe comprenant :
**(ii)**_{**1**} **-** les polymères amphiphiles non-ioniques comportant au moins un motif hydrophile et au moins un motif à chaîne grasse ;
**(ii)**_{**2**} **-** les polymères amphiphiles anioniques comportant au moins un motif hydrophile et au moins un motif à chaîne grasse ;
**(ii)**_{**3**} **-** les polymères amphiphiles cationiques comportant au moins un motif hydrophile et au moins un motif à chaîne grasse ;

2. Composition selon la revendication 1, **caractérisée par le fait que** les colorants directs cationiques de formule (I) sont choisis parmi les composés répondant aux structures (I1) à (I54) suivantes :

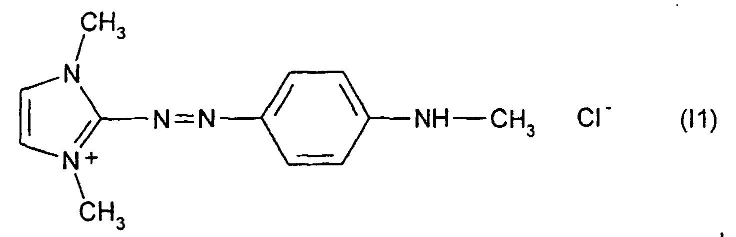

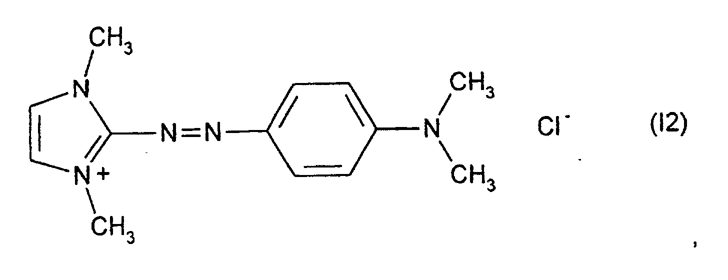

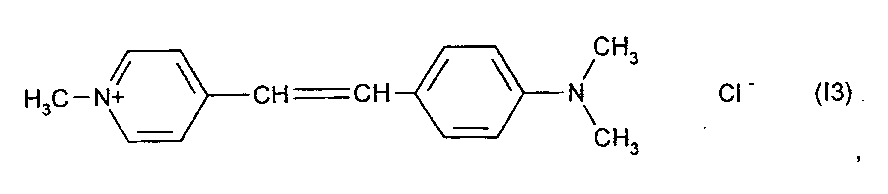

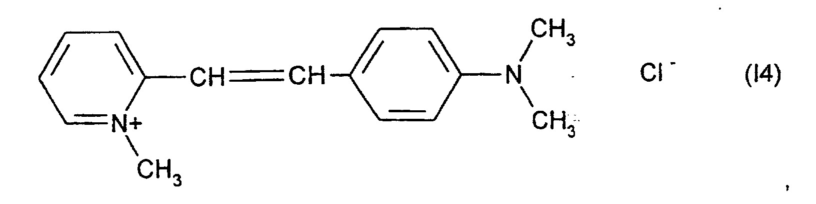

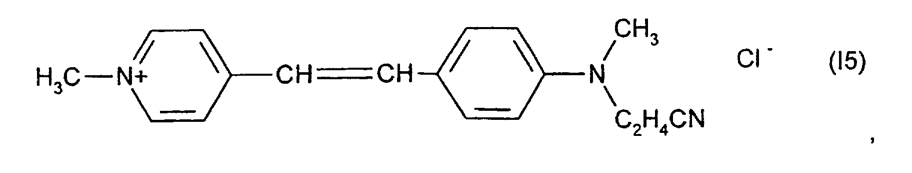

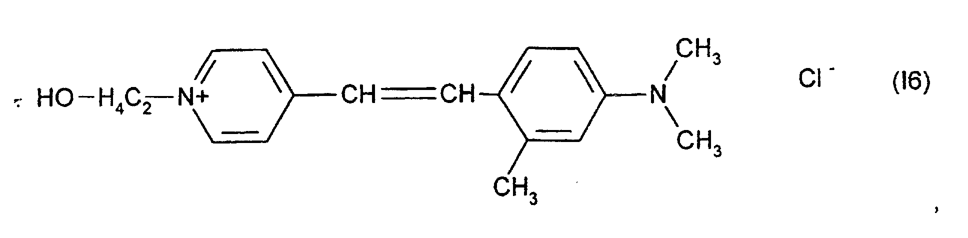

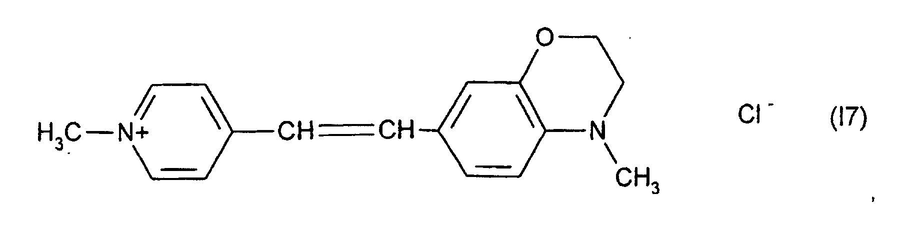

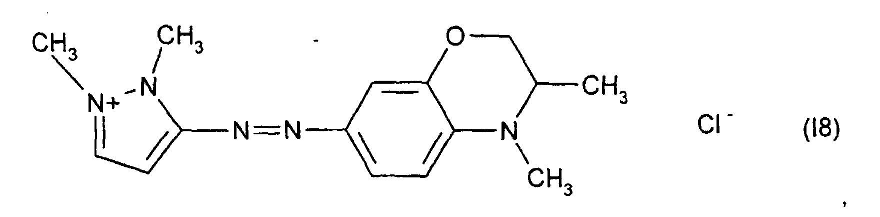

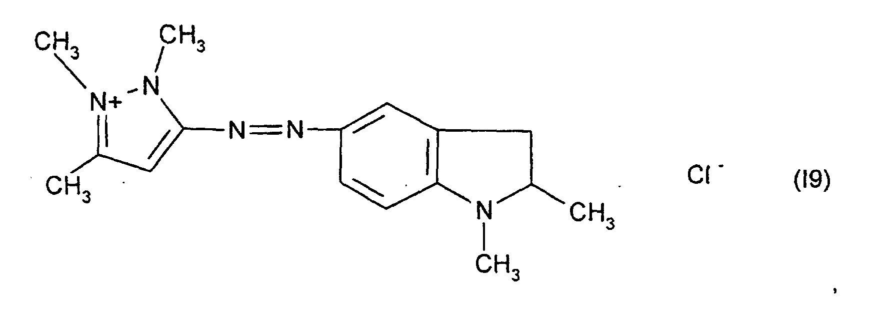

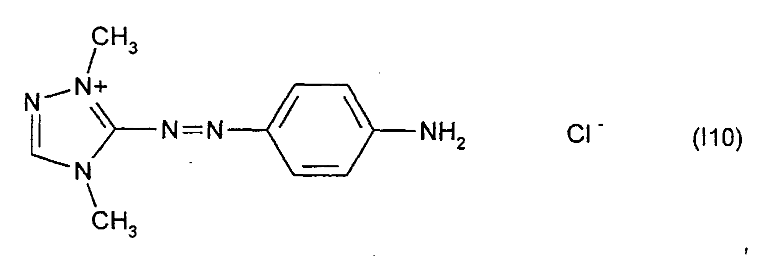

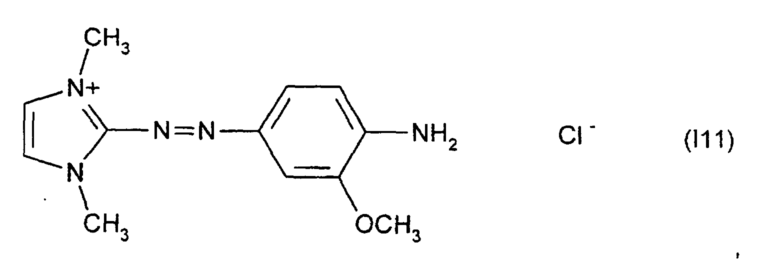

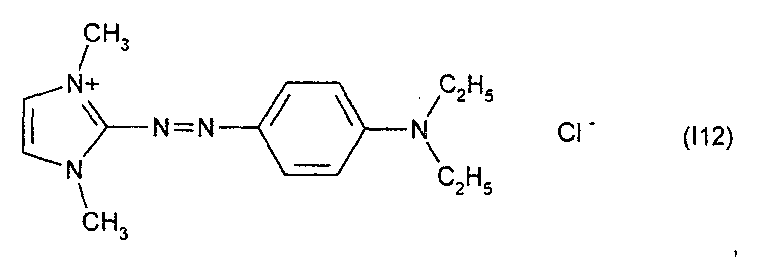

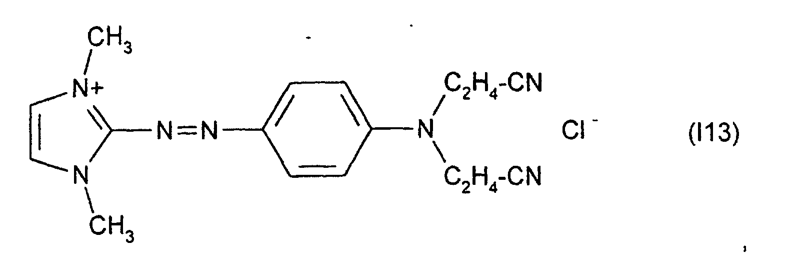

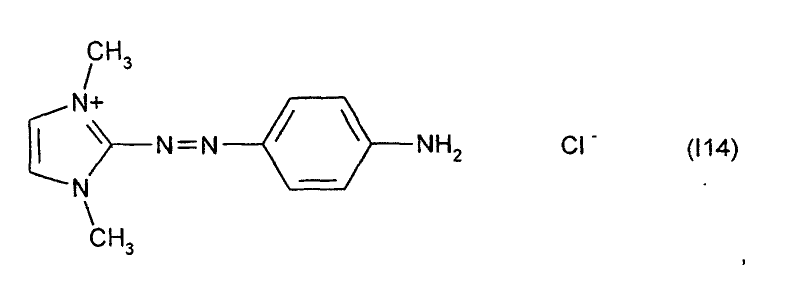

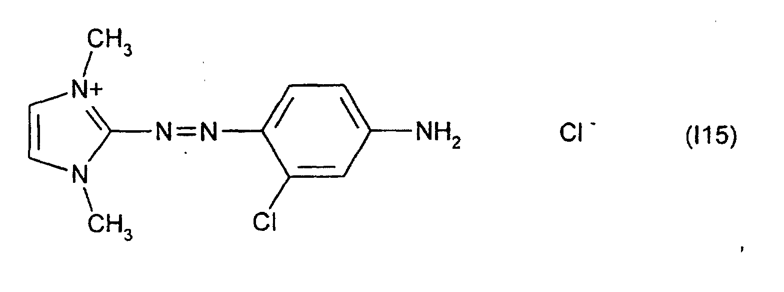

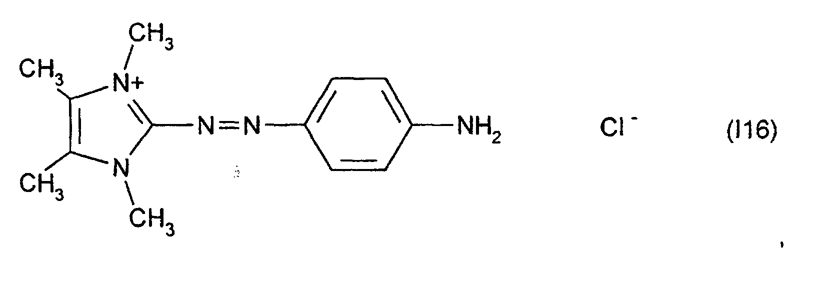

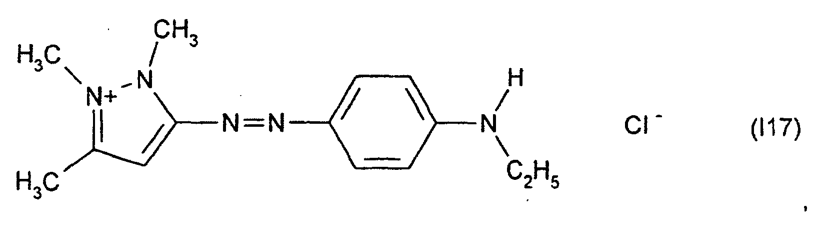

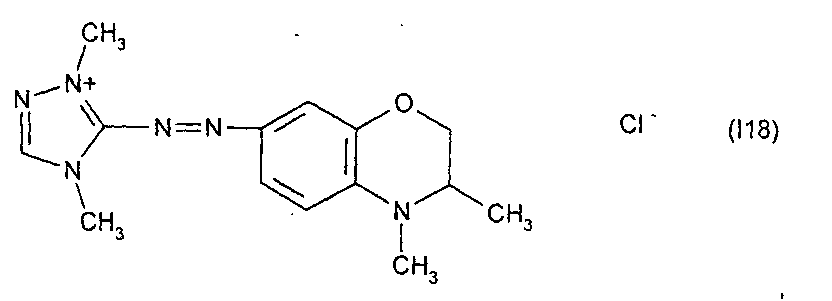

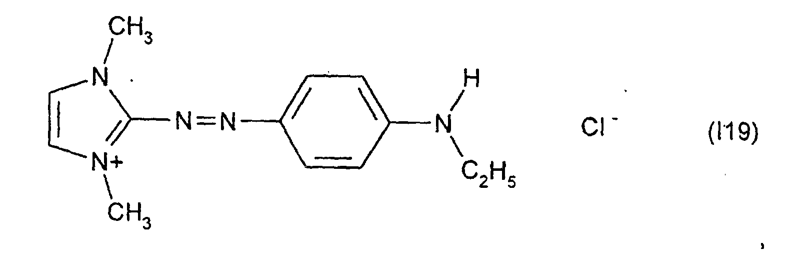

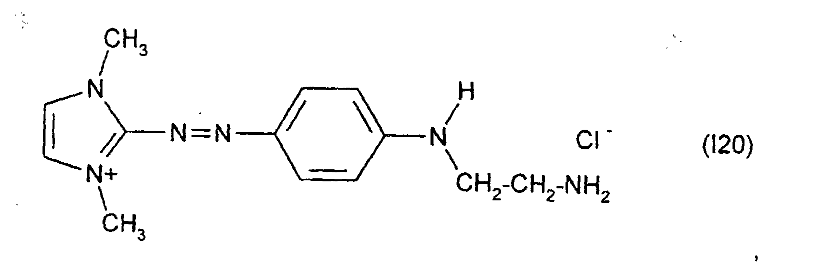

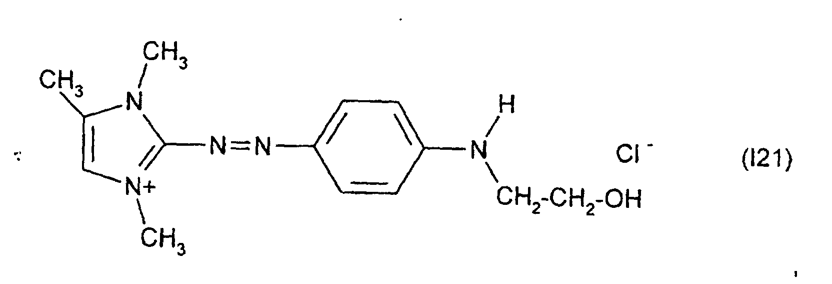

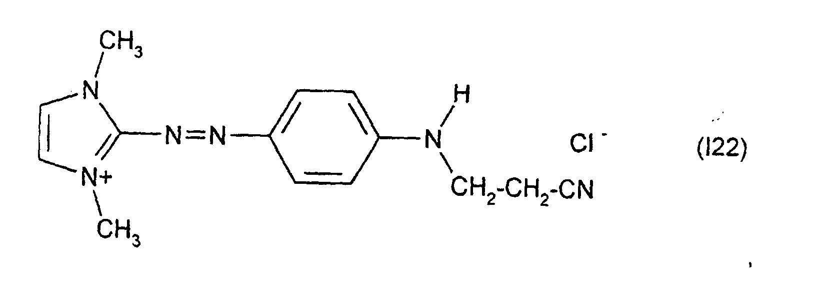

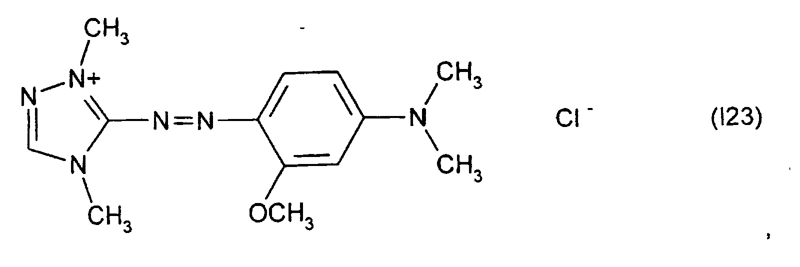

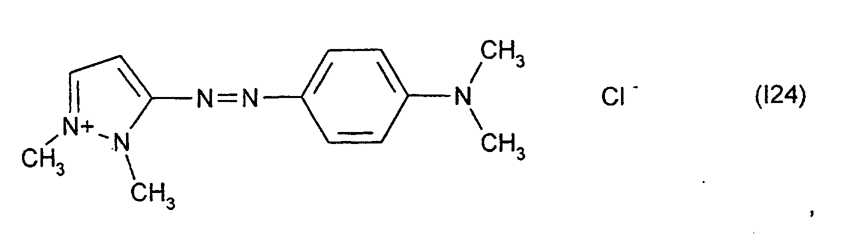

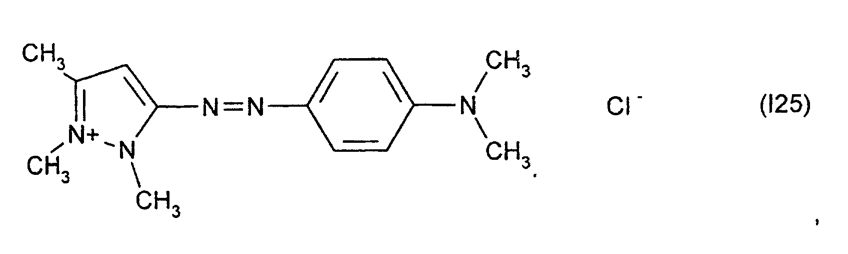

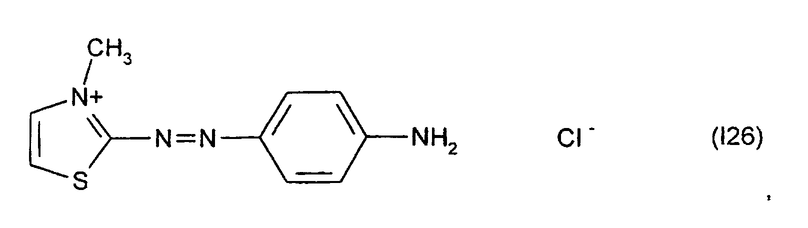

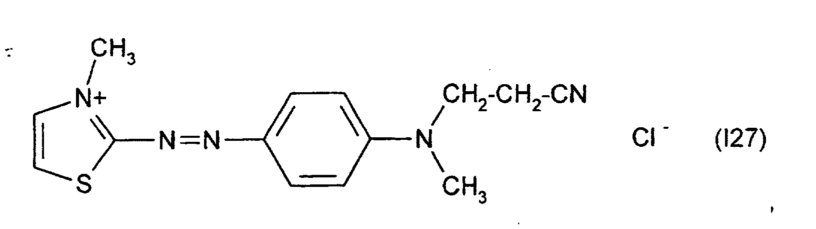

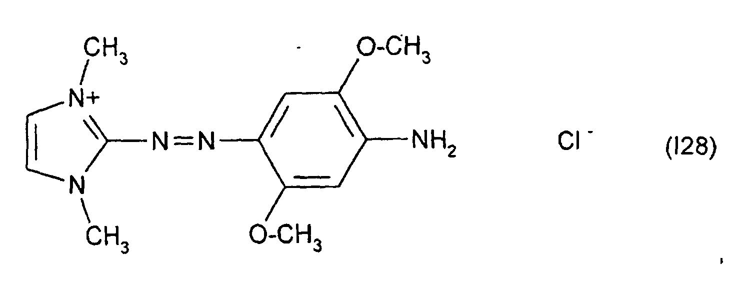

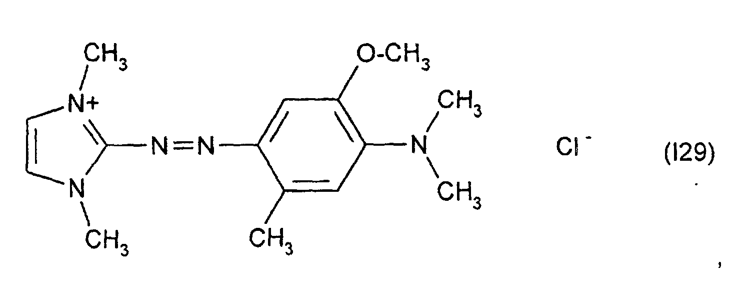

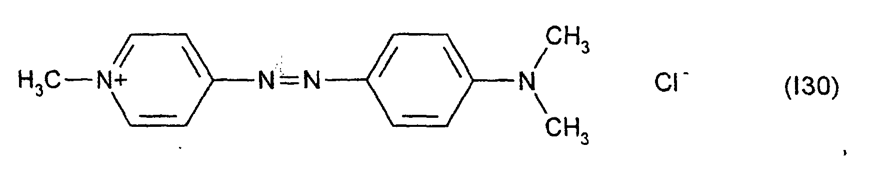

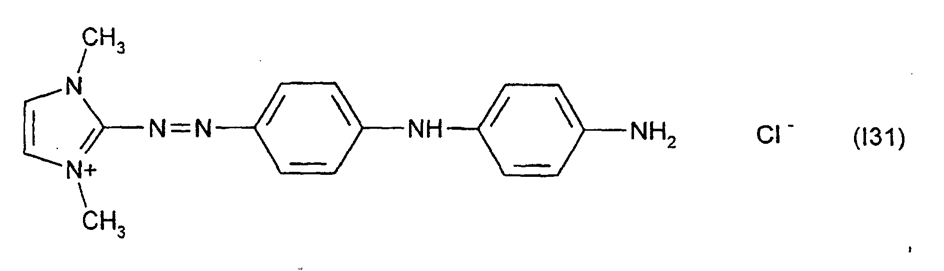

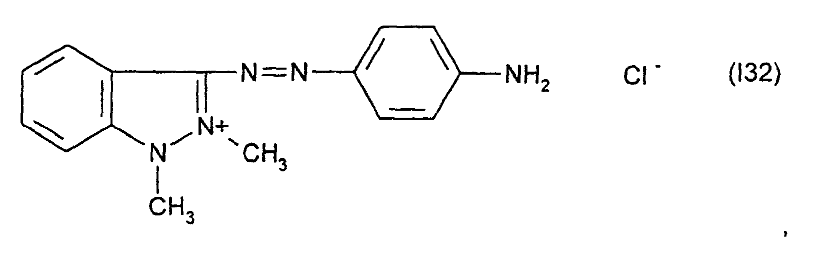

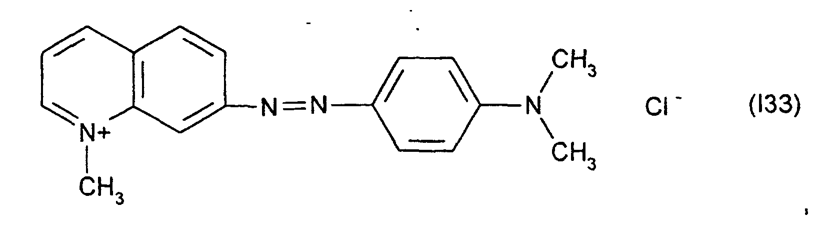

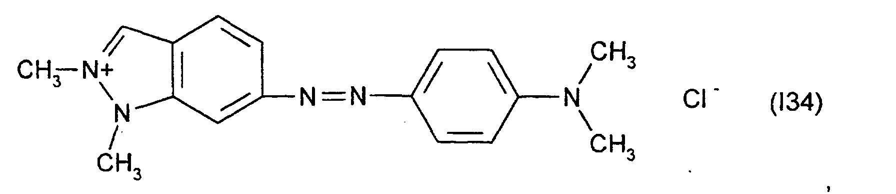

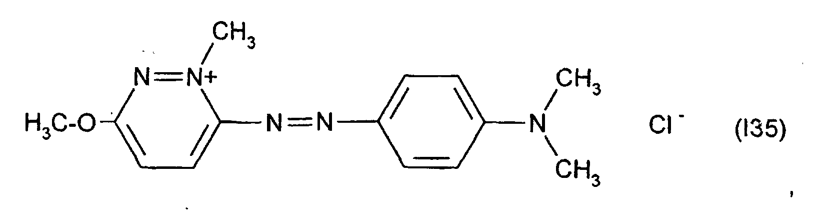

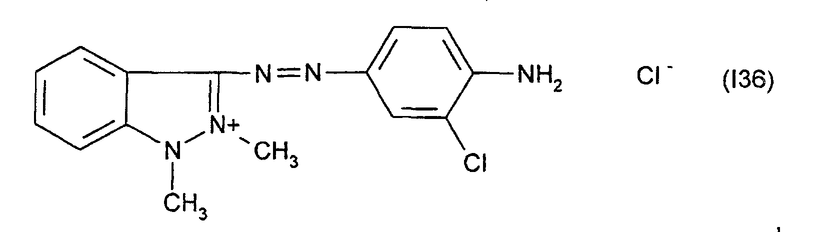

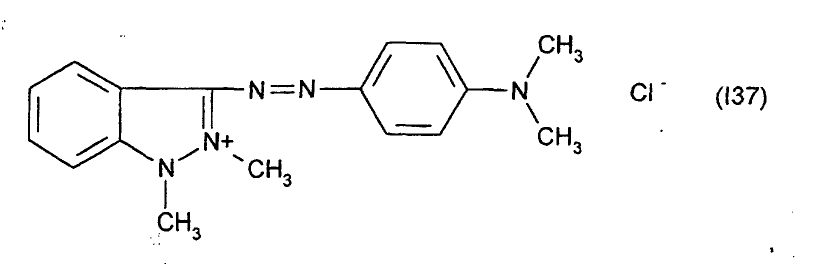

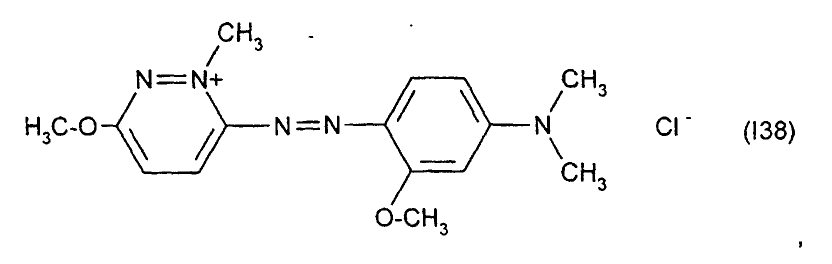

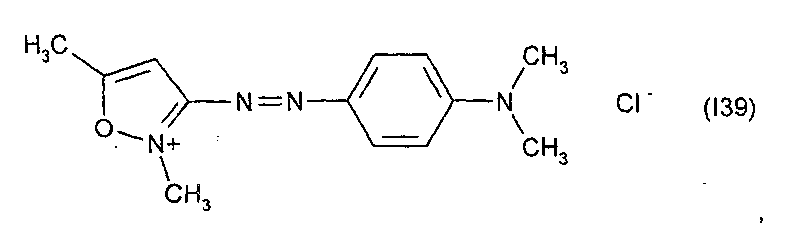

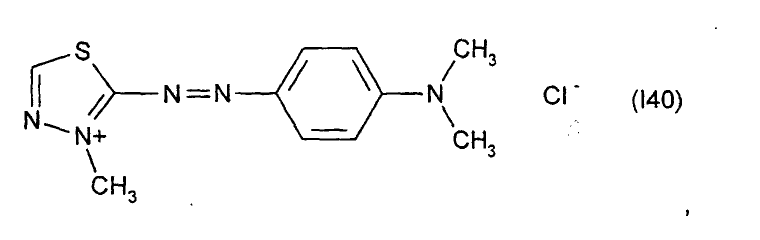

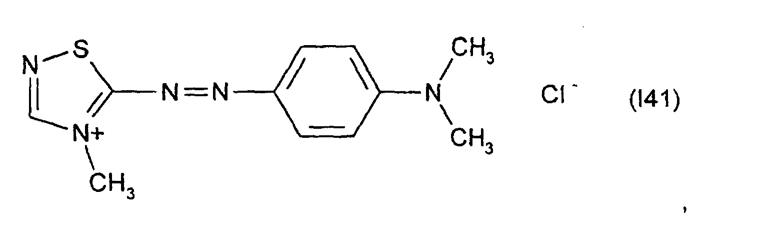

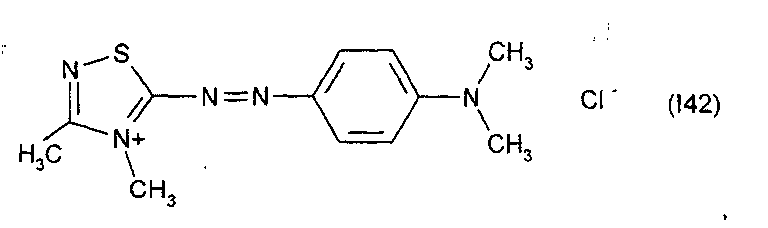

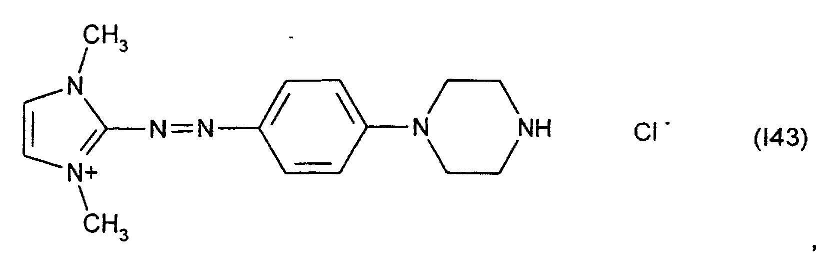

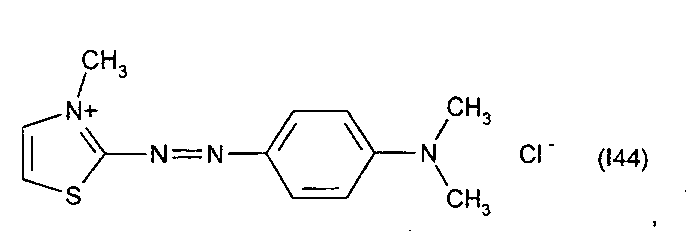

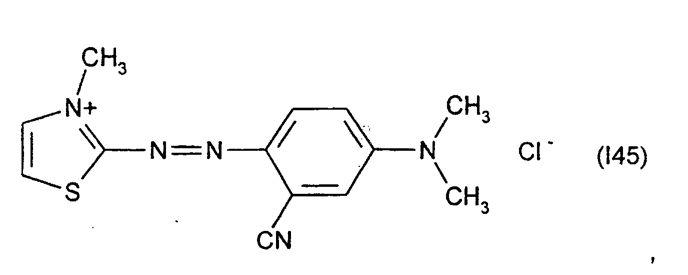

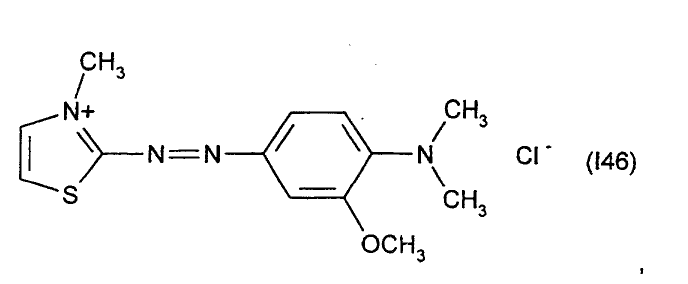

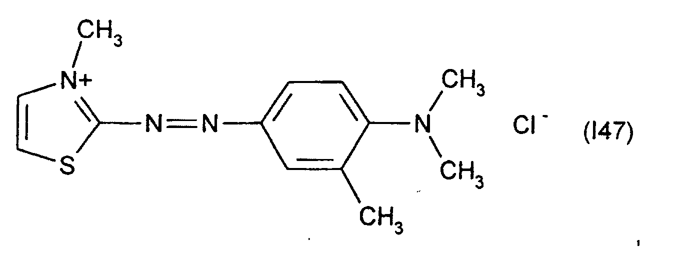

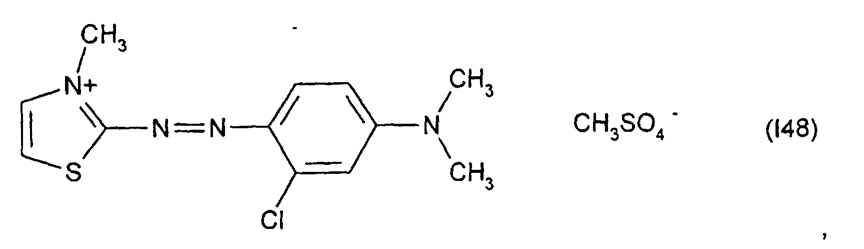

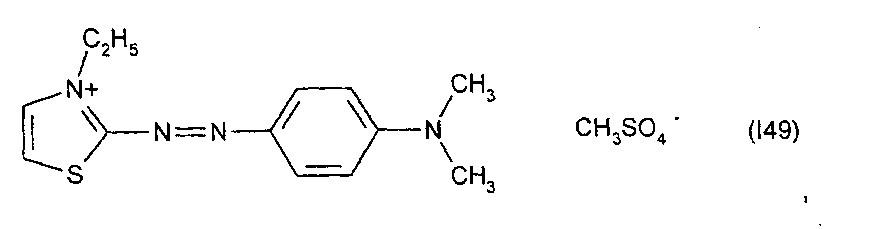

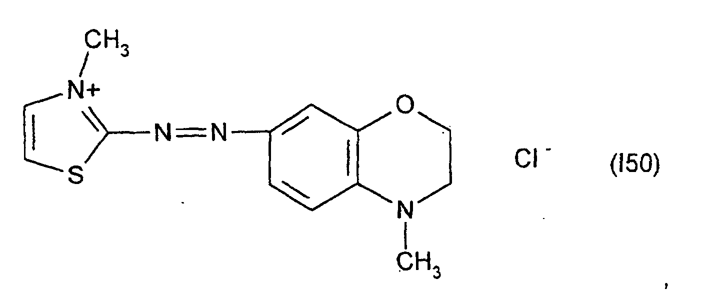

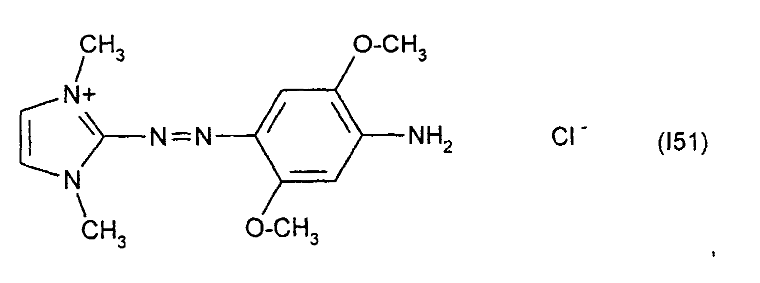

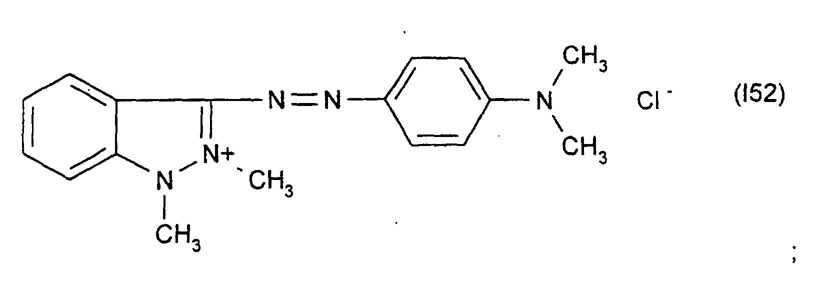

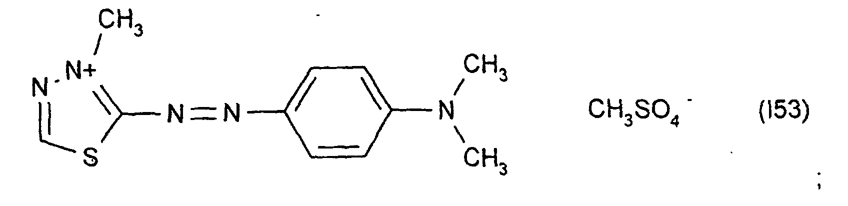

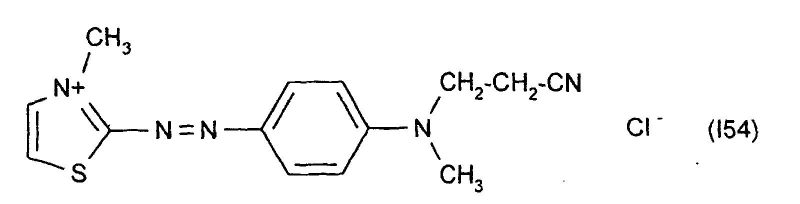

3. Composition selon la revendication 2, **caractérisée par le fait que** les colorants directs cationiques répondent aux structures (I1), (I2), (I14), et (I31).

4. Composition selon la revendication 1, **caractérisée par le fait que** les colorants directs cationiques de formule (II) sont choisis parmi les composés répondant aux structures (II1) à (II9) suivantes : 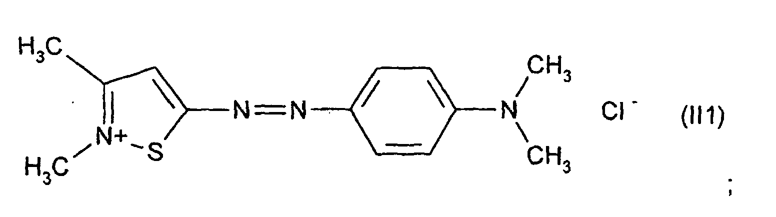 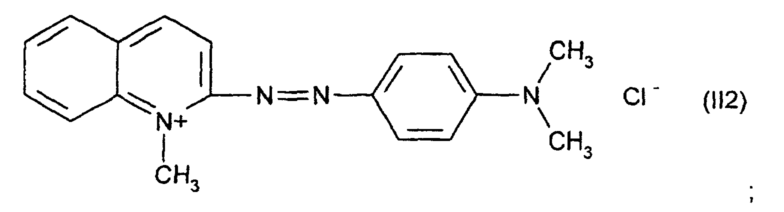 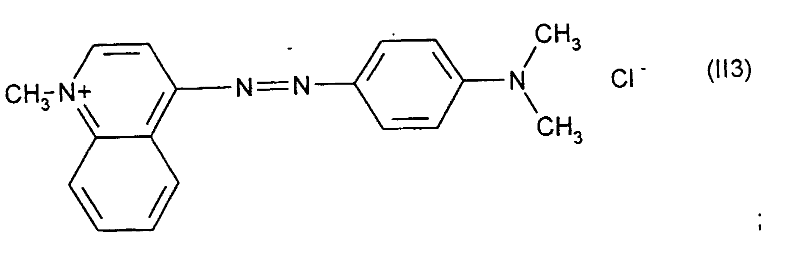 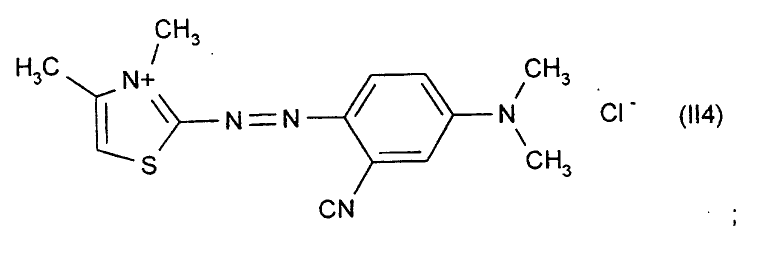 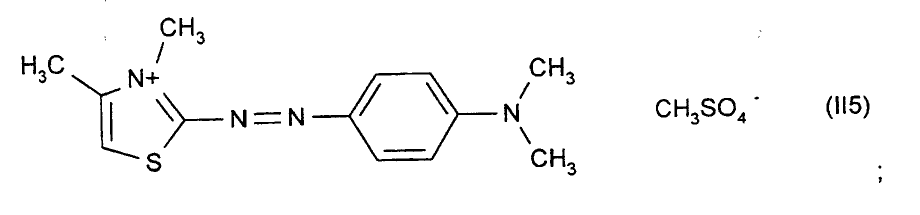 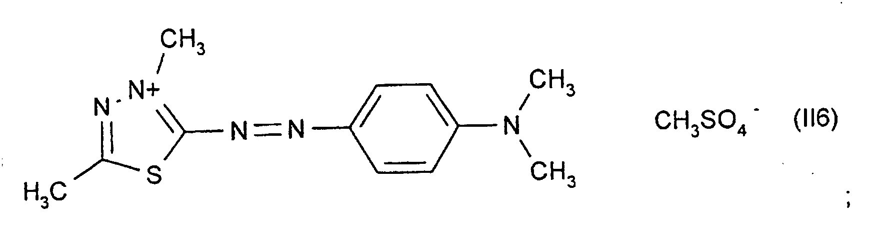 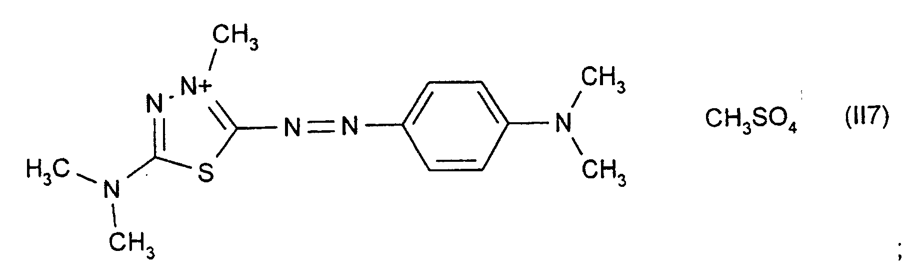 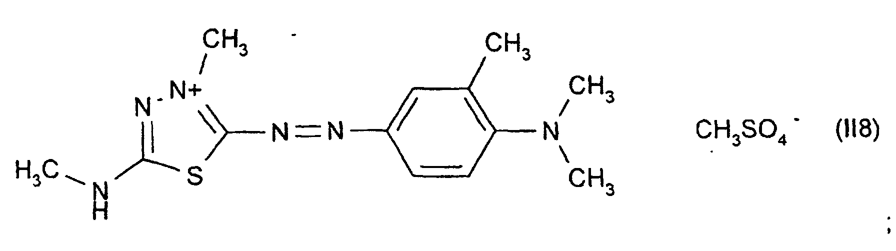 et

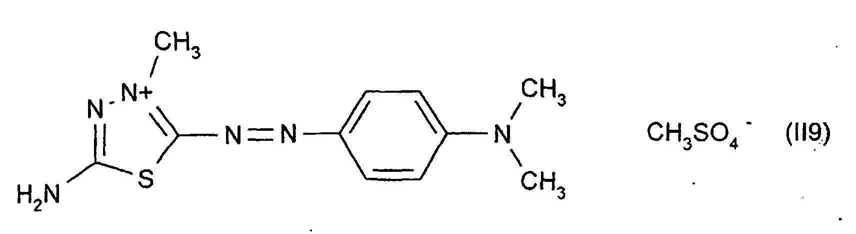

5. Composition selon la revendication 1, **caractérisée par le fait que** les colorants directs cationiques de formule (III) sont choisis parmi les composés répondant aux structures (III1) à (III18) suivantes: 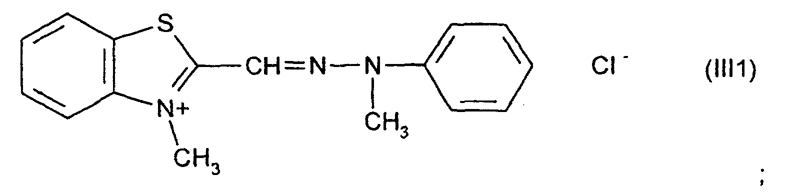 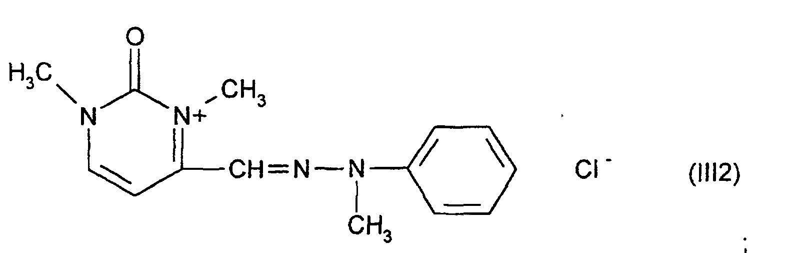 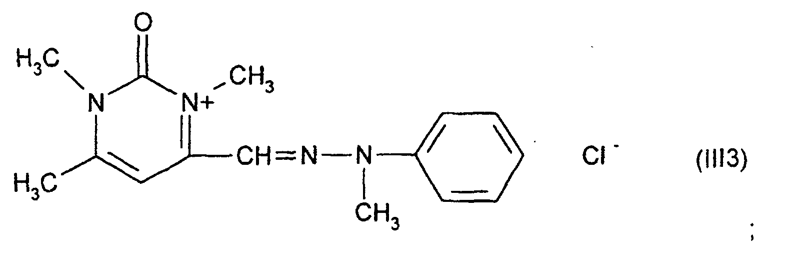 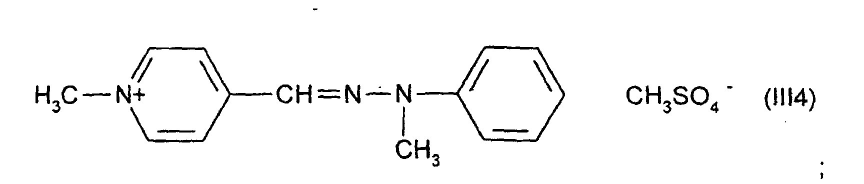 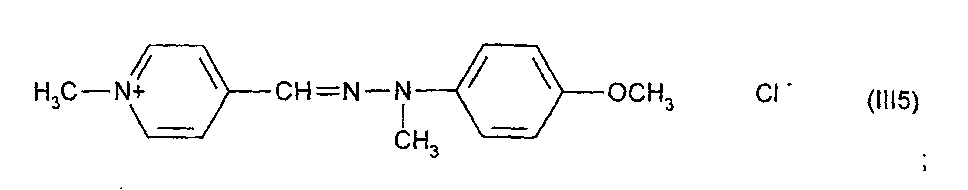 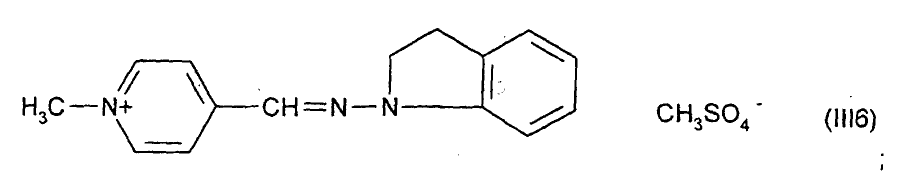 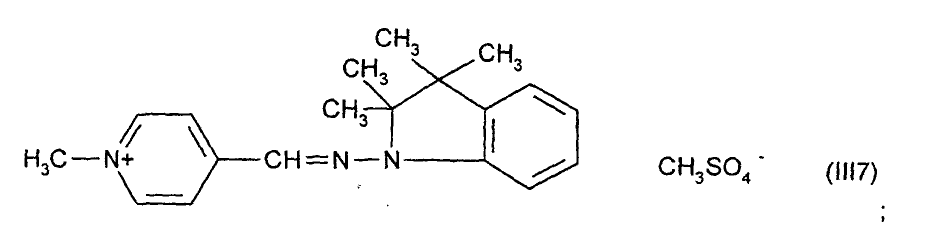 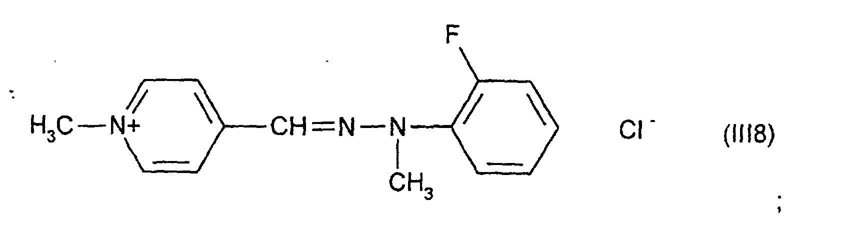 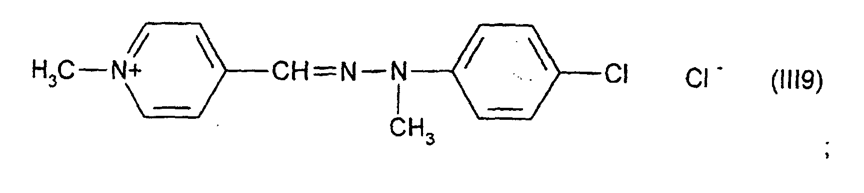 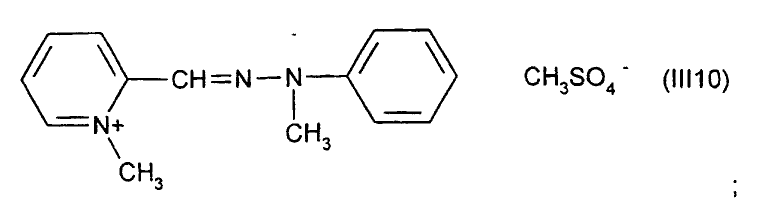 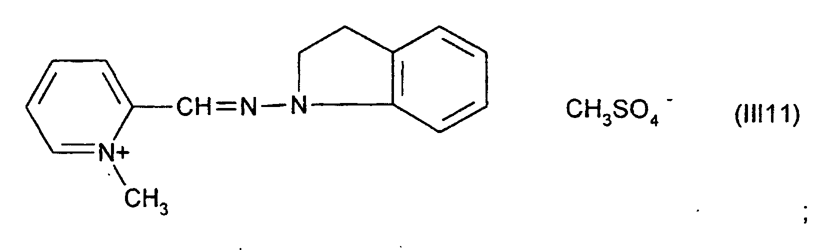 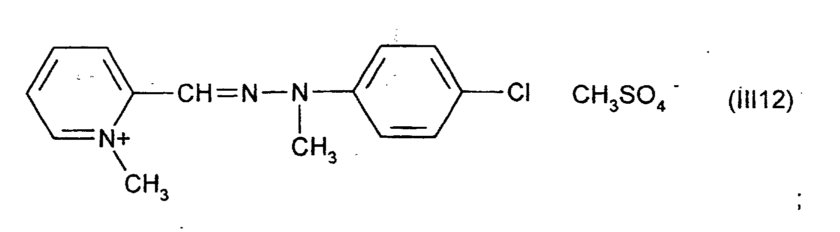 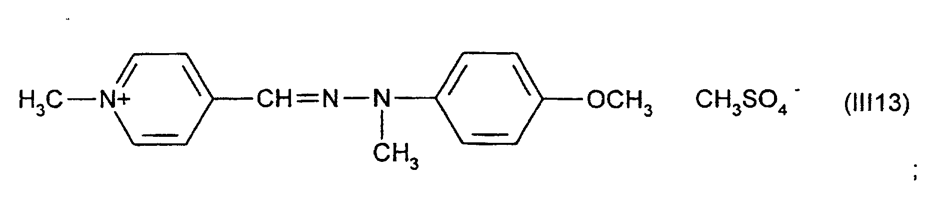 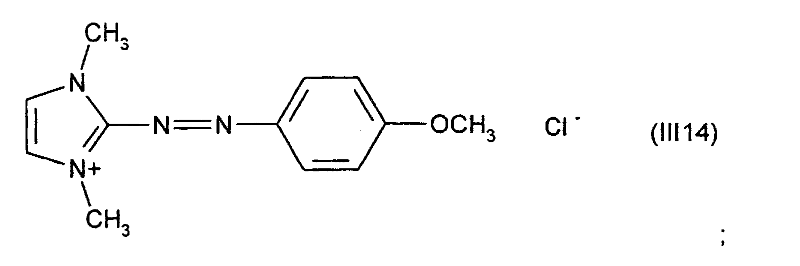 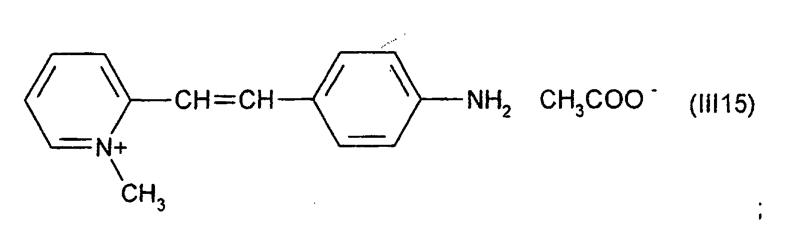 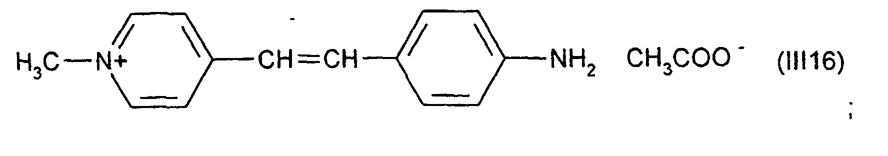 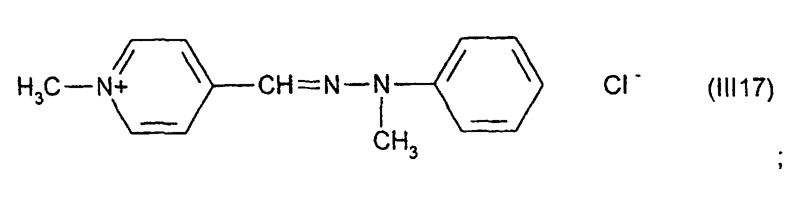 et

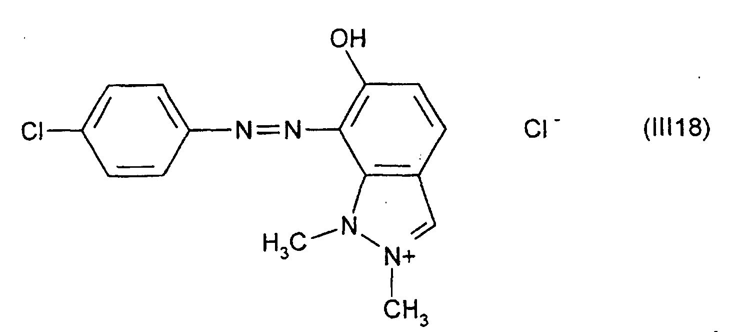

6. Composition selon la revendication 5, **caractérisée par le fait que** les colorants directs cationiques de formule (III) sont choisis parmi les composés répondant aux structures (III4), (III5) et (III13).

7. Composition selon la revendication 1, **caractérisée par le fait que** les colorants directs cationiques de formule (III') sont choisis parmi les composés répondant aux structures (III'1 ) à (III'3) suivantes : 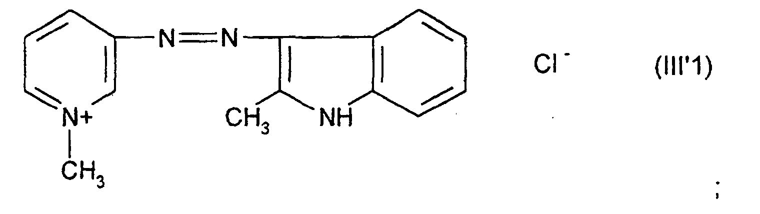 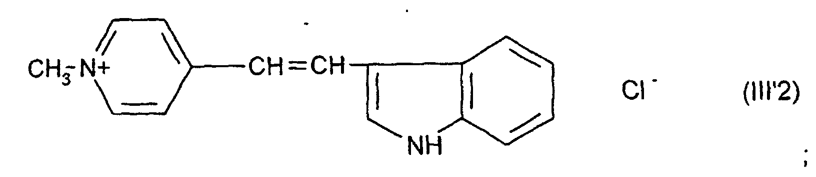 et

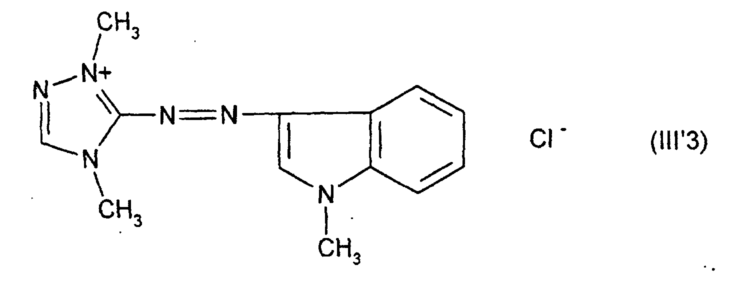

8. Composition selon la revendication 1, **caractérisée par le fait que** les colorants directs cationiques de formule (IV) sont choisis parmi les composés répondant aux structures (IV₁) à (IV₇₇) suivantes :

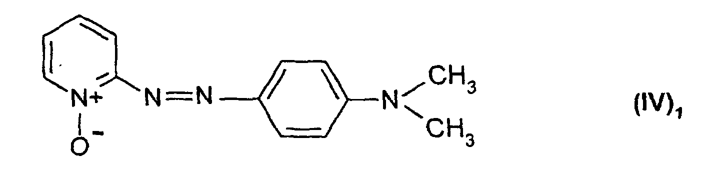

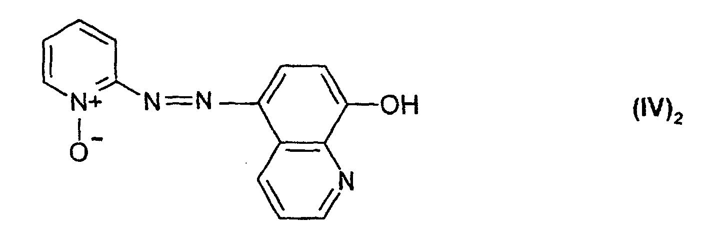

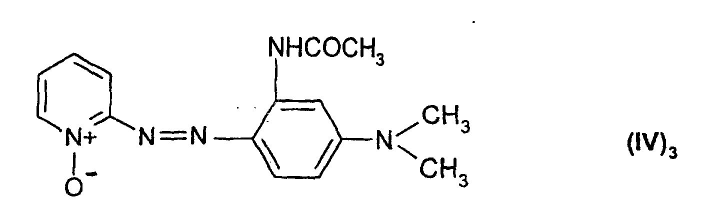

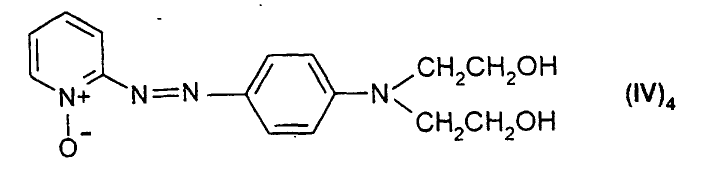

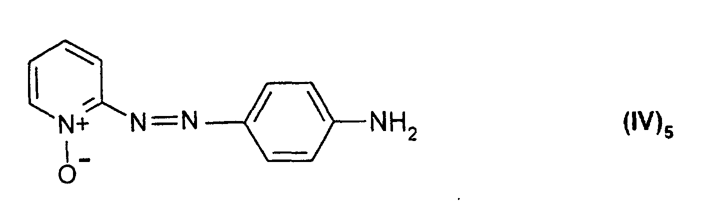

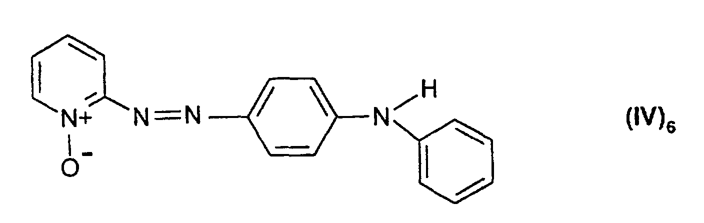

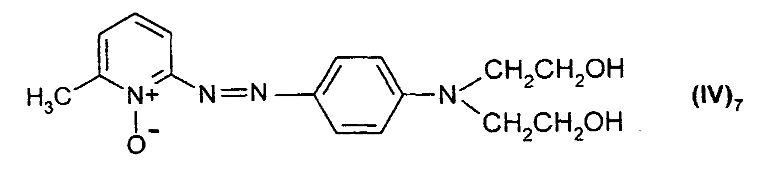

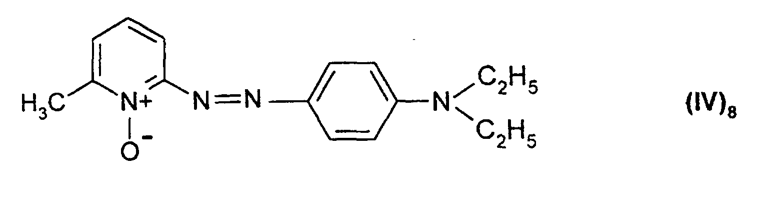

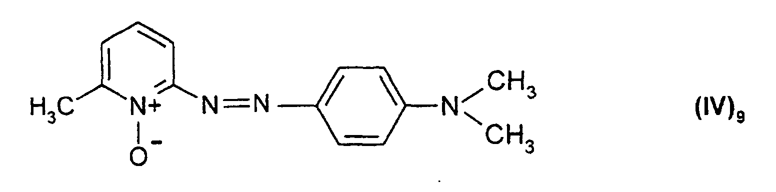

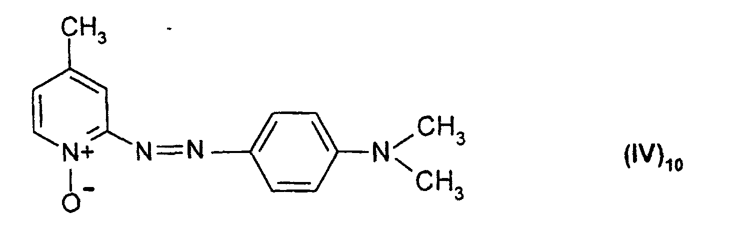

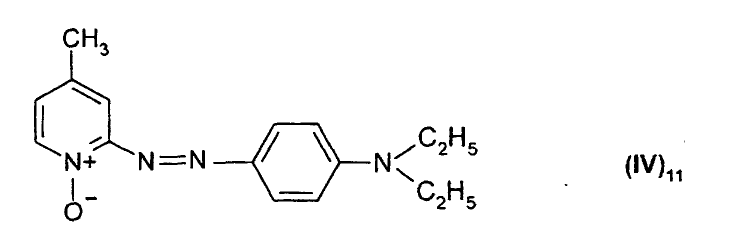

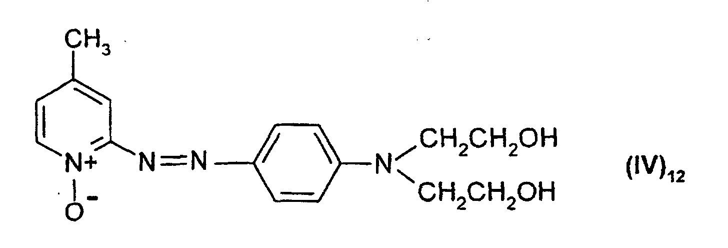

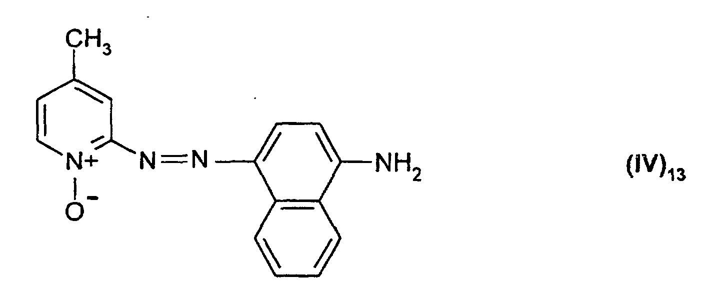

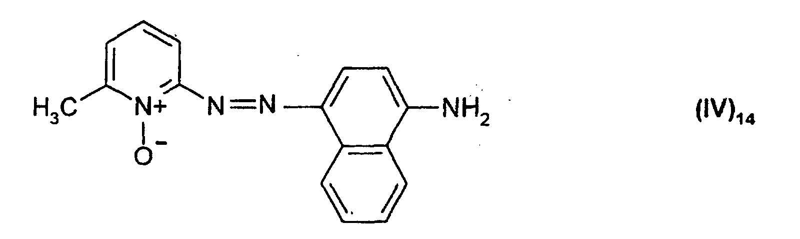

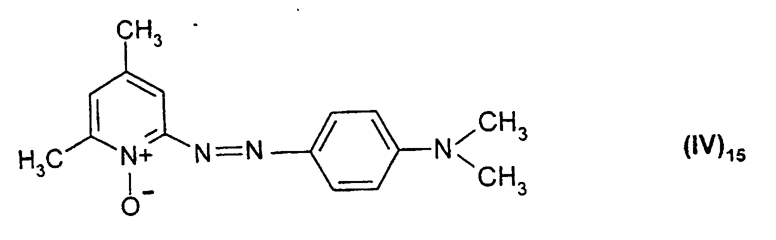

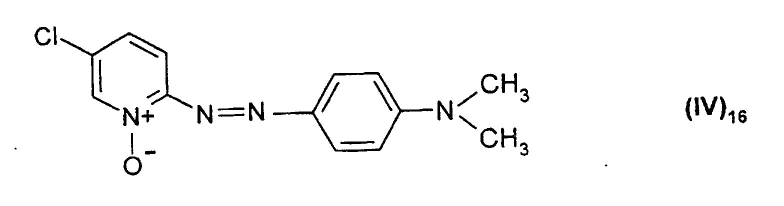

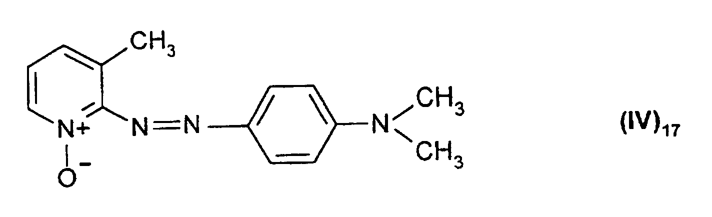

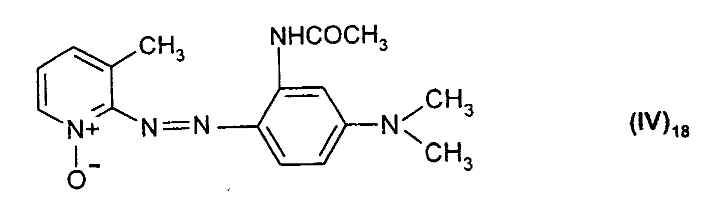

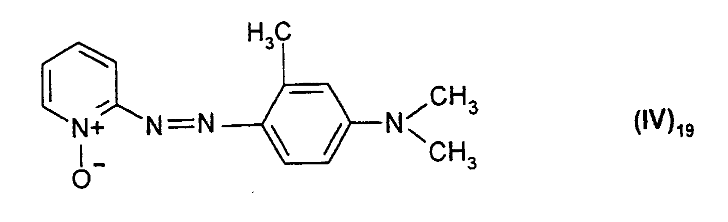

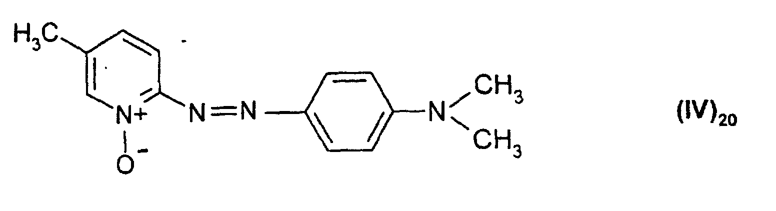

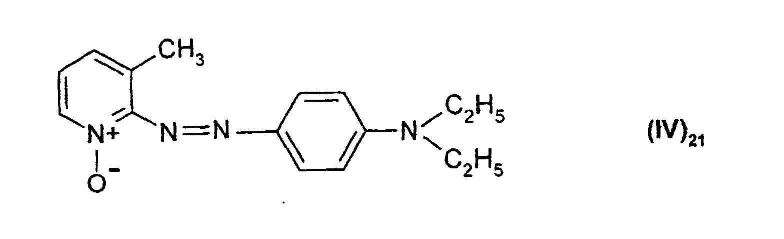

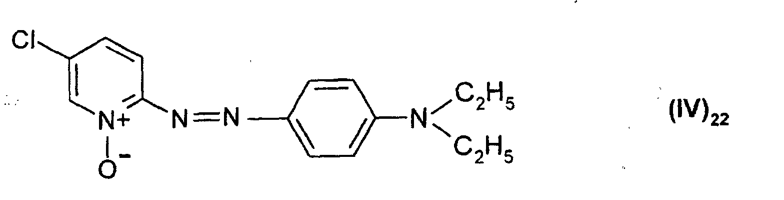

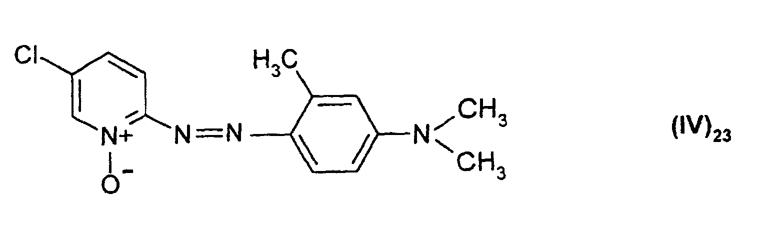

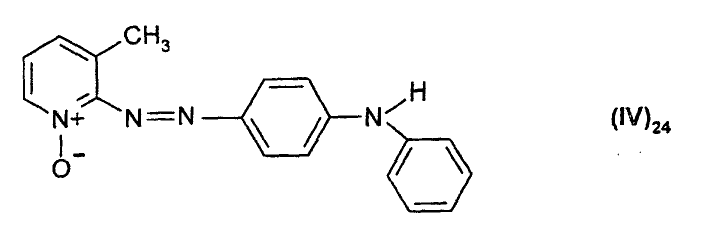

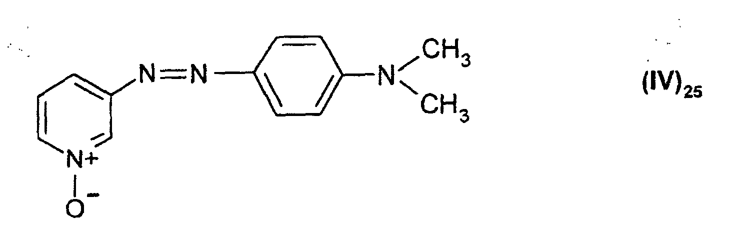

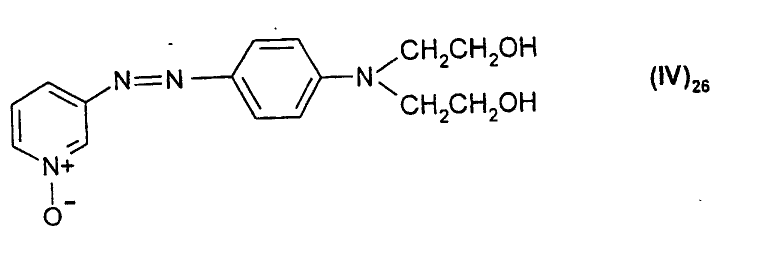

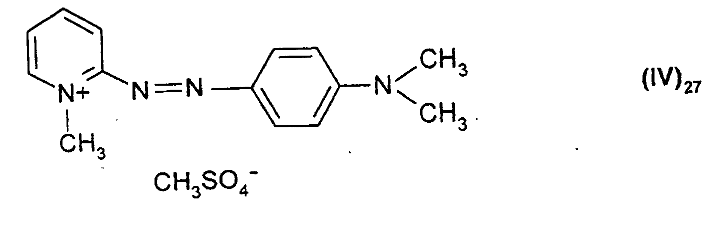

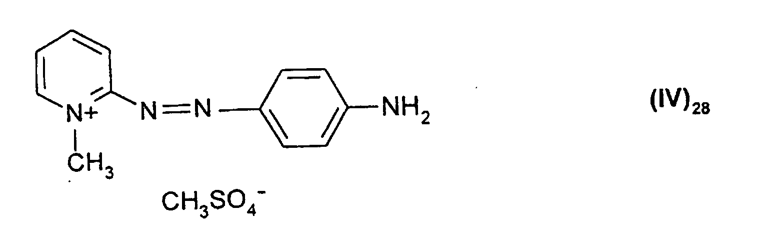

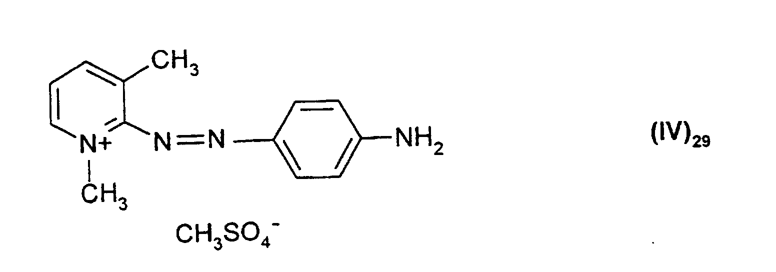

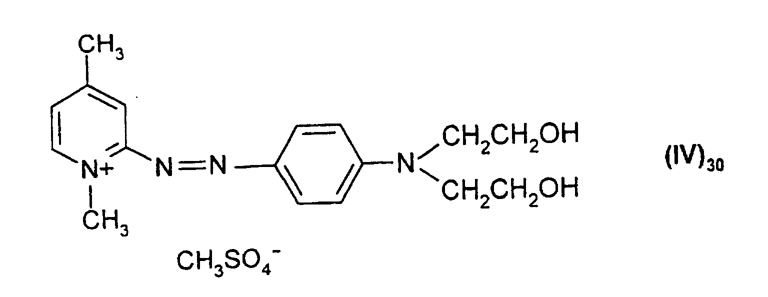

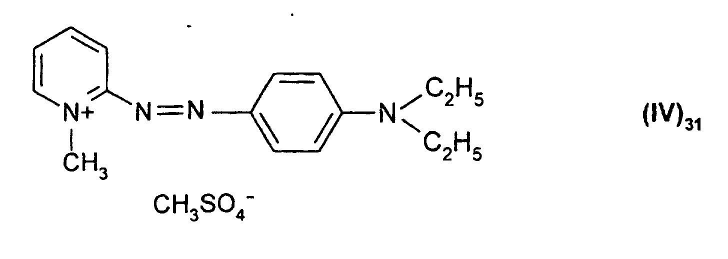

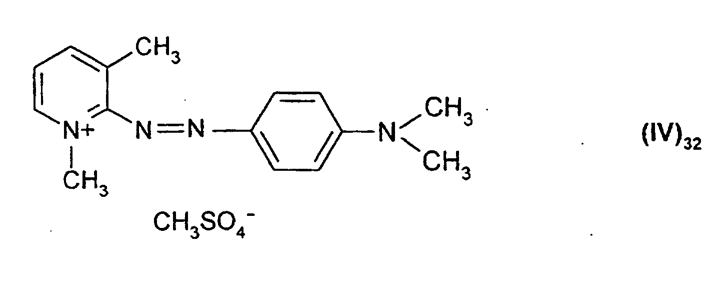

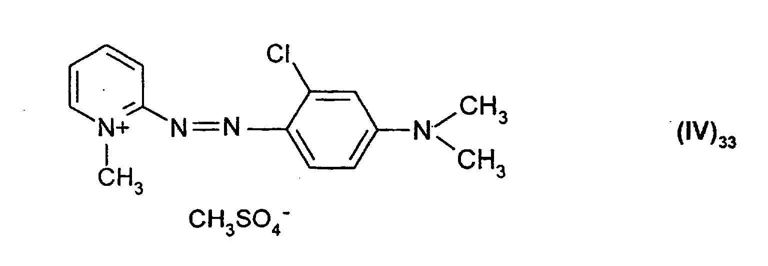

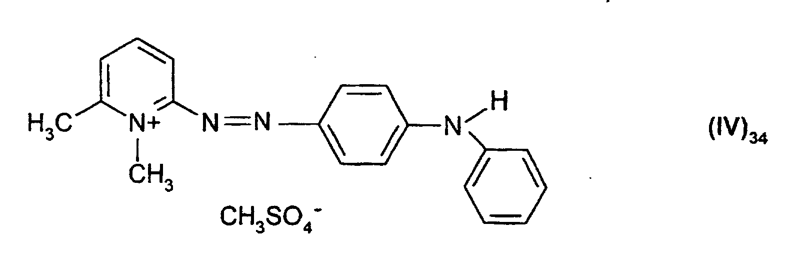

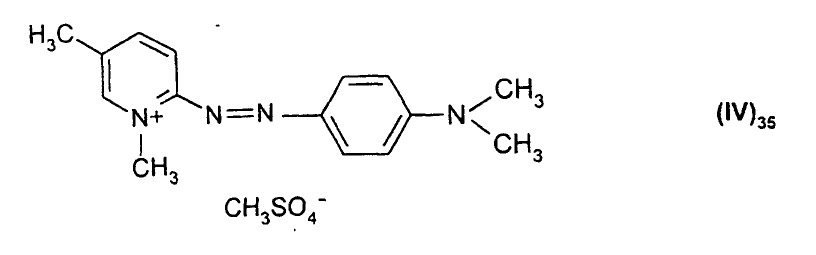

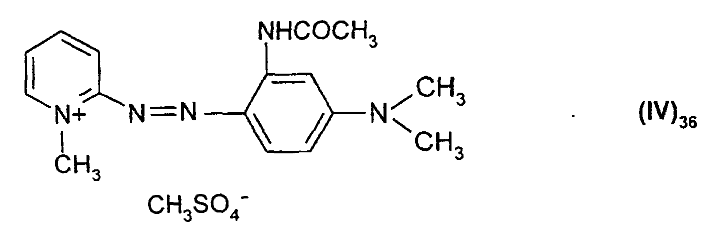

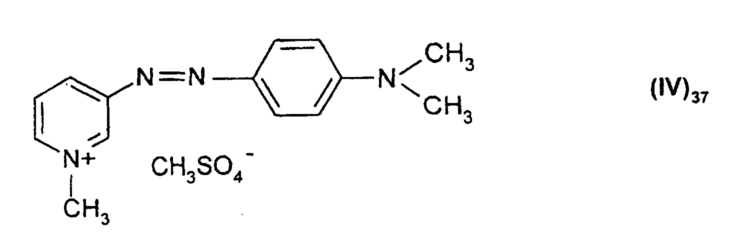

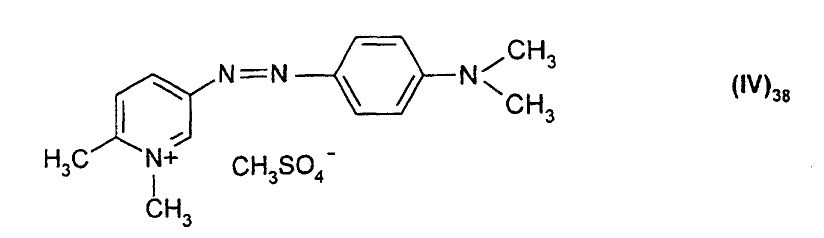

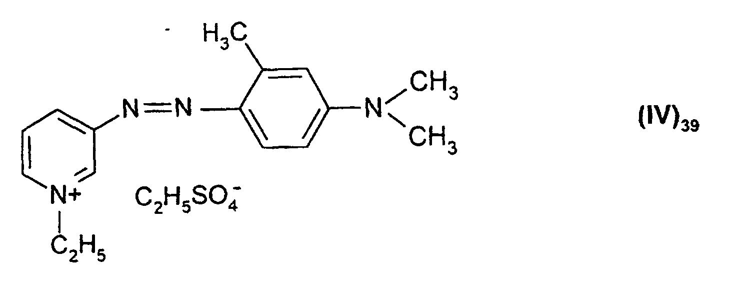

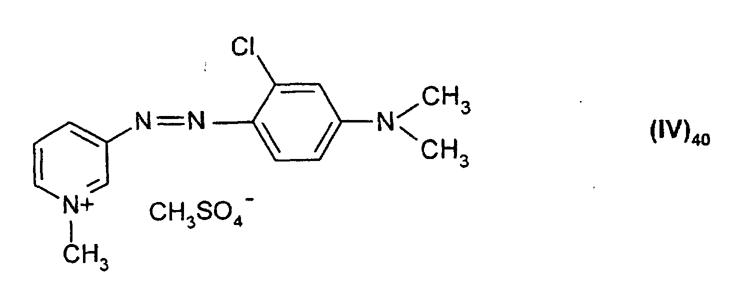

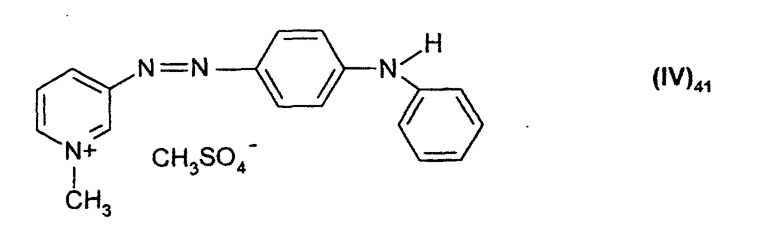

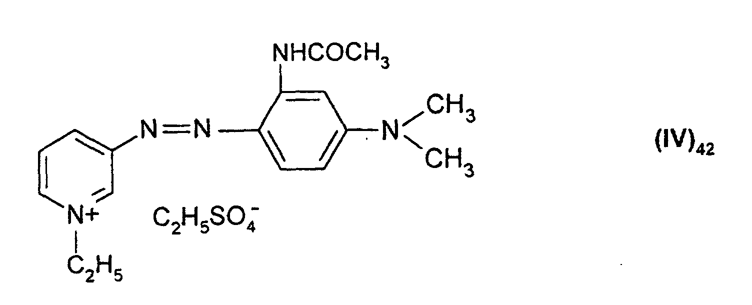

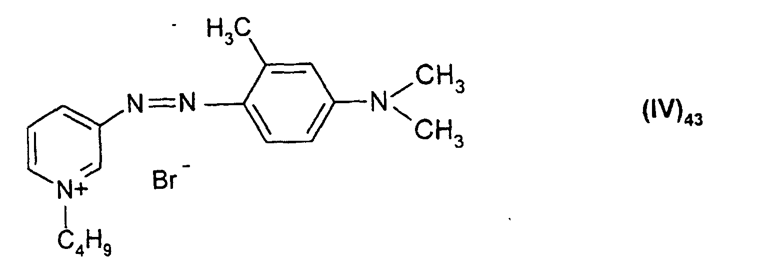

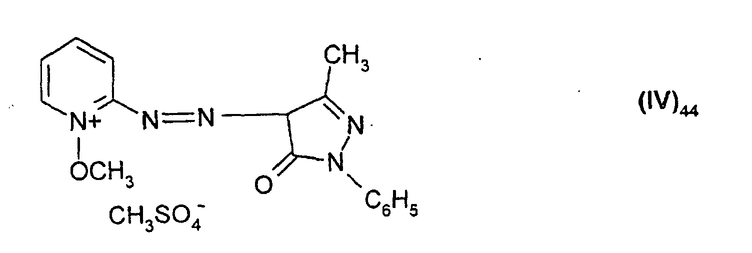

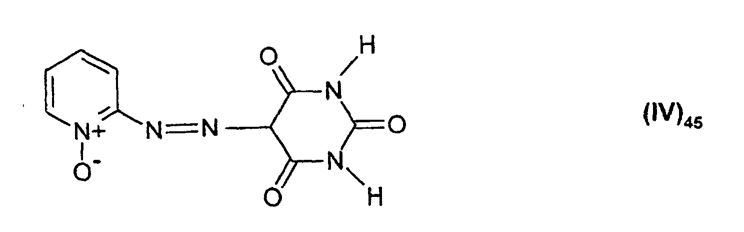

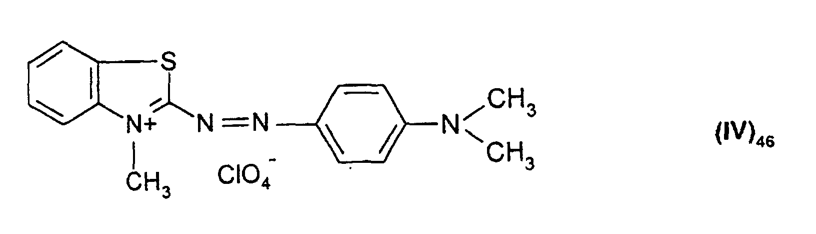

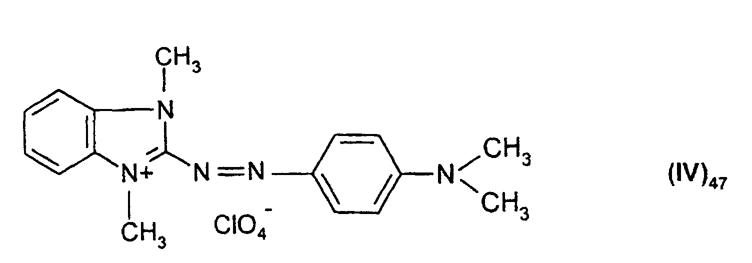

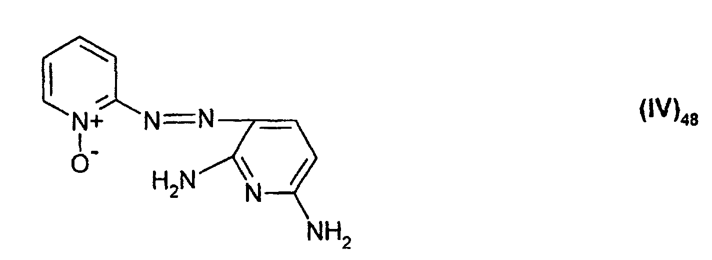

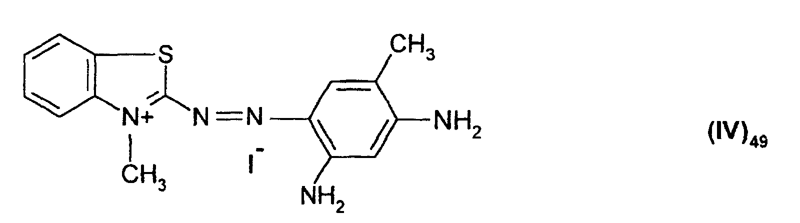

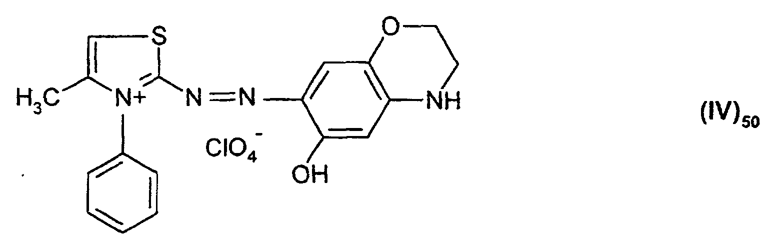

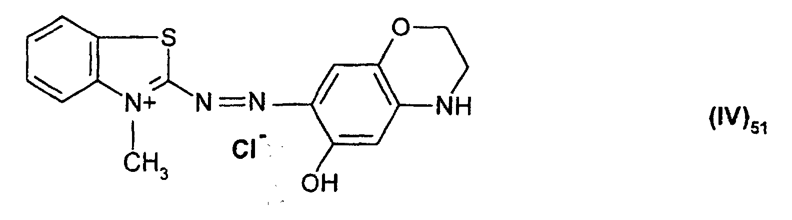

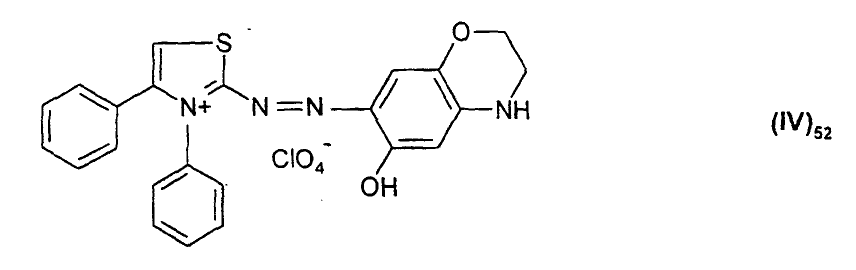

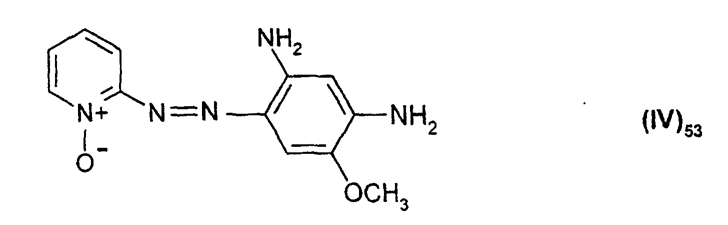

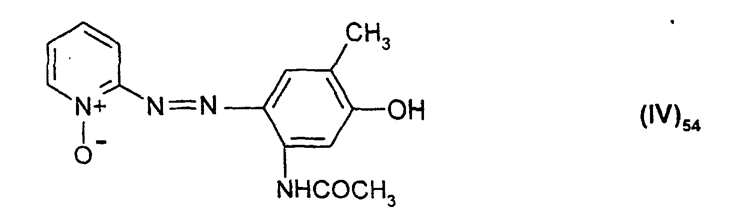

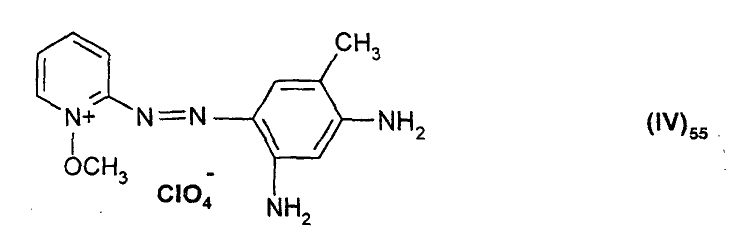

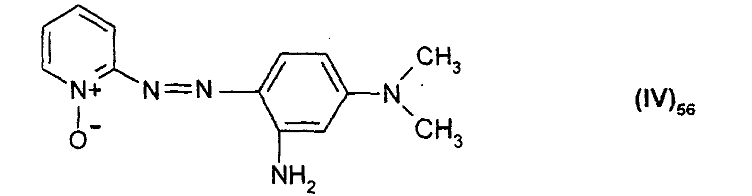

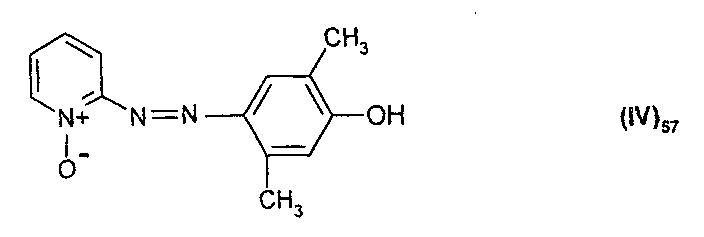

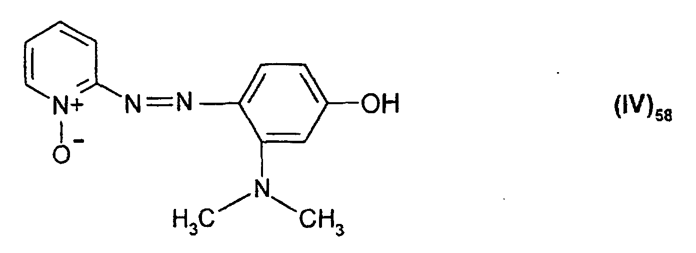

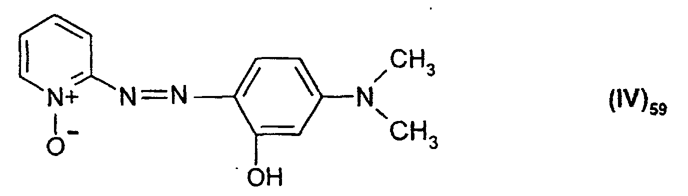

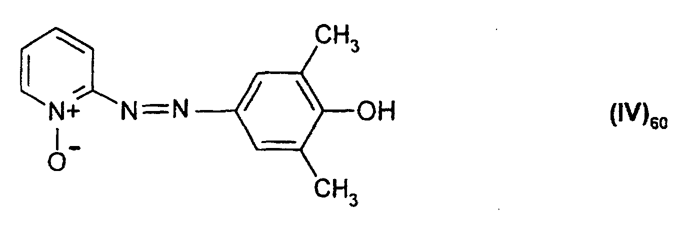

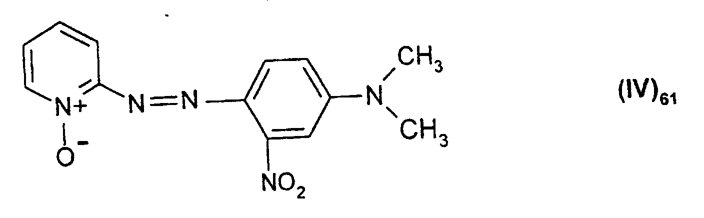

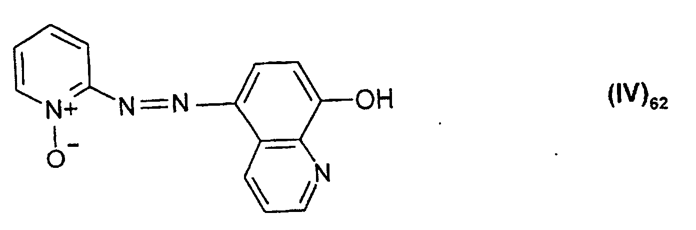

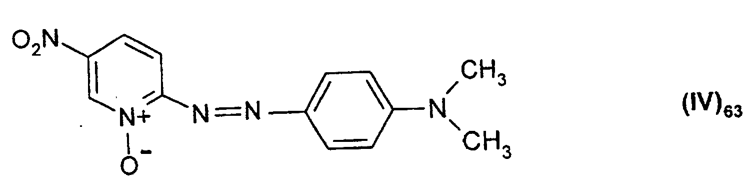

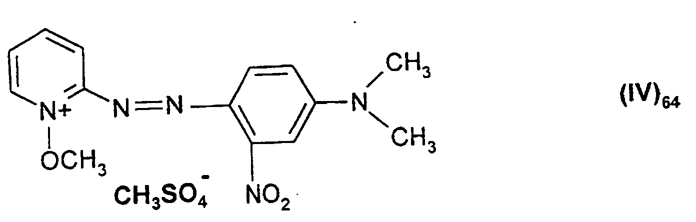

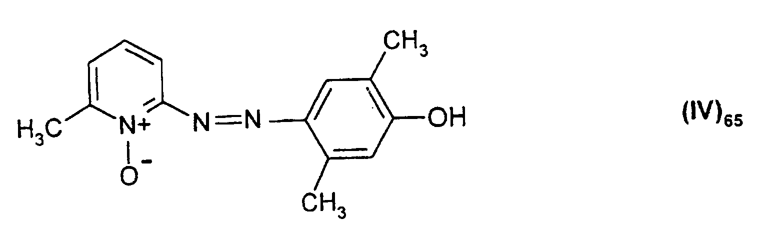

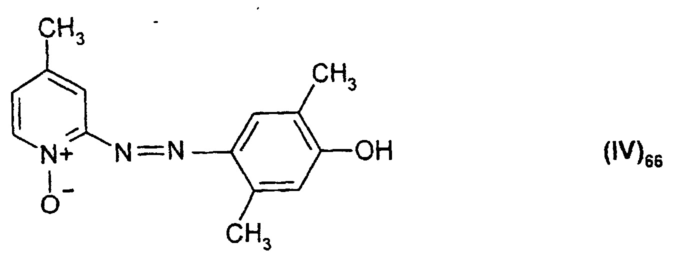

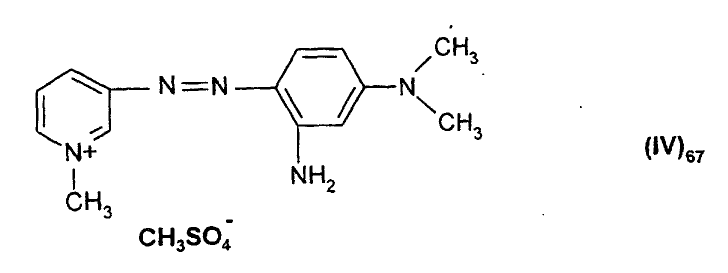

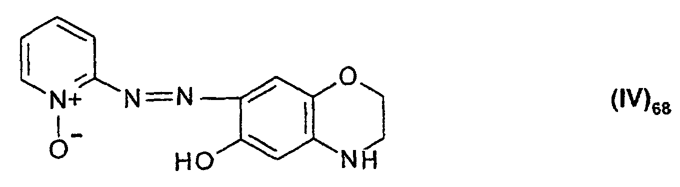

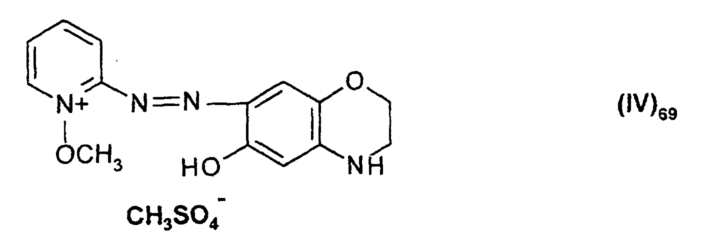

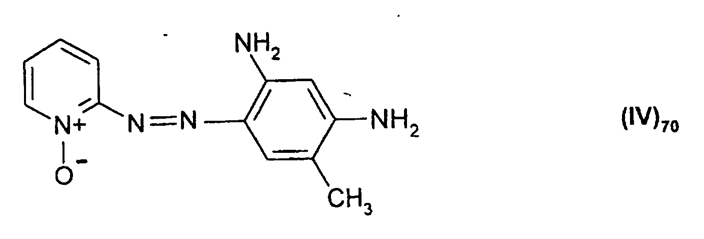

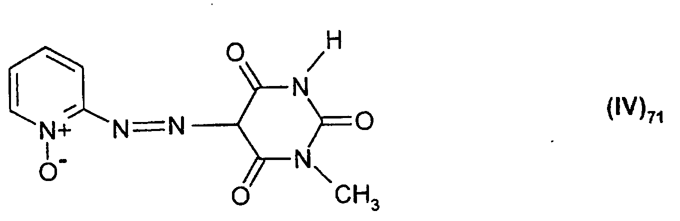

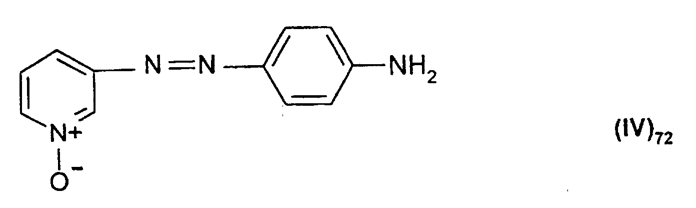

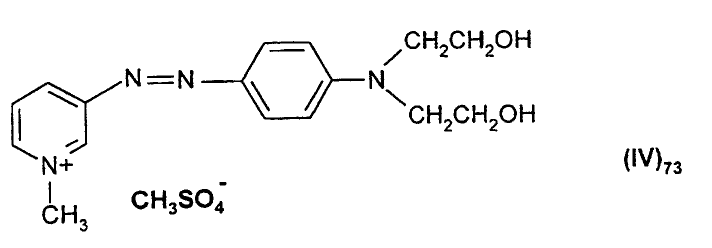

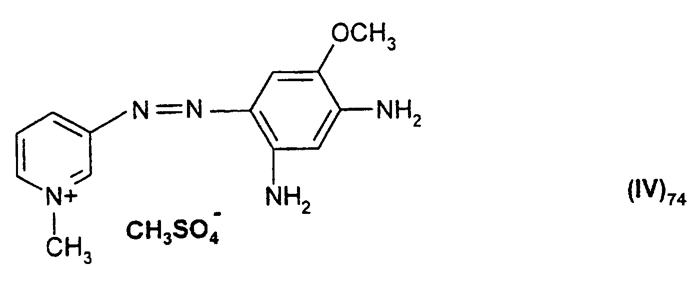

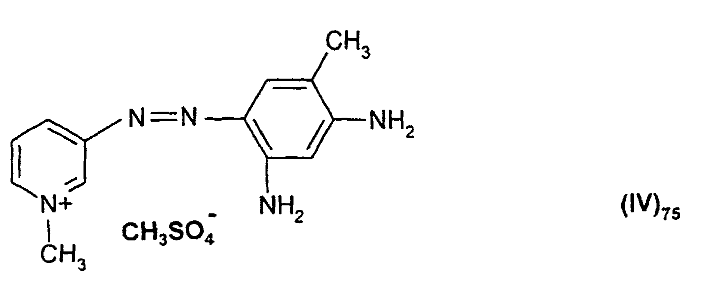

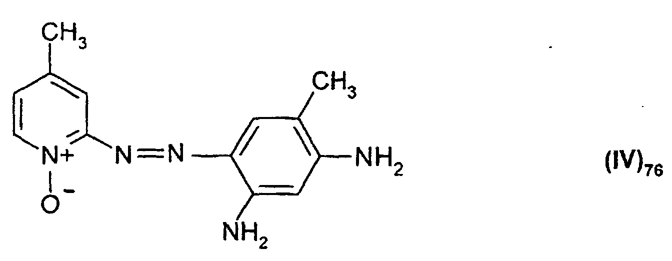

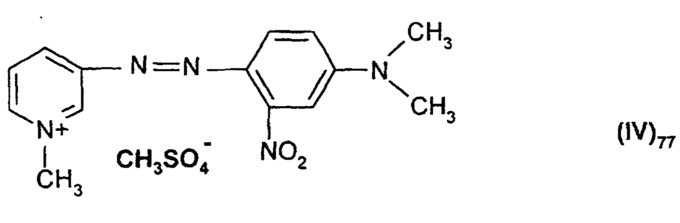

9. Composition selon l'une quelconque des revendications précédentes, **caractérisée par le fait que** le ou les colorants directs cationiques de formules (I), (II), (III), (III') ou (IV) représentent de 0,001 à 10 % en poids du poids total de la composition.

10. Composition selon la revendication 9, **caractérisée par le fait que** le ou les colorants directs cationiques de formules (I), (II), (III) (III') ou (IV) représentent de 0,005 à 5 % en poids du poids total de la composition.

11. Composition selon l'une quelconque des revendications précédentes, **caractérisée par le fait que** le polymère épaississant (ii) de type amphiphile non-ionique comportant au moins un motif hydrophile et au moins un motif à chaîne grasse est une cellulose modifiée par des groupements comportant au moins une chaîne grasse.

12. Composition selon la revendication 11, **caractérisée par le fait qu'**il s'agit d'une hydroxyéthylcellulose modifiée par des groupements alkyle ou aralkyle ou alkylaryle ou leurs mélanges.

13. Composition selon la revendication 12, **caractérisée par le fait que** dans les groupements alkyle, aralkyle ou alkylaryle, le radical alkyle est une chaîne en C₈-C₂₂.

14. Composition selon l'une quelconque des revendications 11 à 13, **caractérisée par le fait que** l'hydroxyéthylcellulose est modifiée par des groupements comportant au moins un groupe alkyle en C₁₆.

15. Composition selon la revendication 11, **caractérisée par le fait qu'**il s'agit d'une hydroxyéthylcellulose modifiée par des groupements comportant au moins un groupe polyalkylène glycol éther d'alkyl phénol.

16. Composition selon la revendication 15, **caractérisée par le fait qu'**il s'agit d'une hydroxyéthylcellulose modifiée par des groupements comportant au moins un groupe polyéthylèneglycol (15) éther de nonyl phénol.

17. Composition selon l'une quelconque des revendications 1 à 10, **caractérisée par le fait que** le polymère épaississant (ii) de type amphiphile non-ionique comportant au moins un motif hydrophile et au moins un motif à chaîne grasse est un uréthane polyéther comportant au moins un groupe alkyle ou alcényle en C₈-C₃₀.

18. Composition selon l'une quelconque des revendications 1 à 10, **caractérisée par le fait que** dans le polymère épaississant (ii) de type amphiphile anionique, le motif hydrophile est constitué par un monomère anionique insaturé éthylénique et le motif à chaîne grasse est un éther d'allyl à chaîne grasse.

19. Composition selon la revendication 18, **caractérisée par le fait que** le monomère anionique insaturé éthylénique est un acide carboxylique vinylique.

20. Composition selon la revendication 19, **caractérisée par le fait qu'**il s'agit d'un acide acrylique, un acide méthacrylique ou leur mélange.

21. Composition selon l'une quelconque des revendications 18 à 20, **caractérisée par le fait que** le motif éther d'allyl à chaîne grasse correspond au monomère de formule (V) suivante :
CH₂ = C R' CH₂ O Bₙ R (V)
dans laquelle R' désigne H ou CH₃, B désigne le radical éthylèneoxy, n est nul ou désigne un entier allant de 1 à 100, R désigne un radical hydrocarboné choisi parmi les radicaux alkyl, cycloalkyl, comprenant 8 à 30 atomes de carbone.

22. Composition selon la revendication 21, **caractérisée par le fait que** dans la formule (V), le radical hydrocarboné est alkyl et comprend de 10 à 24 atomes de carbone.

23. Composition selon la revendication 21 ou 22, **caractérisée par le fait que** dans la formule (V), R' désigne l'hydrogène, n est égal à 10 et R désigne un radical stéaryl.

24. Composition selon l'une quelconque des revendications 18 à 23, **caractérisée par le fait que** le polymère amphiphile anionique est formé par polymérisation en émulsion de 20 à 60% en poids d'acide acrylique et/ou d'acide méthacrylique, de 5 à 60% en poids de (méth)acrylates d'alkyls inférieurs, de 2 à 50% en poids d'éther d'allyl à chaîne grasse de formule (V), et de 0 à 1% en poids d'un agent réticulant.

25. Composition selon la revendication 24, **caractérisée par le fait qu'**il s'agit d'un polymère réticulé comprenant 40% en poids d'acide méthacrylique, 50% en poids d'acrylate d'éthyle, 10% en poids de polyéthylèneglycol (10 OE) éther d'alcool stéarylique (Steareth 10).

26. Composition selon l'une quelconque des revendications 1 à 10, **caractérisée par le fait que** dans le polymère épaississant (ii) de type amphiphile anionique, le motif hydrophile est constitué par un acide carboxylique insaturé oléfinique et le motif à chaîne grasse est un ester d'alkyl (C₁₀-C₃₀) d'acide carboxylique insaturé.

27. Composition selon la revendication 26, **caractérisée par le fait que** le motif hydrophile correspond au monomère de formule (VI) suivante : 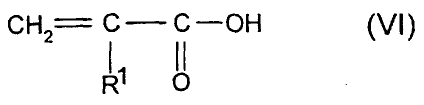 formule dans laquelle, R¹ désigne H ou CH₃ ou C₂H₅.

28. Composition selon la revendication 27, **caractérisée par le fait qu'**il s'agit d'acide acrylique, d'acide méthacrylique ou de leur mélange.

29. Composition selon la revendication 26, **caractérisée par le fait que** le motif à chaîne grasse est un ester correspondant au monomère de formule (VII) suivante : 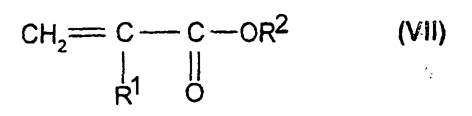 formule dans laquelle, R¹ désigne H ou CH₃ ou C₂H₅, R² désignant un radical alkyl en C₁₀-C₃₀.

30. Composition selon la revendication 29, **caractérisée par le fait que** dans la formule (VII), R¹ désigne H ou CH₃.

31. Composition selon la revendication 29 ou 30, **caractérisée par le fait que** dans la formule (VII), R² désigne un radical alkyl en C₁₂-C₂₂.

32. Composition selon l'une quelconque des revendications 26 à 31, **caractérisée par le fait que** le polymère amphiphile anionique est réticulé.

33. Composition selon l'une quelconque des revendications 26 à 32, **caractérisée par le fait que** le polymère amphiphile anionique est un polymère formé à partir d'un mélange de monomères comprenant essentiellement de l'acide acrylique, un ester de formule (VII) suivante : 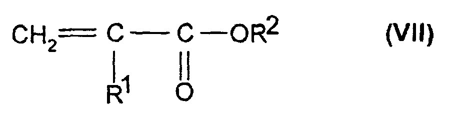 dans laquelle R¹ désigne H ou CH3, R² désignant un radical alkyl ayant de 12 à 22 atomes de carbone, et,
un agent réticulant.

34. Composition selon l'une quelconque des revendications 26 à 33, **caractérisée par le fait que** le polymère amphiphile anionique est un polymère d'acide acrylique et de méthacrylate de lauryle.

35. Composition selon l'une quelconque des revendications précédentes, **caractérisée par le fait que** le polymère épaississant (ii) représente de 0,01 à 10 % en poids du poids total de la composition.

36. Composition selon la revendication 35, **caractérisée par le fait que** le polymère épaississant (ii) représentent de 0,1 à 5% en poids du poids total de la composition.

37. Composition selon l'une quelconque des revendications précédentes, **caractérisée par le fait que** le milieu approprié pour la teinture (ou support) est constitué par de l'eau ou par un mélange d'eau et d'au moins un solvant organique.

38. Composition selon l'une quelconque des revendications précédentes, **caractérisée par le fait qu'**elle présente un pH compris entre 2 et 11, et de préférence entre 5 et 10.

39. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phénylalkylènediamines, les para-aminophénols, les ortho-aminophénols et les bases hétérocycliques.

40. Composition selon la revendication 39, **caractérisée par le fait que** la ou les bases d'oxydation représentent 0,0005 à 12 % en poids du poids total de la composition tinctoriale.

41. Composition selon la revendication 40, **caractérisée par le fait que** la ou les bases d'oxydation représentent 0,005 à 6 % en poids du poids total de la composition tinctoriale.

42. Composition selon l'une quelconque des revendications 39 à 41, **caractérisée par le fait qu'**elle renferme un ou plusieurs coupleurs choisis parmi les métaphénylènediamines, les méta-aminophénols, les métadiphénols et les coupleurs hétérocycliques.

43. Composition selon la revendication 42, **caractérisée par le fait que** le ou les coupleurs représentent de 0,0001 à 10 % en poids du poids total de la composition tinctoriale.

44. Composition selon la revendication 43, **caractérisée par le fait que** le ou les coupleurs représentent de 0,005 à 5 % en poids du poids total de la composition tinctoriale.

45. Composition selon l'une quelconque des revendications précédentes, **caractérisée par le fait qu'**elle est destinée à la teinture directe éclaircissante ou la teinture d'oxydation et qu'elle renferme alors au moins un agent oxydant.

46. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 45, pendant un temps suffisant pour développer la coloration désirée, après quoi on rince, on lave éventuellement au shampooing, on rince à nouveau et on sèche.

47. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 45, pendant un temps suffisant pour développer la coloration désirée, sans rinçage final.

48. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant le polymère épaississant (ii) tel que défini dans les revendications précédentes.

49. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant le polymère épaississant (ii) tel que défini dans les revendications précédentes.

50. Dispositif à plusieurs compartiments ou "kit" de teinture à plusieurs compartiments, **caractérisé par le fait qu'**un premier compartiment renferme la composition (A1) ou (A2) telle que définie à la revendication 48 ou 49 et un second compartiment renferme la composition (B1) ou (B2) telle que définie à la revendication 48 ou 49.

## Patentansprüche

1. Zusammensetzung zum Färben von Keratinfasern und insbesondere menschlichen Keratinfasern, beispielsweise zum Färben des Haares, die in einem zum Färben geeigneten Medium enthält: (i) mindestens eine Verbindung, die unter den Verbindungen der folgenden Formeln (I), (II), (III), (III') oder (IV) ausgewählt ist:
(a) Verbindungen der folgenden Formel (I): 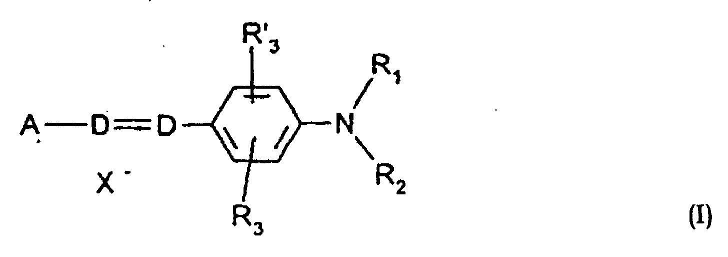 worin bedeuten:
D ein Stickstoffatom oder die Gruppe -CH,
R₁ und R₂, die gleich oder verschieden sind, ein Wasserstoffatom; eine C₁₋₄-Alkylgruppe, die mit einer Gruppe -CN, -OH oder -NH₂ substituiert sein kann, oder sie bilden mit einem Kohlenstoffatom des Benzolrings einen gegebenenfalls sauerstoff- oder stickstoffhaltigen Heterocyclus, der mit einer oder mehreren C₁₋₄-Alkylgruppen substituiert sein kann; oder eine 4'-Aminophenylgruppe,
R₃ und R'₃, die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder Acetyloxy,
X⁻ ein Anion, das vorzugsweise unter Chlorid, Methylsulfat und Acetat ausgewählt ist,
A eine Gruppe, die unter den folgenden Strukturen A1 bis A18 ausgewählt ist: 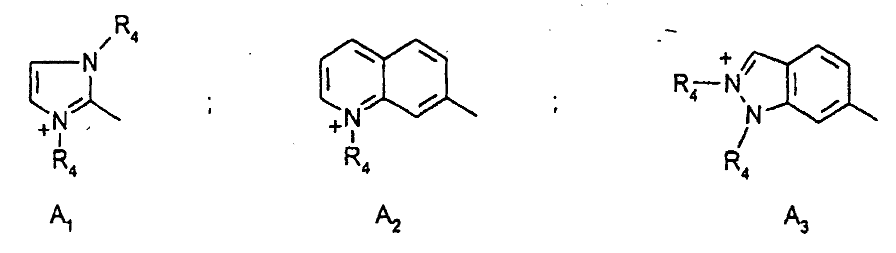 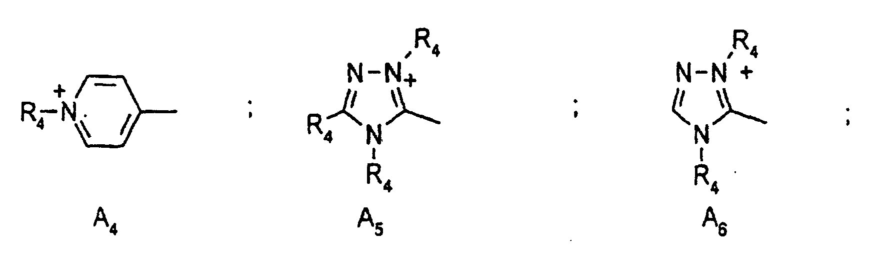 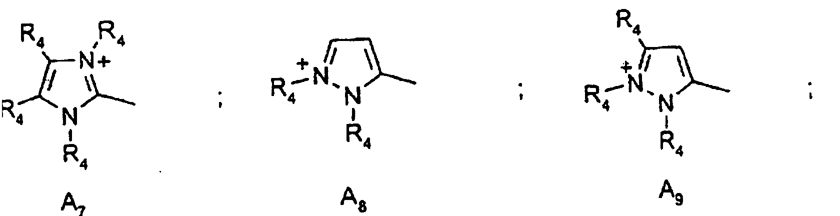 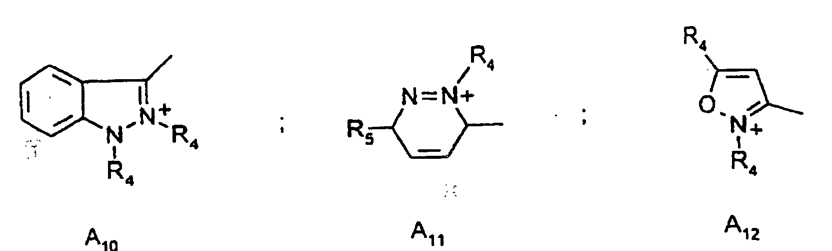 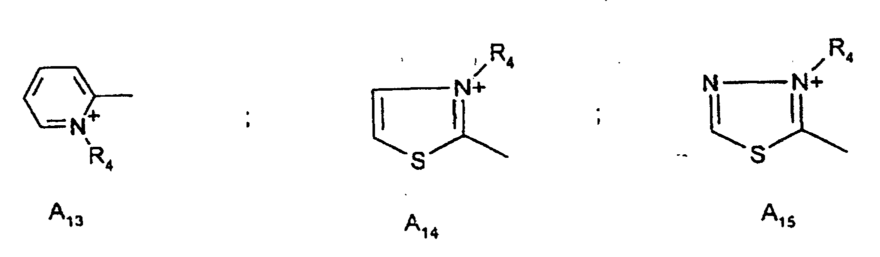 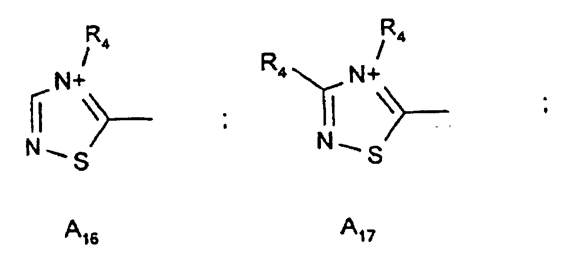 und 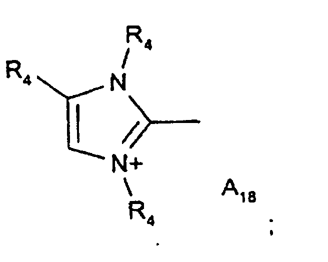 worin die Gruppe R₄ eine C₁₋₄-Alkylgruppe bedeutet, die mit einer Hydroxygruppe substituiert sein kann, und R₅ eine C₁₋₄-Alkoxygruppe ist, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist;
(b) Verbindungen der folgenden Formel (II): 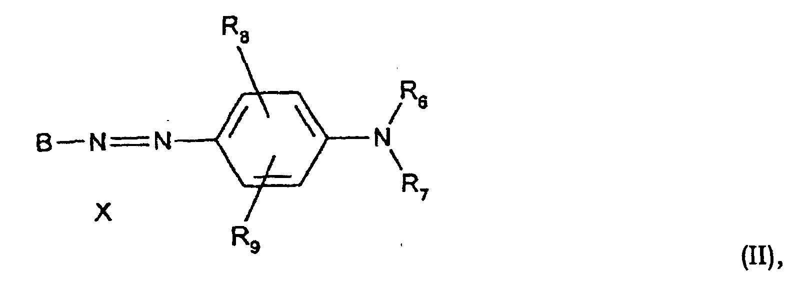 worin bedeuten:
R₆ ein Wasserstoffatom oder eine C₁₋₄-Alkylgruppe,
R₇ ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
R₈ und R₉, die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe -CN,
X⁻ ein Anion, das vorzugsweise unter Chlorid, Methylsulfat und Acetat ausgewählt ist,
B eine Gruppe, die unter den folgenden Strukturen B1 bis B6 ausgewählt ist: 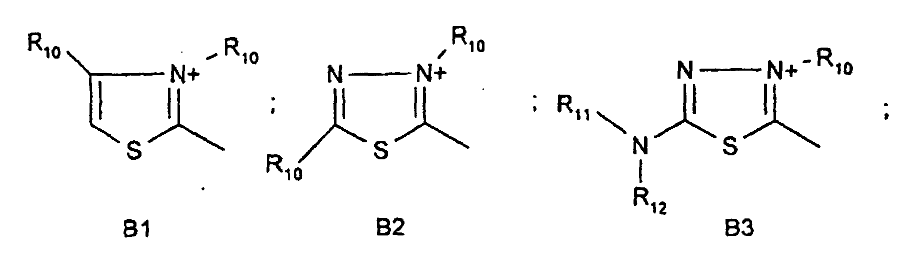 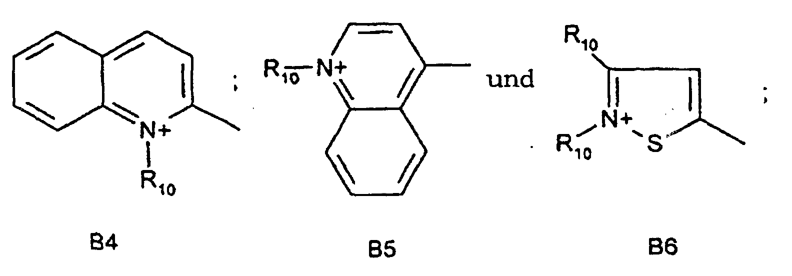 worin die Gruppe R¹⁰ eine C₁₋₄-Alkylgruppe bedeutet und die Gruppen R₁₁ und R₁₂, die gleich oder verschieden sind, Wasserstoff oder C₁₋₄-Alkyl bedeuten;
(c) Verbindungen der folgenden Formel (III) und (III'): 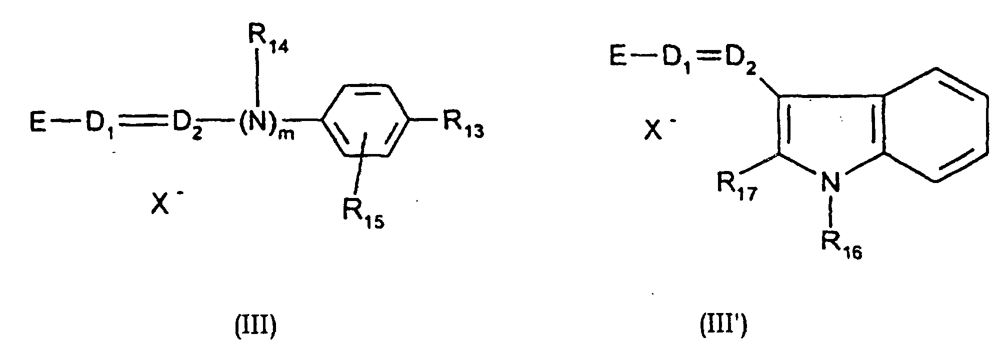 worin bedeuten:
R₁₃ ein Wasserstoffatom, eine C₁₋₄-Alkoxygruppe, ein Halogenatom, wie Brom, Chlor, Iod oder Fluor, oder eine Aminogruppe,
R₁₄ ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig und/oder mit einer oder mehreren C₁₋₄-Alkylgruppen substituiert ist;
R₁₅ ein Wasserstoffatom oder ein Halogenatom, wie Brom, Chlor, Iod oder Fluor,
R₁₆ und R₁₇, die identisch oder voneinander verschieden sind, Wasserstoff oder C₁₋₄-Alkyl,
D₁ und D₂, die gleich oder verschieden sind, ein Stickstoffatom oder die Gruppe -CH,
m = 0 oder 1,
mit der Maßgabe, dass die Gruppen D₁ und D₂ gleichzeitig -CH bedeuten und m = 0, wenn R₁₃ eine unsubstituierte Aminogruppe ist,
X⁻ ein Anion, das vorzugsweise unter Chlorid, Methylsulfat und Acetat ausgewählt ist,
E eine Gruppe, die unter den folgenden Strukturen E1 bis E8 ausgewählt ist: 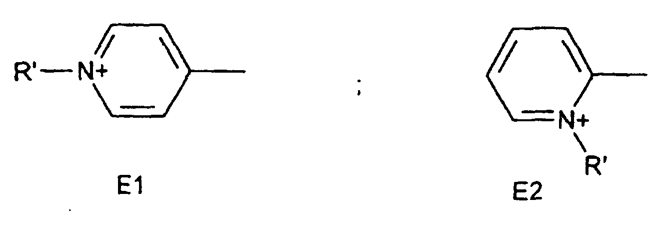 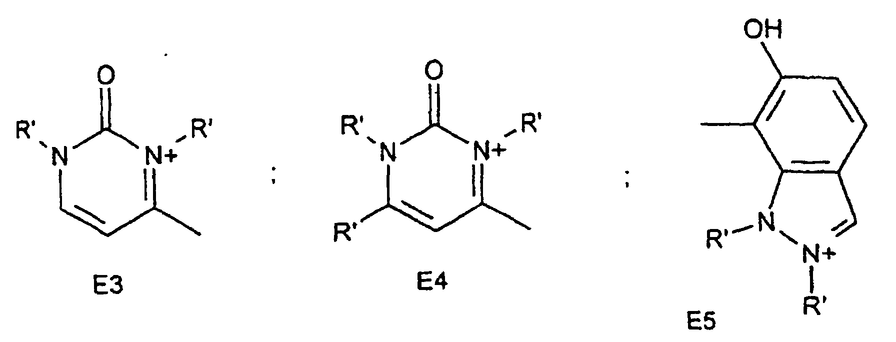 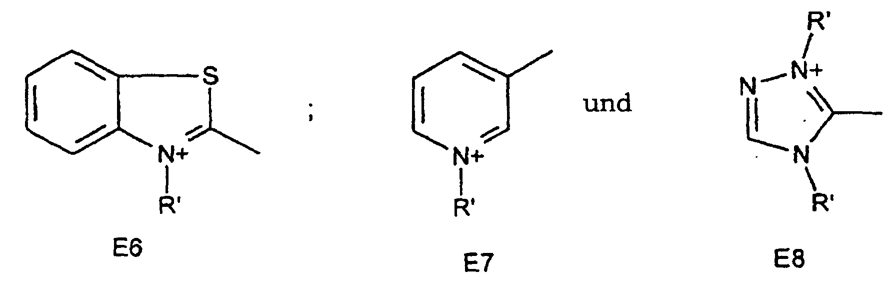 wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 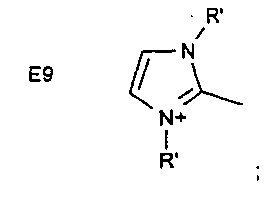 wobei R' eine C₁₋₄-Alkylgruppe bedeutet;
d) Verbindungen der folgenden Formel (IV):
G-N = N-J (IV),
worin bedeuten:
das Symbol G eine Gruppe, die unter den folgenden Strukturen G₁ bis G₃ ausgewählt ist: 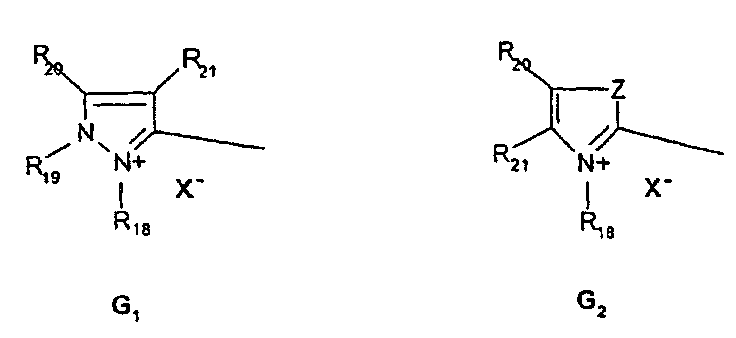 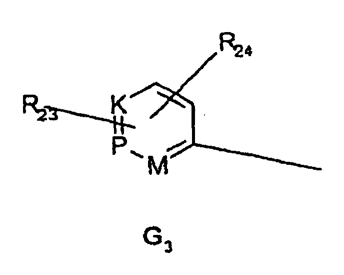 wobei in den Strukturen G₁ bis G₃ bedeuten:
R₁₈ C₁₋₄-Alkyl oder eine Phenylgruppe, die mit einer C₁₋₄-Alkylgruppe oder einem Halogenatom substituiert sein kann, das unter Chlor, Brom, Iod und Fluor ausgewählt ist;
R₁₉ C₁₋₄-Alkyl oder Phenyl;
R₂₀ und R₂₁, die gleich oder verschieden sind, C₁₋₄-Alkyl, Phenyl oder R₂₀ und R₂₁ bilden in G₁ gemeinsam einen Benzolring, der mit einer oder mehreren Gruppen C₁₋₄-Alkyl, C₁₋₄-Alkoxy oder NO₂ substituiert ist, oder bilden in G₂ gemeinsam einen Benzolring, der gegebenenfalls mit einer oder mehreren Gruppen C₁₋₄-Alkyl, C₁₋₄-Alkoxy oder NO₂ substituiert ist; wobei R₂₀ außerdem ein Wasserstoffatom bedeuten kann;
Z ein Sauerstoffatom, ein Schwefelatom oder eine Gruppe -NR₁₉;
M -CH, -CR (wobei R eine C₁₋₄-Alkylgruppe ist) oder -NR₂₂(X⁻)ᵣ;
K -CH, -CR (wobei R eine C₁₋₄-Alkylgruppe bedeutet) oder -NR₂₂(X⁻)ᵣ;
P eine Gruppe -CH, -CR (wobei R eine C₁₋₄-Alkylgruppe bedeutet) oder -NR₂₂(X⁻)ᵣ; wobei r Null oder 1 ist;
R₂₂ ein Atom O⁻, C₁₋₄-Alkoxy oder C₁₋₄-Alkyl;
R₂₃ und R₂₄, die gleich oder verschieden sind, ein Wasserstoffatom, ein Halogenatom, das unter Chlor, Brom, Iod und Fluor ausgewählt ist, eine C₁₋₄-Alkylgruppe, eine C₁₋₄-Alkoxygruppe oder die Gruppe -NO₂;
X- ein Anion, das vorzugsweise unter Chlorid, Iodid, Methylsulfat, Ethylsulfat, Acetat und Perchlorat ausgewählt ist;
mit der Maßgabe, dass
wenn R₂₂ O⁻ bedeutet, r Null ist;
wenn K oder P oder M -N(C₁₋₄)alkyl X⁻ bedeutet, R₂₃ oder R₂₄ von Wasserstoff verschieden ist;
wenn K -NR₂₂(X⁻)ᵣ bedeutet, M = P = -CH, -CR;
wenn M -NR₂₂(X⁻)ᵣ bedeutet, K = P = -CH, -CR;
wenn P -NR₂₂(X⁻)ᵣ bedeutet, K = M = -CH oder -CR bedeutet;
wenn Z ein Schwefelatom und R₂₁ C₁₋₄-Alkyl ist, R₂₀ von Wasserstoff verschieden ist;
wenn Z -NR₁₉ und R₁₉ C₁₋₄-Alkyl bedeutet, mindestens eine der Gruppen R₁₈, R₂₀ oder R₂₁ der Struktur G₂ von einer C₁₋₄-Alkylgruppe verschieden ist;
das Symbol J:
- (a) eine Gruppe der folgenden Struktur J₁: 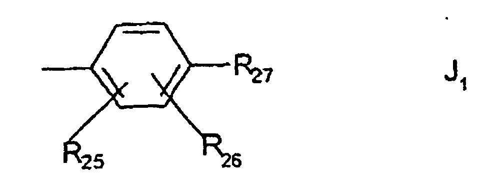 wobei in der Struktur J₁ bedeuten:
R₂₅ ein Wasserstoffatom, ein Halogenatom, das unter Chlor, Brom, Iod und Fluor ausgewählt ist, C₁₋₄-Alkyl, C₁₋₄-Alkoxy, -OH, -NO₂, -NHR₂₈, -NR₂₉R₃₀, -NHCOalkyl (C₁₋₄) oder R₂₅ bildet mit R₂₆ einen 5- oder 6-gliedrigen Ring, der gegebenenfalls ein oder mehrere Heteroatome enthält, die unter Stickstoff, Sauerstoff oder Schwefel ausgewählt sind;
R₂₆ ein Wasserstoffatom, ein Halogenatom, das unter Chlor, Brom, Iod und Fluor ausgewählt ist, C₁₋₄-Alkyl, C₁₋₄-Alkoxy oder R₂₆ bildet mit R₂₇ oder R₂₈ einen 5- oder 6-gliedrigen Ring, der gegebenenfalls ein oder mehrere Heteroatome enthält, die unter Stickstoff, Sauerstoff oder Schwefel ausgewählt sind;
R₂₇ Wasserstoff, -OH, -NHR₂₈ oder -NR₂₉R₃₀;
R₂₈ Wasserstoff, C₁₋₄-Alkyl, C₁₋₄-Monohydroxyalkyl, C₂₋₄-Polyhydroxyalkyl oder Phenyl;
R₂₉ und R₃₀, die gleich oder verschieden sind, C₁₋₄-Alkyl, C₁₋₄-Monohydroxyalkyl oder C₂₋₄-Polyhydroxyalkyl;
- (b) eine stickstoffhaltige, 5- oder 6-gliedrige heterocyclische Gruppe, die weitere Heteroatome und/oder carbonylhaltige Gruppen enthalten und mit einer oder mehreren Gruppen C₁₋₄-Alkyl, Amino oder Phenyl substituiert sein kann, insbesondere eine Gruppe der folgenden Struktur J₂: 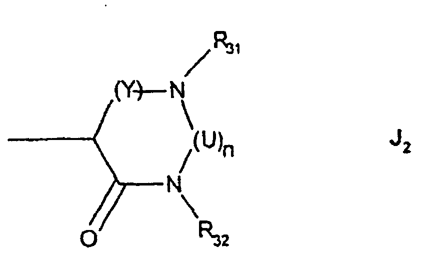 wobei in der Struktur J₂ bedeuten:
R₃₁ und R₃₂, die gleich oder verschieden sind, Wasserstoff, C₁₋₄-Alkyl oder Phenyl;
Y die Gruppe -CO- oder die Gruppe 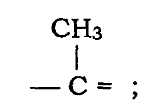
n = 0 oder 1, wobei U die Gruppe -CO- bedeutet, wenn n 1 ist,
wobei die Zusammensetzung **dadurch gekennzeichnet ist, dass** sie ferner enthält:
(ii) mindestens ein verdickendes Polymer, das unter den folgenden Polymeren ausgewählt ist:
(ii)₁ - nichtionischen amphiphilen Polymeren, die mindestens eine hydrophile Einheit und mindestens eine Einheit mit Fettkette enthalten;
(ii)₂ - anionischen amphiphilen Polymeren, die mindestens eine hydrophile Einheit und mindestens eine Einheit mit Fettkette enthalten;
(ii)₃ - kationischen amphiphilen Polymeren, die mindestens eine hydrophile Einheit und mindestens eine Einheit mit Fettkette enthalten.

2. Zusammensetzung nach Anspruch 1, **dadurch gekennzeichnet, dass** die kationischen Direktfarbstoffe der Formel (I) unter den Verbindungen der folgenden Strukturen (I1) bis (I54) ausgewählt sind:

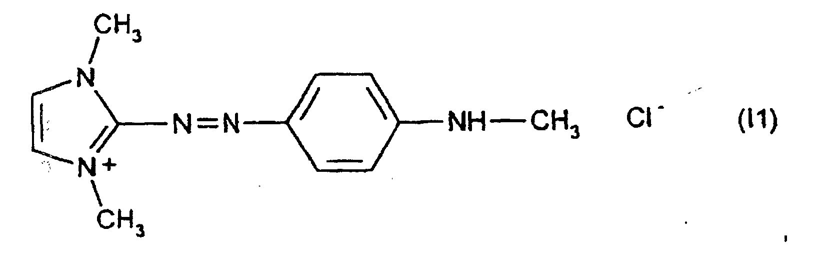

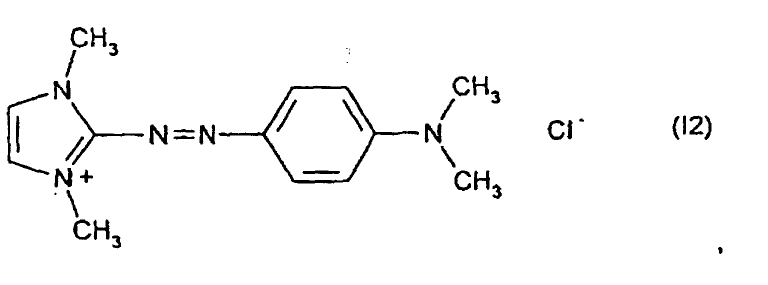

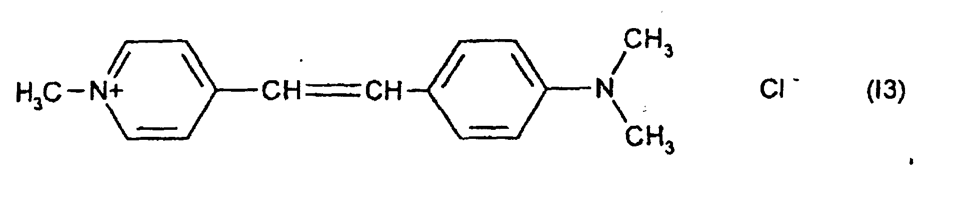

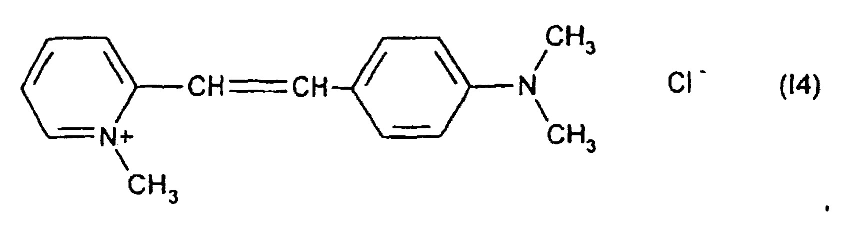

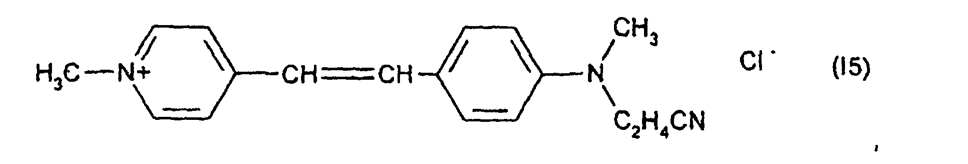

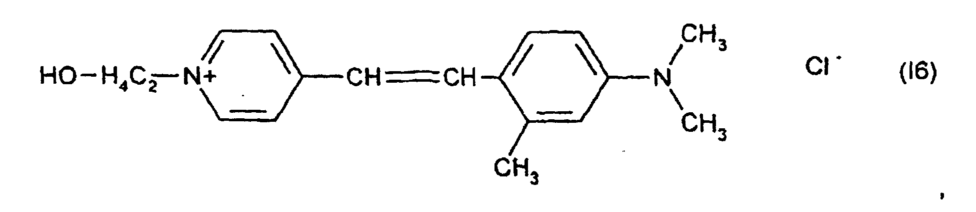

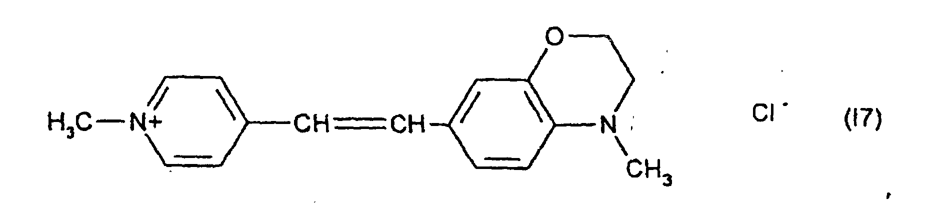

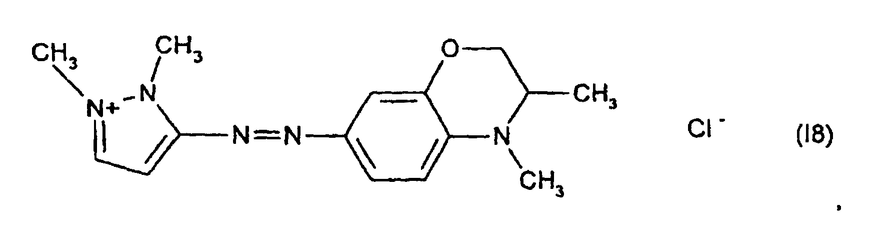

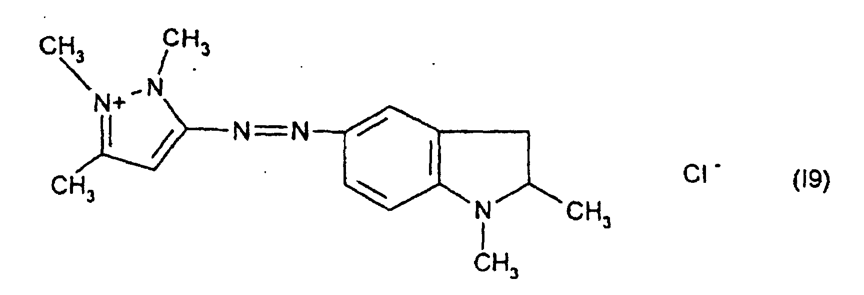

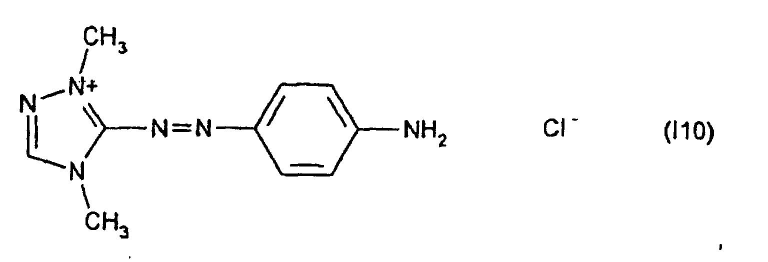

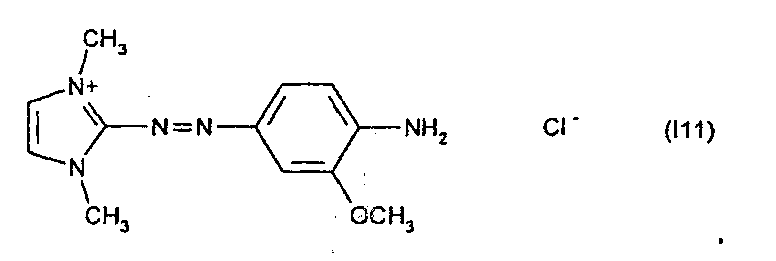

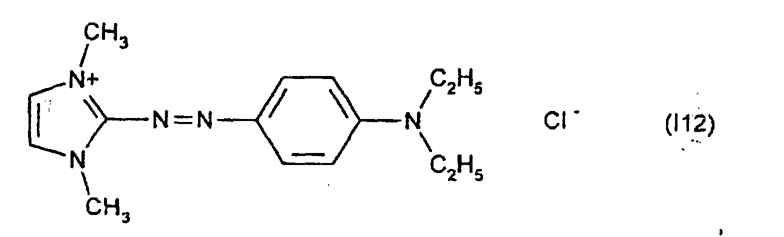

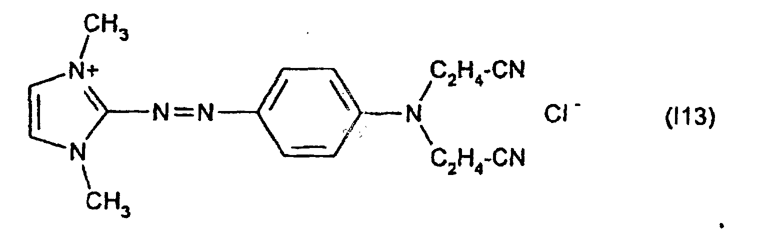

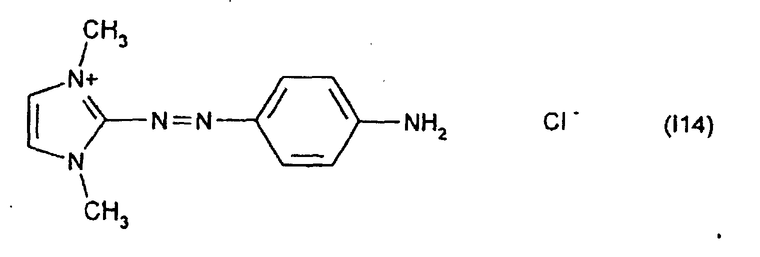

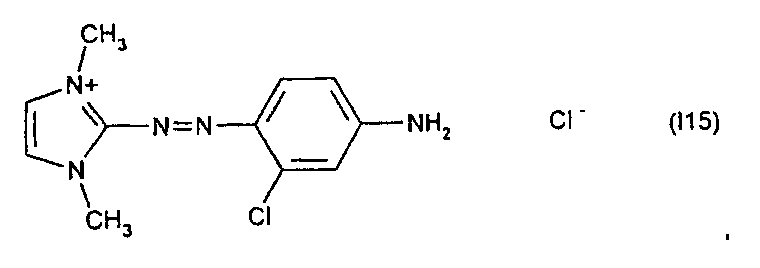

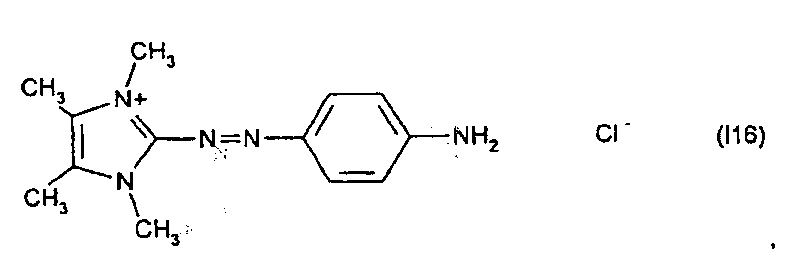

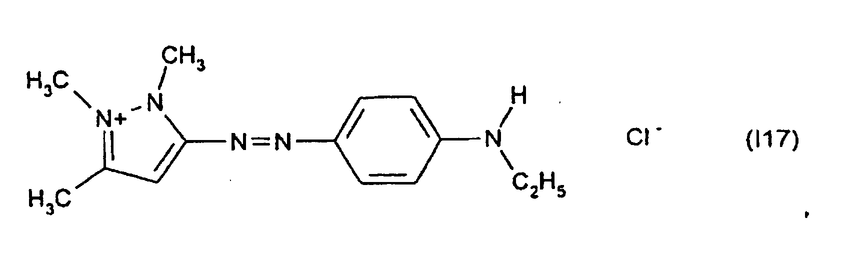

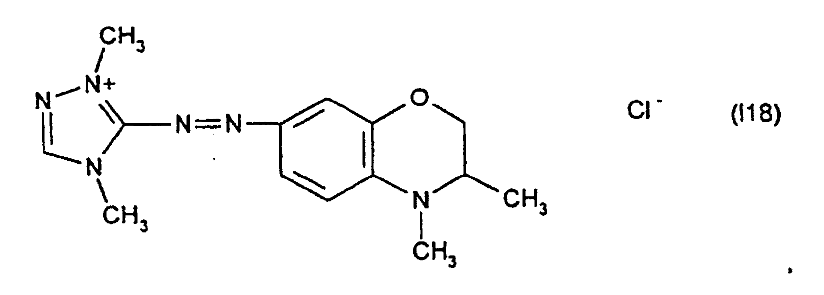

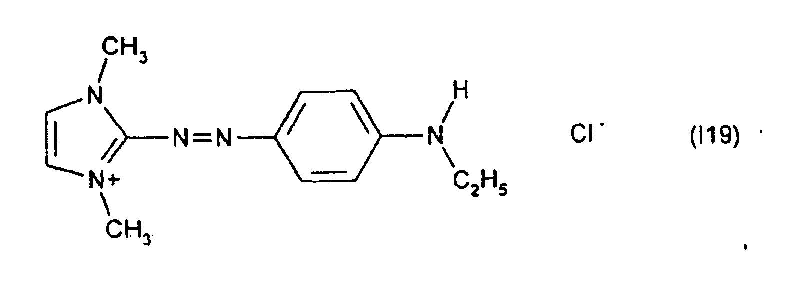

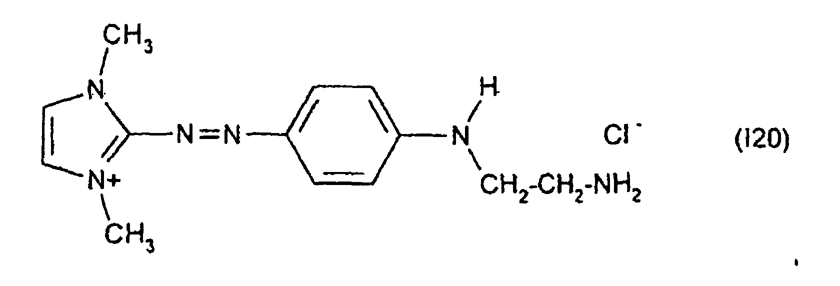

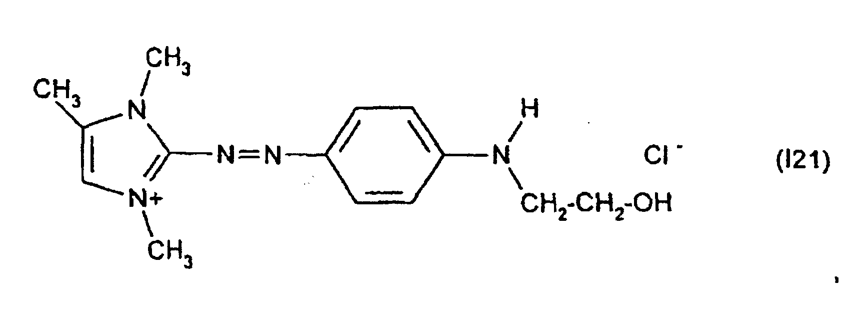

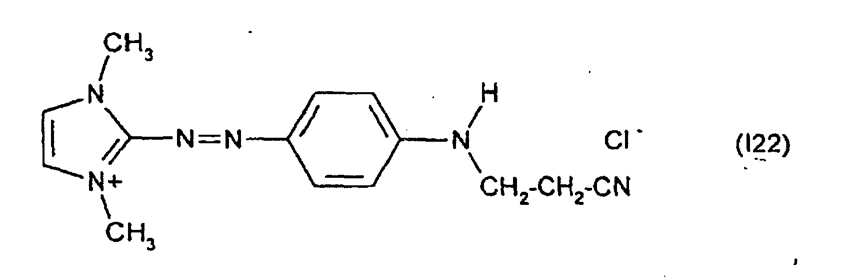

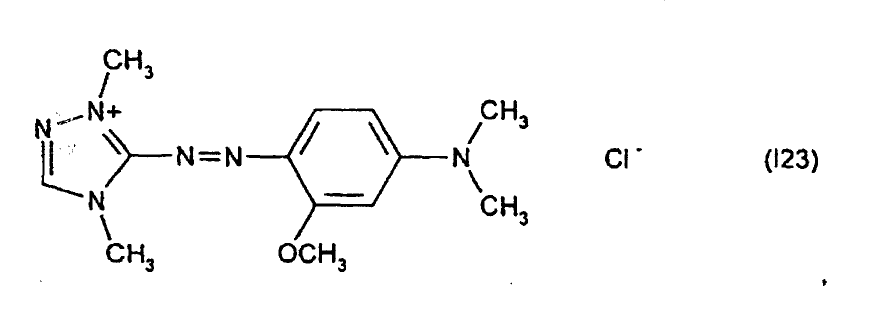

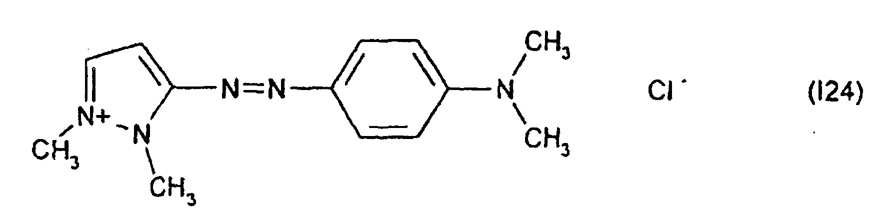

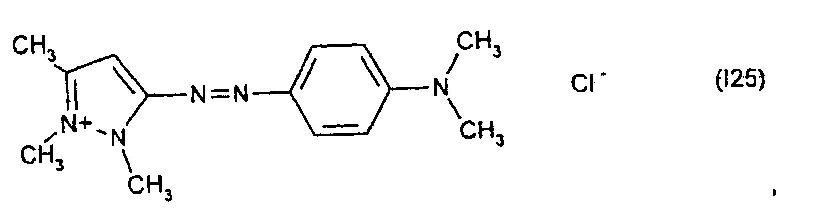

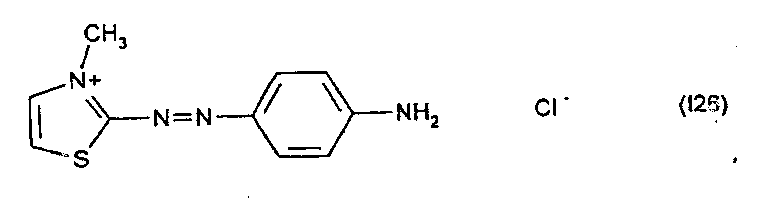

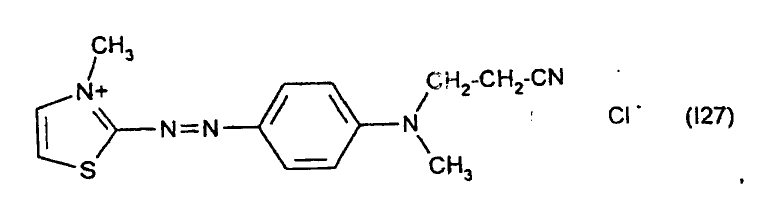

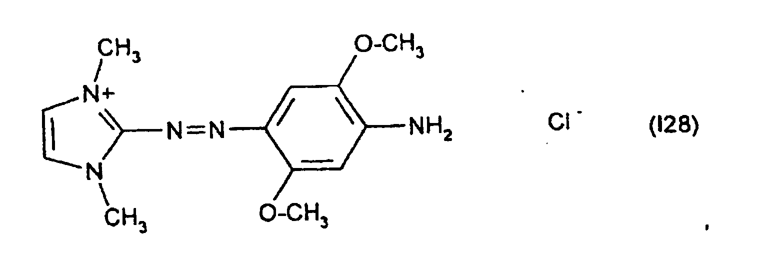

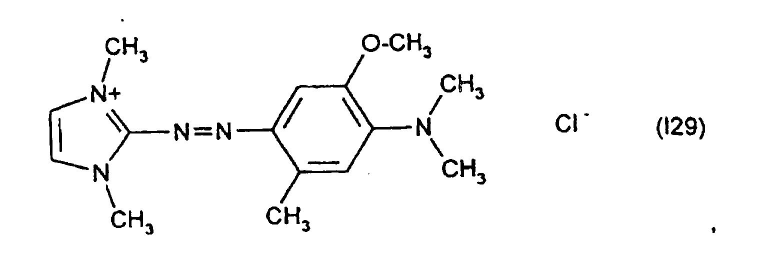

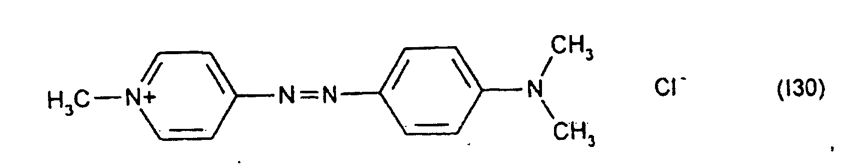

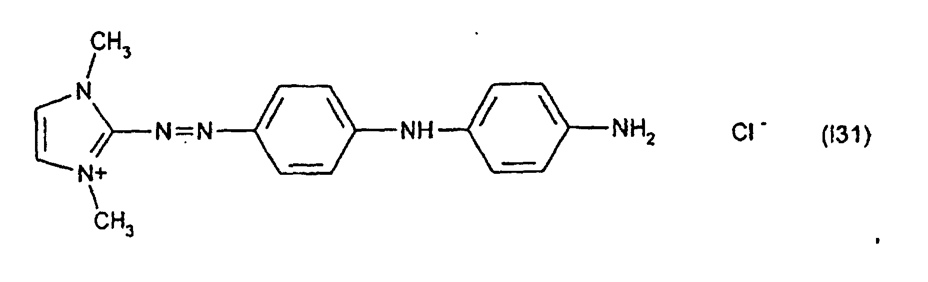

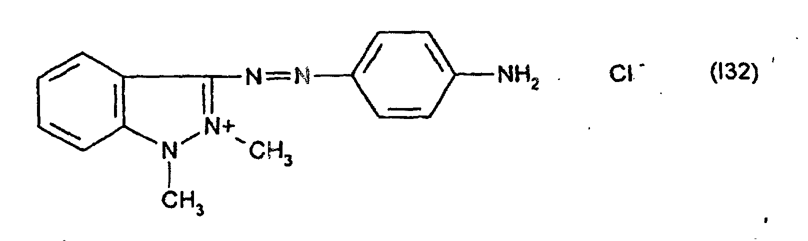

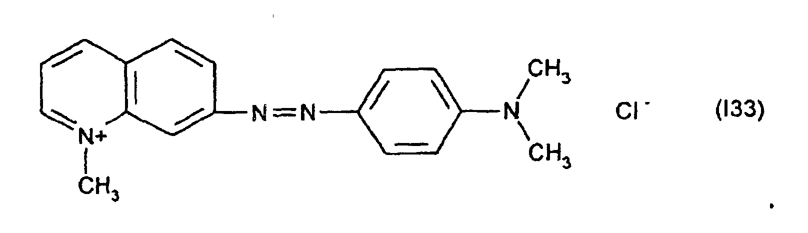

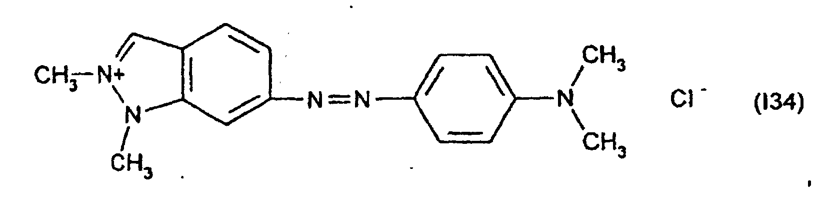

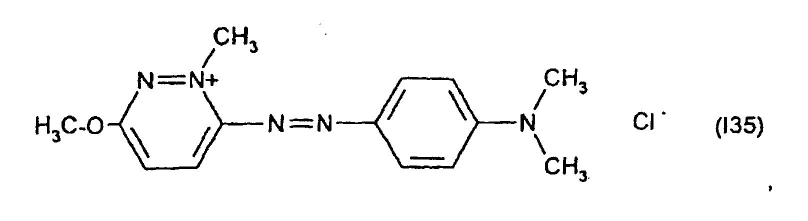

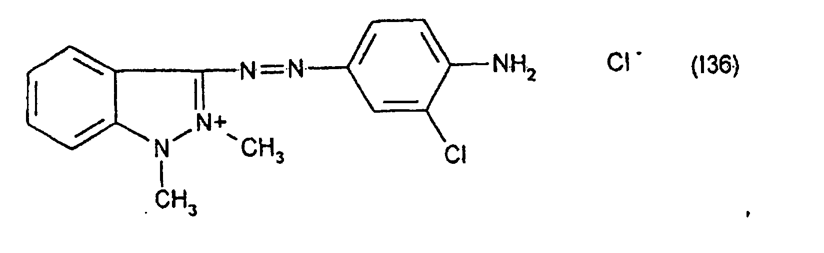

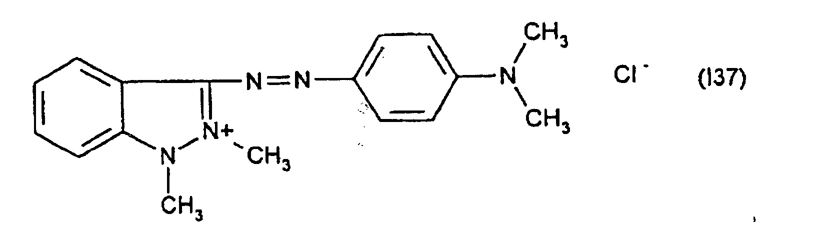

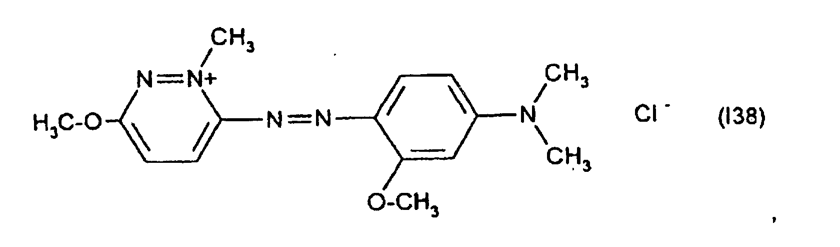

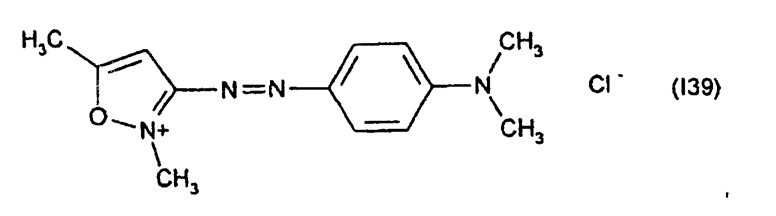

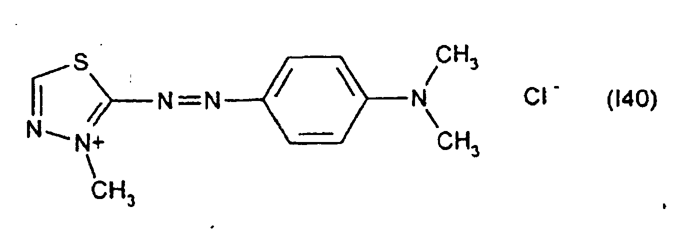

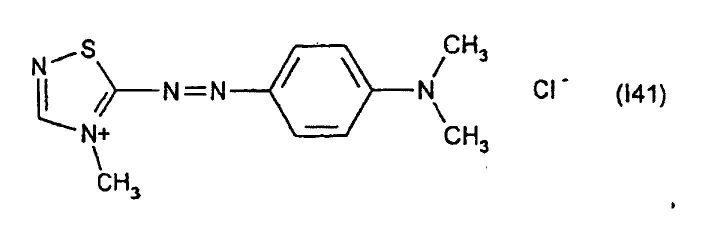

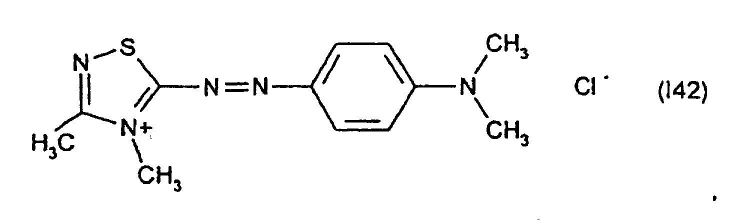

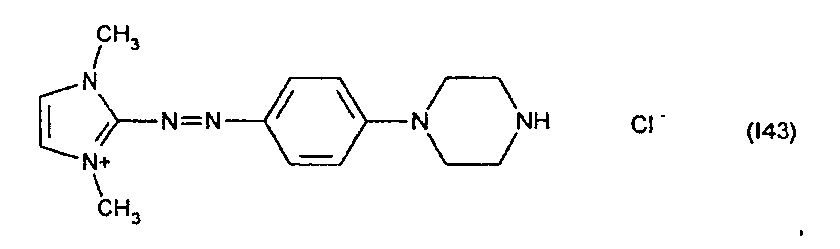

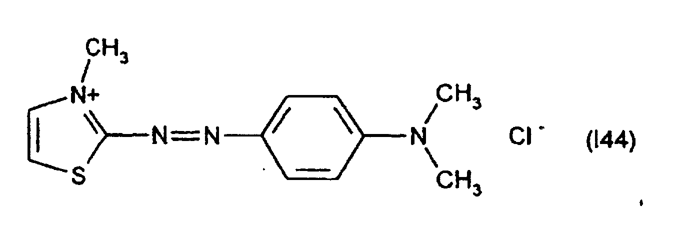

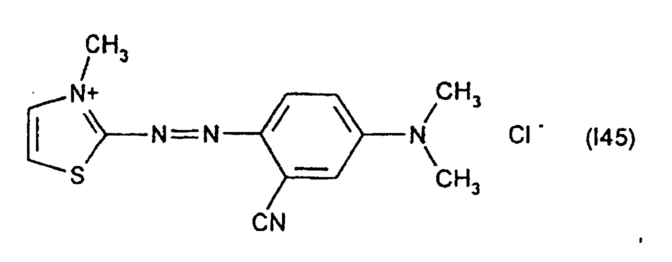

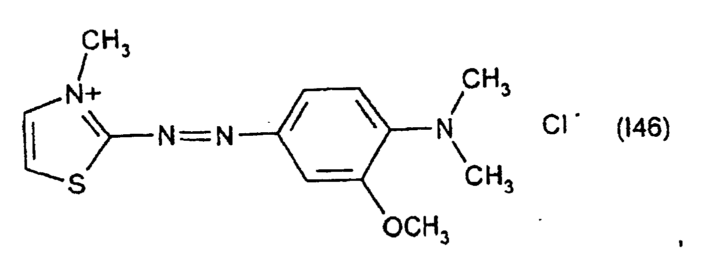

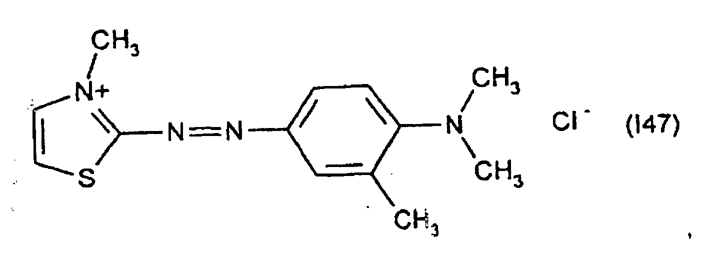

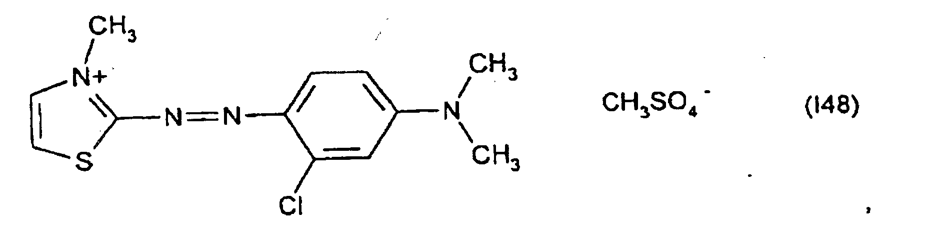

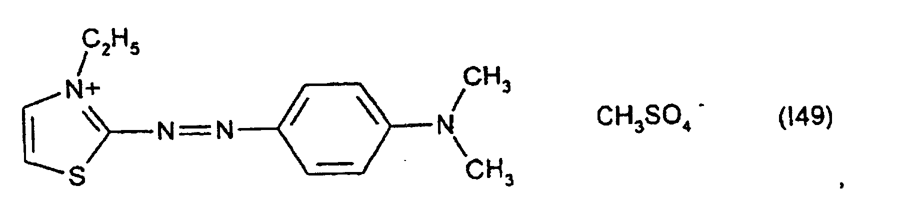

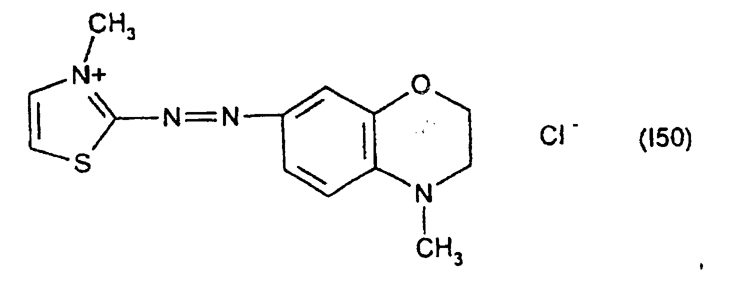

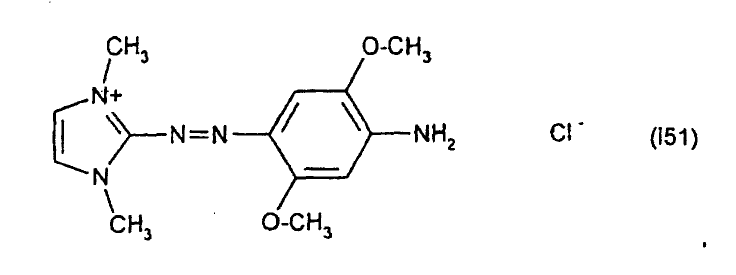

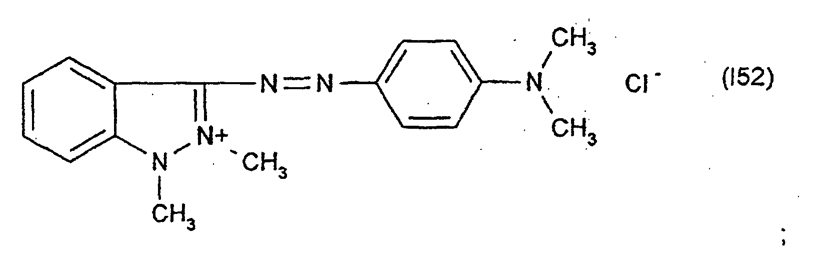

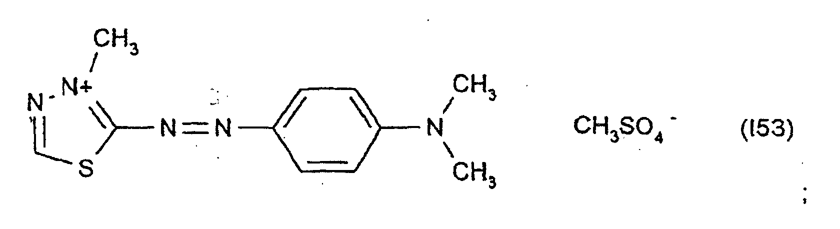

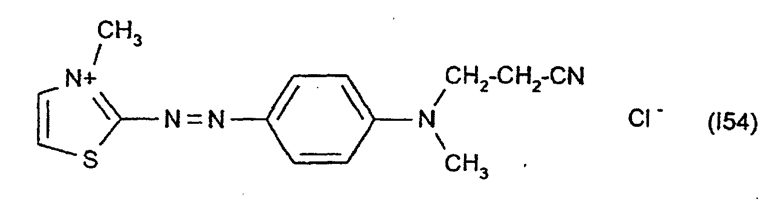

3. Zusammensetzung nach Anspruch 2, **dadurch gekennzeichnet, dass** die kationischen Direktfarbstoffe den Strukturen (I1), (I2), (I14) und (I31) entsprechen.

4. Zusammensetzung nach Anspruch 1, **dadurch gekennzeichnet, dass** die kationischen Direktfarbstoffe der Formel (II) unter den Verbindungen der folgenden Strukturen (II1) bis (II9) ausgewählt sind: 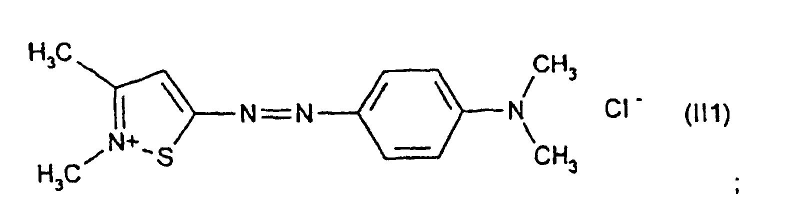 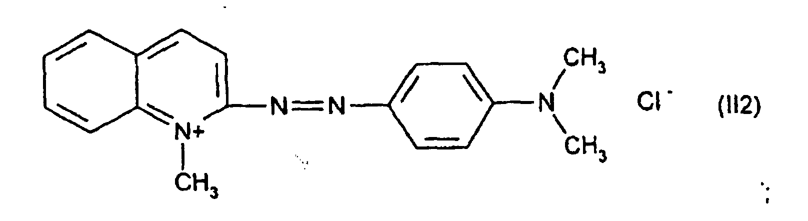 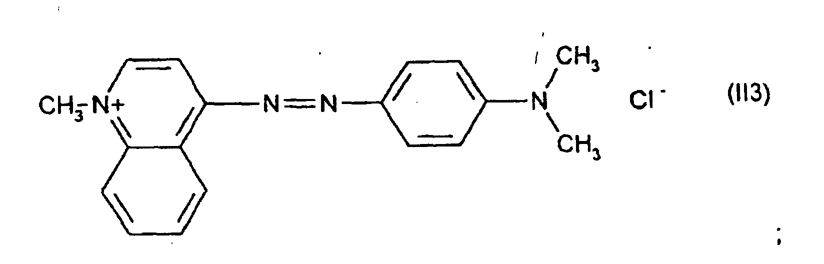 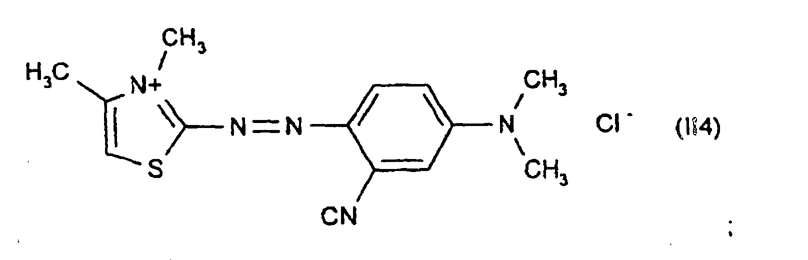 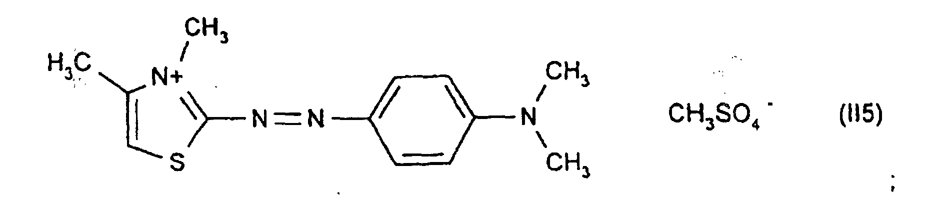 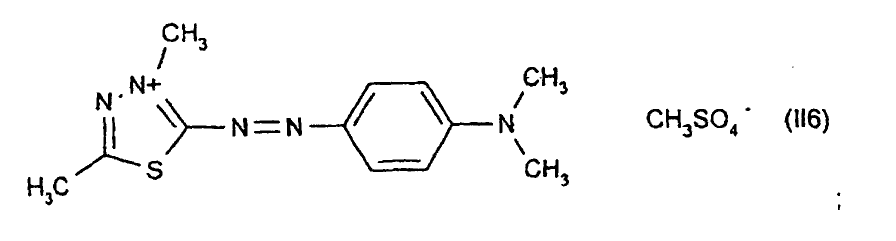 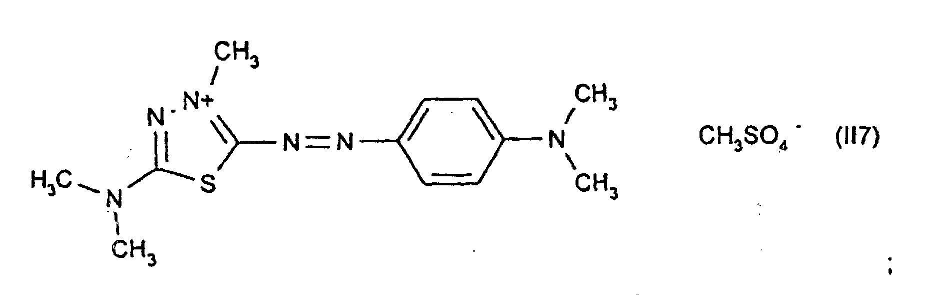 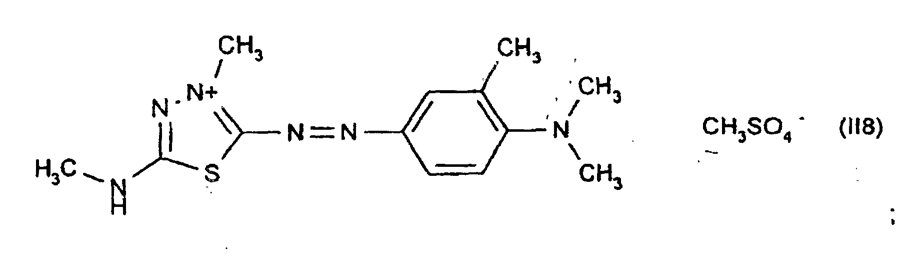 und

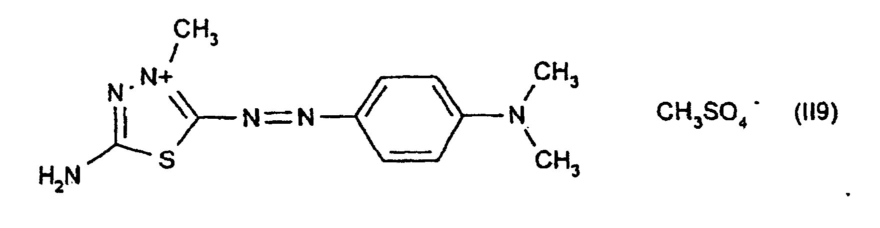

5. Zusammensetzung nach Anspruch 1, **dadurch gekennzeichnet, dass** die kationischen Direktfarbstoffe der Formel (III) unter den Verbindungen der folgenden Strukturen (III1) bis (III18) ausgewählt sind: 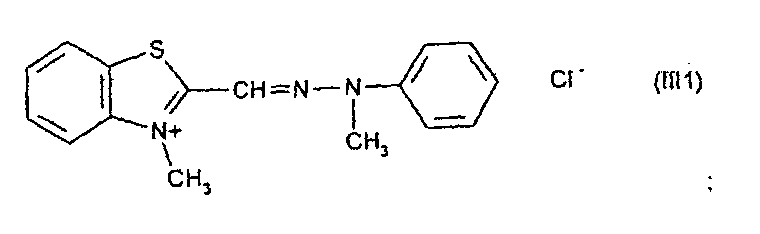 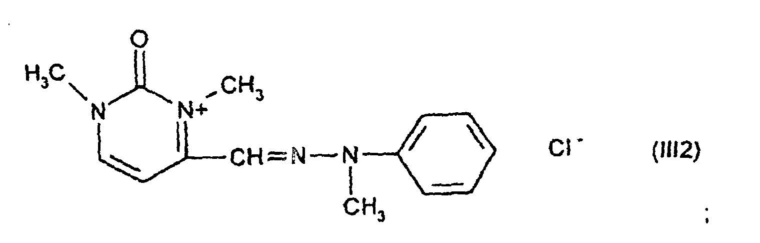 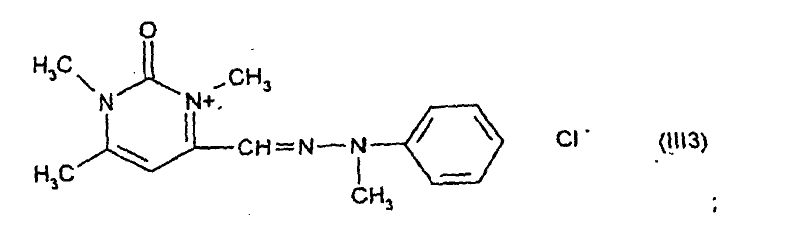 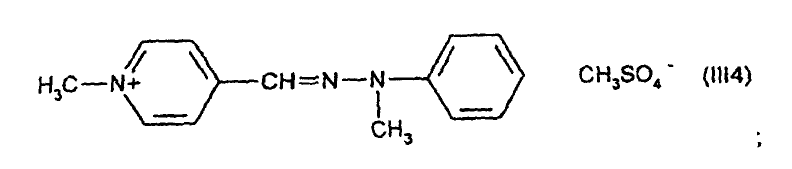 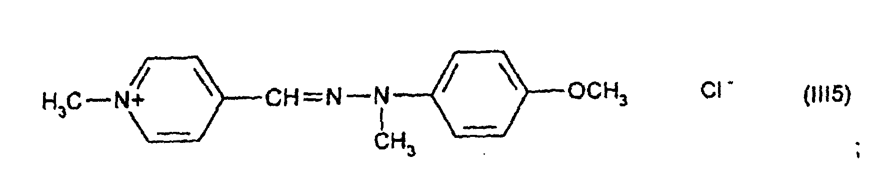 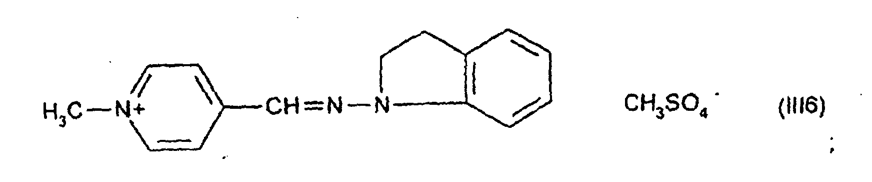 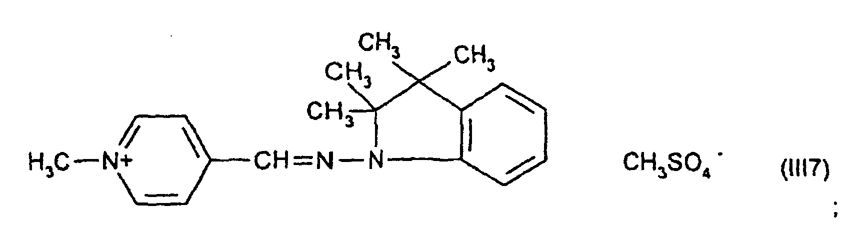 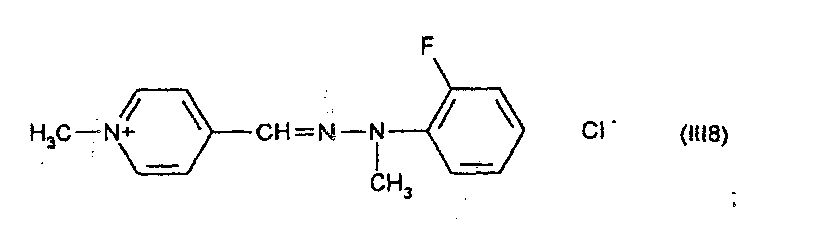 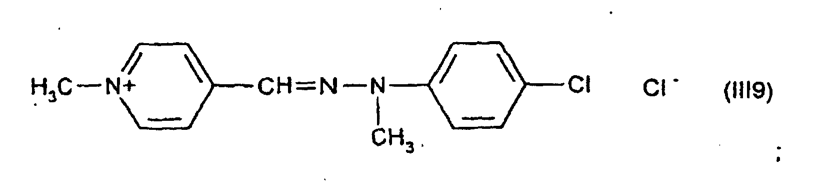 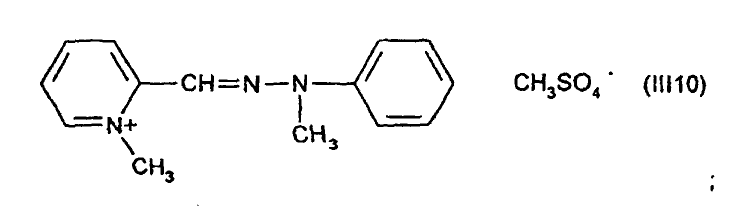 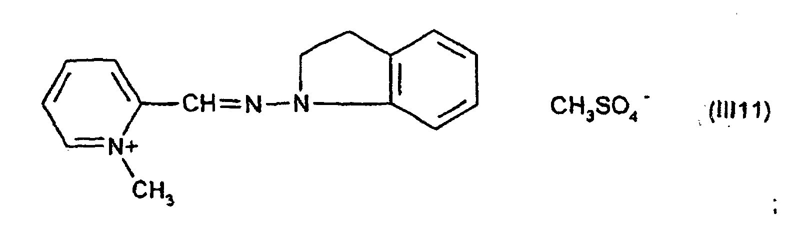 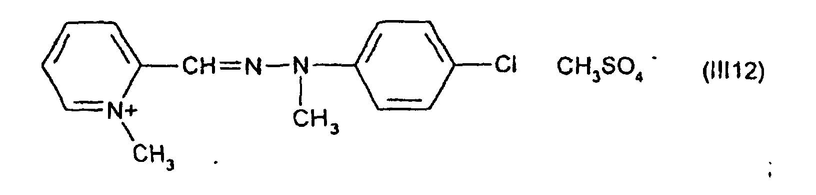 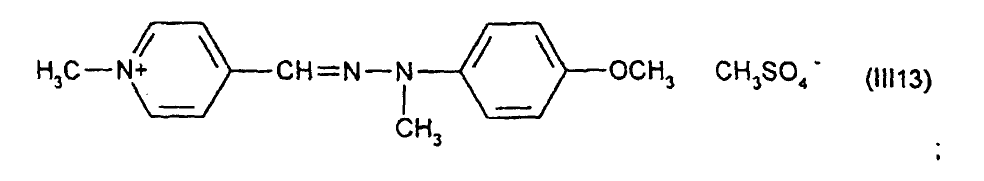 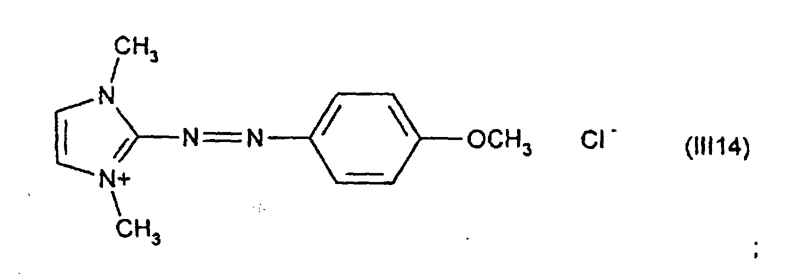 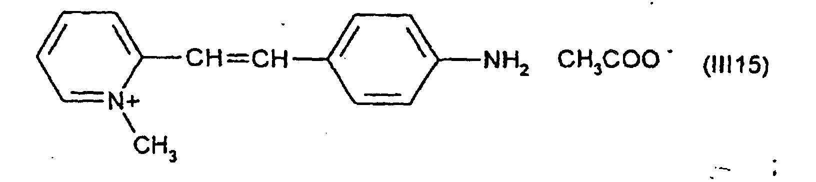 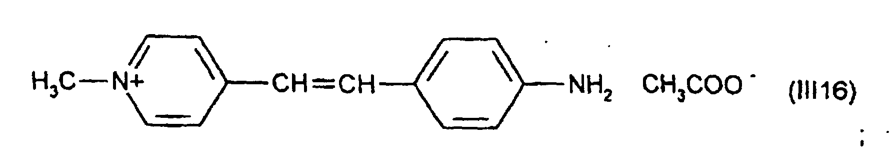 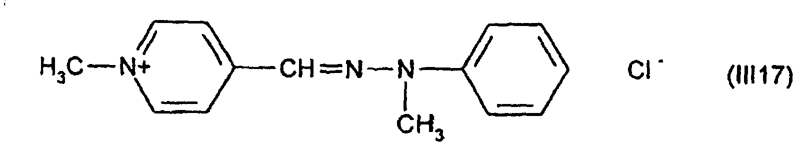 und

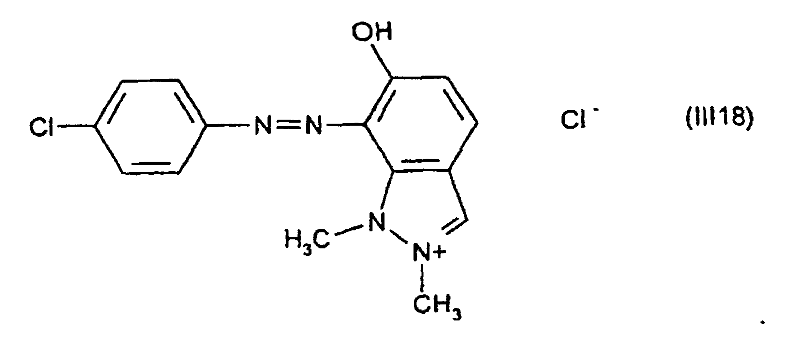

6. Zusammensetzung nach Anspruch 5, **dadurch gekennzeichnet, dass** die kationischen Direktfarbstoffe der Formel (III) den Strukturen (III4), (III5) und (III13) entsprechen.

7. Zusammensetzung nach Anspruch 1, **dadurch gekennzeichnet, dass** die kationischen Direktfarbstoffe der Formel (III') unter den Verbindungen der folgenden Strukturen (III'1) bis (III'3) ausgewählt sind: 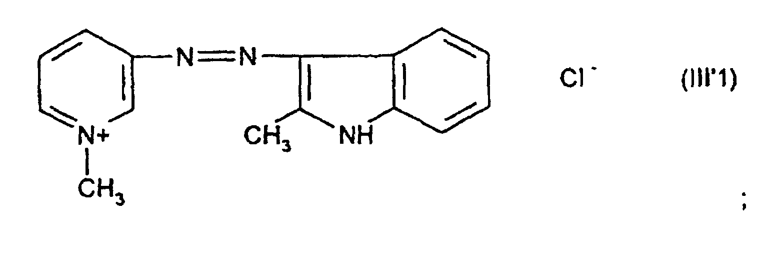 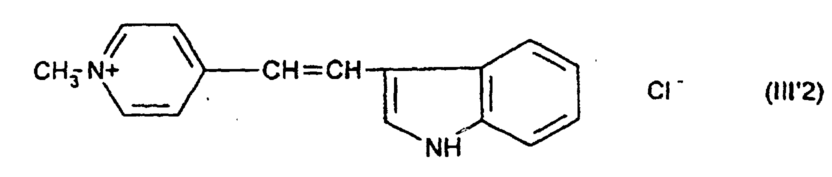 und

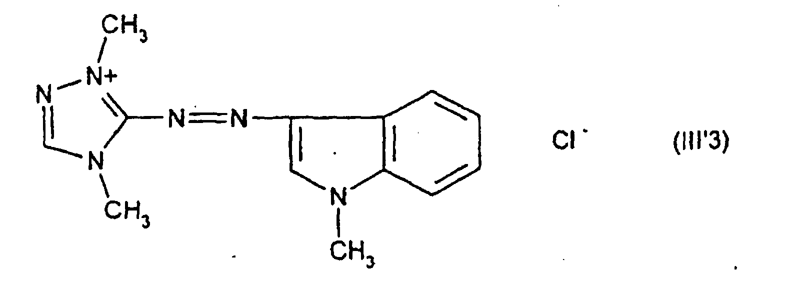

8. Zusammensetzung Anspruch 1, **dadurch gekennzeichnet, dass** die kationischen Direktfarbstoffe der Formel (IV) unter den Verbindungen der folgenden Strukturen (IV₁) bis (IV₇₇) ausgewählt sind:

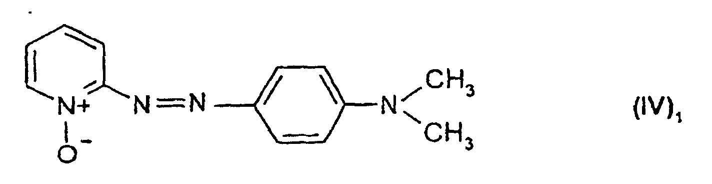

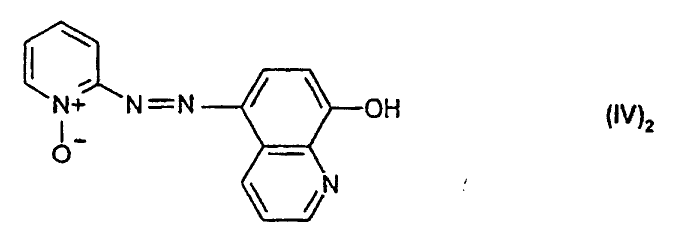

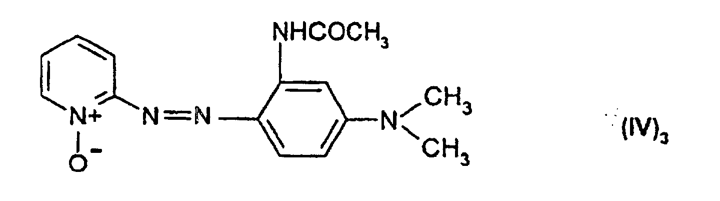

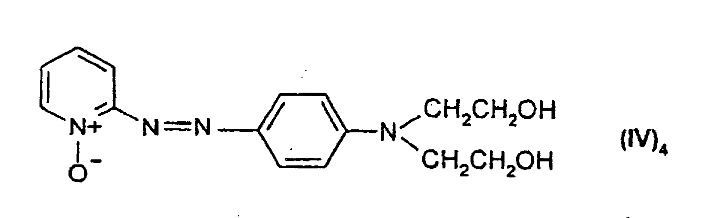

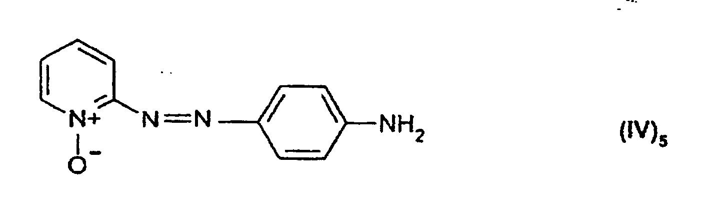

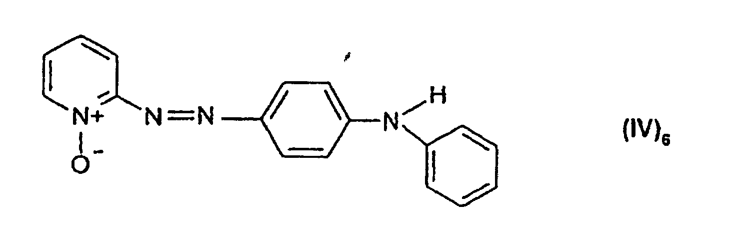

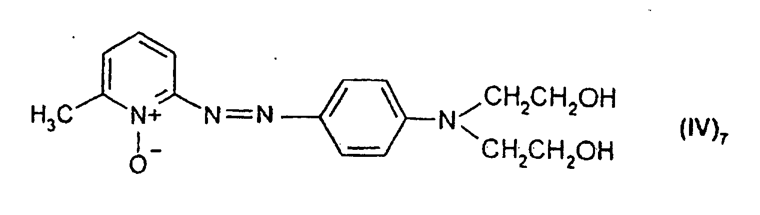

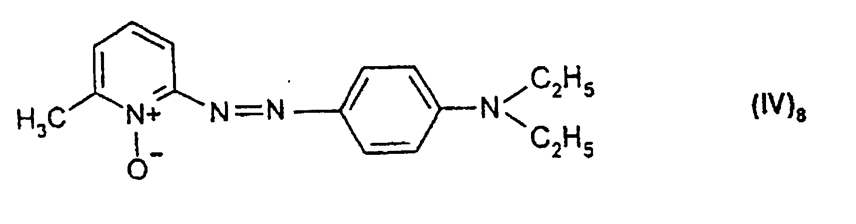

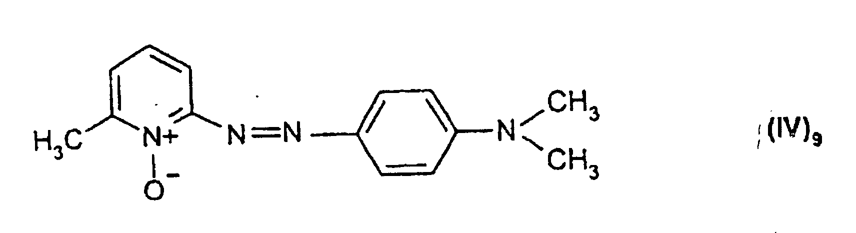

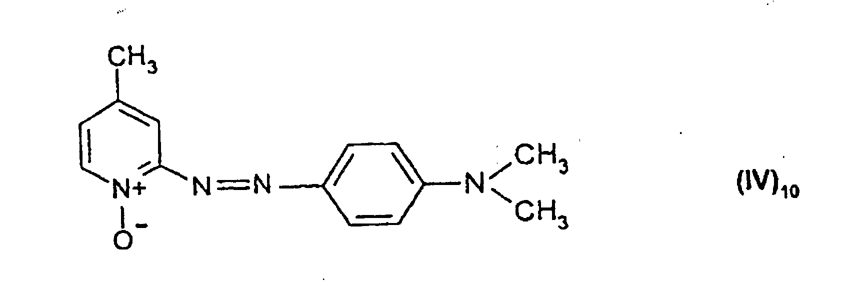

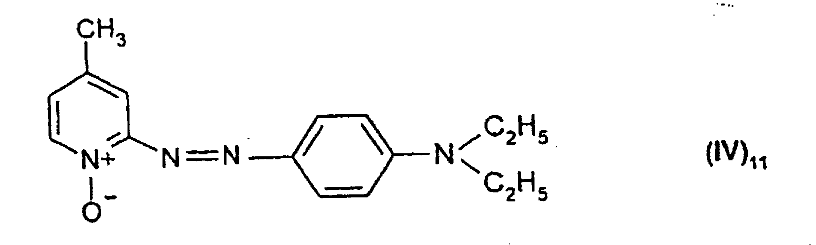

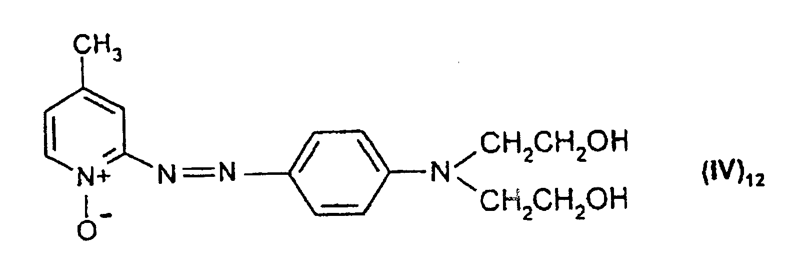

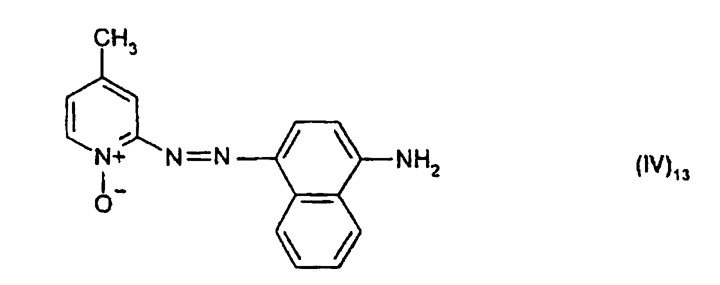

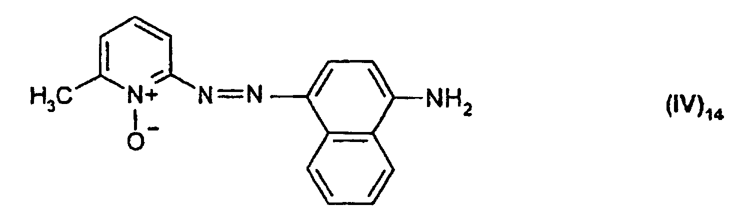

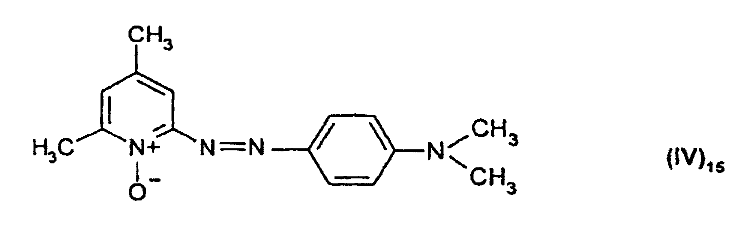

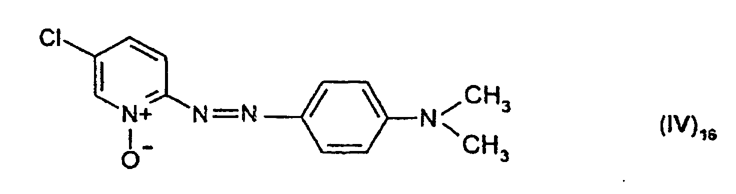

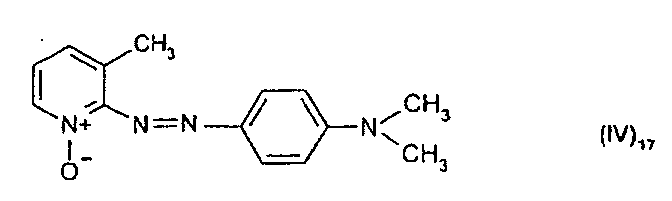

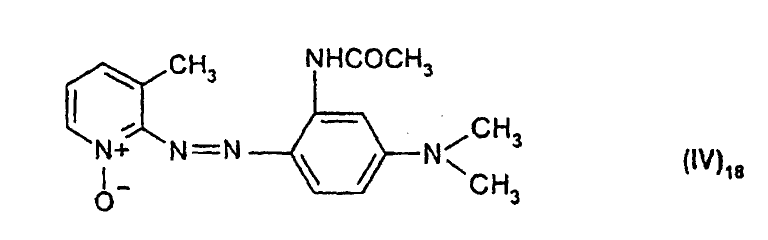

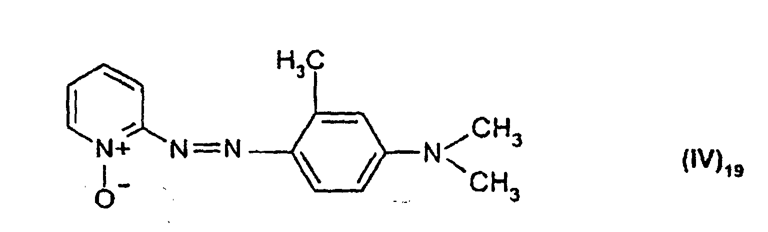

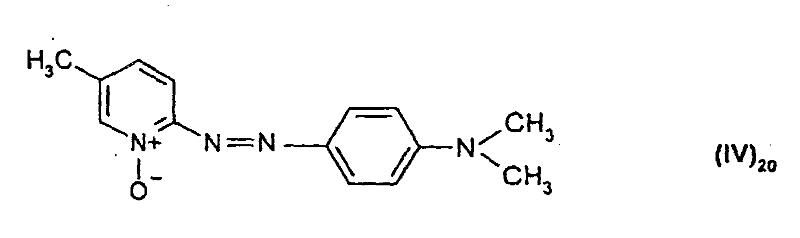

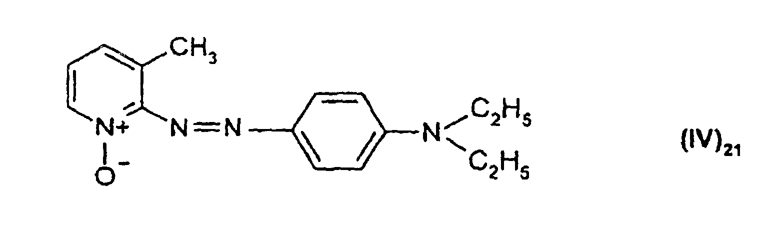

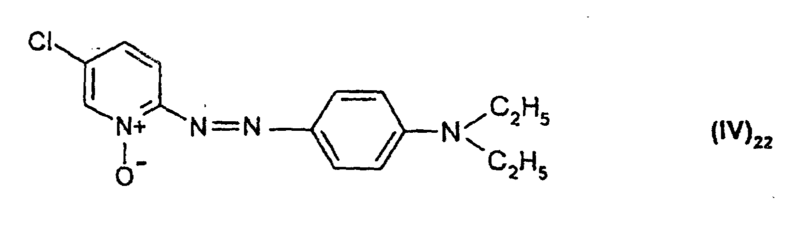

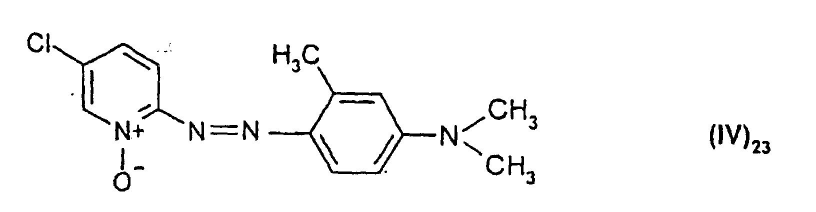

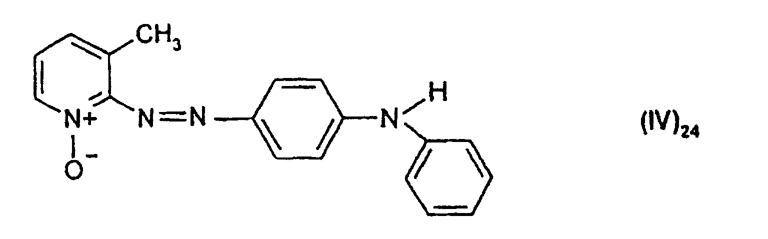

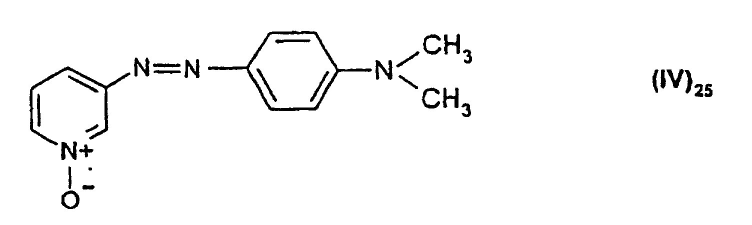

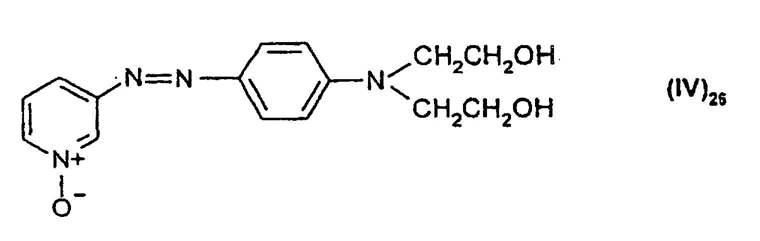

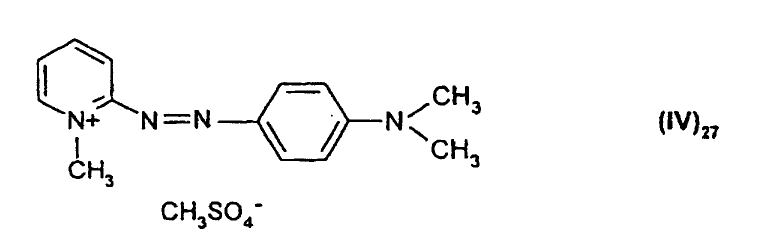

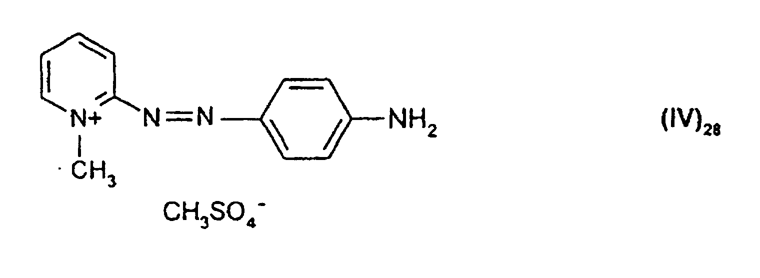

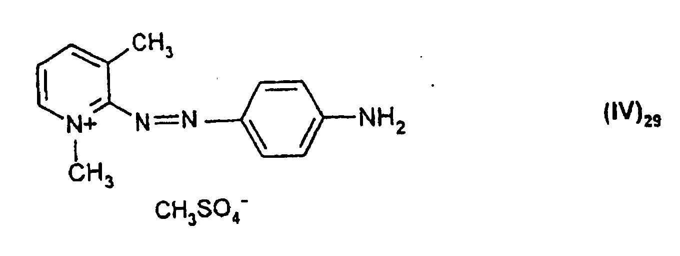

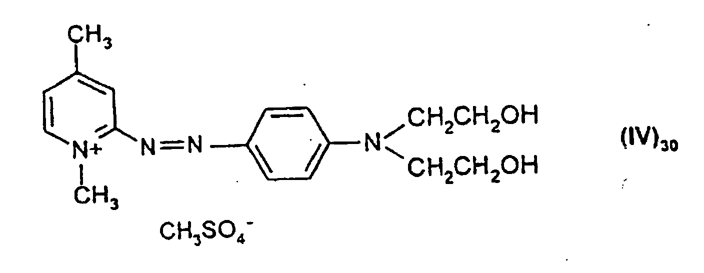

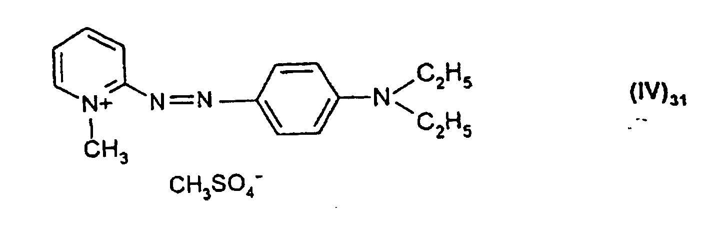

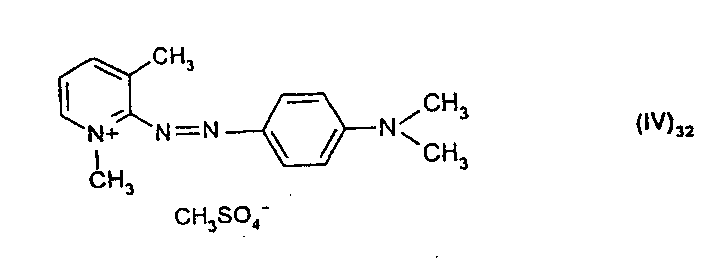

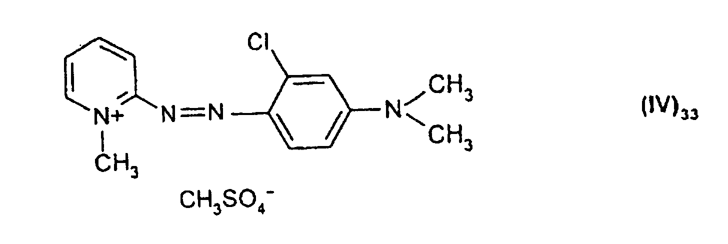

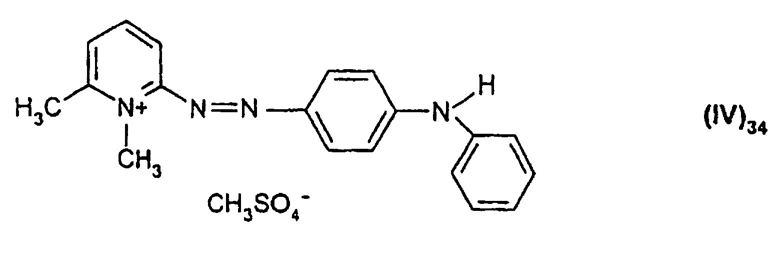

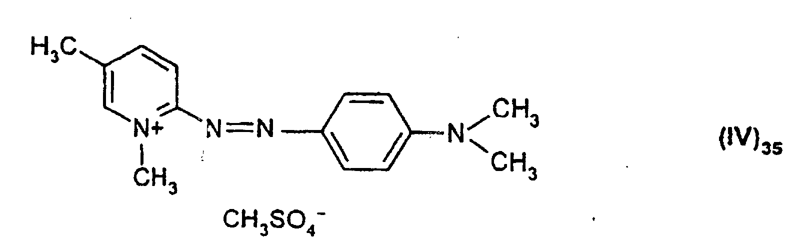

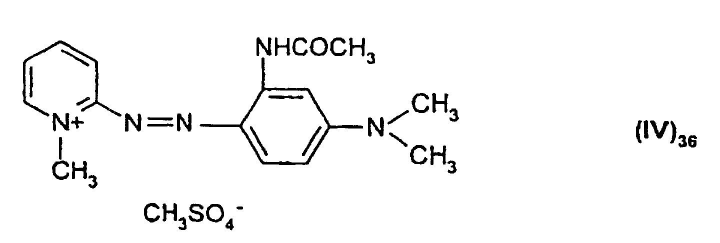

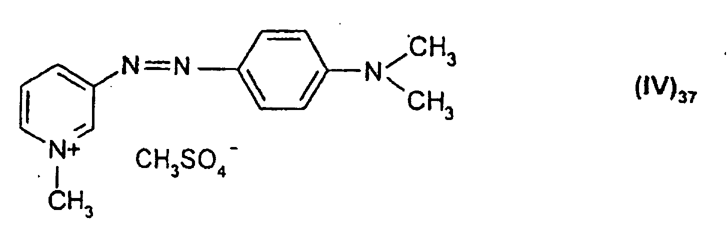

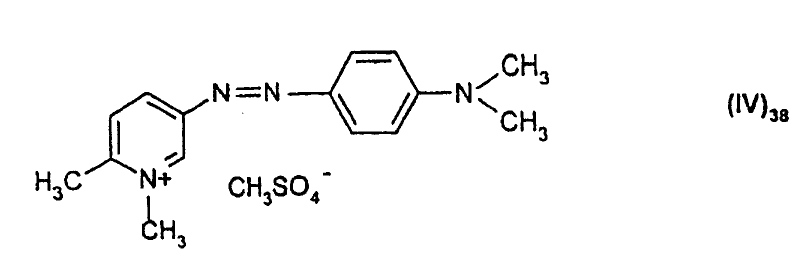

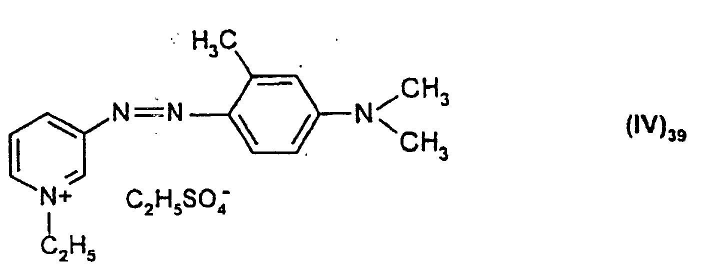

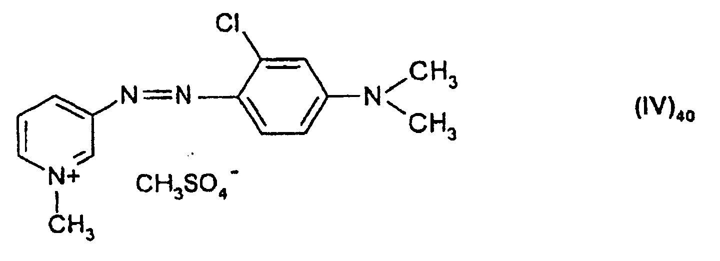

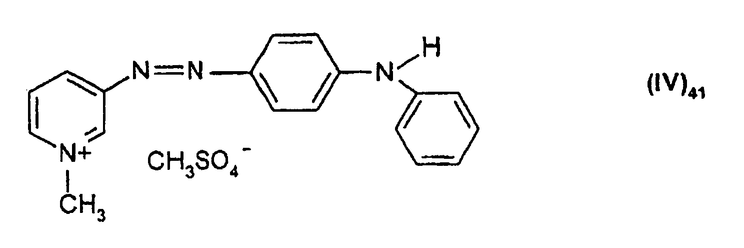

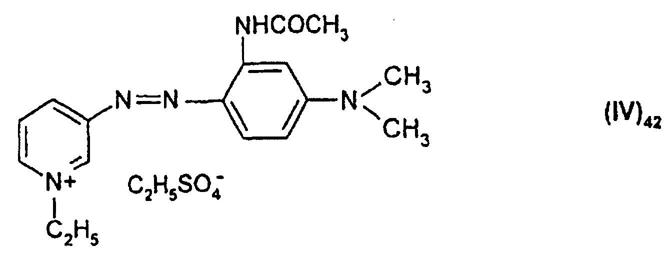

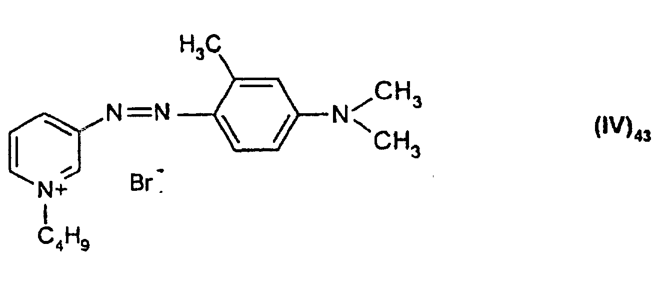

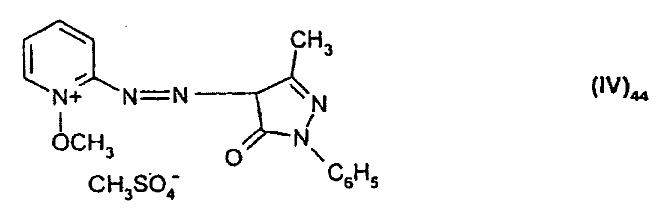

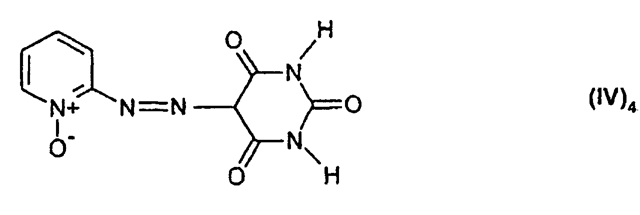

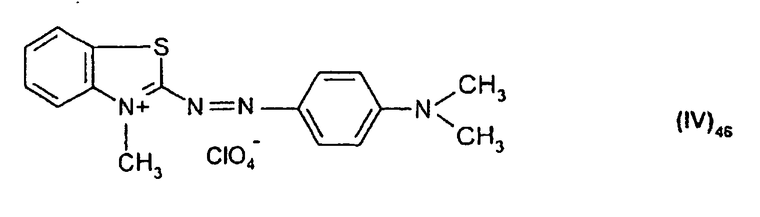

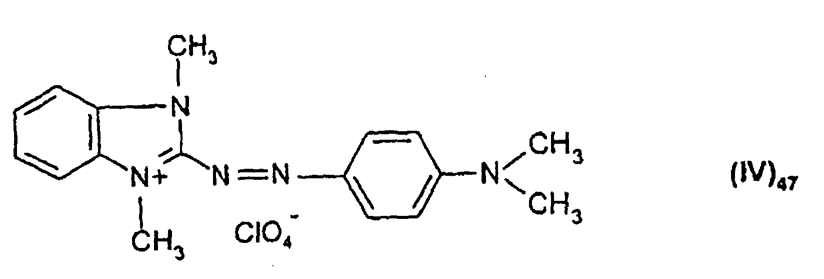

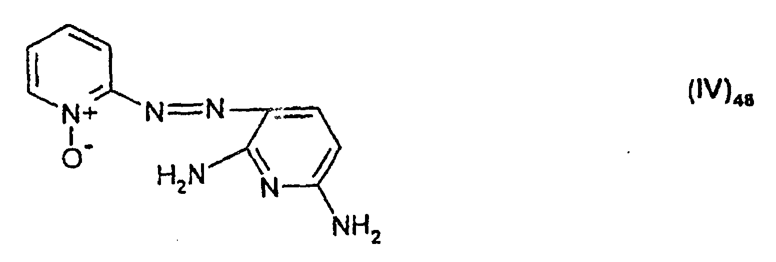

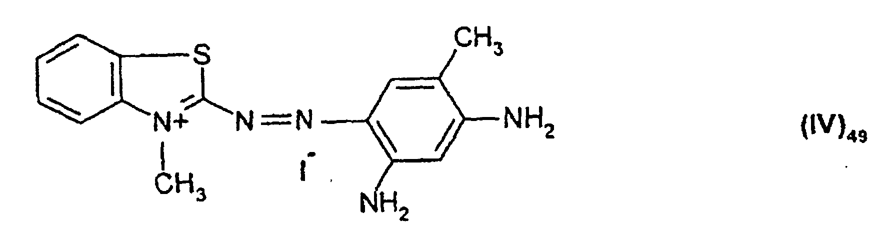

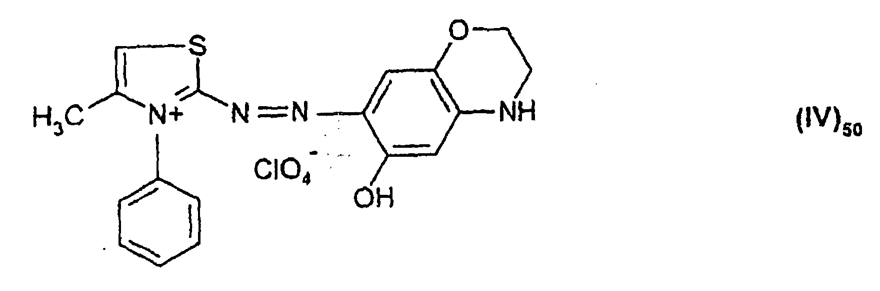

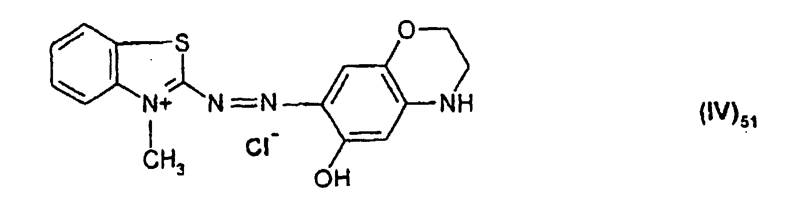

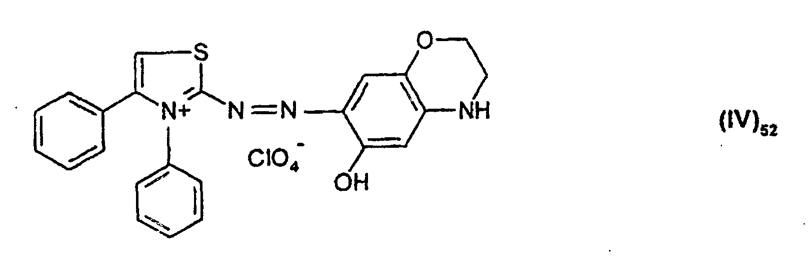

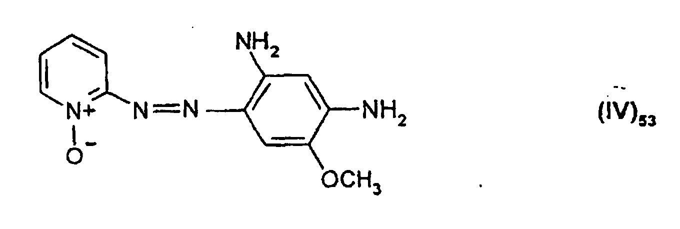

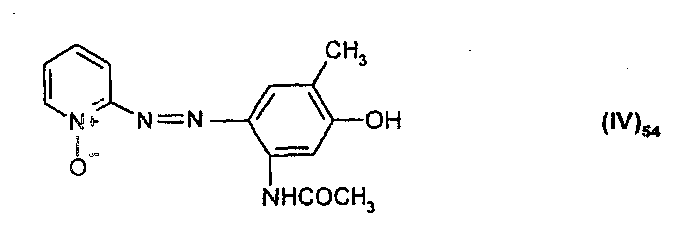

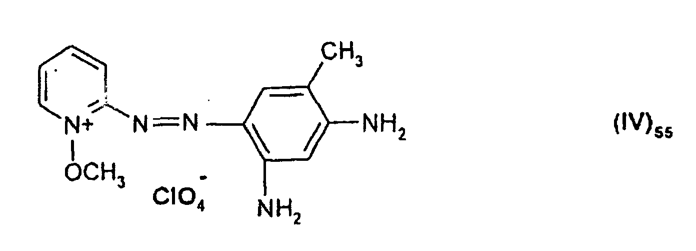

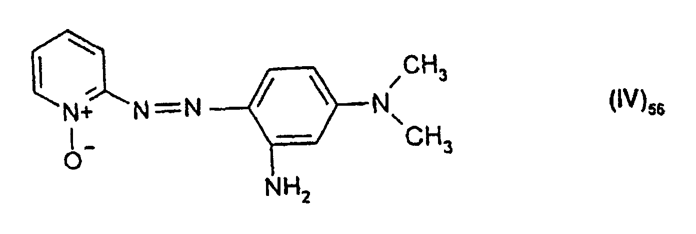

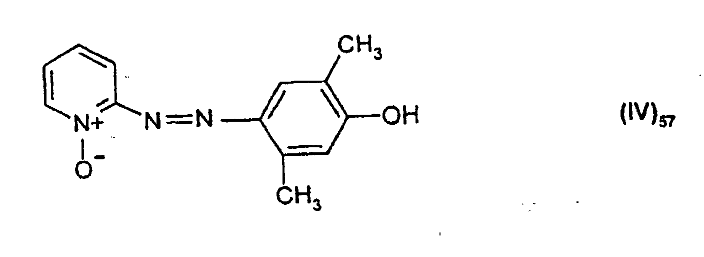

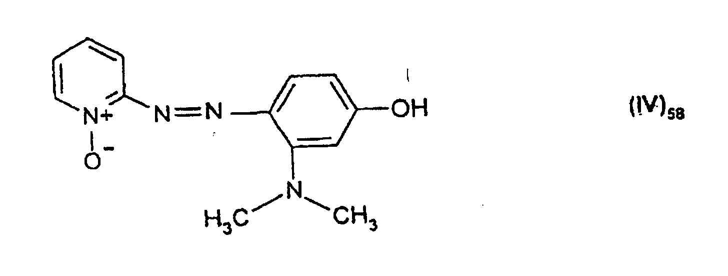

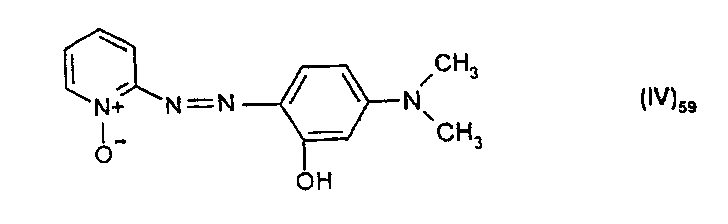

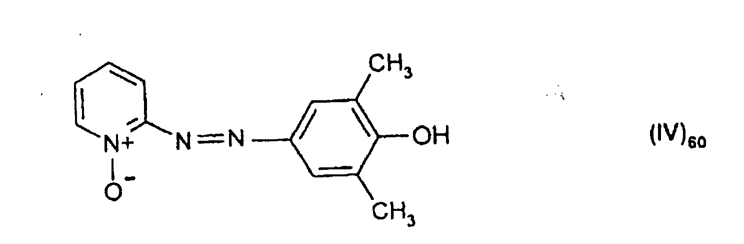

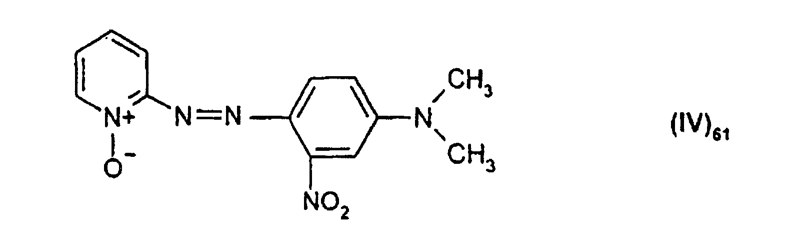

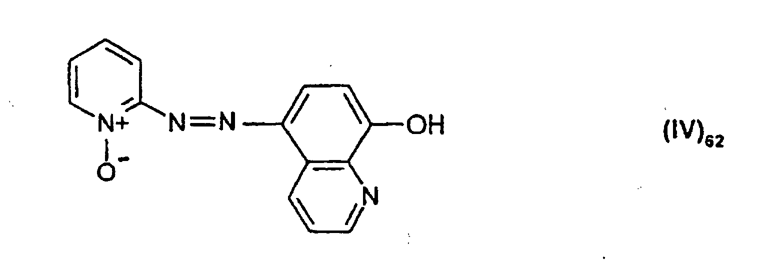

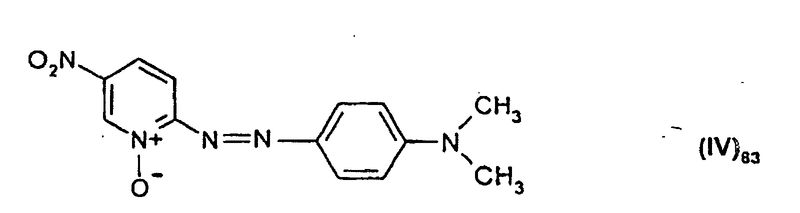

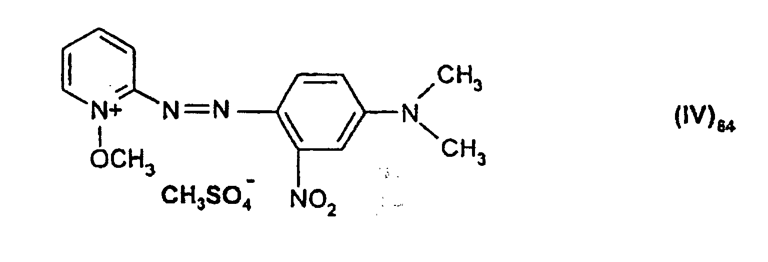

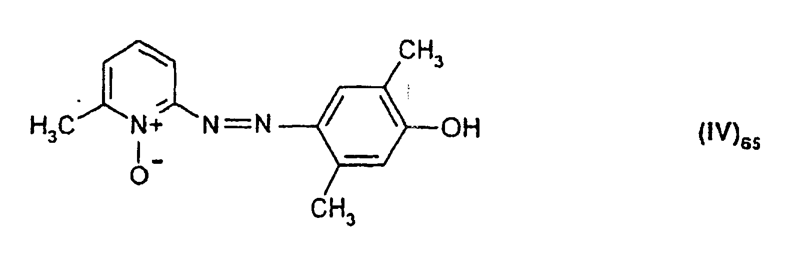

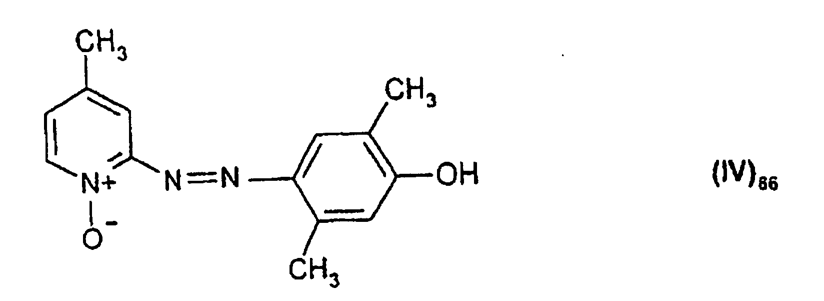

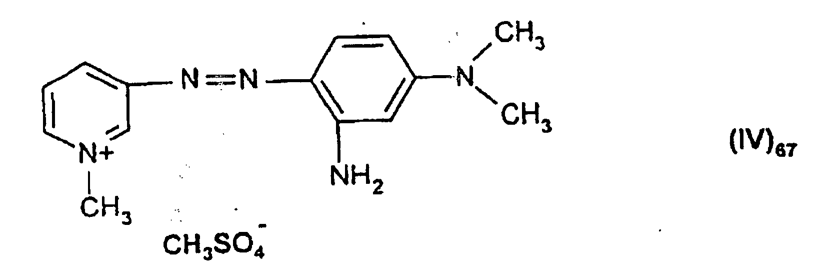

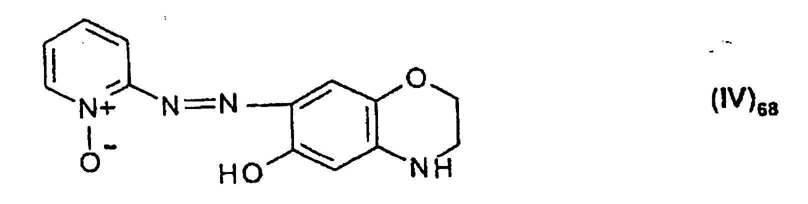

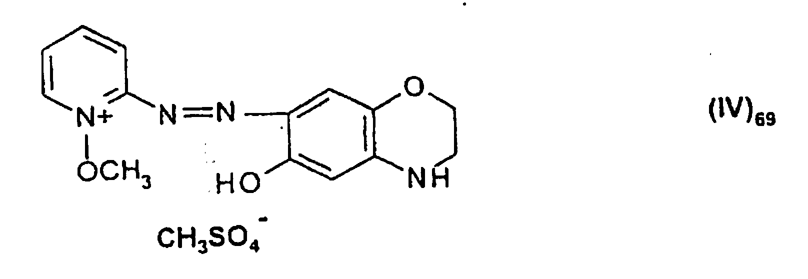

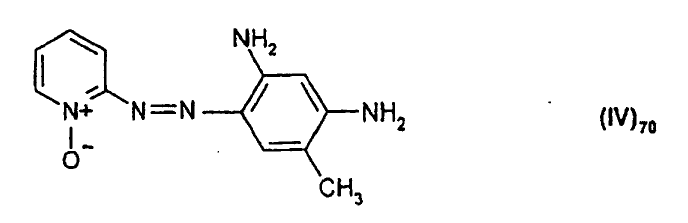

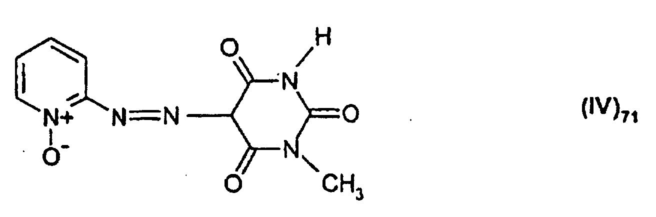

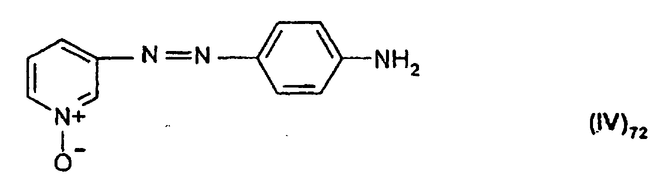

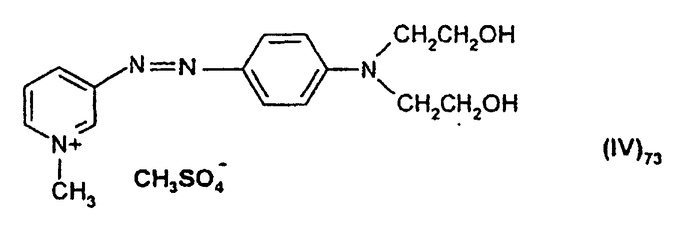

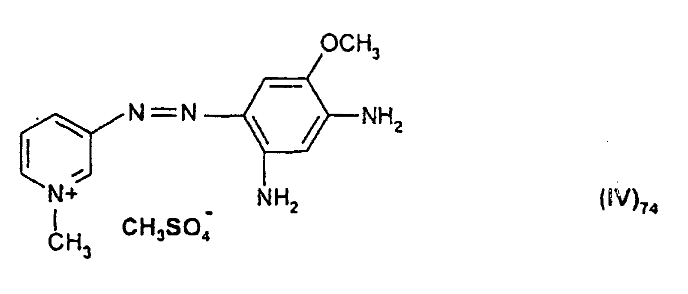

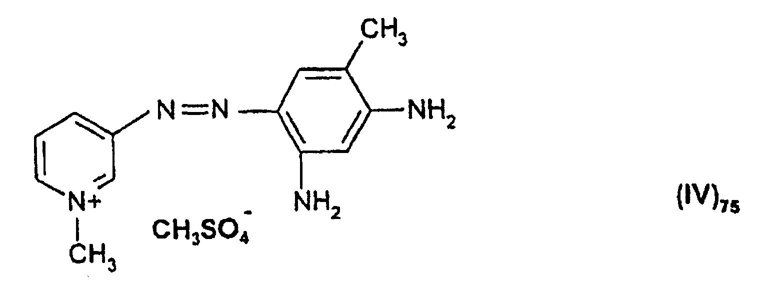

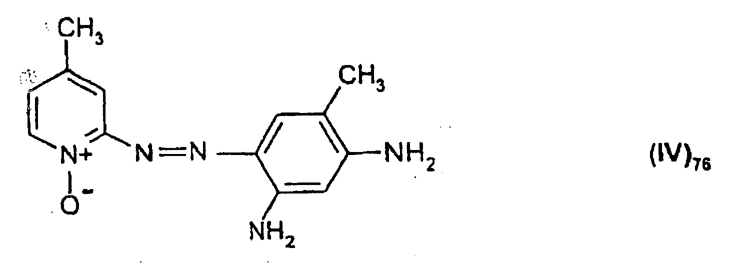

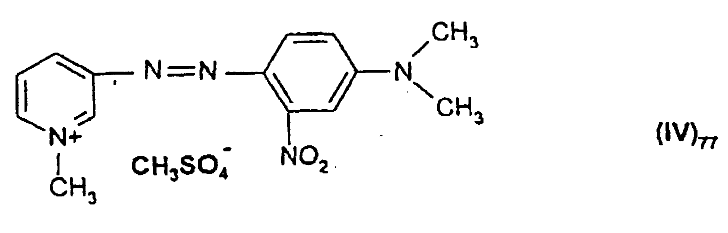

9. Zusammensetzung nach einem der vorhergehenden Ansprüche, **dadurch gekennzeichnet, dass** der oder die kationische(n) Direktfarbstoff(e) der Formeln (I), (II), (III), (III') oder (IV) 0,001 bis 10 Gew.-% des Gesamtgewichts der Zusammensetzung ausmachen.

10. Zusammensetzung nach Anspruch 9, **dadurch gekennzeichnet, dass** der oder die kationische(n) Direktfarbstoff(e) der Formeln (I), (II), (III), (III') oder (IV) 0,005 bis 5 Gew.-% des Gesamtgewichts der Zusammensetzung ausmachen.

11. Zusammensetzung nach einem der vorhergehenden Ansprüche, **dadurch gekennzeichnet, dass** das verdickende Polymer (ii) vom nichtionischen amphiphilen Typ, das mindestens eine hydrophile Einheit und mindestens eine Einheit mit Fettkette enthält, eine Cellulose ist, die mit Gruppen modifiziert ist, die mindestens eine Fettkette aufweisen.

12. Zusammensetzung nach Anspruch 11, **dadurch gekennzeichnet, dass** es sich um eine Hydroxyethylcellulose handelt, die mit Alkyl, Aralkyl oder Alkylaryl oder deren Gemischen modifiziert ist.

13. Zusammensetzung nach Anspruch 12, **dadurch gekennzeichnet, dass** in den Alkyl-, Aralkyl- oder Alkylarylgruppen die Alkylgruppe 8 bis 22 Kohlenstoffatome aufweist.

14. Zusammensetzung nach einem der vorhergehenden Ansprüche 11 bis 13, **dadurch gekennzeichnet, dass** die Hydroxyethylcellulose mit Gruppen modifiziert ist, die mindestens eine C₁₆-Alkylgruppe enthalten.

15. Zusammensetzung nach Anspruch 11, **dadurch gekennzeichnet, dass** es sich um eine Hydroxyethylcellulose handelt, die mit Gruppen modifiziert ist, die mindestens eine Alkylphenolpolyalkylenglykolethergruppe enthalten.

16. Zusammensetzung nach Anspruch 15, **dadurch gekennzeichnet, dass** es sich um eine Hydroxyethylcellulose handelt, die mit Gruppen modifiziert ist, die mindestens eine Nonylphenolpolyethylenglykol(15)ethergruppe enthalten.

17. Zusammensetzung nach einem der Ansprüche 1 bis 10, **dadurch gekennzeichnet, dass** das verdickende Polymer (ii) vom nichtionischen amphiphilen Typ, das mindestens eine hydrophile Einheit und mindestens eine Einheit mit Fettkette enthält, ein Urethanpolyether ist, der mindestens eine Alkyl- oder Alkenylgruppe mit 8 bis 30 Kohlenstoffatome enthält.

18. Zusammensetzung nach einem der Ansprüche 1 bis 10, **dadurch gekennzeichnet, dass** in dem verdickenden Polymer (ii) vom anionischen amphiphilen Typ die hydrophile Einheit aus einem ethylenisch ungesättigten Monomer besteht und die Einheit mit Fettkette ein Allylether mit Fettkette ist.

19. Zusammensetzung nach Anspruch 18, **dadurch gekennzeichnet, dass** das ethylenisch ungesättigte anionische Monomer eine Vinylcarbonsäure ist.

20. Zusammensetzung nach Anspruch 19, **dadurch gekennzeichnet, dass** es sich um Acrylsäure oder Methacrylsäure oder deren Gemische handelt.

21. Zusammensetzung nach einem der Ansprüche 18 bis 20, **dadurch gekennzeichnet, dass** die Allylethereinheit mit Fettkette dem Monomer der folgenden Formel (V) entspricht:
CH₂ = CR'CH₂ O Bₙ R (V),
worin bedeuten: R' H oder CH₃, B Ethylenoxy, n Null oder eine ganze Zahl von 1 bis 100, R eine Kohlenwasserstoffgruppe, die unter Alkyl und Cycloalkyl mit 8 bis 30 Kohlenstoffatomen ausgewählt ist.

22. Zusammensetzung nach Anspruch 21, **dadurch gekennzeichnet, dass** in der Formel (V) die Kohlenwasserstoffgruppe Alkyl bedeutet und 10 bis 24 Kohlenstoffatome enthält.

23. Zusammensetzung nach Anspruch 21 oder 22, **dadurch gekennzeichnet, dass** in der Formel (V) R' Wasserstoff, n 10 und R Stearyl bedeutet.

24. Zusammensetzung nach einem der Ansprüche 18 bis 23, **dadurch gekennzeichnet, dass** das anionische amphiphile Polymer durch Emulsionspolymerisation von 20 bis 60 Gew.-% Acrylsäure und/oder Methacrylsäure, 5 bis 60 Gew.-% Alkyl(meth)acrylaten, wobei es sich um niedere Alkylgruppen handelt, 2 bis 50 Gew.-% Allylether mit Fettkette der Formel (V) und 0 bis 1 Gew.-% Vernetzungsmittel gebildet wurde.

25. Zusammensetzung nach Anspruch 24, **dadurch gekennzeichnet, dass** es sich um ein vernetztes Polymer handelt, das 40 Gew.-% Methacrylsäure, 50 Gew.-% Ethylacrylat, 10 Gew.-% Polyethylenglykolether (10 EO) von Stearylalkohol (Steareth-10) enthält.

26. Zusammensetzung nach einem der Ansprüche 1 bis 10, **dadurch gekennzeichnet, dass** in dem verdickenden Polymer (ii) vom anionischen amphiphilen Typ die hydrophile Einheit aus einer olefinisch ungesättigten Carbonsäure besteht und die Einheit mit Fettkette ein Alkyl(C₁₀₋₃₀)ester einer ungesättigten Carbonsäure ist.

27. Zusammensetzung nach Anspruch 26, **dadurch gekennzeichnet, dass** die hydrophile Einheit dem Monomer der folgenden Formel (VI) entspricht: 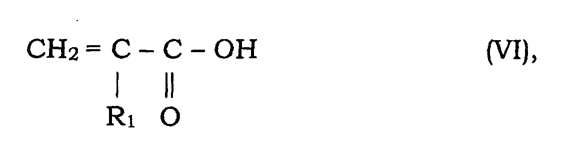 worin die Gruppe R₁ H oder CH₃ oder C₂H₅ bedeutet.

28. Zusammensetzung nach Anspruch 27, **dadurch gekennzeichnet, dass** es sich um Einheiten von Acrylsäure, Methacrylsäure oder deren Gemische handelt.

29. Zusammensetzung nach Anspruch 26, **dadurch gekennzeichnet, dass** die Einheit mit Fettkette ein Ester ist, der dem Monomer der folgenden Formel (VII) entspricht: 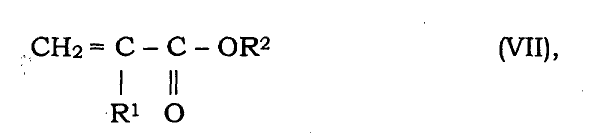 worin die Gruppe R¹ H oder CH₃ oder C₂H₅ und die Gruppe R² eine C₁₀₋₃₀-Alkylgruppe bedeutet.

30. Zusammensetzung nach Anspruch 29, **dadurch gekennzeichnet, dass** in der Formel (VII) R¹ H oder CH₃ bedeutet.

31. Zusammensetzung nach Anspruch 29 oder 30, **dadurch gekennzeichnet, dass** in der Formel (VII) R² C₁₂₋₂₂-Alkyl bedeutet.

32. Zusammensetzung nach einem der Ansprüche 26 bis 31, **dadurch gekennzeichnet, dass** das anionische amphiphile Polymer vernetzt ist.

33. Zusammensetzung nach einem der Ansprüche 26 bis 32, **dadurch gekennzeichnet, dass** das anionische amphiphile Polymer ein Polymer ist, das ausgehend von einem Monomerengemisch erhalten wird, das im Wesentlichen Acrylsäure, einen Ester der folgenden Formel (VII): 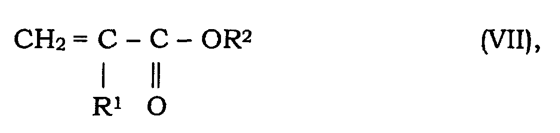 worin die Gruppe R¹ H oder CH₃ und R² eine Alkylgruppe mit 12 bis 22 Kohlenstoffatomen bedeutet, und ein Vernetzungsmittel enthält.

34. Zusammensetzung nach einem der Ansprüche 26 bis 33, **dadurch gekennzeichnet, dass** das anionische amphiphile Polymer ein Polymer von Acrylsäure und Laurylmethacrylat ist.

35. Zusammensetzung nach einem der vorhergehenden Ansprüche, **dadurch gekennzeichnet, dass** das verdickende Polymer (ii) 0,01 bis 10 Gew.-% des Gesamtgewichts der Zusammensetzung ausmacht.

36. Zusammensetzung nach Anspruch 35, **dadurch gekennzeichnet, dass** das verdickende Polymer (ii) 0,1 bis 5 Gew.-% des Gesamtgewichts der Zusammensetzung ausmacht.

37. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

38. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

39. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

40. Zusammensetzung nach Anspruch 39, **dadurch gekennzeichnet, dass** die Oxidationsbase(n) 0,0005 bis 12 Gew.-% des Gesamtgewichts der Farbmittelzusammensetzung ausmachen.

41. Zusammensetzung nach Anspruch 40, **dadurch gekennzeichnet, dass** die Oxidationsbase(n) 0,005 bis 6 Gew.-% des Gesamtgewichts der Farbmittelzusammensetzung ausmachen.

42. Zusammensetzung nach einem der Ansprüche 39 bis 41, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

43. Zusammensetzung nach Anspruch 42, **dadurch gekennzeichnet, dass** der oder die Kuppler 0,0001 bis 10 Gew.-% des Gesamtgewichts der Farbmittelzusammensetzung ausmachen.

44. Zusammensetzung nach Anspruch 43, **dadurch gekennzeichnet, dass** der oder die Kuppler 0,005 bis 5 Gew.-% des Gesamtgewichts der Farbmittelzusammensetzung ausmachen.

45. Zusammensetzung nach einem der vorhergehenden Ansprüche, **dadurch gekennzeichnet, dass** sie für die aufhellende Direktfärbung oder zum oxidativen Färben vorgesehen ist und mindestens ein Oxidationsmittel enthält.

46. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 45 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

47. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 45 während einer Zeitspanne, die zur Bildung der gewünschten Färbung ausreichend ist, aufgebracht wird, ohne dass nochmals gespült wird.

48. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A1) oder die Zusammensetzung (B1) das in den vorhergehenden Ansprüchen definierte verdickende Polymer (ii) enthält.

49. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten kationischen Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) das in den vorhergehenden Ansprüchen definierten verdickende Polymer (ii) enthält.

50. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 48 oder 49 und eine andere Abteilung die Zusammensetzung (B1) oder (B2) nach Anspruch 48 oder 49 enthält.

## Claims

1. Composition for dyeing keratin fibres, and in particular human keratin fibres such as the hair, containing, in a medium which is suitable for dyeing, **(i)** at least one compound chosen from those of formulae (I), (II), (III), (III') and (IV) below:
**a) the compounds of formula (I) below:** 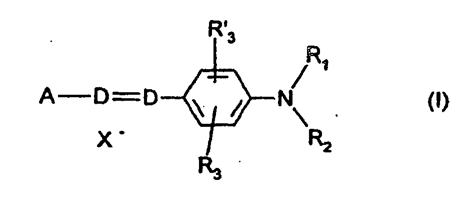 in which:
D represents a nitrogen atom or a -CH group,
R₁ and R₂, which may be identical or different, represent a hydrogen atom; a C₁-C₄ alkyl radical which can be substituted with a -CN, -OH or -NH₂ radical or form, with a carbon atom of the benzene ring, a heterocycle optionally containing oxygen or nitrogen, which can be substituted with one or more C₁-C₄ alkyl radicals; a 4'-aminophenyl radical,
R₃ and R'₃, which may be identical or different, represent a hydrogen atom, a halogen atom chosen from chlorine, bromine, iodine and fluorine, a cyano radical, or a C₁-C₄ alkyl, C₁-C₄ alkoxy or acetyloxy radical,
X⁻ represents an anion preferably chosen from chloride, methyl sulphate and acetate,
A represents a group chosen from the structures A1 to A18 below: 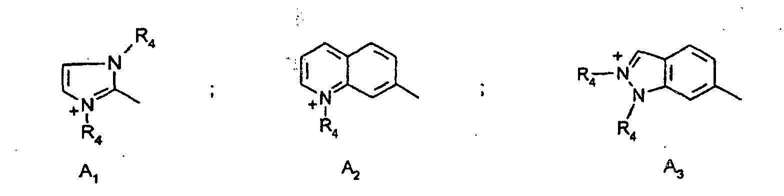 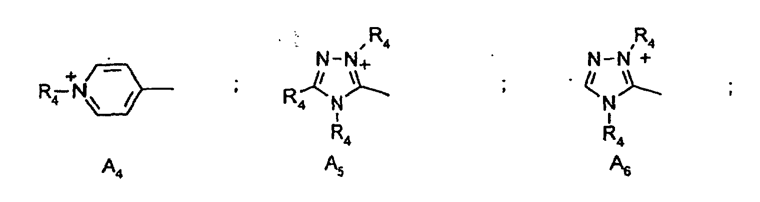 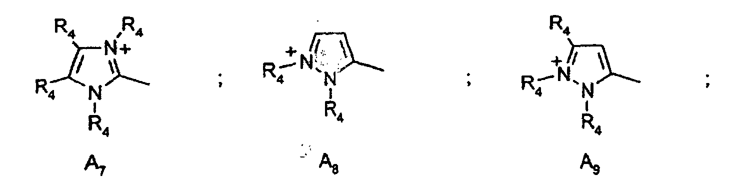 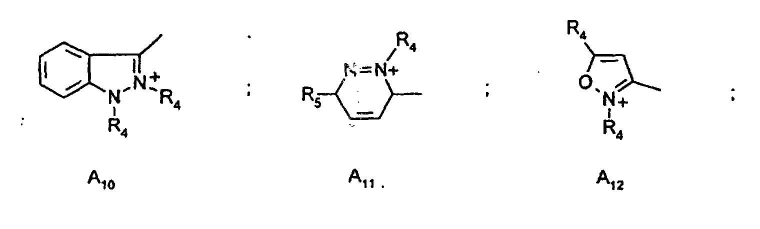 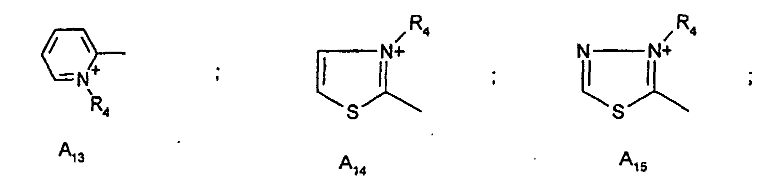 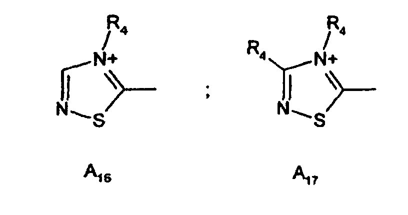 and 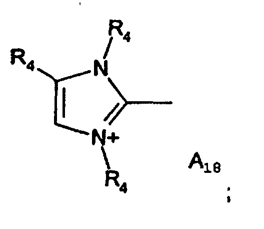 in which R₄ represents a C₁-C₄ alkyl radical which can be substituted with a hydroxyl radical and R₅ represents a C₁-C₄ alkoxy radical, with the proviso that when D represents -CH, when A represents A₄ or A₁₃ and when R₃ is other than an alkoxy radical, then R₁ and R₂ do not simultaneously denote a hydrogen atom;
**b) the compounds of formula (II) below:** 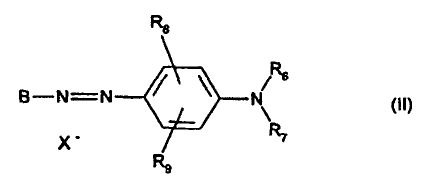 in which:
R₆ represents a hydrogen atom or a C₁-C₄ alkyl radical,
R₇ represents a hydrogen atom, an alkyl radical which can be substituted with a -CN radical or with an amino group, a 4'-aminophenyl radical or forms with R₆ a heterocycle optionally containing oxygen and/or nitrogen, which can be substituted with a C₁-C₄ alkyl radical,
R₈ and R₉, which may be identical or different, represent a hydrogen atom, a halogen atom such as bromine, chlorine, iodine or fluorine, a C₁-C₄ alkyl or C₁-C₄ alkoxy radical or a -CN radical,
X- represents an anion preferably chosen from chloride, methyl sulphate and acetate,
B represents a group chosen from the structures B1 to B6 below: 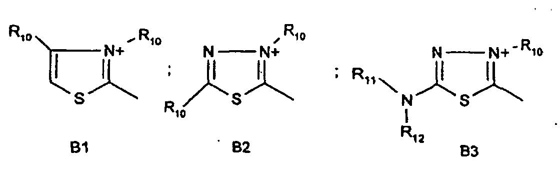 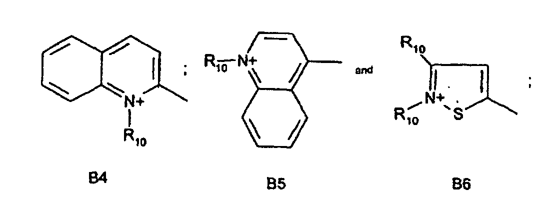 in which R₁₀ represents a C₁-C₄ alkyl radical, R₁₁ and R₁₂, which may be identical or different, represent a hydrogen atom or a C₁-C₄ alkyl radical;
**c) the compounds of formulae (III) and (III') below:** 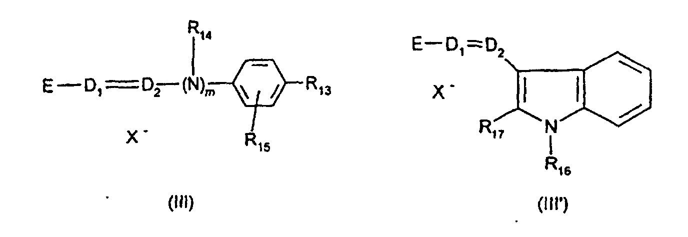 in which:
R₁₃ represents a hydrogen atom, a C₁-C₄ alkoxy radical, a halogen atom such as bromine, chlorine, iodine or fluorine, or an amino radical,
R₁₄ represents a hydrogen atom, a C₁-C₄ alkyl radical or forms, with a carbon atom of the benzene ring, a heterocycle optionally containing oxygen and/or substituted with one or more C₁-C₄ alkyl groups,
R₁₅ represents a hydrogen atom or a halogen atom such as bromine, chlorine, iodine or fluorine,
R₁₆ and R₁₇, which may be identical or different, represent a hydrogen atom or a C₁-C₄ alkyl radical,
D₁ and D₂, which may be identical or different, represent a nitrogen atom or a -CH group,
m = 0 or 1,
it being understood that when R₁₃ represents an unsubstituted amino group, then D₁ and D₂ simultaneously represent a -CH group and m = 0,
X⁻ represents an anion preferably chosen from chloride, methyl sulphate and acetate,
E represents a group chosen from the structures E1 to E8 below: 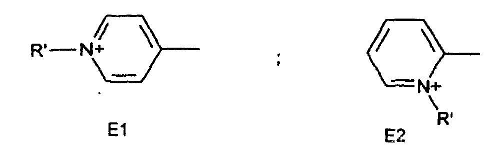 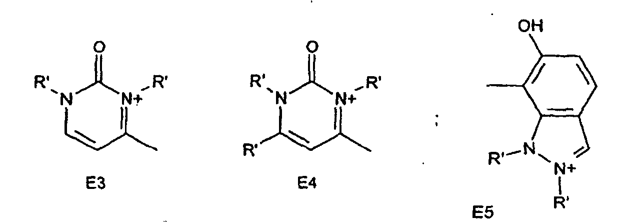 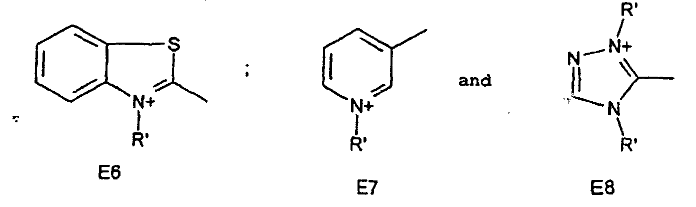 in which R' represents a C₁-C₄ alkyl radical;
when m = 0 and when D₁ represents a nitrogen atom, then E can also denote a group of structure E9 below: 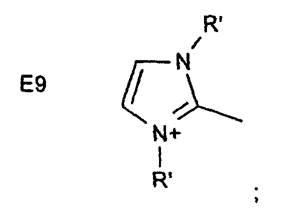 in which R' represents a C₁-C₄ alkyl radical;
**d) the compounds of formula (IV) below:**
G-N = N-J (IV)
in which:
**the symbol G** represents a group chosen from the structures G₁ to G₃ below: 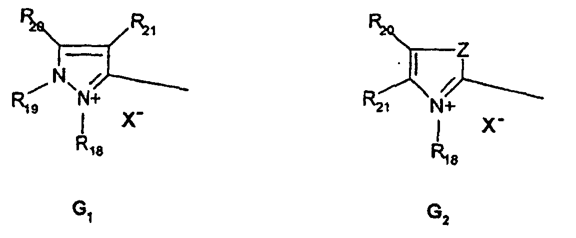 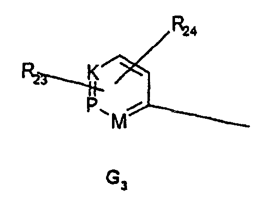 in which structures G₁ to G₃,
R₁₈ denotes a C₁-C₄ alkyl radical, a phenyl radical which can be substituted with a C₁-C₄ alkyl radical or a halogen atom chosen from chlorine, bromine, iodine and fluorine;
R₁₉ denotes a C₁-C₄ alkyl radical or a phenyl radical;
R₂₀ and R₂₁, which may be identical or different, represent a C₁-C₄ alkyl radical, a phenyl radical or together form, in G₁, a benzene ring substituted with one or more C₁-C₄ alkyl, C₁-C₄ alkoxy or NO₂ radicals or together form, in G₂, a benzene ring optionally substituted with one or more C₁-C₄ alkyl, C₁-C₄ alkoxy or NO₂ radicals;
R₂₀ can also denote a hydrogen atom;
Z denotes an oxygen or sulphur atom or a group -NR₁₉;
M represents a -CH, -CR (R denoting C₁-C₄ alkyl) or -NR₂₂(X⁻)ᵣ group;
K represents a -CH, -CR (R denoting C₁-C₄ alkyl) or -NR₂₂(X⁻)ᵣ group;
P represents a -CH, -CR (R denoting C₁-C₄ alkyl) or -NR₂₂(X⁻)ᵣ group; r denotes zero or 1;
R₂₂ represents an atom O⁻, a C₁-C₄ alkoxy radical or a C₁-C₄ alkyl radical;
R₂₃ and R₂₄, which may be identical or different, represent a hydrogen atom, a halogen atom chosen from chlorine, bromine, iodine and fluorine, a C₁-C₄ alkyl or C₁-C₄ alkoxy radical or an -NO₂ radical;
X⁻ represents an anion preferably chosen from chloride, iodide, methyl sulphate, ethyl sulphate, acetate and perchlorate;
with the proviso that,
if R₂₂ denotes O⁻, then r denotes zero;
if K or P or M denote N-C₁-C₄ alkyl X⁻, then R₂₃ or R₂₄ is other than a hydrogen atom;
if K denotes -NR₂₂(X⁻)ᵣ, then M=P= -CH, -CR;
if M denotes -NR₂₂(X⁻)ᵣ, then K=P= -CH, -CR;
if P denotes -NR₂₂(X⁻)ᵣ, then K=M and denote -CH or -CR;
if Z denotes a sulphur atom with R₂₁ denoting C₁-C₄ alkyl, then R₂₀ is other than a hydrogen atom;
if Z denotes -NR₁₉ with R₁₉ denoting C₁-C₄ alkyl, then at least one of the radicals R₁₈, R₂₀ or R₂₁ of G₂ is other than a C₁-C₄ alkyl radical;
**the symbol J** represents:
- **(a)** a group of structure J₁ below: 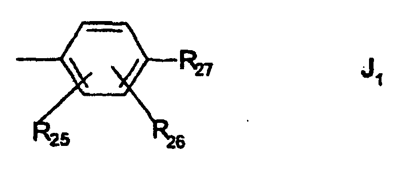 in which structure J₁,
R₂₅ represents a hydrogen atom, a halogen atom chosen from chlorine, bromine, iodine and fluorine, a C₁-C₄ alkyl or C₁-C₄ alkoxy radical, a radical -OH, -NO₂, -NHR₂₈, -NR₂₉R₃₀, -NHCO (C₁-C₄) alkyl, or forms with R₂₆ a 5- or 6-membered ring which may or may not contain one or more hetero atoms chosen from nitrogen, oxygen and sulphur;
R₂₆ represents a hydrogen atom, a halogen atom chosen from chlorine, bromine, iodine and fluorine, a C₁-C₄ alkyl or C₁-C₄ alkoxy radical or forms, with R₂₇ or R₂₈, a 5- or 6-membered ring which may or may not contain one or more hetero atoms chosen from nitrogen, oxygen and sulphur;
R₂₇ represents a hydrogen atom, an -OH radical, a radical -NHR₂₈ or a radical -NR₂₉R₃₀;
R₂₈ represents a hydrogen atom, a C₁-C₄ alkyl radical, a C₁-C₄ monohydroxyalkyl radical, a C₂-C₄ polyhydroxyalkyl radical or a phenyl radical;
R₂₉ and R₃₀, which may be identical or different, represent a C₁-C₄ alkyl radical, a C₁-C₄ monohydroxyalkyl radical or a C₂-C₄ polyhydroxyalkyl radical;
- **(b)** a 5- or 6-membered nitrogenous heterocyclic group which can contain other hetero atoms and/or carbonyl groups and which can be substituted with one or more C₁-C₄ alkyl, amino or phenyl radicals, and in particular a group of structure J₂ below: 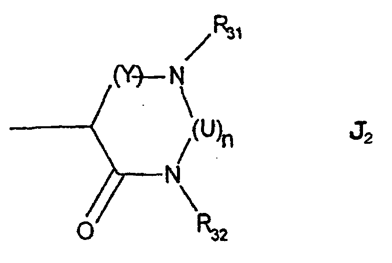
in which structure J₂,
R₃₁ and R₃₂, which may be identical or different, represent a hydrogen atom, a C₁-C₄ alkyl radical or a phenyl radical;
Y denotes the -CO- radical or the radical 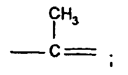 n = 0 or 1, with, when n denotes 1, U denoting a -CO- radical,
the said composition being **characterized in that** it also contains
**(ii)** at least one thickening polymer chosen from the group comprising:
**(ii)**_{**1**} **-** nonionic amphiphilic polymers comprising at least one hydrophilic unit and at least one unit containing a fatty chain;
**(ii)**_{**2**} **-** anionic amphiphilic polymers comprising at least one hydrophilic unit and at least one unit containing a fatty chain;
**(ii)**_{**3**} - cationic amphiphilic polymers comprising at least one hydrophilic unit and at least one unit containing a fatty chain.

2. Composition according to Claim 1, **characterized in that** the cationic direct dyes of formula (I) are chosen from the compounds corresponding to the structures (I1) to (I54) below:

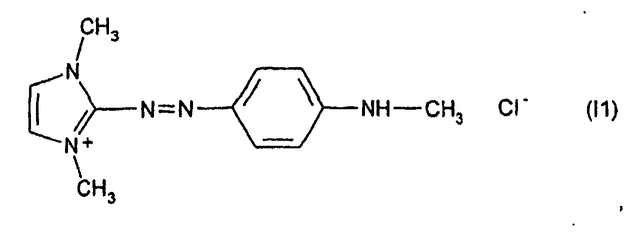

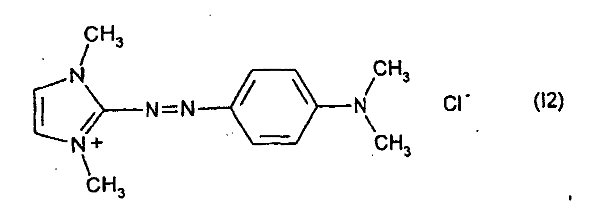

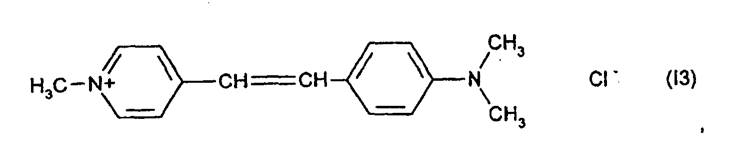

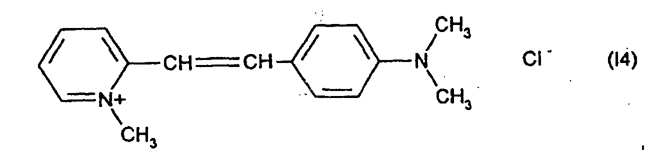

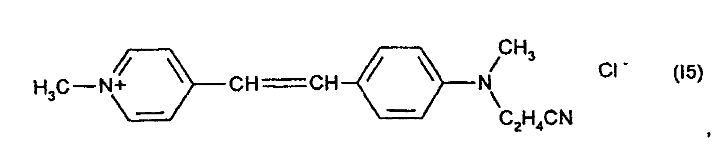

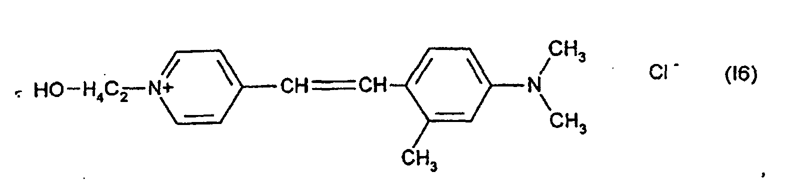

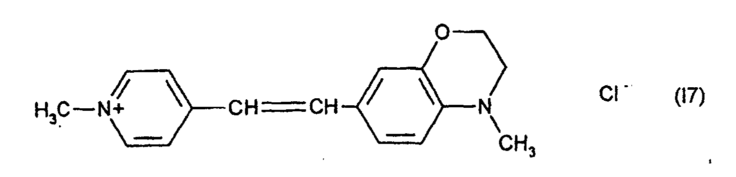

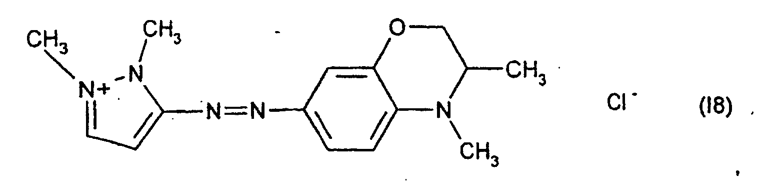

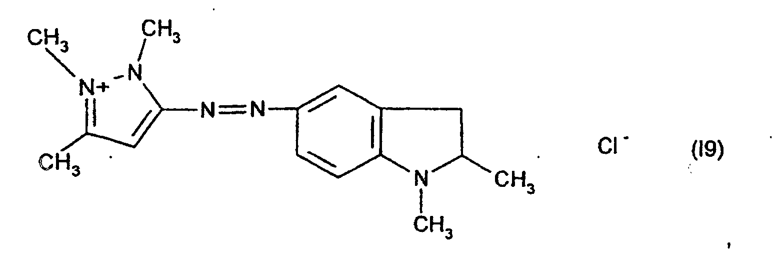

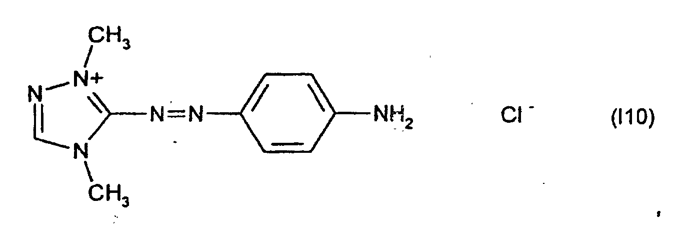

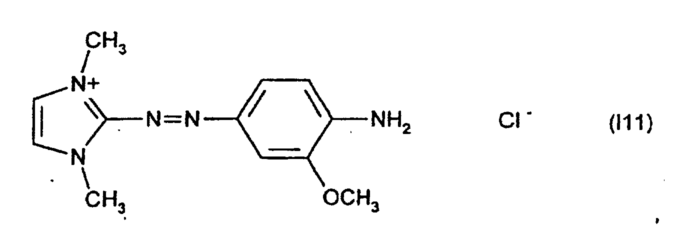

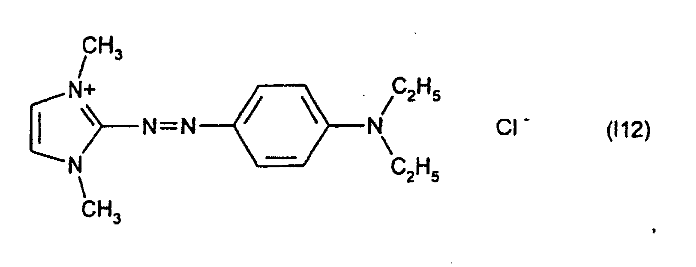

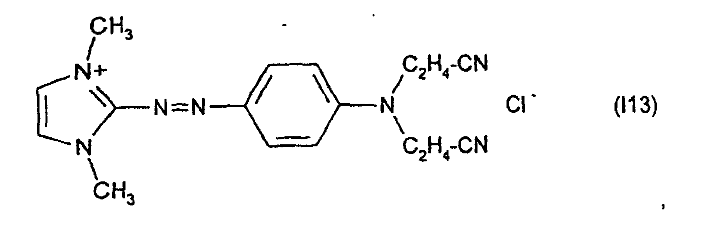

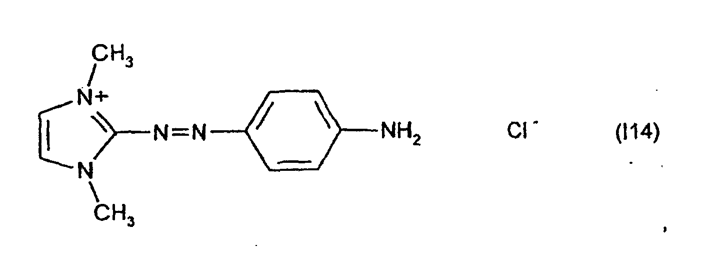

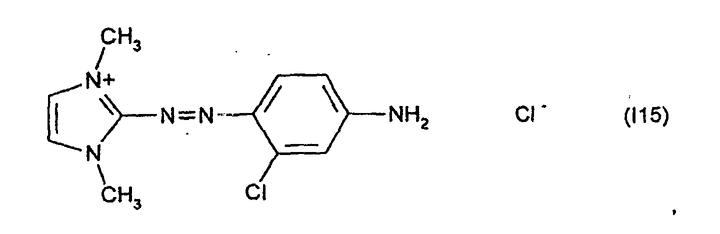

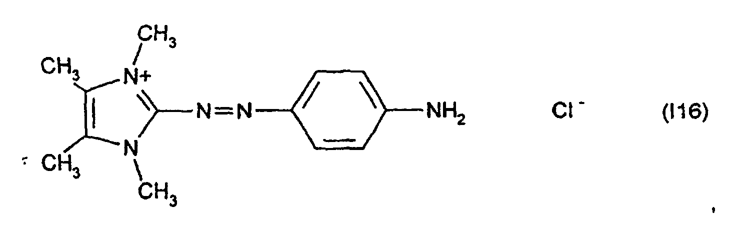

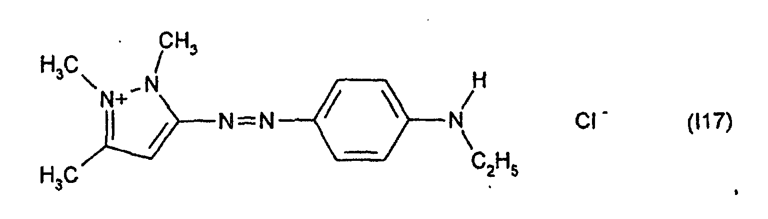

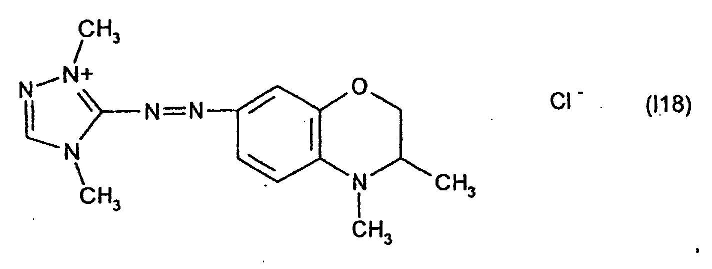

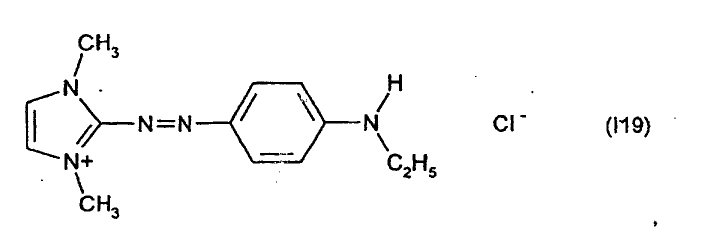

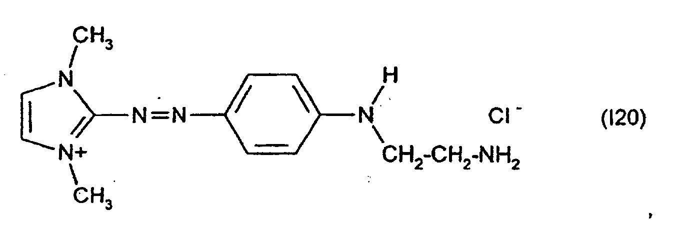

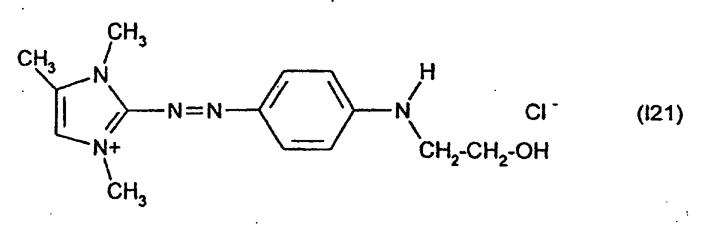

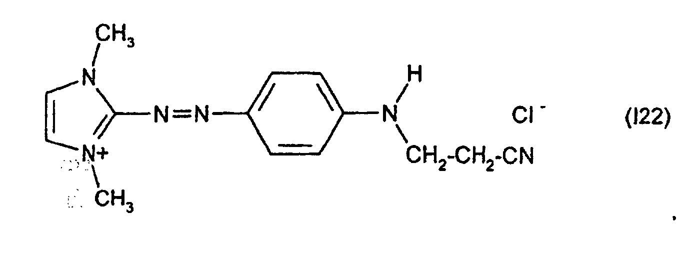

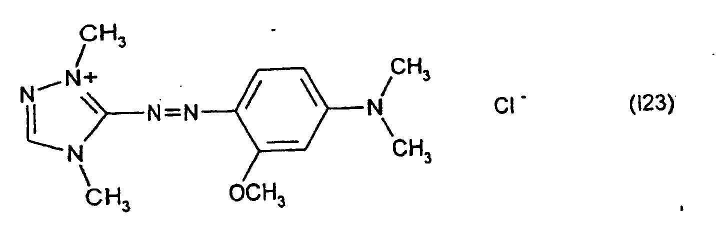

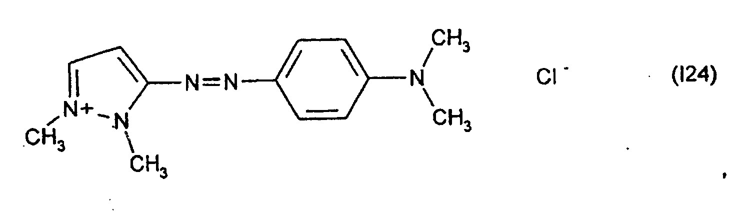

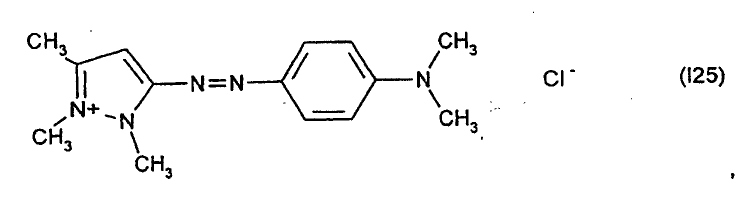

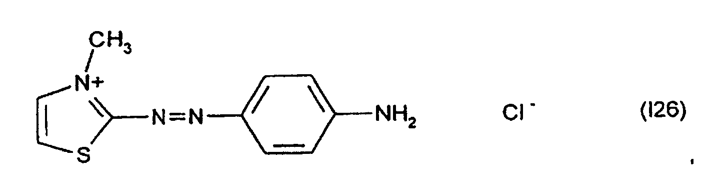

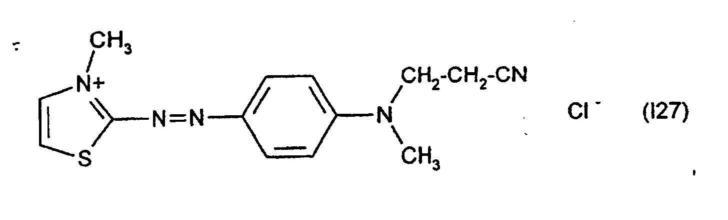

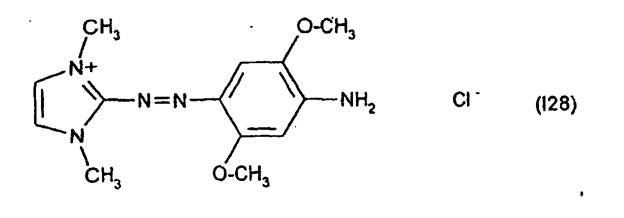

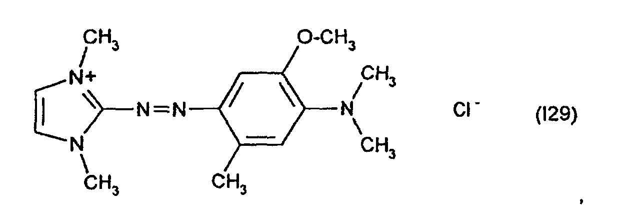

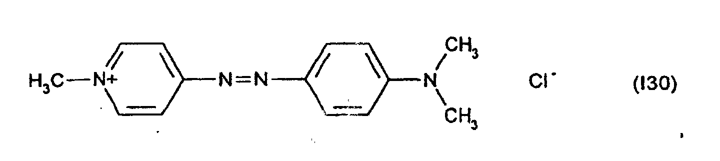

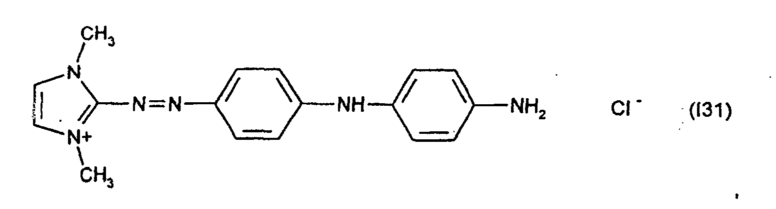

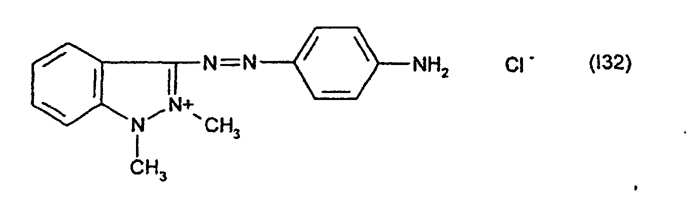

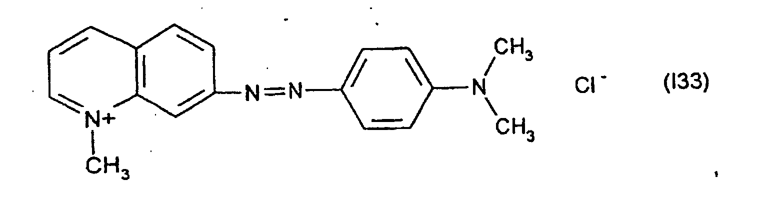

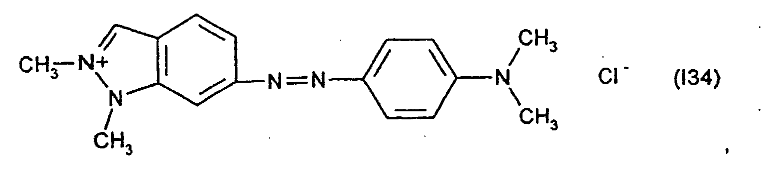

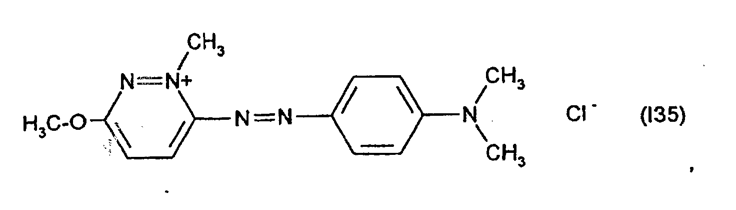

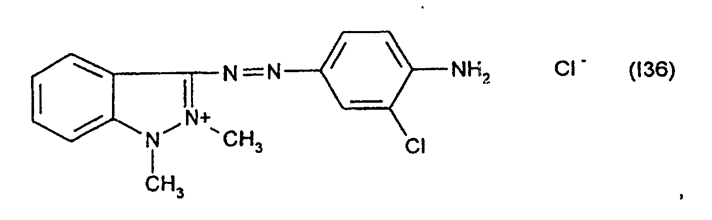

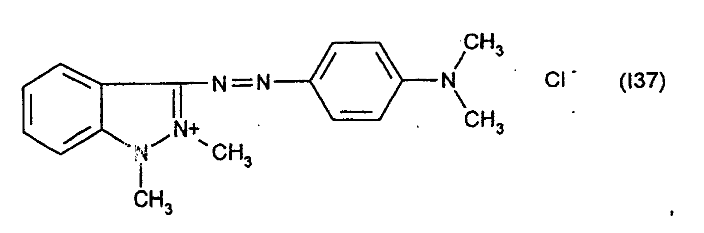

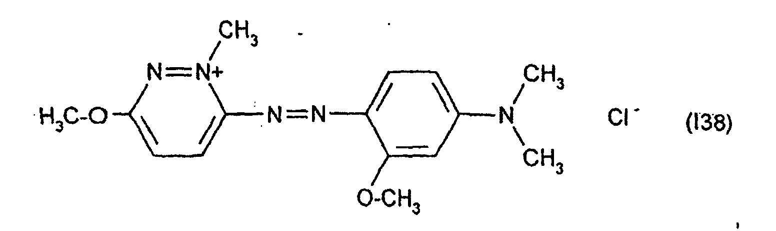

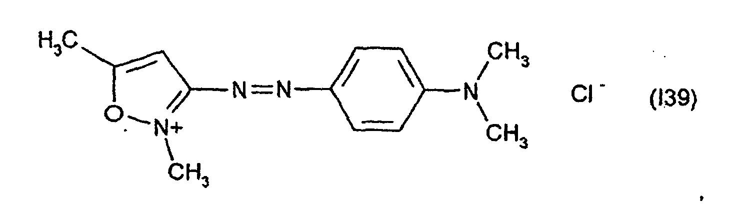

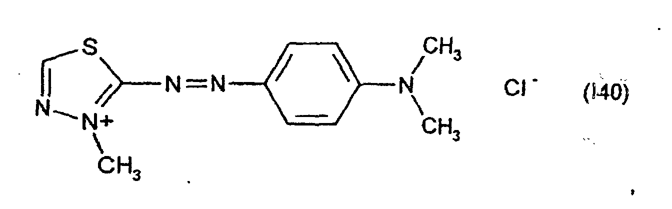

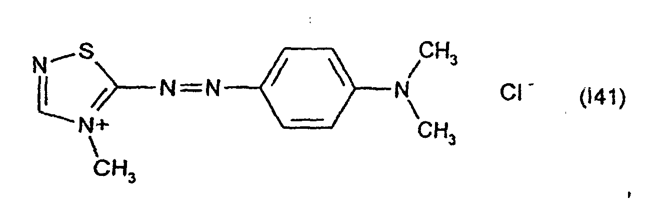

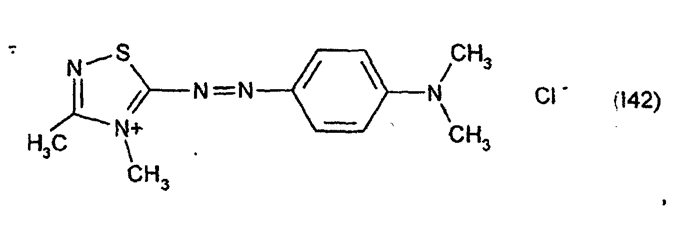

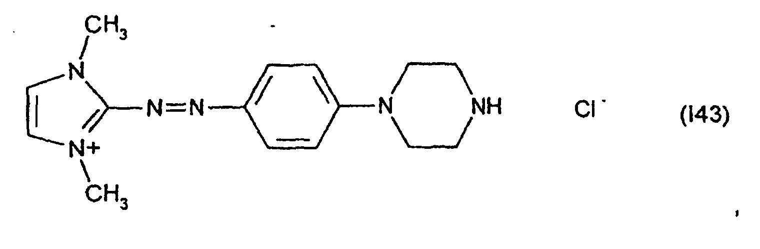

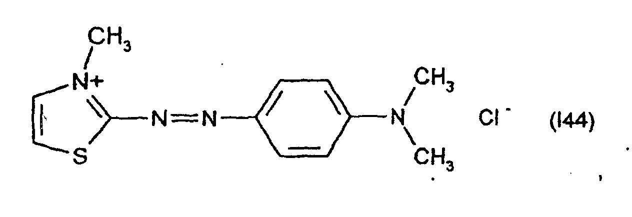

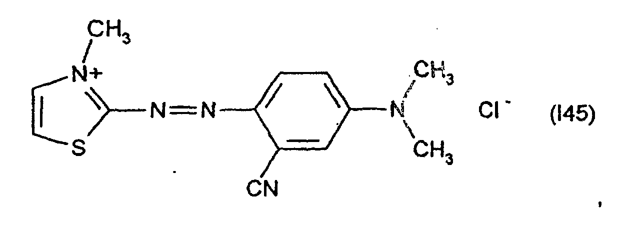

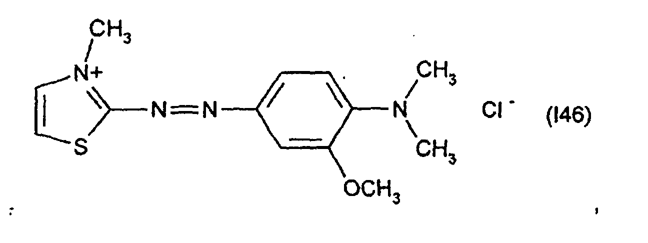

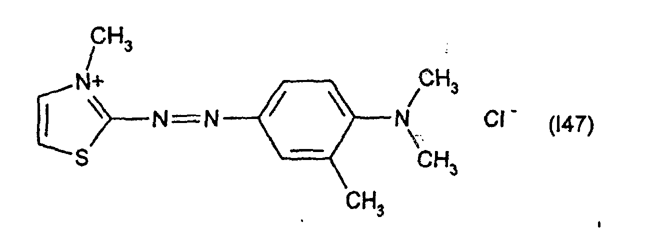

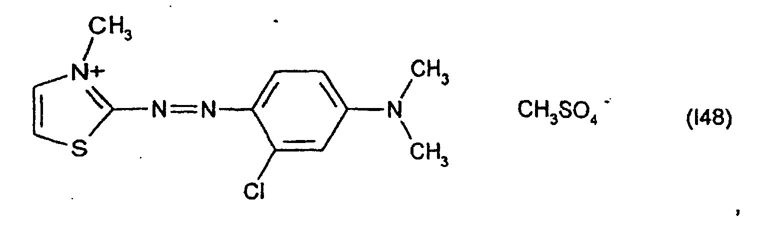

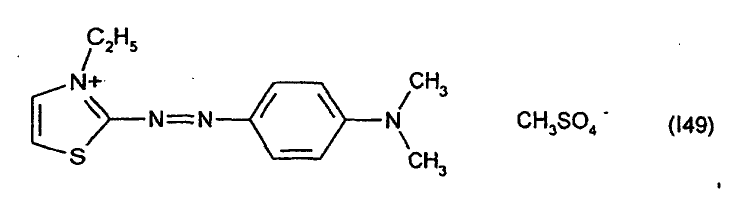

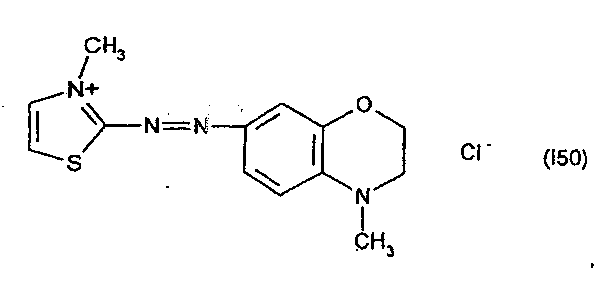

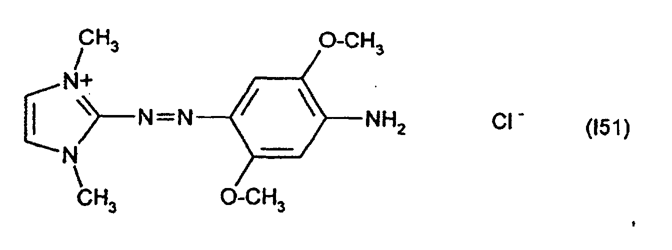

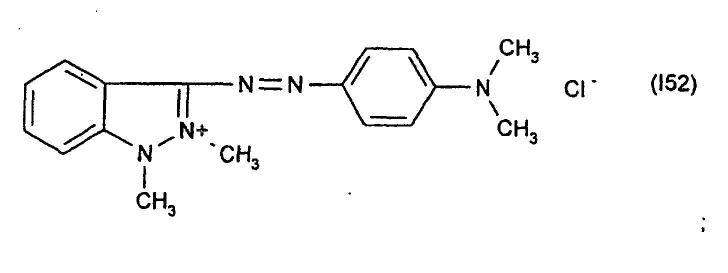

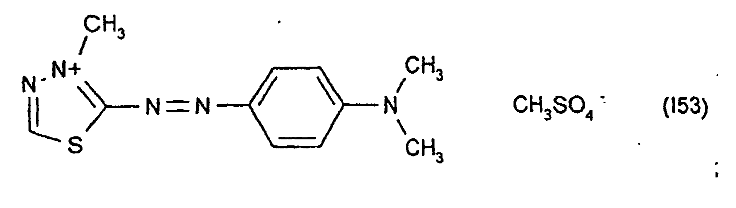

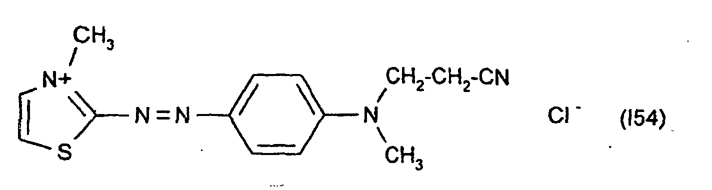

3. Composition according to Claim 2, **characterized in that** the cationic direct dyes correspond to the structures (I1), (I2), (I14) and (I31).

4. Composition according to Claim 1, **characterized in that** the cationic direct dyes of formula (II) are chosen from the compounds corresponding to the structures (II1) to (II9) below: 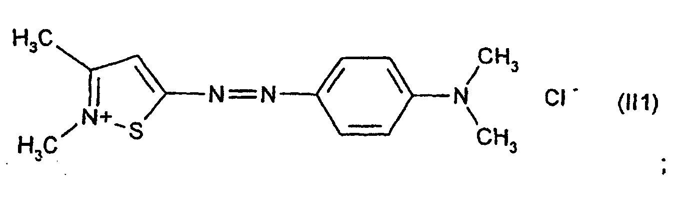 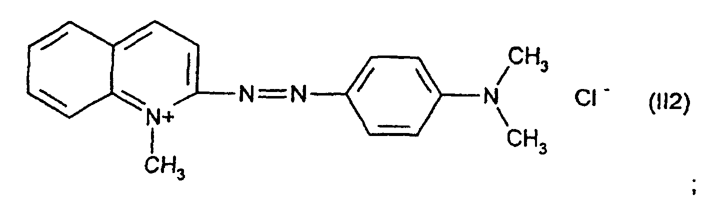 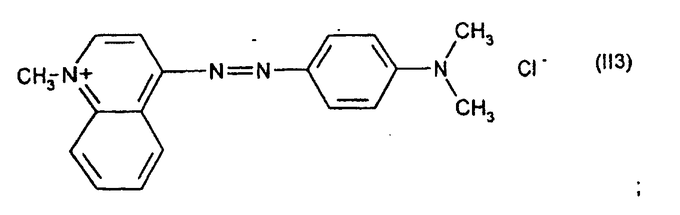 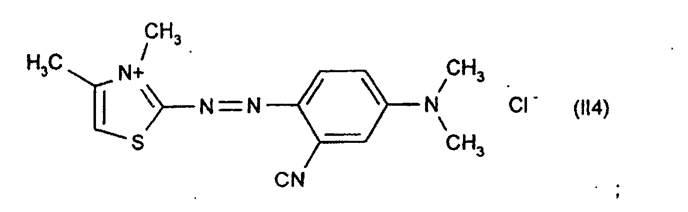 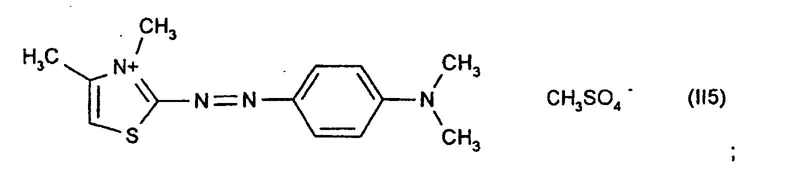 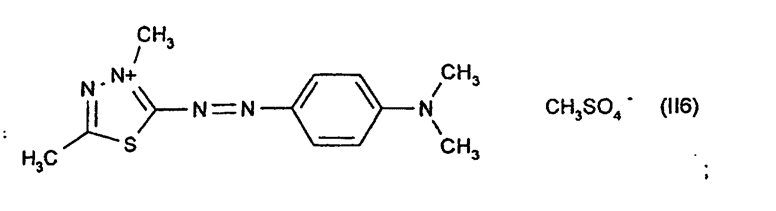 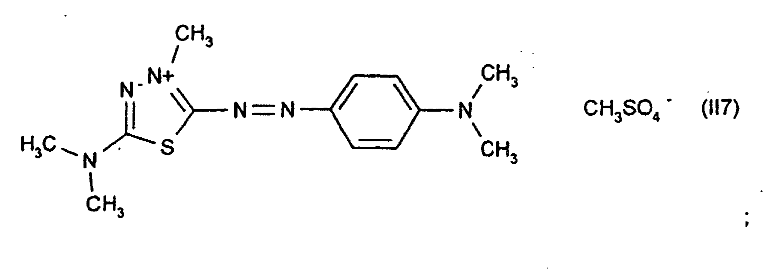 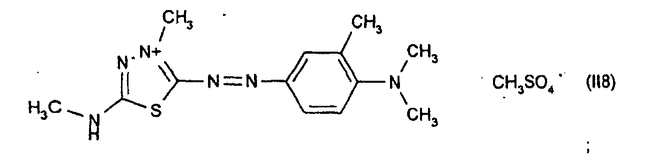 and

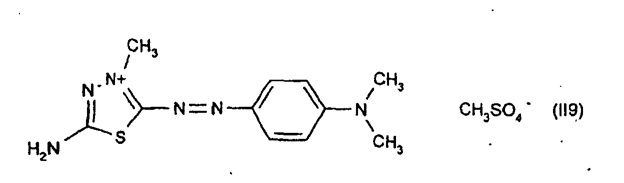

5. Composition according to Claim 1, **characterized in that** the cationic direct dyes of formula (III) are chosen from the compounds corresponding to the structures (III1) to (III18) below: 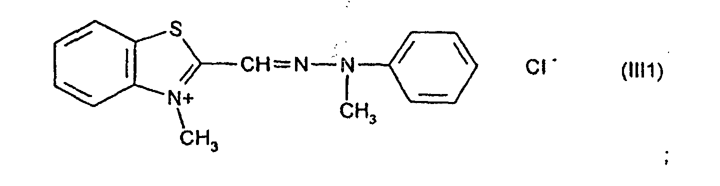 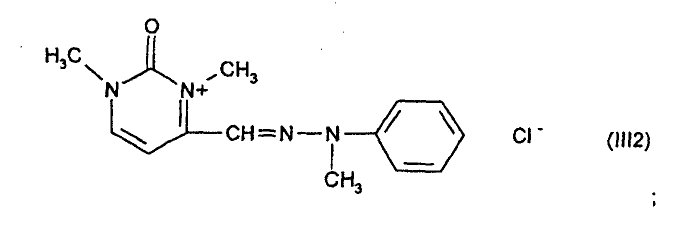 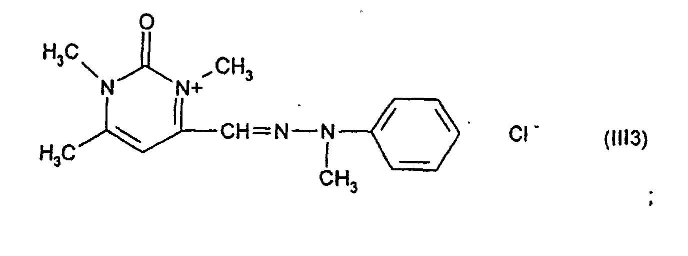 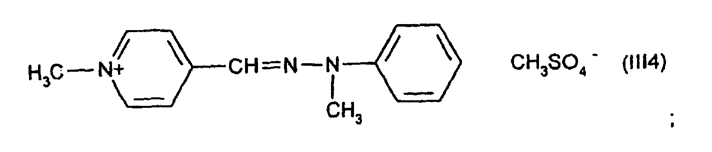 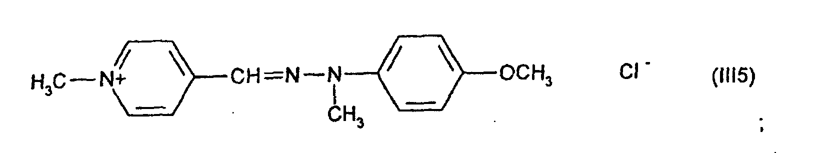 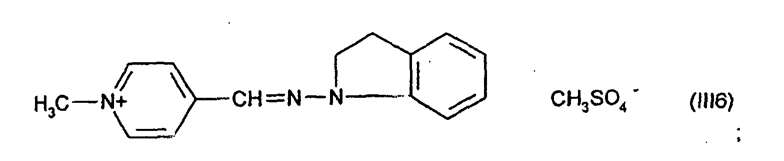 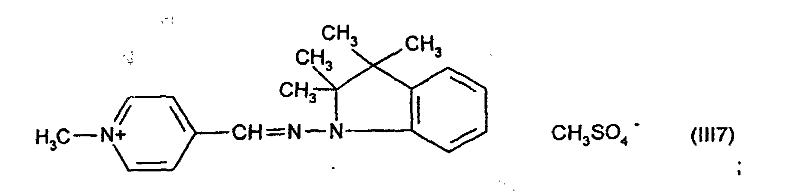 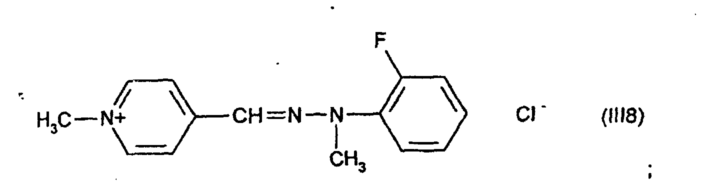 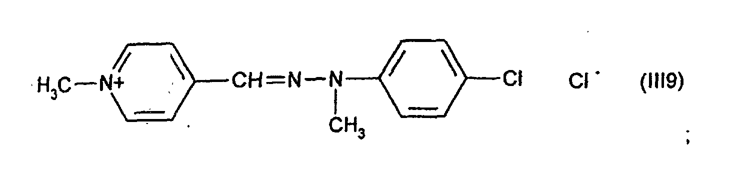 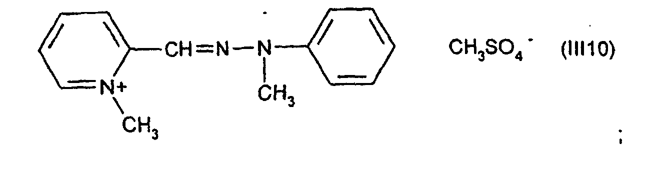 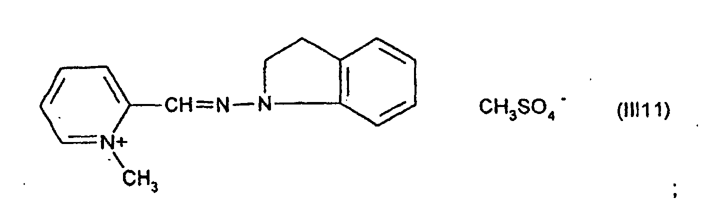 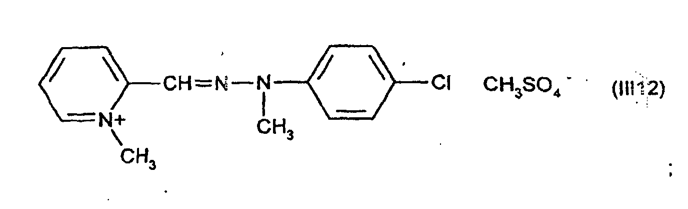 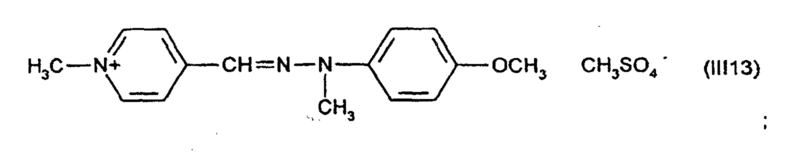 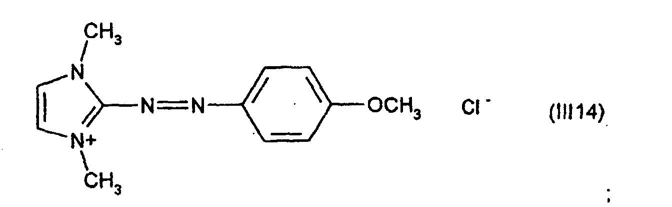 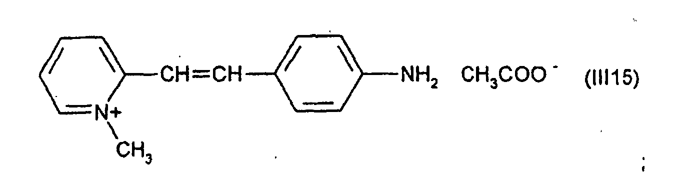 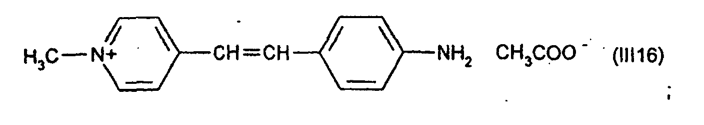 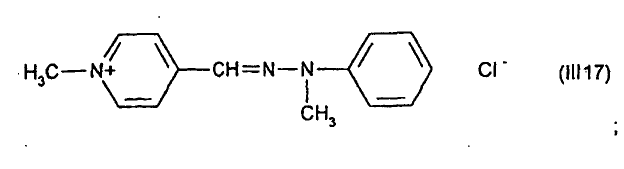 and

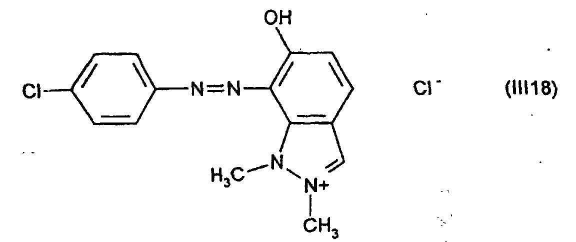

6. Composition according to Claim 5, **characterized in that** the cationic direct dyes of formula (III) are chosen from the compounds corresponding to the structures (III4), (III5) and (III13).

7. Composition according to Claim 1, **characterized in that** the cationic direct dyes of formula (III') are chosen from the compounds corresponding to the structures (III'1) to (III'3) below: 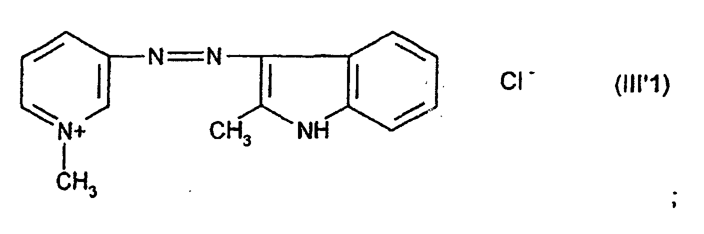 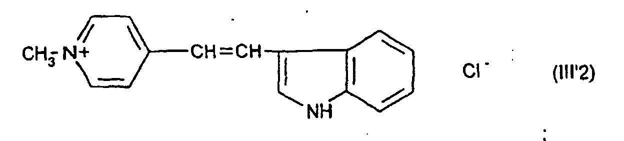 and

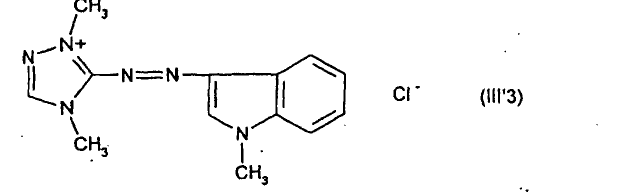

8. Composition according to Claim 1, **characterized in that** the cationic direct dyes of formula (IV) are chosen from the compounds corresponding to the structures (IV)₁ to (IV)₇₇ below:

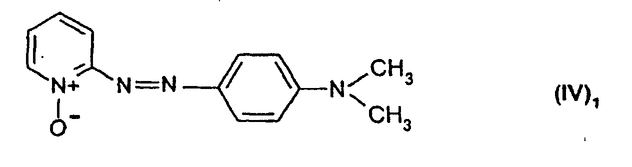

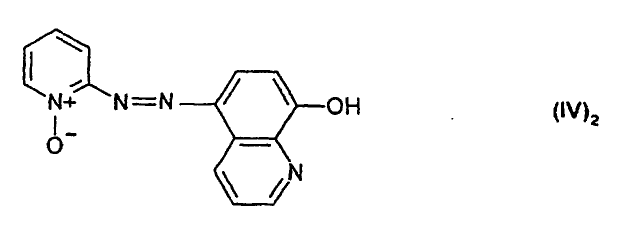

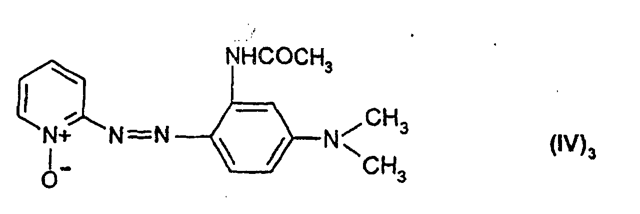

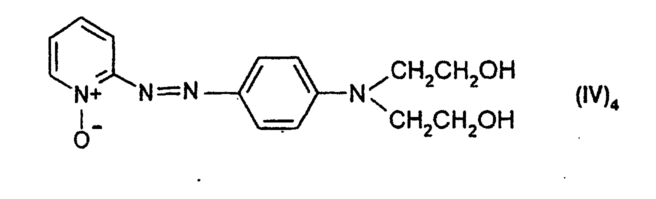

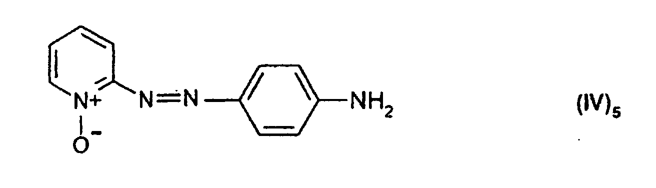

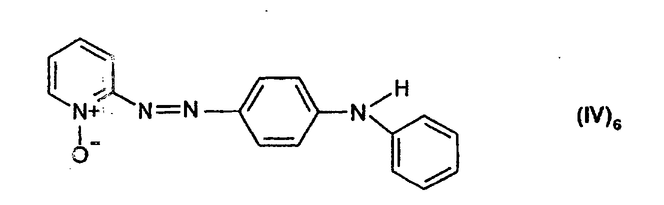

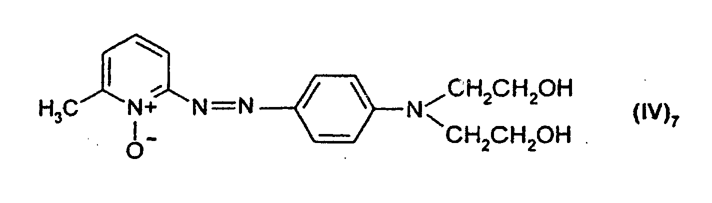

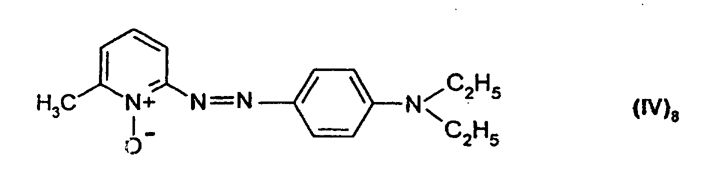

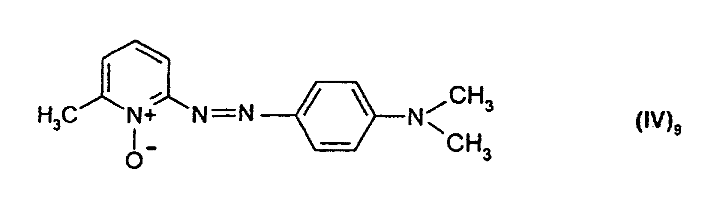

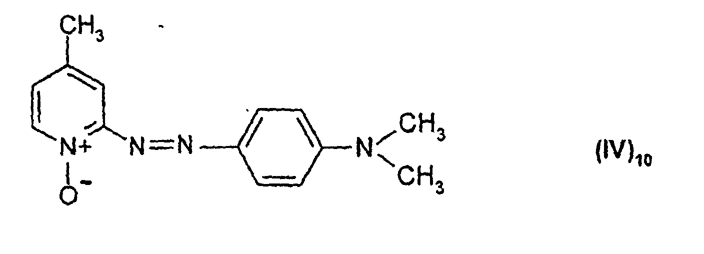

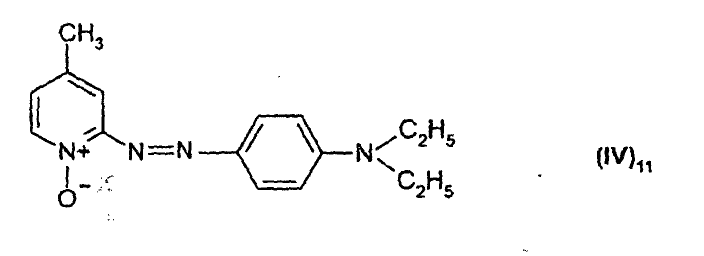

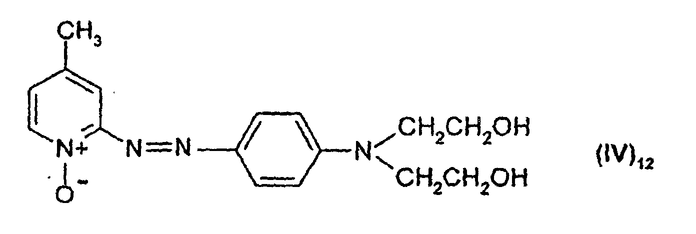

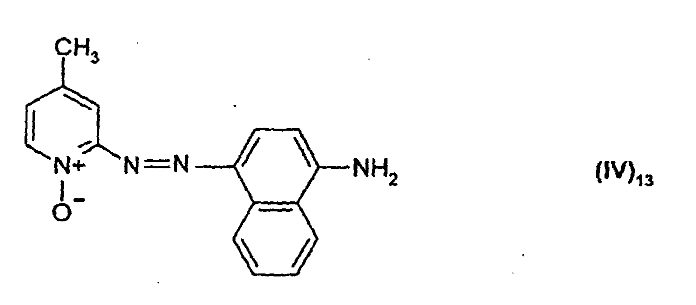

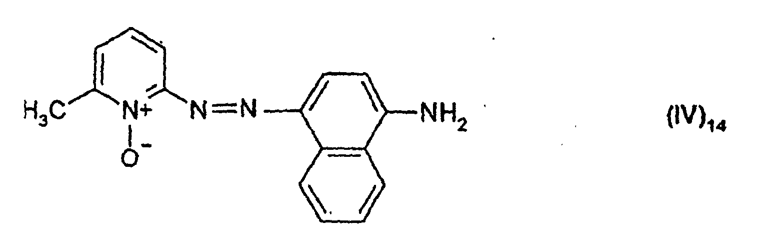

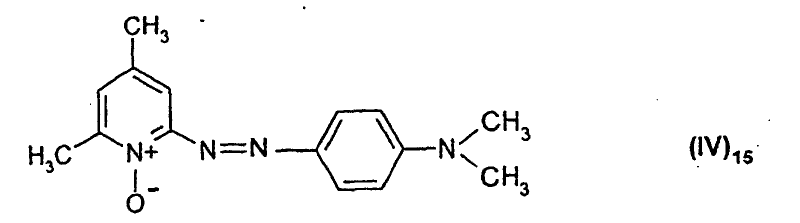

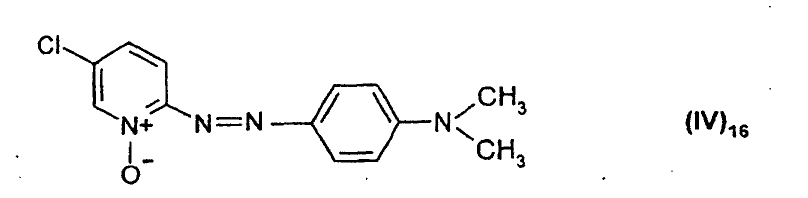

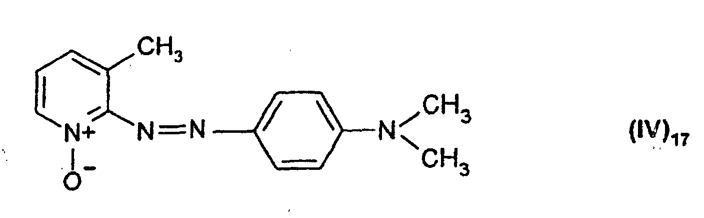

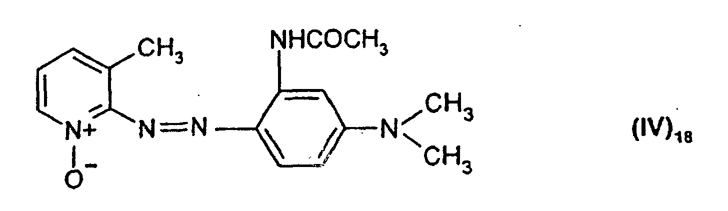

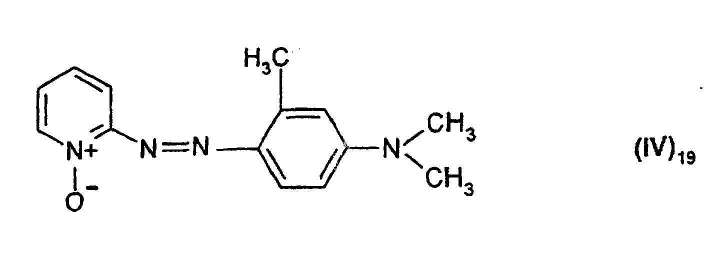

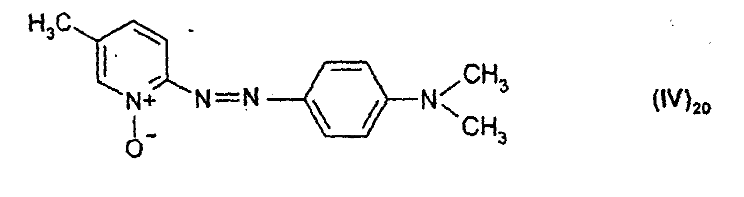

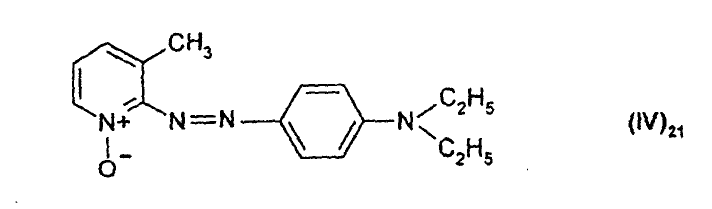

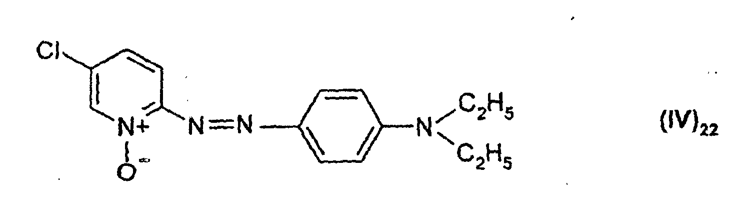

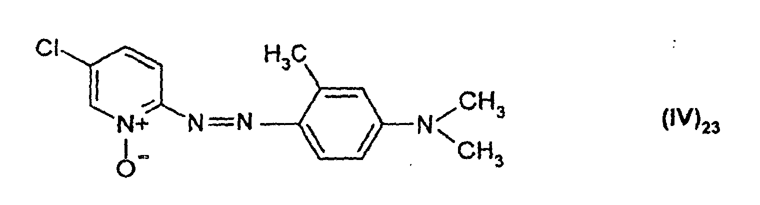

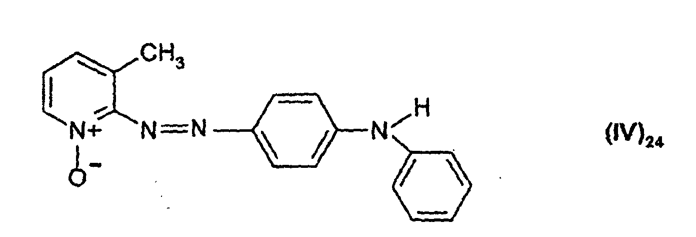

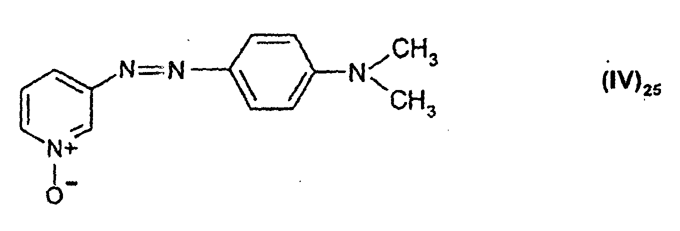

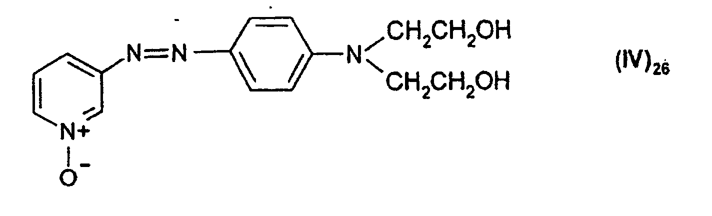

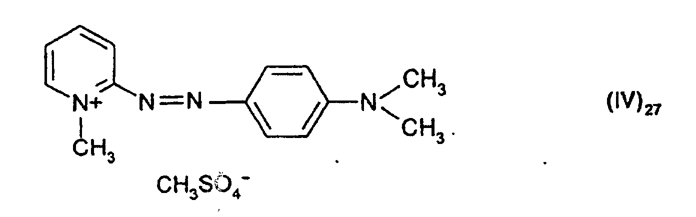

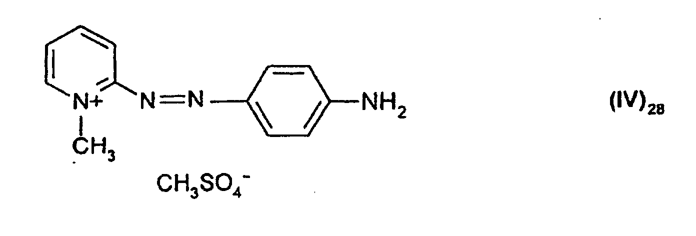

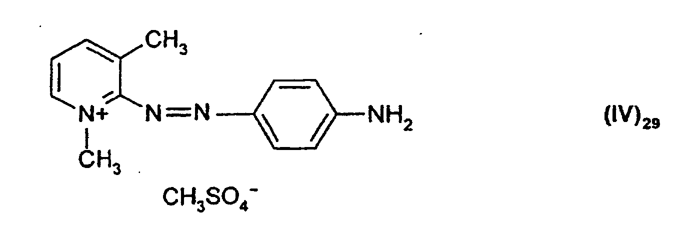

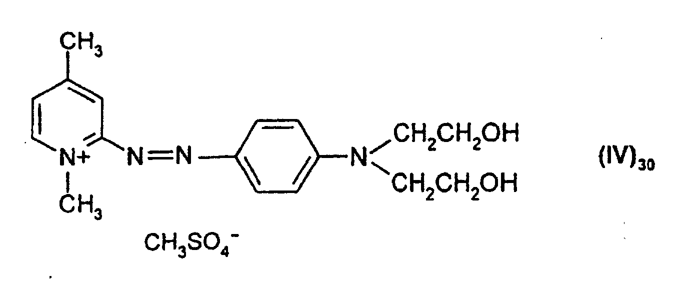

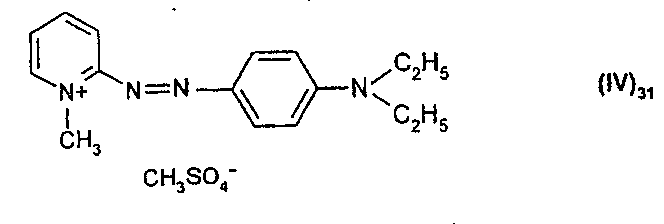

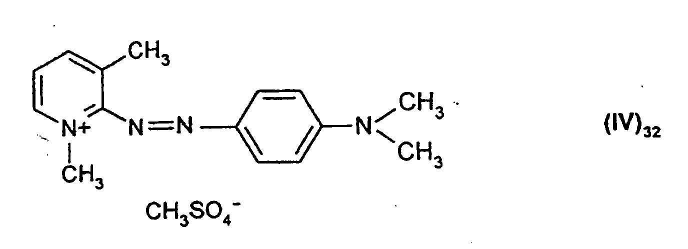

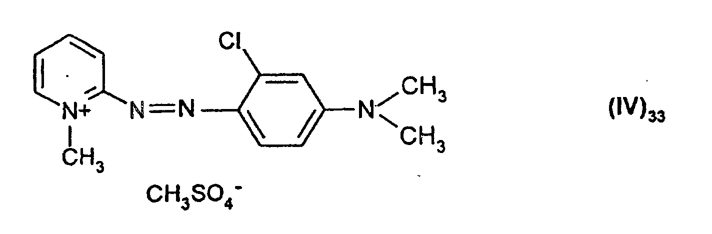

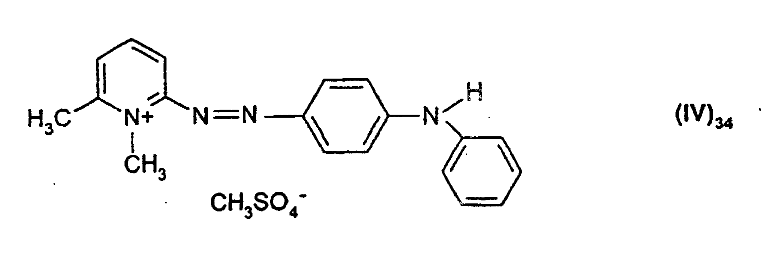

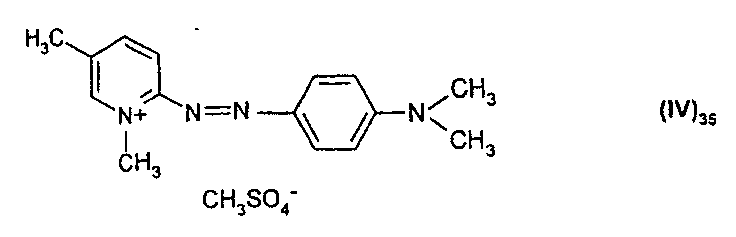

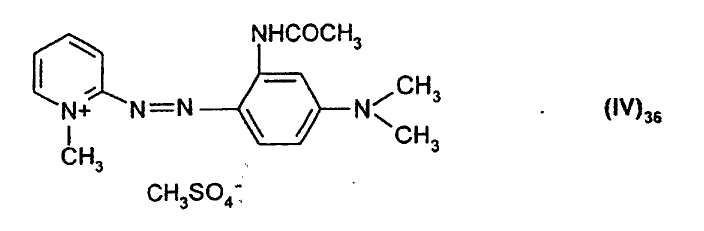

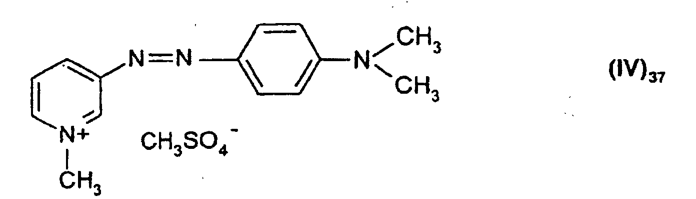

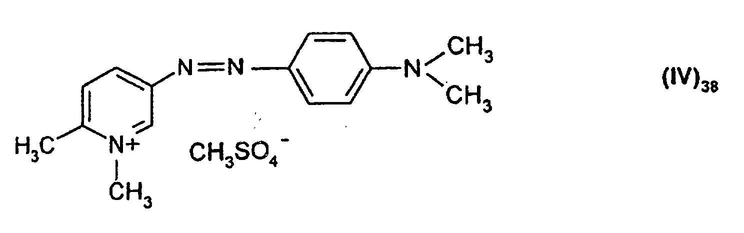

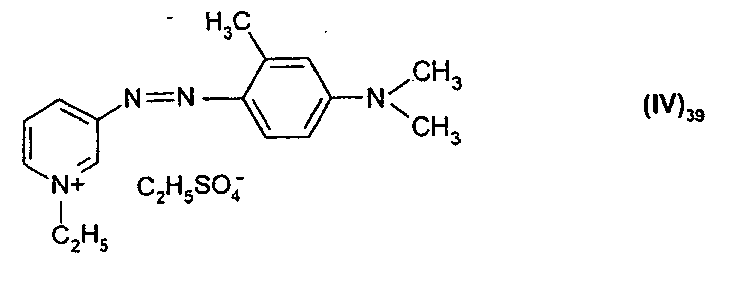

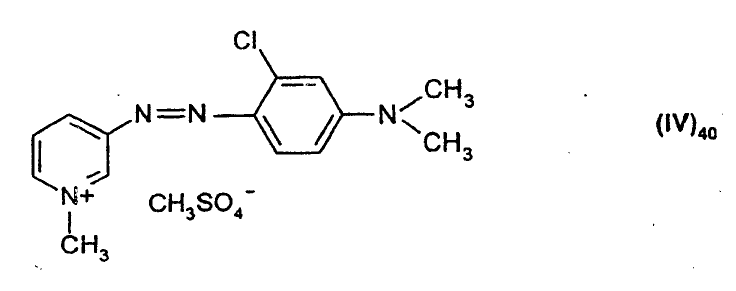

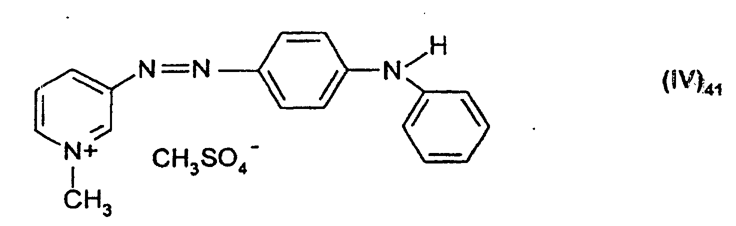

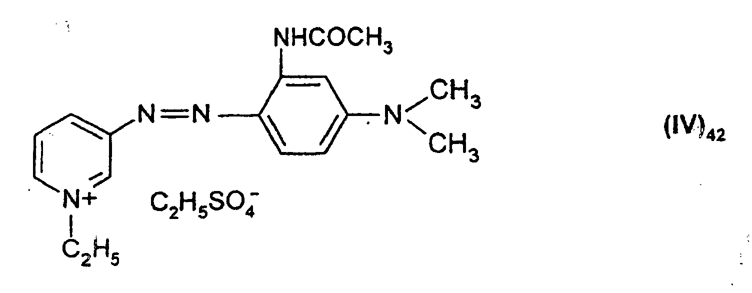

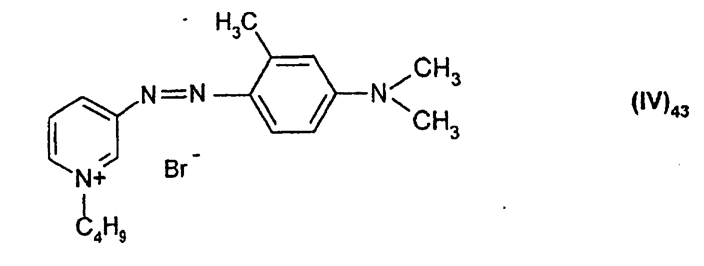

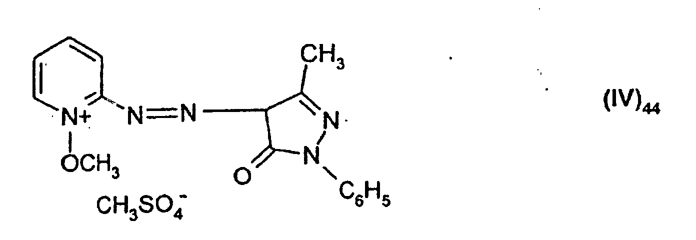

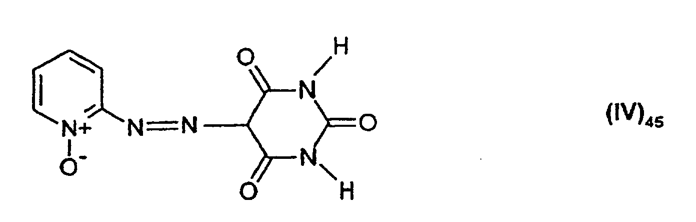

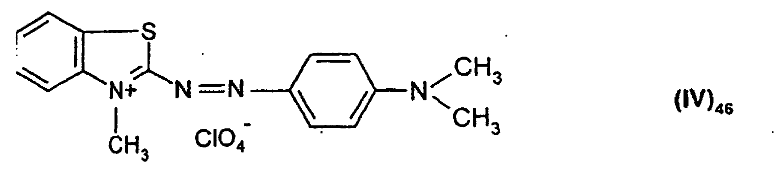

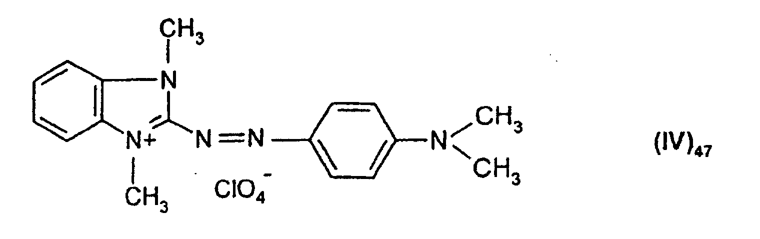

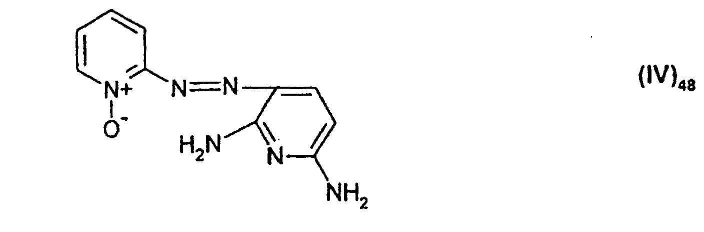

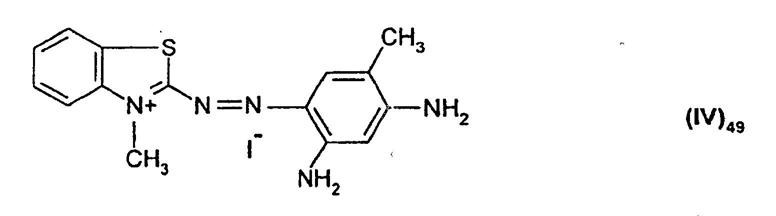

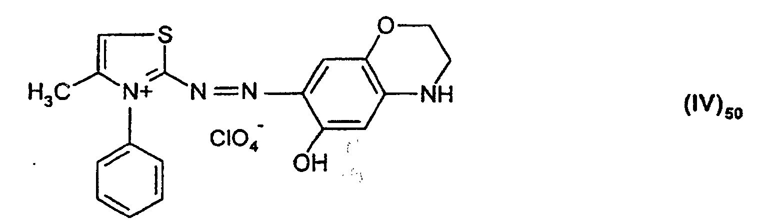

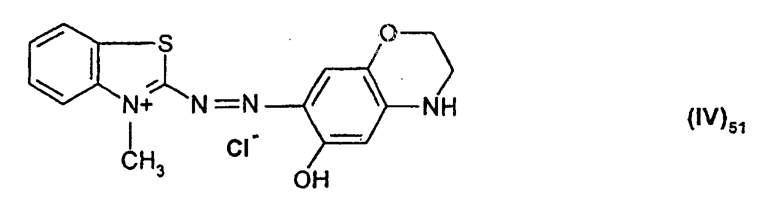

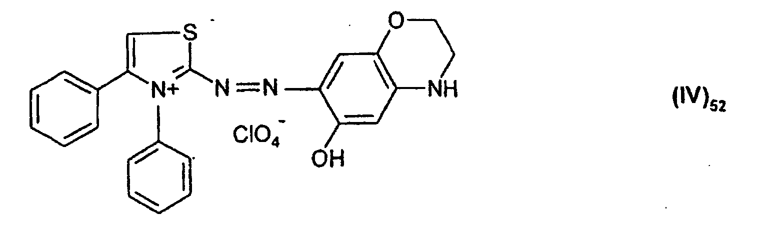

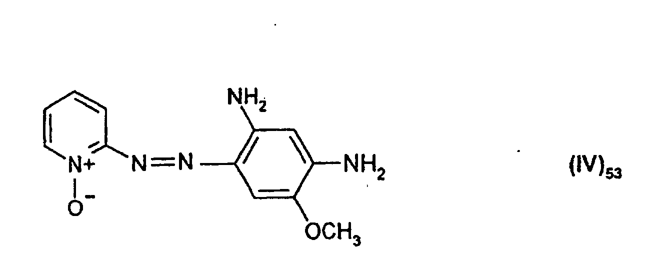

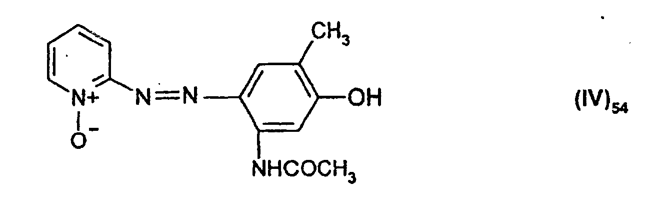

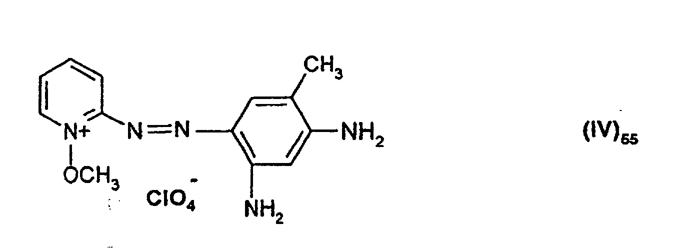

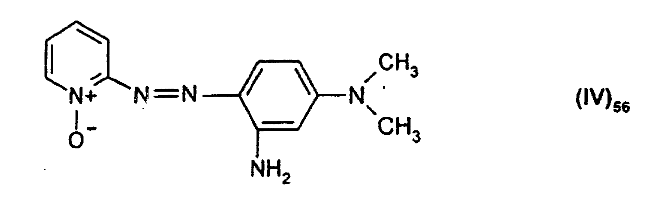

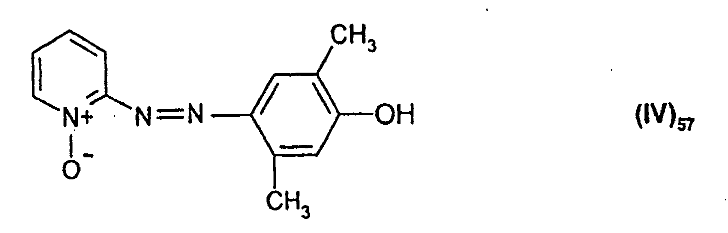

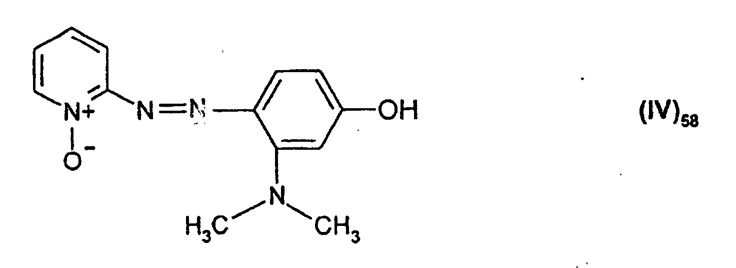

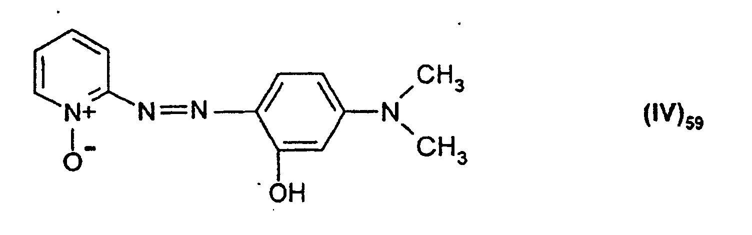

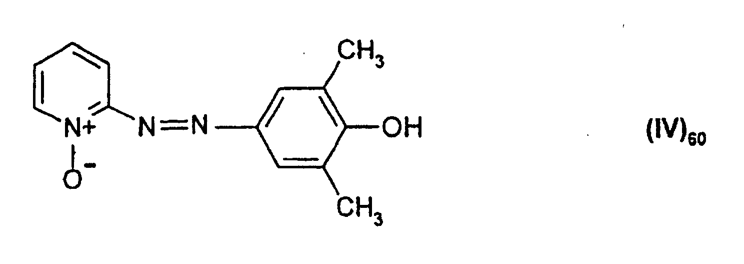

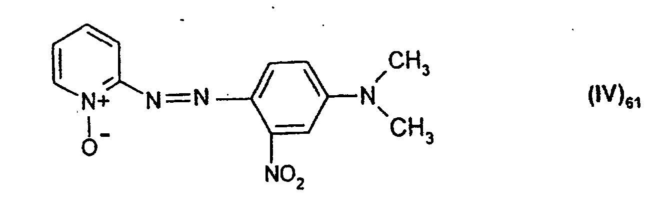

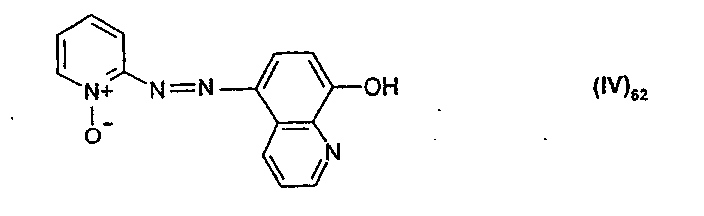

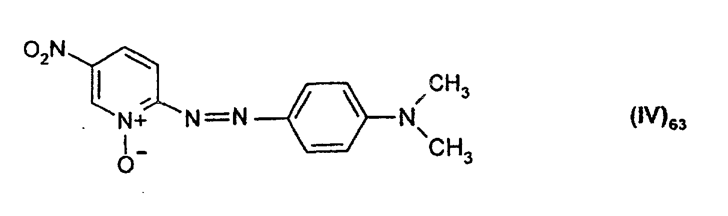

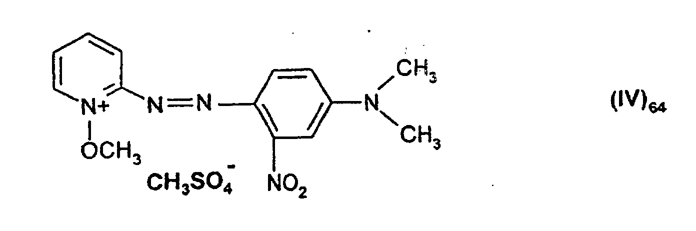

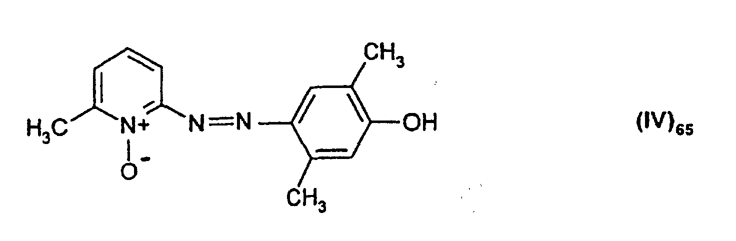

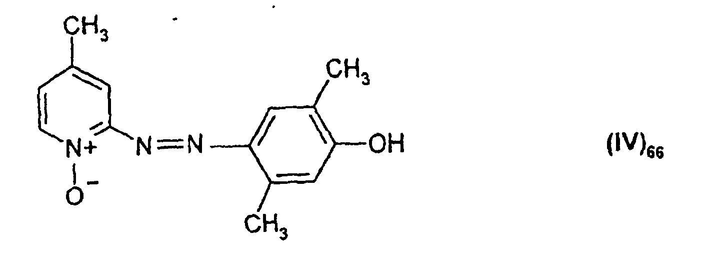

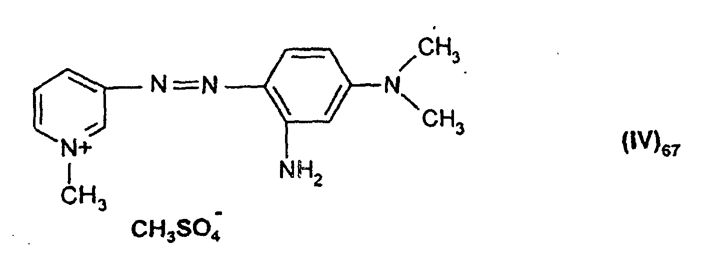

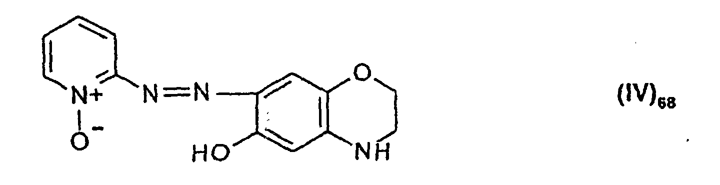

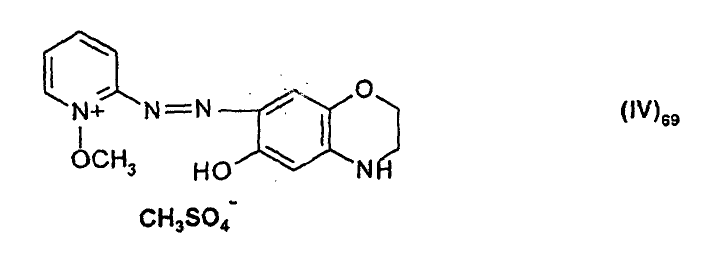

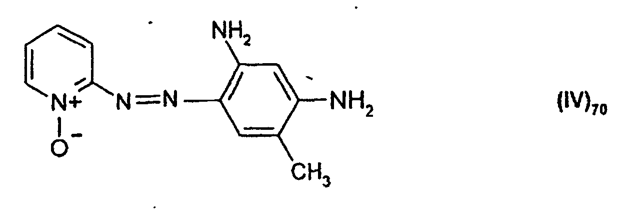

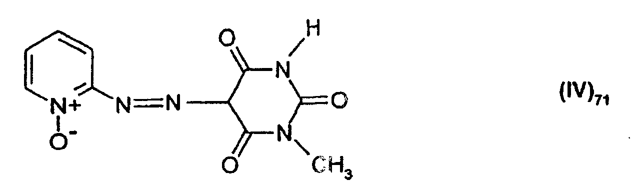

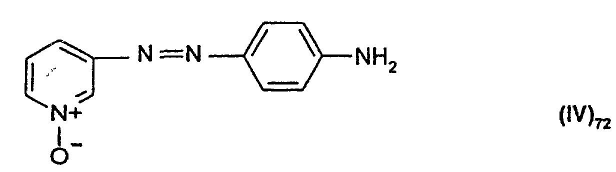

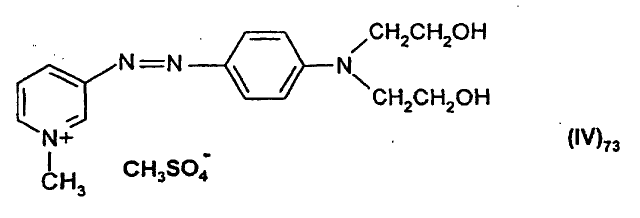

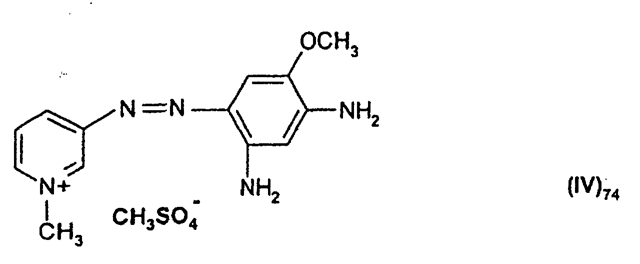

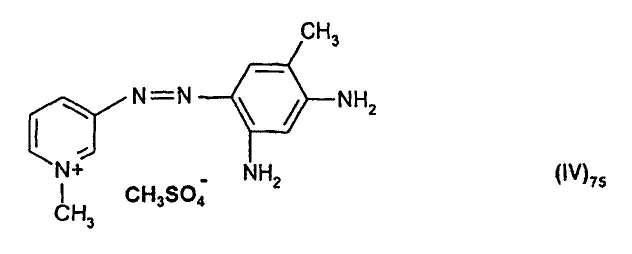

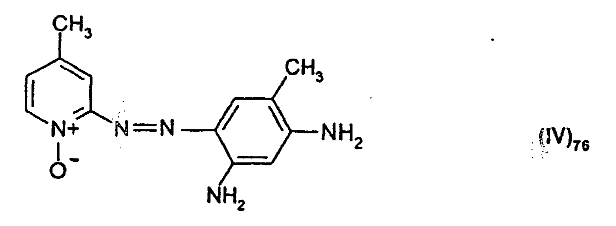

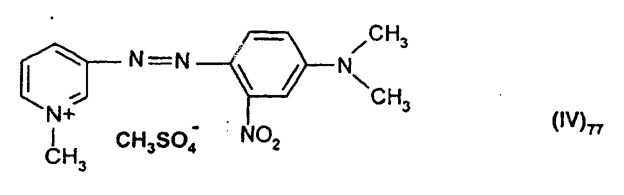

9. Composition according to any one of the preceding claims, **characterized in that** the cationic direct dye(s) of formula (I), (II), (III), (III') or (IV) represent(s) from 0.001 to 10% by weight relative to the total weight of the composition.

10. Composition according to Claim 9, **characterized in that** the cationic direct dye(s) of formula (I), (II), (III), (III') or (IV) represent(s) from 0.005 to 5% by weight relative to the total weight of the composition.

11. Composition according to any one of the preceding claims, **characterized in that** the thickening polymer (ii) of nonionic amphiphilic type comprising at least one hydrophilic unit and at least one unit containing a fatty chain is a cellulose modified with groups comprising at least one fatty chain.

12. Composition according to Claim 11, **characterized in that** it is a hydroxyethylcellulose modified with alkyl or aralkyl or alkylaryl groups or mixtures thereof.

13. Composition according to Claim 12, **characterized in that**, in the alkyl, aralkyl or alkylaryl groups, the alkyl radical is a C₈-C₂₂ chain.

14. Composition according to any one of Claims 11 to 13, **characterized in that** the hydroxyethylcellulose is modified with groups comprising at least one C₁₆ alkyl group.

15. Composition according to Claim 11, **characterized in that** it is a hydroxyethylcellulose modified with groups comprising at least one polyalkylene glycol alkylphenyl ether group.

16. Composition according to Claim 15, **characterized in that** it is a hydroxyethylcellulose modified with groups comprising at least one polyethylene glycol (15) nonylphenyl ether group.

17. Composition according to any one of Claims 1 to 10, **characterized in that** the thickening polymer (ii) of nonionic amphiphilic type comprising at least one hydrophilic unit and at least one unit containing a fatty chain is a polyurethane ether comprising at least one C₈-C₃₀ alkyl or alkenyl group.

18. Composition according to any one of Claims 1 to 10, **characterized in that**, in the thickening polymer (ii) of anionic amphiphilic type, the hydrophilic unit consists of an unsaturated ethylenic anionic monomer and the unit containing a fatty chain is an allyl ether containing a fatty chain.

19. Composition according to Claim 18, **characterized in that** the unsaturated ethylenic anionic monomer is a vinylcarboxylic acid.

20. Composition according to Claim 19, **characterized in that** it is an acrylic acid, a methacrylic acid or a mixture thereof.

21. Composition according to any one of Claims 18 to 20, **characterized in that** the allyl ether unit containing a fatty chain corresponds to the monomer of formula (V) below:
CH₂ = C R' CH₂ O Bₙ R (V)
in which R' denotes H or CH₃, B denotes an ethylenoxy radical, n is zero or denotes an integer ranging from 1 to 100 and R denotes a hydrocarbon-based radical chosen from alkyl and cycloalkyl radicals comprising from 8 to 30 carbon atoms.

22. Composition according to Claim 21, **characterized in that**, in formula (V), the hydrocarbon-based radical is alkyl and comprises from 10 to 24 carbon atoms.

23. Composition according to Claim 21 or 22, **characterized in that**, in formula (V), R' denotes hydrogen, n is equal to 10 and R denotes a stearyl radical.

24. Composition according to any one of Claims 18 to 23, **characterized in that** the anionic amphiphilic polymer is formed by emulsion polymerization of from 20 to 60% by weight of acrylic acid and/or methacrylic acid, from 5 to 60% by weight of lower alkyl (meth)acrylates, from 2 to 50% by weight of allyl ether containing a fatty chain of formula (V), and from 0 to 1% by weight of a crosslinking agent.

25. Composition according to Claim 24, **characterized in that** it is a crosslinked polymer comprising 40% by weight of methacrylic acid, 50% by weight of ethyl acrylate and 10% by weight of polyethylene glycol (10 EO) stearyl ether (Steareth-10).

26. Composition according to any one of Claims 1 to 10, **characterized in that**, in the thickening polymer (ii) of anionic amphiphilic type, the hydrophilic unit consists of an unsaturated olefinic carboxylic acid and the unit containing a fatty chain is a (C₁₀-C₃₀)alkyl ester of an unsaturated carboxylic acid.

27. Composition according to Claim 26, **characterized in that** the hydrophilic unit corresponds to the monomer of formula (VI) below: 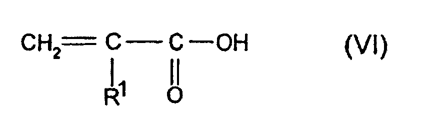 in which formula R¹ denotes H or CH₃ or C₂H₅.

28. Composition according to Claim 27, **characterized in that** it is acrylic acid, methacrylic acid or a mixture thereof.

29. Composition according to Claim 26, **characterized in that** the unit containing a fatty chain is an ester corresponding to the monomer of formula (VII) below: 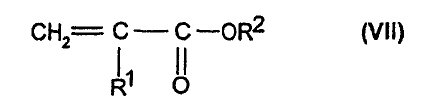 in which formula R¹ denotes H or CH₃ or C₂H₅, R² denoting a C₁₀-C₃₀ alkyl radical.

30. Composition according to Claim 29, **characterized in that**, in formula (VII), R¹ denotes H or CH₃.

31. Composition according to Claim 29 or 30, **characterized in that**, in formula (VII), R² denotes a C₁₂-C₂₂ alkyl radical.

32. Composition according to any one of Claims 26 to 31, **characterized in that** the anionic amphiphilic polymer is crosslinked.

33. Composition according to any one of Claims 26 to 32, **characterized in that** the anionic amphiphilic polymer is a polymer formed from a mixture of monomers essentially comprising acrylic acid, an ester of formula (VII) below: 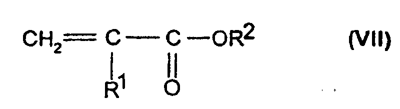 in which R¹ denotes H or CH₃, R² denoting an alkyl radical containing from 12 to 22 carbon atoms, and a crosslinking agent.

34. Composition according to any one of Claims 26 to 33, **characterized in that** the anionic amphiphilic polymer is a polymer of acrylic acid and of lauryl methacrylate.

35. Composition according to any one of the preceding claims, **characterized in that** the thickening polymer (ii) represents from 0.01 to 10% by weight relative to the total weight of the composition.

36. Composition according to Claim 35, **characterized in that** the thickening polymer (ii) represents from 0.1 to 5% by weight relative to the total weight of the composition.

37. Composition according to any one of the preceding claims, **characterized in that** the medium which is suitable for dyeing (or support) consists of water or of a mixture of water and at least one organic solvent.

38. Composition according to any one of the preceding claims, **characterized in that** it has a pH of between 2 and 11 and preferably between 5 and 10.

39. Composition according to any one of the preceding claims, **characterized in that** it is intended for oxidation dyeing and **in that** it contains one or more oxidation bases chosen from para-phenylenediamines, bis(phenyl)alkylenediamines, para-aminophenols, ortho-aminophenols and heterocyclic bases.

40. Composition according to Claim 39, **characterized in that** the oxidation base(s) represent(s) from 0.0005 to 12% by weight relative to the total weight of the dye composition.

41. Composition according to Claim 40, **characterized in that** the oxidation base(s) represent(s) from 0.005 to 6% by weight relative to the total weight of the dye composition.

42. Composition according to any one of Claims 39 to 41, **characterized in that** it contains one or more couplers chosen from meta-phenylenediamines, meta-aminophenols, meta-diphenols and heterocyclic couplers.

43. Composition according to Claim 42, **characterized in that** the coupler(s) represent (s) from 0.0001 to 10% by weight relative to the total weight of the dye composition.

44. Composition according to Claim 43, **characterized in that** the coupler(s) represent(s) from 0.005 to 5% by weight relative to the total weight of the dye composition.

45. Composition according to any one of the preceding claims, **characterized in that** it is intended for lightening direct dyeing or oxidation dyeing and **in that** it contains at least one oxidizing agent.

46. Process for dyeing keratin fibres, and in particular human keratin fibres such as the hair, **characterized in that** at least one dye composition as defined in any one of Claims 1 to 45 is applied to the fibres, for a period which is sufficient to develop the desired coloration, after which the fibres are rinsed, optionally washed with shampoo, rinsed again and dried.

47. Process for dyeing keratin fibres, and in particular human keratin fibres such as the hair, **characterized in that** at least one dye composition as defined in any one of Claims 1 to 45 is applied to the fibres, for a period which is sufficient to develop the desired coloration, without final rinsing.

48. Process for dyeing keratin fibres, and in particular human keratin fibres such as the hair, **characterized in that** it comprises a first step which consists in separately storing, on the one hand, a composition (A1) comprising, in a medium which is suitable for dyeing, at least one cationic direct dye (i) as defined in the preceding claims and at least one oxidation base, and, on the other hand, a composition (B1) comprising, in a medium which is suitable for dyeing, at least one oxidizing agent, and then in mixing them together at the time of use, after which this mixture is applied to the keratin fibres, the composition (A1) or the composition (B1) containing the thickening polymer (ii) as defined in the preceding claims.

49. Process for dyeing keratin fibres, and in particular human keratin fibres such as the hair, **characterized in that** it comprises a first step which consists in separately storing, on the one hand, a composition (A2) comprising, in a medium which is suitable for dyeing, at least one cationic direct dye (i) as defined in the preceding claims and, on the other hand, a composition (B2) comprising, in a medium which is suitable for dyeing, at least one oxidizing agent, and then in mixing them together at the time of use, after which this mixture is applied to the keratin fibres, the composition (A2) or the composition (B2) containing the thickening polymer (ii) as defined in the preceding claims.

50. Multi-compartment dyeing device or multicompartment dyeing "kit", **characterized in that** a first compartment comprises the composition (A1) or (A2) as defined in Claim 48 or 49 and a second compartment comprises the composition (B1) or (B2) as defined in Claim 48 or 49.
